# EUROPEAN PATENT APPLICATION

(11) **EP 4 714 942 A1**
(43) Date of publication of application: **25.03.2026**
(21) Application number: 25187390.7
(22) Date of filing: 28.07.2021
(51) Int. Cl.: C07D 211/46

(54) **SUBSTITUTED HALOALKYL SULFONANILIDE HERBICIDES**

(30) Priority: 29.07.2020 US 202063058459 P
(62) Divisional of application: 21758233.7
(71) Applicant: FMC Corporation, Philadelphia, Pennsylvania 19104 (US)
(72) Inventor: SELBY, Thomas, Paul, Philadelphia, 19104 (US); STEVENSON, Thomas, Martin, Philadelphia, 19104 (US); LEVENS, Alison, Mary, Philadelphia, 19104 (US); HOLMES, Michael, Philadelphia, 19104 (US); ZHANG, Wandi, Philadelphia, 19104 (US)
(74) Representative: Dehns

(57) **Abstract**

Disclosed are compounds of Formula **1,** all stereoisomers, N-oxides, and salts thereof, 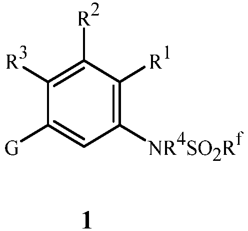 wherein G is selected from

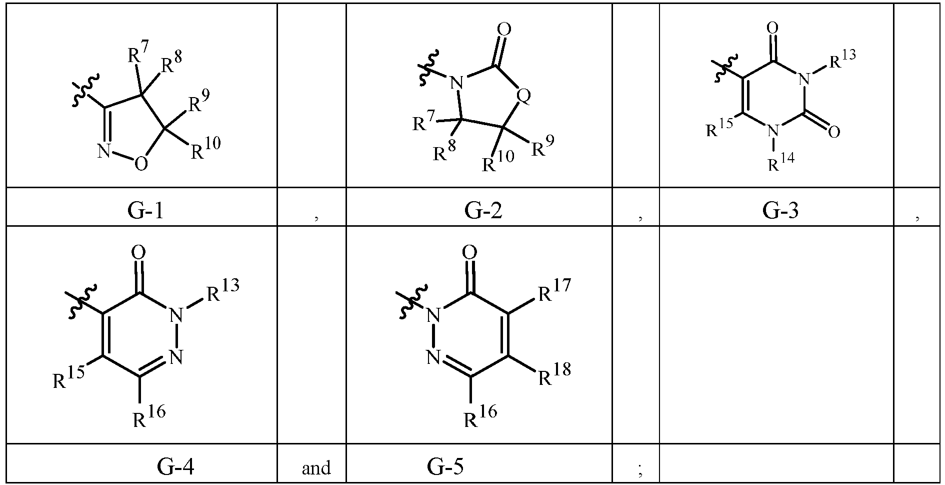

and R¹ through R¹⁸, R^{f} and G are as defined in the Disclosure.

Also disclosed are compositions containing the compounds of Formula **1** and methods for controlling undesired vegetation comprising contacting the undesired vegetation or its environment with an effective amount of a compound or a composition of the invention.

## Description

### FIELD OF THE DISCLOSURE

This invention relates to certain haloalkyl sulfonanilides, their N-oxides, salts and compositions, and methods of their use for controlling undesirable vegetation.

### BACKGROUND OF THE DISCLOSURE

The control of undesired vegetation is extremely important in achieving high crop efficiency. Achievement of selective control of the growth of weeds especially in such useful crops as rice, soybean, sugar beet, maize, potato, wheat, barley, tomato and plantation crops, among others, is very desirable. Unchecked weed growth in such useful crops can cause significant reduction in productivity and thereby result in increased costs to the consumer. The control of undesired vegetation in noncrop areas is also important. Many products are commercially available for these purposes, but the need continues for new compounds that are more effective, less costly, less toxic, environmentally safer or have different sites of action.

### SUMMARY OF THE DISCLOSURE

This invention is directed to compounds of Formula **1,** all stereoisomers, *N*-oxides, and salts thereof, agricultural compositions containing them and their use as herbicides: 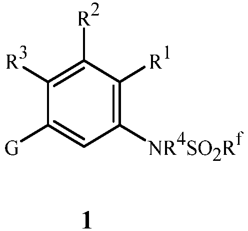 wherein
G is CONR⁵R⁶ or selected from

| | | | | | |
|---|---|---|---|---|---|
| 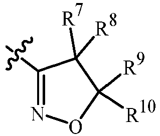 | | 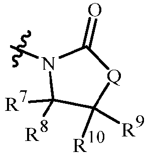 | | 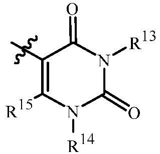 | |
| G-1 | , | G-2 | , | G-3 | , |
| 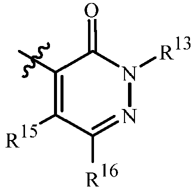 | | 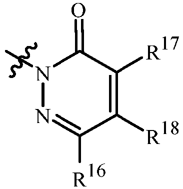 | | | |
| G-4 | and | G-5 | ; | | |

R¹ is H, C₁-C₇ alkyl, halogen, CN, C₂-C₆ alkenyl, C₂-C₆ alkynyl, C₃-C₇ cycloalkyl, C₂-C₃ cyanoalkyl, C₁-C₇ haloalkyl, C₂-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl;
R² is H, C₁-C₇ alkyl, halogen, CN, C₁-C₇ haloalkyl, C₁-C₇ alkoxy, C₃-C₇ cycloalkyl or C₁-C₅ alkylthio;
R³ is H, C₁-C₇ alkyl, halogen, CN, C₂-C₆ alkenyl, C₂-C₇ alkynyl, C₃-C₇ cycloalkyl, C₂-C₃ cyanoalkyl, C₁-C₇ haloalkyl, C₂-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl;
R⁴ is H, C(=O)R¹⁹, -C(=S)R¹⁹, -CO₂R¹⁹, -C(=O)SR¹⁹, -S(O)₂R¹⁹, C(=O)NR¹⁹R²⁰, -S(O)₂NR¹⁹R²⁰, S(OH)₂NR¹⁹R²⁰, CH₂OC(=O)OR¹⁹, CH₂OC(=O)NR¹⁹R²⁰ or CH₂OC(=O)R¹⁹;
R⁵ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R⁶ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl; or
R⁵ and R⁶ are taken together with the nitrogen atom to which they are attached to form a 3- to 7-membered ring, containing carbon atoms and optionally 1 to 3 oxygen, sulfur or nitrogen atoms as ring members, wherein up to 2 carbon atom ring members are independently selected from C(=O) and C(=S), and the sulfur atom ring member is selected from S, S(O) or S(O)₂, said ring optionally substituted with up to 5 substituents independently selected from (R^{v})ᵣ and r is the number of the substituents;
R^{v} is independently selected from the group consisting of H, halogen, cyano, nitro, C₁-C₄ alkyl, C₃-C₆ cycloalkyl, C₁-C₄ haloalkyl, C₁-C₄ alkoxy and C₁-C₄ haloalkoxy; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1 to 3 oxygen, sulfur or nitrogen atoms as ring members, wherein up to 2 carbon atom ring members are independently selected from C(=O) and C(=S), and the sulfur atom ring member is selected from S, S(O) or S(O)₂, said ring being unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R⁷ is H, C₁-C₇ alkyl, halogen, CN, C₁-C₇ haloalkyl or C₁-C₇ alkoxy;
R⁸ is H, C₁-C₇ alkyl; or
R⁷ and R⁸ may be taken together to form a 3- to 7-membered ring, containing carbon atoms and optionally 1-2 oxygen, sulfur or nitrogen atoms as the ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R⁹ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₂-C₇ haloalkenyl, C₂-C₇ alkoxyalkyl, C₃-C₇ alkylthioalkyl, C₁-C₇ alkoxy or C₄-C₇ alkylcycloalkyl;
R⁷ and R⁹ may be taken together to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R¹⁰ is H or C₁-C₇ alkyl; or
R⁹ and R¹⁰ may be taken together with the carbon atom to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂, said ring optionally substituted with up to 5 substituents independently selected from (R^{v})ᵣ and r is the number of the substituents; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂;
Q is O, S, CR¹¹R¹² or NR¹³;
R¹¹ and R¹² are taken together with the carbon atom to which they are attached to form a 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy; or
R⁹ and R¹¹ are taken together with the carbon atom to which they are attached to form a 6-membered aromatic ring, said ring optionally substituted with up to 4 substituents independently selected from R^{w};
R^{w} is C₁-C₅ alkyl, halogen, C₁-C₇ haloalkyl or C₁-C₇ alkoxy;
r is 0, 1, 2, 3, 4 or 5;
R¹³ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R¹⁴ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₁-C₇ thioalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R¹⁵ is H, C₁-C₇ alkyl, halogen, C₁-C₇ haloalkyl or C₁-C₇ alkoxy;
R¹⁶ is H, cyano, C₁-C₇ alkyl, halogen, C₁-C₄ alkylthio, C₁-C₇ haloalkyl or C₁-C₇ alkoxy;
R¹⁷ is H, C₁-C₇ alkyl, halogen, CN, C₂-C₆ alkenyl, C₂-C₆ alkynyl, C₃-C₇ cycloalkyl, C₂-C₃ cyanoalkyl, C₁-C₇ haloalkyl, C₂-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl;
R¹⁸ is H, C₁-C₇ alkyl, halogen, C₁-C₇ haloalkyl or C₁-C₇ alkoxy;
R¹⁹ is C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl, C₄-C₇ alkylcycloalkyl;
R²⁰ is H or C₁-C₇ haloalkyl; and
R^{f} is C₁-C₇ haloalkyl.

More particularly, this invention pertains to a compound of Formula **1,** all stereoisomers, an N-oxide or a salt thereof. This invention also relates to a herbicidal composition comprising a compound of the disclosure (i.e. in a herbicidally effective amount) and at least one component selected from the group consisting of surfactants, solid diluents and liquid diluents. This invention further relates to a method for controlling the growth of undesired vegetation comprising contacting the vegetation or its environment with a herbicidally effective amount of a compound of the disclosure (e.g., as a composition described herein).

This invention also includes a herbicidal mixture comprising (a) a compound selected from Formula **1,** all stereoisomers, N-oxides, and salts thereof, and (b) at least one additional active ingredient selected from (b1) through (b 16), and salts of compounds of (b1) through (b16), as described below.

### DETAILS OF THE INVENTION

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process, method, article or apparatus that comprises a list of elements is not necessarily limited to only those elements but may include other elements not expressly listed or inherent to such composition, mixture, process, method, article or apparatus.

The transitional phrase "consisting of" excludes any element, step or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition, method or apparatus that includes materials, steps, features, components or elements, in addition to those literally disclosed, provided that these additional materials, steps, features, components or elements do not materially affect the basic and novel characteristic(s) of the claimed invention. The term "consisting essentially of" occupies a middle ground between "comprising" and "consisting of".

Where applicants have defined an invention or a portion thereof with an open-ended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As referred to herein, the term "seedling", used either alone or in a combination of words means a young plant developing from the embryo of a seed.

As referred to herein, the term "broadleaf" used either alone or in words such as "broadleaf weed" means dicot or dicotyledon, a term used to describe a group of angiosperms characterized by embryos having two cotyledons.

In the above recitations, the term "alkyl", used either alone or in compound words such as "alkylthio" or "haloalkyl" includes straight-chain or branched alkyl, such as, methyl, ethyl, n-propyl, i-propyl or the different butyl, pentyl or hexyl isomers. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl. "Alkynylalkyl" denotes alkynyl substitution on alkyl. Examples of "alkynylalkyl" include CH≡CCH₂, CH₃C≡≡CCH₂, CH≡CCH₂CH₂, CH≡CCH(CH₃)CH₂ and the different alkynylalkyl isomers. "Alkylene" denotes a straight-chain or branched alkanediyl. Examples of "alkylene" include CH₂, CH₂CH₂, CH(CH₃), CH₂CH₂CH₂, CH₂CH(CH₃) and the different butylene isomers. "Alkenylene" denotes a straight-chain or branched alkenediyl containing one olefinic bond. Examples of "alkenylene" include CH=CH, CH₂CH=CH, CH=C(CH₃) and the different butenylene isomers. "Alkynylene" denotes a straight-chain or branched alkynediyl containing one triple bond. Examples of "alkynylene" include C=C, CH₂C≡C, C≡CCH₂ and the different butynylene isomers.

"Alkoxy" includes, for example, methoxy, ethoxy, n-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Alkoxyalkoxy" denotes alkoxy substitution on alkoxy. "Alkenyloxy" includes straight-chain or branched alkenyloxy moieties. Examples of "alkenyloxy" include H₂C=CHCH₂O, (CH₃)₂C=CHCH₂O, (CH₃)CH=CHCH₂O, (CH₃)CH=C(CH₃)CH₂O and CH₂=CHCH₂CH₂O. "Alkynyloxy" includes straight-chain or branched alkynyloxy moieties. Examples of "alkynyloxy" include HC≡CCH₂O, CH₃C≡CCH₂O and CH₃C≡CCH₂CH₂O. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include CH₃S(O)-, CH₃CH₂S(O)-, CH₃CH₂CH₂S(O)-, (CH₃)₂CHS(O)- and the different butylsulfinyl, pentylsulfinyl and hexylsulfinyl isomers. Examples of "alkylsulfonyl" include CH₃S(O)₂-, CH₃CH₂S(O)₂-, CH₃CH₂CH₂S(O)₂-, (CH₃)₂CHS(O)₂-, and the different butylsulfonyl, pentylsulfonyl and hexylsulfonyl isomers. "Alkylthioalkyl" denotes alkylthio substitution on alkyl. Examples of "alkylthioalkyl" include CH₃SCH₂, CH₃SCH₂CH₂, CH₃CH₂SCH₂, CH₃CH₂CH₂CH₂SCH₂ and CH₃CH₂SCH₂CH₂. "Alkylthioalkoxy" denotes alkylthio substitution on alkoxy. "Alkyldithio" denotes branched or straight-chain alkyldithio moieties. Examples of "alkyldithio" include CH₃SS-, CH₃CH₂SS-, CH₃CH₂CH₂SS-, (CH₃)₂CHSS- and the different butyldithio and pentyldithio isomers. "Cyanoalkyl" denotes an alkyl group substituted with one cyano group. Examples of "cyanoalkyl" include NCCH₂, NCCH₂CH₂ and CH₃CH(CN)CH₂. "Alkylamino", "dialkylamino", "alkenylthio", "alkenylsulfinyl", "alkenylsulfonyl", "alkynylthio", "alkynylsulfinyl", "alkynylsulfonyl", and the like, are defined analogously to the above examples.

"Cycloalkyl" includes, for example, cyclopropyl, cyclobutyl, cyclopentyl and cyclohexyl. The term "alkylcycloalkyl" denotes alkyl substitution on a cycloalkyl moiety and includes, for example, ethylcyclopropyl, i-propylcyclobutyl, 3-methylcyclopentyl and 4-methylcyclohexyl. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to straight-chain or branched alkyl groups. The term "cycloalkoxy" denotes cycloalkyl linked through an oxygen atom such as cyclopentyloxy and cyclohexyloxy. "Cycloalkylalkoxy" denotes cycloalkylalkyl linked through an oxygen atom attached to the alkyl chain. Examples of "cycloalkylalkoxy" include cyclopropylmethoxy, cyclopentylethoxy, and other cycloalkyl moieties bonded to straight-chain or branched alkoxy groups. "Cyanocycloalkyl" denotes a cycloalkyl group substituted with one cyano group. Examples of "cyanocycloalkyl" include 4-cyanocyclohexyl and 3-cyanocyclopentyl. "Cycloalkenyl" includes groups such as cyclopentenyl and cyclohexenyl as well as groups with more than one double bond such as 1,3- and 1,4-cyclohexadienyl.

The term "halogen", either alone or in compound words such as "haloalkyl" or when used in descriptions such as "alkyl substituted with halogen" includes fluorine, chlorine, bromine or iodine. Further, when used in compound words such as "haloalkyl" or when used in descriptions such as "alkyl substituted with halogen" said alkyl may be partially or fully substituted with halogen atoms which may be the same or different. Examples of "haloalkyl" or "alkyl substituted with halogen" include F₃C, ClCH₂, CF₃CH₂ and CF₃CCl₂. The terms "halocycloalkyl", "haloalkoxy", "haloalkylthio", "haloalkenyl", "haloalkynyl", and the like, areis defined analogously to the term "haloalkyl". Examples of "haloalkoxy" include CF₃O-, CCl₃CH₂O-, HCF₂CH₂CH₂O- and CF₃CH₂O-. Examples of "haloalkylthio" include CCl₃S-, CF₃S-, CCl₃CH₂S- and ClCH₂CH₂CH₂S-. Examples of "haloalkylsulfinyl" include CF₃S(O)-, CCl₃S(O)-, CF₃CH₂S(O)- and CF₃CF₂S(O)-. Examples of "haloalkylsulfonyl" include CF₃S(O)₂-, CCl₃S(O)₂-, CF₃CH₂S(O)₂- and CF₃CF₂S(O)₂-. Examples of "haloalkenyl" include (Cl)₂C=CHCH₂- and CF₃CH₂CH=CHCH₂-. Examples of "haloalkynyl" include HC≡CCHCl-, CF₃C≡C-, CCl₃C≡C- and FCH₂C≡CCH₂-. Examples of "haloalkoxyalkoxy" include CF₃OCH₂O-, ClCH₂CH₂OCH₂CH₂O-, Cl₃CCH₂OCH₂O- as well as branched alkyl derivatives.

"Alkylcarbonyl" denotes a straight-chain or branched alkyl moieties bonded to a C(=O) moiety. Examples of "alkylcarbonyl" include CH₃C(=O)-, CH₃CH₂CH₂C(=O)- and (CH₃)₂CHC(=O)-. Examples of "alkoxycarbonyl" include CH₃OC(=O)-, CH₃CH₂OC(=O)-, CH₃CH₂CH₂OC(=O)-, (CH₃)₂CHOC(=O)- and the different butoxy- or pentoxycarbonyl isomers.

The total number of carbon atoms in a substituent group is indicated by the "Cᵢ-Cⱼ" prefix where i and j are numbers from 1 to 7. For example, C₁-C₄ alkylsulfonyl designates methylsulfonyl through butylsulfonyl; C₂ alkoxyalkyl designates CH₃OCH₂-; C₃ alkoxyalkyl designates, for example, CH₃CH(OCH₃)-, CH₃OCH₂CH₂- or CH₃CH₂OCH₂-; and C₄ alkoxyalkyl designates the various isomers of an alkyl group substituted with an alkoxy group containing a total of four carbon atoms, examples including CH₃CH₂CH₂OCH₂- and CH₃CH₂OCH₂CH₂-·

When a compound is unsubstituted or substituted with a substituent bearing a subscript that indicates the number of said substituents, said substituents are independently selected from the group of defined substituents, e.g., [(R^{v})ᵣ], r is 0, 1, 2, 3, 4 or 5. For example, when r is 0, it indicates that the compound is unsubstituted, then hydrogen may be at the position even if not recited in the variable group definition. As another example, when r is 2, it indicates that the compound is substituted with two R^{v} which are independently selected from the group of defined substituents.

When a group contains a substituent which can be hydrogen, for example R¹ or R², then when this substituent is taken as hydrogen, it is recognized that this is equivalent to said group being unsubstituted. When one or more positions on a group are said to be "not substituted" or "unsubstituted", then hydrogen atoms are attached to take up any free valency.

Unless otherwise indicated, a "ring" as a component of Formula **1** (e.g., two R^{v} taken together with the carbon atom they are attached to form a ring) is carbocyclic or heterocyclic. The term "ring member" refers to an atom or other moiety (e.g., C(=O), C(=S), S(O) or S(O)₂) forming the backbone of a ring or ring system.

The terms "carbocyclic", "carbocycle" or "carbocyclic ring system" denote a ring or ring system wherein the atoms forming the ring backbone are selected only from carbon. Unless otherwise indicated, a carbocyclic ring can be a saturated, partially unsaturated or fully unsaturated ring. When a fully unsaturated carbocyclic ring satisfies Hückel's rule, then said ring is also called an "aromatic ring". "Saturated carbocyclic" refers to a ring having a backbone consisting of carbon atoms linked to one another by single bonds; unless otherwise specified, the remaining carbon valences are occupied by hydrogen atoms.

The terms "heterocyclic ring", "heterocycle" or "heterocyclic ring system" denote a ring or ring system in which at least one atom forming the ring backbone is not carbon, e.g., nitrogen, oxygen or sulfur. Typically a heterocyclic ring contains no more than 4 nitrogens, no more than 2 oxygens and no more than 2 sulfurs. Unless otherwise indicated, a heterocyclic ring can be a saturated, partially unsaturated or fully unsaturated ring. When a fully unsaturated heterocyclic ring satisfies Hückel's rule, then said ring is also called a "heteroaromatic ring" or "aromatic heterocyclic ring". Unless otherwise indicated, heterocyclic rings and ring systems can be attached through any available carbon or nitrogen by replacement of a hydrogen on said carbon or nitrogen.

"Aromatic" indicates that each of the ring atoms is essentially in the same plane and has a p-orbital perpendicular to the ring plane, and that (4n + 2) π electrons, where n is a positive integer, are associated with the ring to comply with Hückel's rule. The term "aromatic ring system" denotes a carbocyclic or heterocyclic ring system in which at least one ring of the ring system is aromatic. The term "aromatic carbocyclic ring system" denotes a carbocyclic ring system in which at least one ring of the ring system is aromatic. The term "aromatic heterocyclic ring system" denotes a heterocyclic ring system in which at least one ring of the ring system is aromatic. The term "nonaromatic ring system" denotes a carbocyclic or heterocyclic ring system that may be fully saturated, as well as partially or fully unsaturated, provided that none of the rings in the ring system are aromatic. The term "nonaromatic carbocyclic ring system" in which no ring in the ring system is aromatic. The term "nonaromatic heterocyclic ring system" denotes a heterocyclic ring system in which no ring in the ring system is aromatic.

The term "optionally substituted" in connection with the heterocyclic rings refers to groups which are unsubstituted or have at least one non-hydrogen substituent that does not extinguish the biological activity possessed by the unsubstituted analog. As used herein, the following definitions shall apply unless otherwise indicated. The term "optionally substituted" is used interchangeably with the phrase "substituted or unsubstituted" or with the term "(un)substituted." Unless otherwise indicated, an optionally substituted group may have a substituent at each substitutable position of the group, and each substitution is independent of the other.

In Formula **1,** when G is CONR⁵R⁶, NR⁵R⁶ can be (among others) J. Some non-limiting examples of J are illustrated in the table of Exhibit 1 wherein each structure is associated with a J-# and the # is a number.

### Exhibit 1

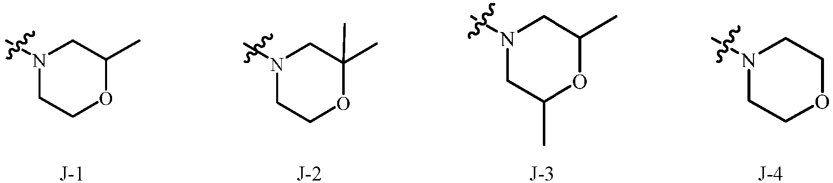

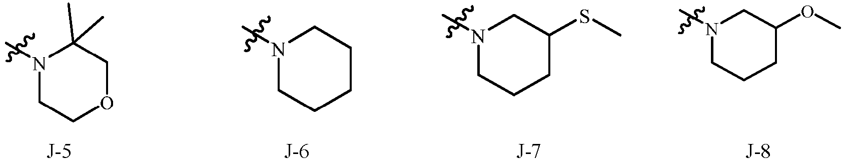

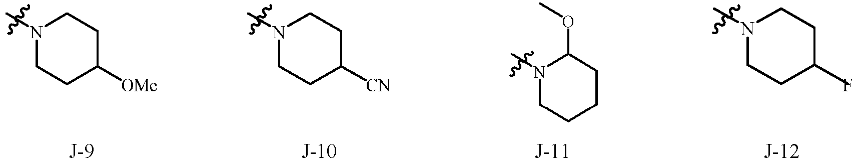

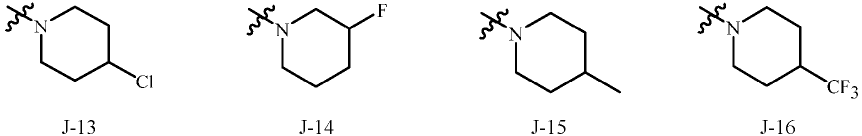

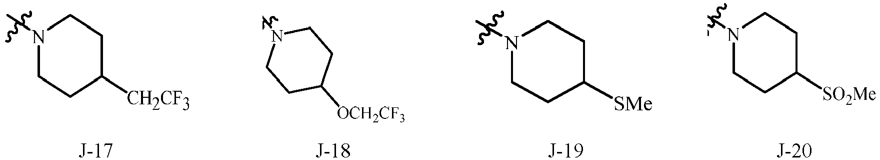

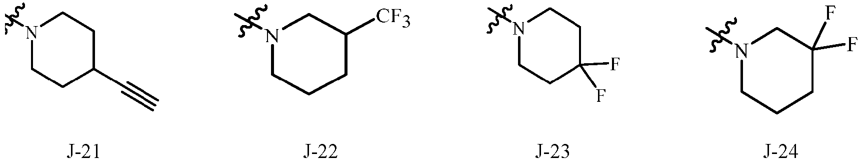

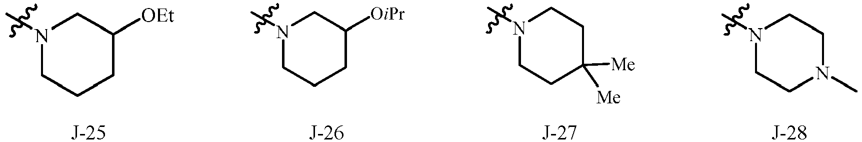

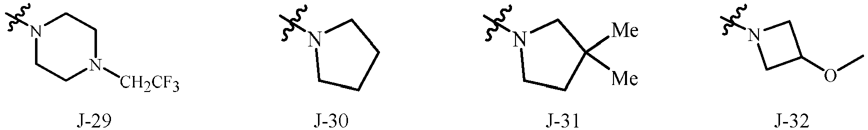

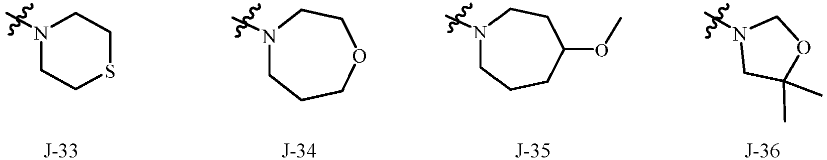

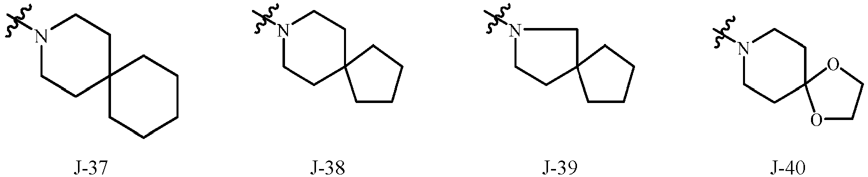

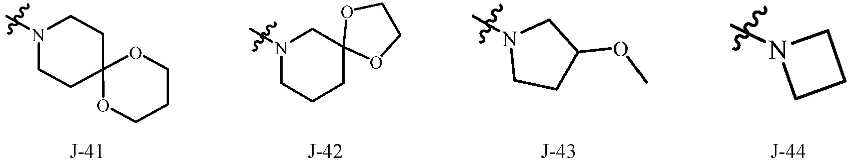

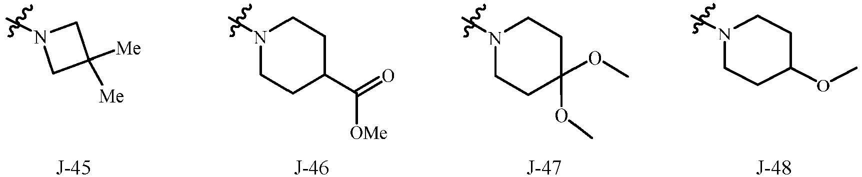

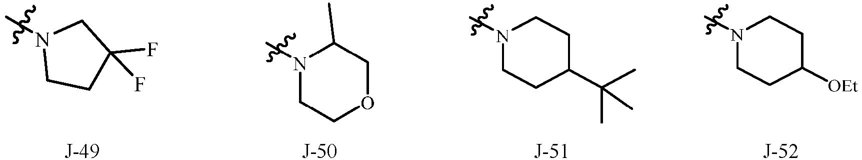

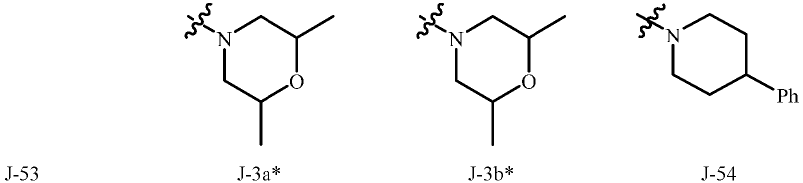

* "a" indicates the two CH₃ moieties are in the *cis* configuration; and "b" indicates the two CH₃ moieties are in the *trans* configuration.

In Formula **1,** when G is CONR⁵R⁶, NR⁵R⁶ can also be (among others) K. Some non-limiting examples of K are illustrated in the table of Exhibit 2 wherein each structure is associated with a K-# and the # is a number.

### Exhibit 2

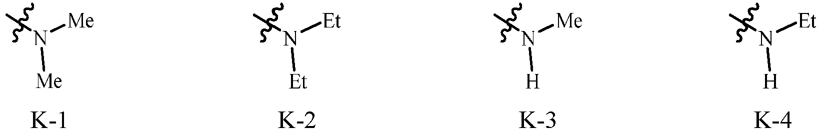

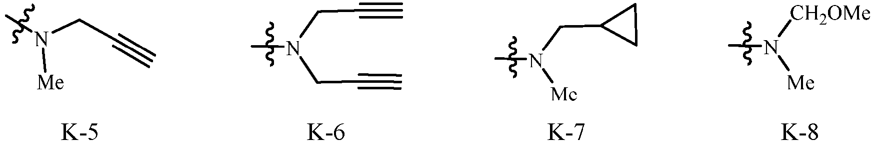

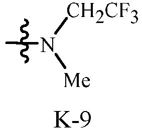

In Formula **1,** when G is G-1, some non-limiting examples of G-1 are illustrated in the table of Exhibit 3 wherein each structure is associated with a G-1-# and # is a number.

### Exhibit 3

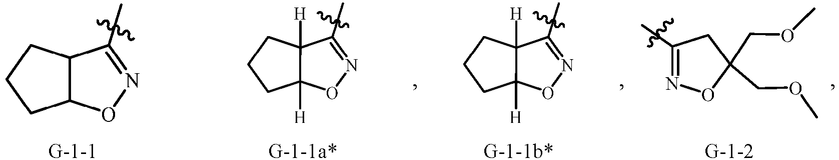

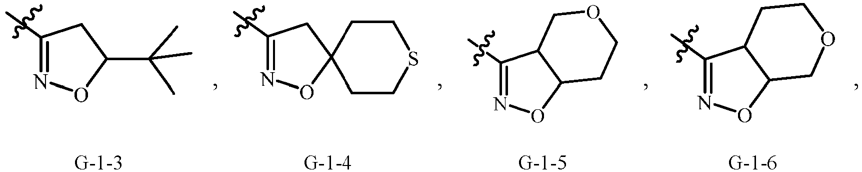

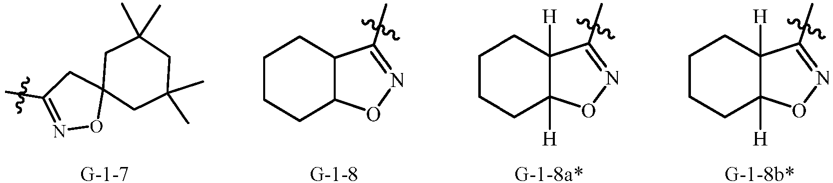

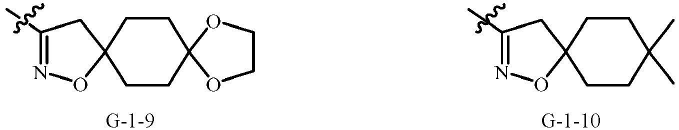

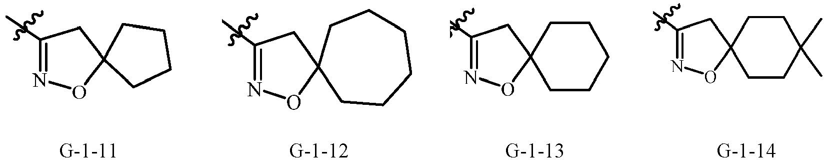

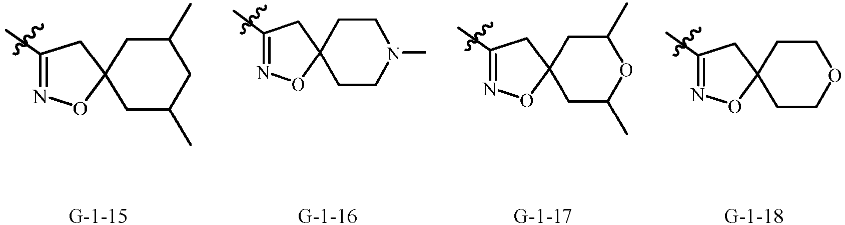

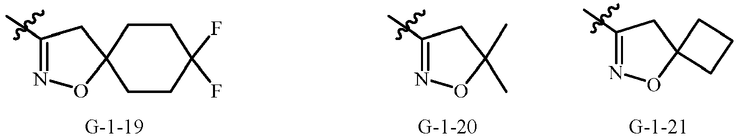

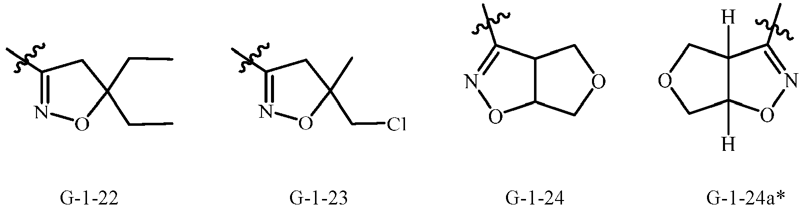

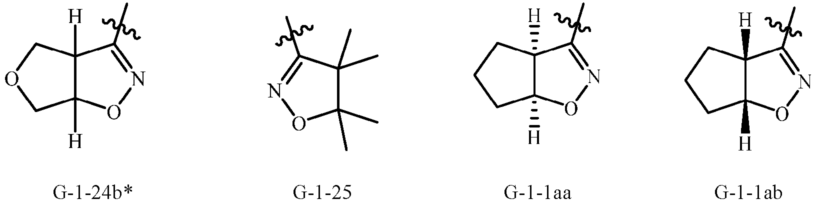

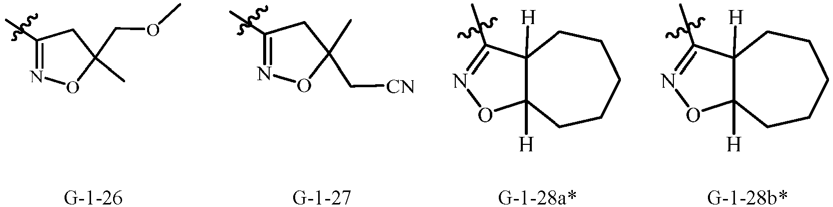

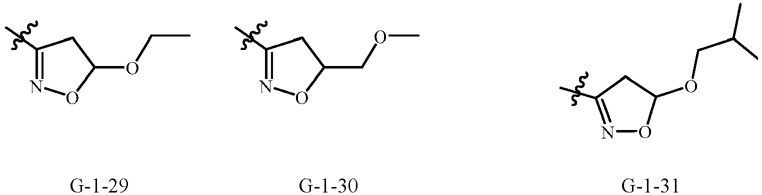

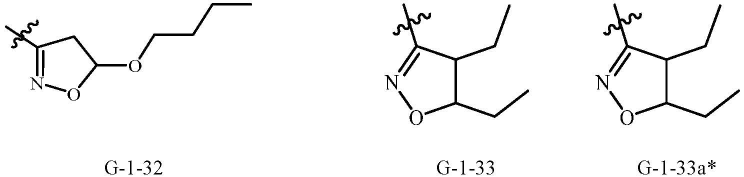

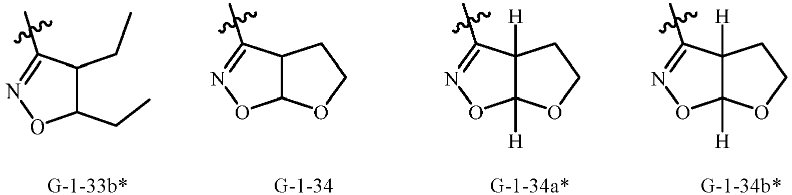

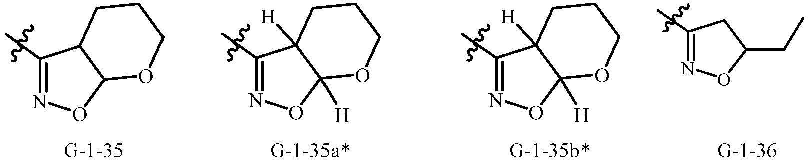

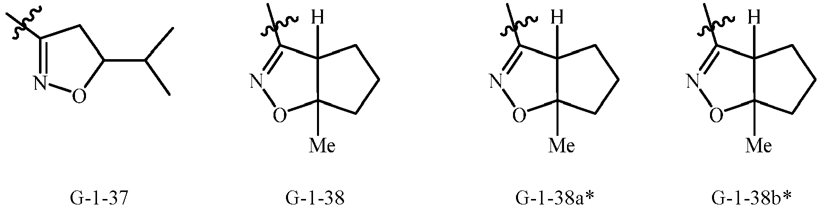

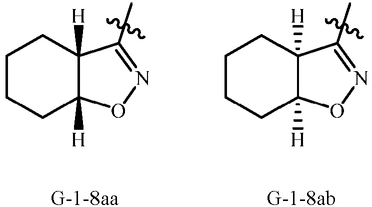

* "a" indicates the two H's are in *cis* configuration; and "b" indicates the two H's are in *trans* configuration, except in the following compounds; for G-1-33a and G-1-33b where "a" indicates the two Et's are in *cis* configuration, and "b" indicates the two Et's are in *trans* configuration; for G-1-38a and G-1-38b, "a" indicates that the H and Me shown are in *cis* configuration, and "b" indicates that the H and Me shown are in *trans* configuration.

In Formula **1,** when G is G-2, some non-limiting examples of G-2 are illustrated in the table of Exhibit 4 wherein each structure is associated with a G-2-# and # is a number.

### Exhibit 4

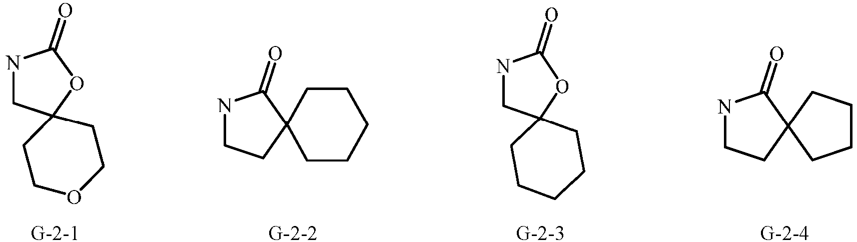

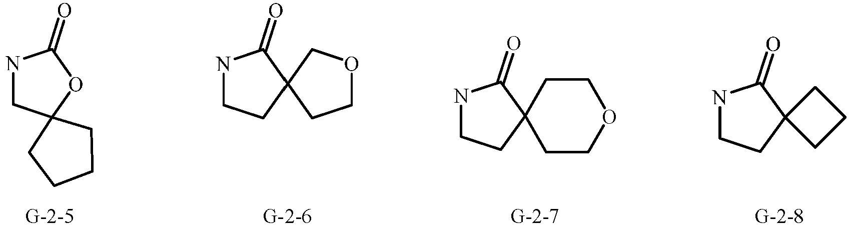

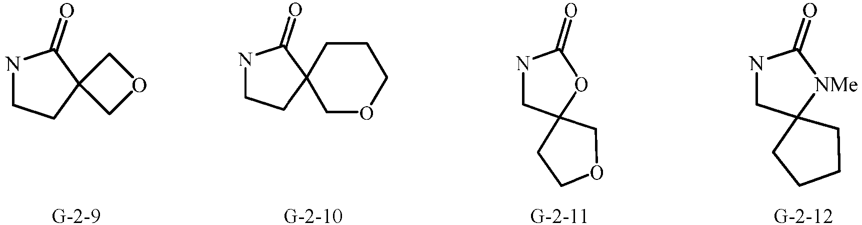

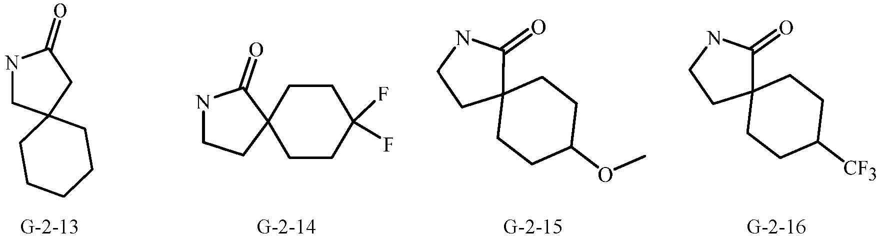

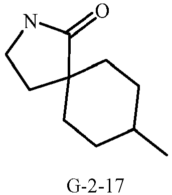

A wide variety of synthetic methods are known in the art to enable preparation of aromatic and nonaromatic heterocyclic rings and ring systems; for extensive reviews see the eight volume set of Comprehensive Heterocyclic Chemistry, A. R. Katritzky and C. W. Rees editors-in-chief, Pergamon Press, Oxford, 1984 and the twelve volume set of Comprehensive Heterocyclic Chemistry II, A. R. Katritzky, C. W. Rees and E. F. V. Scriven editors-in-chief, Pergamon Press, Oxford, 1996.

Compounds of this invention can exist as one or more stereoisomers. The various stereoisomers include enantiomers, diastereomers, atropisomers and geometric isomers. Stereoisomers are isomers of identical constitution but differing in the arrangement of their atoms in space and include enantiomers, diastereomers, cis-trans isomers (also known as geometric isomers) and atropisomers. Atropisomers result from restricted rotation about single bonds where the rotational barrier is high enough to permit isolation of the isomeric species. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, individual stereoisomers or as an optically active form.

For example, when G is G-1 and R7 and R9 are taken together with the carbon atoms they are attached to form a c-pentyl ring, the compound of Formula **1** can have at least two stereoisomers. The two stereoisomers are depicted as Formula **1'** and Formula **1"** with the chiral center identified with an asterisk (*). For a comprehensive discussion of all aspects of stereoisomerism, see Ernest L. Eliel and Samuel H. Wilen, Stereochemistry of Organic Compounds, John Wiley & Sons, 1994.

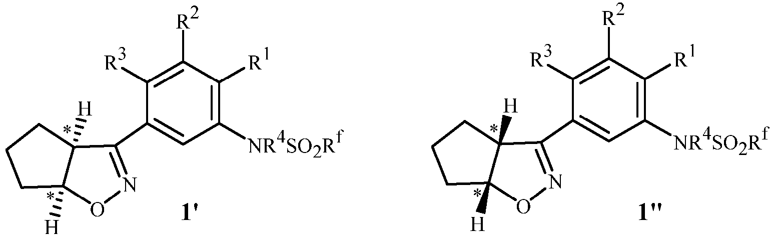

As another example, when G is CONR⁵R⁶, R⁵ and R⁶ are taken together with the nitrogen atom they are attached to form a piperidinyl ring, which possesses at least one chiral center, the compound of Formula **1** can have at least two stereoisomers with the chiral center identified with an asterisk (*).

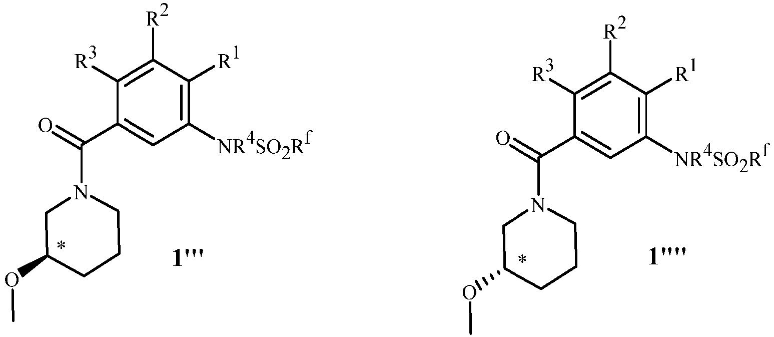

Molecular depictions drawn herein follow standard conventions for depicting stereochemistry. To indicate stereoconfiguration, bonds rising from the plane of the drawing and towards the viewer are denoted by solid wedges wherein the broad end of the wedge is attached to the atom rising from the plane of the drawing towards the viewer. Bonds going below the plane of the drawing and away from the viewer are denoted by dashed wedges wherein the broad end of the wedge is attached to the atom further away from the viewer. Constant width lines indicate bonds with a direction opposite or neutral relative to bonds shown with solid or dashed wedges; constant width lines also depict bonds in molecules or parts of molecules in which no particular stereoconfiguration is intended to be specified.

This invention comprises racemic mixtures, for example, equal amounts of the enantiomers of Formulae **1'** and **1"** or equal amounts of the enantiomers of Formulae **1‴** and **1ʺʺ**. In addition, this invention includes compounds that are enriched compared to the racemic mixture in an enantiomer of Formula **1.** Also included are the essentially pure enantiomers of compounds of Formula **1,** for example, Formula **1'** or Formula **1".**

When enantiomerically enriched, one enantiomer is present in greater amounts than the other, and the extent of enrichment can be defined by an expression of enantiomeric excess ("ee"), which is defined as (2x-1)·100 %, where x is the mole fraction of the dominant enantiomer in the mixture (e.g., an ee of 20 % corresponds to a 60:40 ratio of enantiomers).

Preferably the compositions of this invention have at least a 50 % enantiomeric excess; more preferably at least a 75 % enantiomeric excess; still more preferably at least a 90 % enantiomeric excess; and the most preferably at least a 94 % enantiomeric excess of the more active isomer. Of particular note are enantiomerically pure embodiments of the more active isomer.

Compounds of Formula **1** can comprise additional chiral centers. For example, substituents and other molecular constituents such as R^{v} may themselves contain chiral centers. This invention comprises racemic mixtures as well as enriched and essentially pure stereoconfigurations at these additional chiral centers.

Compounds of this invention can exist as one or more conformational isomers due to any restricted bond rotation in Formula **1.** This invention comprises mixtures of conformational isomers. In addition, this invention includes compounds that are enriched in one conformer relative to others.

Compounds of Formula **1** typically exist in more than one form, and Formula 1 thus include all crystalline and non-crystalline forms of the compounds they represent. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound of Formula 1 can exhibit beneficial effects (e.g., suitability for preparation of useful formulations, improved biological performance) relative to another polymorph or a mixture of polymorphs of the same compound of Formula **1.** Preparation and isolation of a particular polymorph of a compound of Formula **1** can be achieved by methods known to those skilled in the art including, for example, crystallization using selected solvents and temperatures. For a comprehensive discussion of polymorphism see R. Hilfiker, Ed., Polymorphism in the Pharmaceutical Industry, Wiley-VCH, Weinheim, 2006.

One skilled in the art will appreciate that not all nitrogen-containing heterocycles can form N-oxides since the nitrogen requires an available lone pair for oxidation to the oxide; one skilled in the art will recognize those nitrogen-containing heterocycles which can form N-oxides. One skilled in the art will also recognize that tertiary amines can form N-oxides. Synthetic methods for the preparation of N-oxides of heterocycles and tertiary amines are very well known by one skilled in the art including the oxidation of heterocycles and tertiary amines with peroxy acids such as peracetic and m-chloroperbenzoic acid (MCPBA), hydrogen peroxide, alkyl hydroperoxides such as t-butyl hydroperoxide, sodium perborate, and dioxiranes such as dimethyldioxirane. These methods for the preparation of N-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of a compound of Formula 1 are useful for control of undesired vegetation (i.e. are agriculturally suitable). The salts of a compound of Formula 1 include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula **1** contains an acidic moiety such as a carboxylic acid or phenol, salts also include those formed with organic or inorganic bases such as pyridine, triethylamine or ammonia or amides, hydrides, hydroxides or carbonates of sodium, potassium, lithium, calcium, magnesium or barium. Accordingly, the present invention comprises compounds selected from Formula **1,** N-oxides and agriculturally suitable salts thereof.

Embodiments of the present invention as described in the Summary of the Disclosure include those wherein a compound of Formula **1** is as described in any of the following Embodiments:
Embodiment 1. A compound of Formula **1,** stereoisomers, N-oxides, and salts thereof, agricultural compositions containing them and their use as herbicides as described in the Summary of the Disclosure.
Embodiment 1a. A compound of Embodiment 1 wherein G is CONR⁵R⁶.
Embodiment 1b. A compound of Embodiment 1 wherein G is G-1.
Embodiment 1c. A compound of Embodiment 1 wherein G is G-2.
Embodiment 1d. A compound of Embodiment 1 wherein G is G-3.
Embodiment 1e. A compound of Embodiment 1 wherein G is G-4.
Embodiment 1f. A compound of Embodiment 1 wherein G is G-5.
Embodiment 2. A compound of Formula **1** or any one of the preceding Embodiments wherein R¹ is H, C₁-C₇ alkyl, halogen, C₂-C₆ alkenyl, C₂-C₆ alkynyl, C₃-C₇ cycloalkyl or C₁-C₇ haloalkyl.
Embodiment 2a. A compound of Embodiment 2 wherein R¹ is H, C₁-C₇ alkyl, halogen or C₃-C₇ cycloalkyl;
Embodiment 2b. A compound of Embodiment 2a wherein R¹ is H, C₁-C₃ alkyl, halogen or C₃-C₄ cycloalkyl.
Embodiment 2c. A compound of Embodiment 2b wherein R¹ is H, Me, halogen or cyclopropyl.
Embodiment 2d. A compound of Embodiment 2c wherein R¹ is H, Me, F, Cl, Br or cyclopropyl.
Embodiment 2e. A compound of Embodiment 2d wherein R¹ is Me or Cl.
Embodiment 2f. A compound of Embodiment 2e wherein R¹ is Me.
Embodiment 2g. A compound of Embodiment 2e wherein R¹ is Cl.
Embodiment 3. A compound of Formula 1 or any one of the preceding Embodiments wherein R² is H, C₁-C₇ alkyl, halogen, -CN, C₁-C₇ haloalkyl, C₁-C₇ alkoxy, C₃-C₇ cycloalkyl or C₁-C₅ alkylthio.
Embodiment 3a. A compound of Embodiment 3 wherein R² is H, C₁-C₇ alkyl, C₃-C₆ cycloalkyl, halogen or CN.
Embodiment 3b. A compound of Embodiment 3a wherein R² is H, Me, F, Cl or CN.
Embodiment 3c. A compound of Embodiment 3b wherein R² is H, Me or F.
Embodiment 3d. A compound of Embodiment 3c wherein R² is H.
Embodiment 3e. A compound of Embodiment 3c wherein R² is F.
Embodiment 3f. A compound of Embodiment 3c wherein R² is Me.
Embodiment 4. A compound of Formula 1 or any one of the preceding Embodiments wherein R³ is H, C₁-C₇ alkyl, halogen, -CN, C₂-C₆ alkenyl, C₃-C₇ alkynyl, C₃-C₇ cycloalkyl, C₂-C₃ cyanoalkyl, C₁-C₇ haloalkyl, C₃-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl.
Embodiment 4a. A compound of Embodiment 4 wherein R³ is H, C₁-C₇ alkyl, halogen, CN, C₁-C₇ alkoxy or C₁-C₇ haloalkyl.
Embodiment 4b. A compound of Embodiment 4a wherein R³ is H, Me, F, Cl, -CN, OMe or CF₃.
Embodiment 4c. A compound of Embodiment 4b wherein R³ is Me or F.
Embodiment 4d. A compound of Embodiment 4c wherein R³ is Me.
Embodiment 4f. A compound of Embodiment 4c wherein R³ is Cl.
Embodiment 4g. A compound of Embodiment 4c wherein R³ is F.
Embodiment 5. A compound of Formula **1** or any one of the preceding Embodiments wherein R⁴ is H, C(=O)R¹⁹, C(=S)R¹⁹, C(=O)OR¹⁹, C(=O)SR¹⁹, S(O)₂R¹⁹, C(=O)NR¹⁹R²⁰, S(O)₂NR¹⁹R²⁰, -S(OH)₂NR¹⁹R²⁰, CH₂OC(=O)OR¹⁹, CH₂OC(=O)NR¹⁹R²⁰ or CH₂OC(=O)R¹⁹.
Embodiment 5a. A compound of Embodiment 5 wherein R⁴ is H, C(=O)R¹⁹, CO₂R¹⁹, C(=O)SR¹⁹, S(O)₂R19 or CH₂OCOR¹⁹.
Embodiment 5b. A compound of Embodiment 5a wherein R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe.
Embodiment 5c. A compound of Embodiment 5a wherein R⁴ is H, CH₂OCOR¹⁹ or -S(O)₂R¹⁹.
Embodiment 5d. A compound of Embodiment 5c wherein R⁴ is H, CH₂OCO-*t*-Bu or SO₂CF₃.
Embodiment 6. A compound of Formula **1** or any one of the preceding Embodiments wherein R⁵ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy, C₂-C₇ alkoxyalkyl or C₄-C₇ alkylcycloalkyl.
Embodiment 6a. A compound of Embodiment 6 wherein R⁵ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl or C₂-C₃ cyanoalkyl.
Embodiment 6b. A compound of Embodiment 6a wherein R⁵ is H, C₁-C₃ alkyl, C₂-C₃ alkenyl, C₂-C₃ alkynyl, C₃-C₆ cycloalkyl, C₄-C₇ cycloalkylalkyl, C₃-C₆ alkenylalkyl, C₃-C₆ alkynylalkyl or C₂-C₃ cyanoalkyl.
Embodiment 6c. A compound of Embodiment 6b wherein R⁵ is H, methyl, ethyl, propyl, cyanomethyl, CH₂CCH or c-propylmethyl.
Embodiment 6d. A compound of Embodiment 6c wherein R⁵ is H or methyl.
Embodiment 6e. A compound of Embodiment 6c wherein R⁵ is methyl.
Embodiment 7. A compound of Formula **1** or any one of the preceding Embodiments wherein R⁶ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy, C₂-C₇ alkoxyalkyl or C₄-C₇ alkylcycloalkyl.
Embodiment 7a. A compound of Embodiment 7 wherein R⁶ is H, C₁-C₇ alkyl, C₂-C₆ alkenyl, C₂-C₆ alkynyl, C₃-C₇ cycloalkyl, C₄-C₇ cycloalkylalkyl, C₃-C₇ alkenylalkyl, C₃-C₇ alkynylalkyl or C₂-C₃ cyanoalkyl.
Embodiment 7b. A compound of Embodiment 7a wherein R⁶ is H, C₁-C₃ alkyl, C₂-C₃ alkenyl, C₂-C₃ alkynyl, C₃-C₆ cycloalkyl, C₄-C₇ cycloalkylalkyl, C₃-C₆ alkenylalkyl, C₃-C₆ alkynylalkyl or C₂-C₃ cyanoalkyl.
Embodiment 7c. A compound of Embodiment 7b wherein R⁶ is H, methyl, ethyl, propyl, cyanomethyl, CH₂CCH or c-propylmethyl.
Embodiment 7d. A compound of Embodiment 7c wherein R⁶ is H or methyl.
Embodiment 7e. A compound of Embodiment 7e wherein R⁶ is methyl.
Embodiment 8. A compound of Formula **1** or any one of Embodiments wherein R⁵ and R⁶ are taken together with the nitrogen atom to which they are attached to form a 3- to 7-membered ring, containing carbon atoms and optionally 1 to 3 oxygen, sulfur or nitrogen atoms as ring members, wherein up to 2 carbon atom ring members are independently selected from C(=O) and C(=S), and the sulfur atom ring member is selected from S, S(O) or S(O)₂, said ring optionally substituted with up to 5 substituents independently selected from (R^{v})ᵣ and r is the number of the substituents.
Embodiment 8a. A compound of Embodiment 8 wherein the 3- to 7-membered ring is a 5-membered ring.
Embodiment 8b. A compound of Embodiment 8a wherein the 5-membered ring is unsubstituted.
Embodiment 8c. A compound of Embodiment 8a wherein the 5-membered ring is substituted with at least one halogen, OMe, SMe or methyl.
Embodiment 8d. A compound of any one of Embodiments 8a through 8c wherein the 5-membered ring is a pyrrolidinyl or oxazolidinyl.
Embodiment 8e. A compound of Embodiment 8 wherein the 3- to 7-membered ring is a 6-membered ring.
Embodiment 8f. A compound of Embodiment 8e wherein the 6-membered ring is unsubstituted.
Embodiment 8g. A compound of Embodiment 8e wherein the 6-membered ring is substituted with at least one halogen, OMe, SMe or methyl.
Embodiment 8h. A compound of Embodiments 8e through 8g wherein the 6-membered ring is a morpholinyl, thiomorpholinyl, piperidinyl or piperazinyl.
Embodiment 8i. A compound of Embodiment 8 wherein the 3- to 7-membered ring is a 4-membered ring.
Embodiment 8j. A compound of Embodiment 8i wherein the 4-membered ring is unsubstituted.
Embodiment 8k. A compound of Embodiment 8i wherein the 4-membered ring is substituted with at least one halogen, OMe, SMe or methyl.
Embodiment 8l. A compound of any one of Embodiments 8i through 8k wherein the 4-membered ring is an azetidinyl.
Embodiment 8m. A compound of Embodiment 8 wherein the 3- to 7-membered ring is a 7-membered ring.
Embodiment 8n. A compound of Embodiment 8m wherein the 7-membered ring is unsubstituted.
Embodiment 8o. A compound of Embodiment 8m wherein the 7-membered ring is substituted with at least one halogen, OMe, SMe or methyl.
Embodiment 8p. A compound of any one of Embodiments 8m through 8o wherein the 7-membered ring is an azepanyl or 1,4-oxazepanyl.
Embodiment 9. A compound of Formula **1** or any one of the preceding Embodiments wherein R^{v} is independently selected from the group consisting of H, halogen, cyano, nitro, C₁-C₄ alkyl, C₃-C₆ cycloalkyl, C₁-C₄ haloalkyl, C₁-C₄ alkoxy and C₁-C₄ haloalkoxy.
Embodiment 9a. A compound of Embodiment 9 wherein R^{v} is independently selected from the group consisting of H, halogen, methyl, ethyl, propyl, *c*-propylmethyl, propargyl, OMe or cyano.
Embodiment 9b. A compound of Embodiment 9a wherein R^{v} is methyl.
Embodiment 9b1. A compound of Embodiment 9a wherein R^{v} is OMe.
Embodiment 9b2. A compound of Embodiment 9a wherein R^{v} is H.
Embodiment 9c. A compound of Formula **1** or any one of the preceding Embodiments 1 through 8p wherein two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1 to 3 oxygen, sulfur or nitrogen atoms as ring members, wherein up to 2 carbon atom ring members are independently selected from C(=O) and C(=S), and the sulfur atom ring member is selected from S, S(O) or S(O)₂, said ring being unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy.
Embodiment 9d. A compound of Embodiment 9c wherein the two R^{v} are attached to the same carbon atom, said two R^{v} are taken together with the carbon atom to which they are attached to form a 3- to 7-membered ring.
Embodiment 9e. A compound of Embodiment 9d wherein 3- to 7-membered ring is a 5-membered ring.
Embodiment 9f. A compound of Embodiment 9e wherein the 5-membered ring is a 1,3-dioxolanyl or c-pentyl.
Embodiment 9g. A compound of Embodiment 9d wherein 3- to 7-membered ring is a 6-membered ring.
Embodiment 9h. A compound of Embodiment 9g wherein the 6-membered ring is a 1,3-dioxanyl or c-hexyl.
Embodiment 9i. A compound of Embodiment 9c wherein the two R^{v} are attached to two adjacent carbon atoms, said two R^{v} are taken together with the carbon atoms to which they are attached to form a 3- to 7-membered ring.
Embodiment 9j. A compound of Embodiment 9i wherein 3- to 7-membered ring is a 5-membered ring.
Embodiment 9k. A compound of Embodiment 9j wherein the 5-membered ring is a 1,3-dioxolanyl or *c*-pentyl.
Embodiment 9l. A compound of Embodiment 9i wherein the 3- to 7-membered ring is a 6-membered ring.
Embodiment 9m. A compound of Embodiment 9l wherein the 6-membered ring is a 1,3-dioxanyl or *c*-hexyl.
Embodiment 10. A compound of Formula **1** or any one of the preceding Embodiments wherein R⁷ is H, C₁-C₇ alkyl, halogen, -CN, C₁-C₇ haloalkyl or C₁-C₇ alkoxy.
Embodiment 10a. A compound of Embodiment 10 wherein R⁷ is H, methyl, F or Cl.
Embodiment 10b. A compound of Embodiment 10a wherein R⁷ is H.
Embodiment 11. A compound of Formula **1** or any one of the preceding Embodiments wherein R⁸ is H or C₁-C₇ alkyl.
Embodiment 11a. A compound of Embodiment 11 wherein R⁸ is H or Me.
Embodiment 11b. A compound of Embodiment 11a wherein R⁸ is H.
Embodiment 12. A compound of Formula **1** or any one of the preceding Embodiments wherein R⁷ and R⁸ may be taken together to form a 3- to 7-membered ring, containing carbon atoms and optionally 1-2 oxygen, sulfur or nitrogen atoms as the ring members.
Embodiment 12a. A compound of Embodiment 12 wherein the 3- to 7-membered ring is a 5-membered ring.
Embodiment 12b. A compound of Embodiment 12 wherein the 3- to 7-membered ring is a 6-membered ring.
Embodiment 13. A compound of Formula 1 or any one of the preceding Embodiments wherein R⁹ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl.
Embodiment 13a. A compound of Embodiment 13 wherein R⁹ is H, C₁-C₇ alkyl, C₁-C₇ haloalkyl or C₂-C₇ alkoxyalkyl.
Embodiment 13b. A compound of Embodiment 13a wherein R⁹ is methy, ethyl, t-butyl, chloromethyl or methoxymethyl.
Embodiment 15. A compound of Formula **1** or any one of the preceding Embodiments wherein R⁷ and R⁹ are taken together to form a fused 3- to 7-membered ring, containing carbon atoms and optionally 1-2 oxygen, sulfur or nitrogen atom members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy.
Embodiment 15a. A compound of Embodiment 15 wherein the 3- to 7-membered ring is a 5- or 6-membered ring.
Embodiment 15b. A compound of Embodiment 15a wherein the 5-membered ring is *c*-pentyl.
Embodiment 15c. A compound of Embodiment 15 wherein the 3- to 7-membered ring is a 6-membered ring.
Embodiment 15d. A compound of Embodiment 15c wherein the 6-membered ring is *c*-hexyl or tetrahydropyran.
Embodiment 15e. A compound of any one of Embodiments 15 to 15d wherein said ring unsubstituted or substituted with at least one substituent independently selected from H, halogen or C₁-C₄ alkyl.
Embodiment 15f. A compound of Embodiment 15e wherein said ring is unsubstituted.
Embodiment 16. A compound of Formula **1** or any one of the preceding Embodiments wherein R¹⁰ is H or C₁-C₇ alkyl.
Embodiment 16a. A compound of Embodiment 16 wherein R¹⁰ is methyl or ethyl.
Embodiment 16b. A compound of Embodiment 16 wherein R¹⁰ is H.
Embodiment 17. A compound of Formula **1** or any one of the preceding Embodiments wherein R⁹ and R¹⁰ are taken together with the carbon atom to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂, said ring optionally substituted with up to 5 substituents independently selected from (R^{v})ᵣ and r is the number of the substituents; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1-2 oxygen, sulfur or nitrogen atoms as the ring members, wherein up to 2 carbon atom ring members are independently selected from C(=O) and C(=S) and the sulfur atom ring member is selected from S, S(O) or S(O)₂.
Embodiment 17a. A compound of Embodiment 17 wherein the 3- to 7-membered ring is a 5- or 6-membered ring.
Embodiment 17b. A compound of Embodiment 17a wherein the 5-membered ring is cyclopentane.
Embodiment 17c. A compound of Embodiment 17 wherein the 3- to 7-membered ring is a 6-membered ring.
Embodiment 17d. A compound of Embodiment 17c wherein the 6-membered ring is cyclohexane, tetrahydro-2H-pyran or tetrahydro-2H-thiopyran.
Embodiment 17e. A compound of Embodiment 17d wherein the 6-membered ring is cyclohexane.
Embodiment 17f. A compound of Embodiment 17 wherein the 3- to 7-membered ring is a 4-membered ring.
Embodiment 17f. A compound of Embodiment 17 wherein the 3- to 7-membered ring is a 7-membered ring.
Embodiment 18. A compound of Formula **1** or any one of the preceding Embodiments wherein Q is O, S, CR¹¹R¹² or NR¹³.
Embodiment 18a. A compound of Embodiment 18 wherein Q is O or S.
Embodiment 18b. A compound of Embodiment 18 wherein Q is O.
Embodiment 18c. A compound of Embodiment 18 wherein Q is CR¹¹R¹².
Embodiment 18d. A compound of Embodiment 18 wherein Q is NR¹³.
Embodiment 18e. A compound of Embodiment 18 wherein Q is O, S or CR¹¹R¹².
Embodiment 18f. A compound of Embodiment 18 wherein Q is other than NR¹³.
Embodiment 19. A compound of Formula **1** or any one of the preceding Embodiments wherein R¹¹ and R¹² are taken together with the carbon atom to which they are attached to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy.
Embodiment 19a. A compound of Embodiment 19 wherein the 3- to 7-membered ring is a 5-membered ring.
Embodiment 19b. A compound of Embodiment 19a wherein the 5-membered ring is cyclopentane.
Embodiment 19c. A compound of Embodiment 19 wherein the 3- to 7-membered ring is a 6-membered ring.
Embodiment 19d. A compound of Embodiment 19c wherein the 6-membered ring is cyclohexane.
Embodiment 19e. A compound of Embodiment 19 wherein the ring is an unsubstituted 5- or 6-membered ring.
Embodiment 20. A compound of Formula **1** or any one of the preceding Embodiments wherein r is 0, 1, 2 or 3.
Embodiment 20a. A compound of Embodiment 20 wherein r is 0.
Embodiment 20b. A compound of Embodiment 20 wherein r is 1 or 2.
Embodiment 20c. A compound of Embodiment 20 wherein r is 2.
Embodiment 20d. A compound of Embodiment 20 wherein r is 3.
Embodiment 20e. A compound of Embodiment 20 wherein r is 1.
Embodiment 21. A compound of Formula **1** or any one of the preceding Embodiments wherein R¹³ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
Embodiment 21a. A compound of Embodiment 21wherein R¹³ is H or C₁-C₇ alkyl.
Embodiment 21b. A compound of Embodiment 21a wherein R¹³ is Me or Et.
Embodiment 21c. A compound of Embodiment 21b wherein R¹³ is Me.
Embodiment 21d. A compound of Embodiment 21d wherein R¹³ is Et.
Embodiment 22. A compound of Formula **1** or any one of the preceding Embodiments wherein R¹⁴ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₁-C₇ thioalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl.
Embodiment 22a. A compound of Embodiment 22wherein R¹⁴ is H, C₁-C₄ alkyl, C₃-C₇ cycloalkyl, C₄-C₇ cycloalkylalkyl, C₁-C₄ haloalkyl, C₁-C₄ haloalkyl or C₁-C₇ alkoxy.
Embodiment 22b. A compound of Embodiment 22a wherein R¹⁴ is C₁-C₄ alkyl.
Embodiment 22c. A compound of Embodiment 22b wherein R¹⁴ is Me.
Embodiment 22d. A compound of Embodiment 22b wherein R¹⁴ is Et.
Embodiment 22e. A compound of Embodiment 22b wherein R¹⁴ is CH₂CF₃.
Embodiment 23. A compound of Formula 1 or any one of the preceding Embodiments wherein R¹⁵ is H, C₁-C₇ alkyl, halogen, C₁-C₇ haloalkyl or C₁-C₇ alkoxy.
Embodiment 23a. A compound of Embodiment 23wherein R¹⁵ is H, C₁-C₃ alkyl or C₁-C₃ alkoxy.
Embodiment 23b. A compound of Embodiment 23a wherein R¹⁵ is H or OMe.
Embodiment 23c. A compound of Embodiment 23b wherein R¹⁵ is H.
Embodiment 23d. A compound of Embodiment 23b wherein R¹⁵ is OMe.
Embodiment 24. A compound of Formula **1** or any one of the preceding Embodiments wherein R¹⁶ is H, cyano, C₁-C₇ alkyl, halogen, C₁-C₄ alkylthio, C₁-C₇ haloalkyl or C₁-C₇ alkoxy.
Embodiment 24a. A compound of Embodiment 24 wherein R¹⁶ is H, cyano, C₁-C₄ alkyl, halogen, C₁-C₄ alkylthio, C₁-C₄ haloalkyl or C₁-C₄ alkoxy.
Embodiment 24b. A compound of Embodiment 24a wherein R¹⁶ is H or C₁-C₄ alkyl.
Embodiment 24c. A compound of Embodiment 24a wherein R¹⁶ is H, cyano, methyl, ethyl, propyl, i-propyl, halogen, SMe, CF₃ or OMe.
Embodiment 24d. A compound of Embodiment 24c wherein R¹⁶ is H, cyano, methyl, F, SMe, CF₃ or OMe.
Embodiment 25. A compound of Formula **1** or any one of the preceding Embodiments wherein R¹⁷ is H, C₁-C₇ alkyl, halogen, -CN, C₂-C₆ alkenyl, C₂-C₆ alkynyl, C₃-C₇ cycloalkyl, C₂-C₃ cyanoalkyl, C₁-C₇ haloalkyl, C₃-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl.
Embodiment 25a. A compound of Embodiment 25 wherein R¹⁷ is H, C₁-C₄ alkyl, halogen or C₁-C₄ alkoxy.
Embodiment 25b. A compound of Embodiment 25a wherein R¹⁷ is H, methyl, Cl or OMe.
Embodiment 26. A compound of Formula **1** or any one of the preceding Embodiments wherein R¹⁸ is H, C₁-C₇ alkyl, halogen, C₁-C₇ haloalkyl or C₁-C₇ alkoxy;
Embodiment 26a. A compound of Embodiment 26 wherein R¹⁸ is H or C₁-C₃ alkoxy;
Embodiment 26b. A compound of Embodiment 26 wherein R¹⁸ is H, methyl or OMe.
Embodiment 26c. A compound of Embodiment 26b wherein R¹⁸ is H.
Embodiment 26d. A compound of Embodiment 26b wherein R¹⁸ is OMe.
Embodiment 27. A compound of Formula **1** or any one of the preceding Embodiments wherein R¹⁹ is C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl.
Embodiment 27a. A compound of Embodiment 27 wherein R¹⁹ is C₁-C₇ alkyl or C₁-C₇ haloalkyl.
Embodiment 27b. A compound of Embodiment 27a wherein R¹⁹ is C₁-C₇ alkyl.
Embodiment 27c. A compound of Embodiment 27b wherein R¹⁹ is methyl, ethyl, i-propyl, t-butyl, n-butyl, s-butyl, i-butyl, c-pentyl or c-hexyl.
Embodiment 27d. A compound of Embodiment 27c wherein R¹⁹ is t-butyl.
Embodiment 27e. A compound of Embodiment 27a wherein R¹⁹ is C₁-C₃ haloalkyl.
Embodiment 27f. A compound of Embodiment 27e wherein R¹⁹ is CF₃.
Embodiment 28. A compound of Formula 1 or any one of the preceding Embodiments wherein R^{f} is C₁-C₇ alkyl or C₁-C₇ haloalkyl.
Embodiment 28a. A compound of Embodiment 28 wherein R^{f} is C₁-C₃ haloalkyl.
Embodiment 28b. A compound of Embodiment 28a wherein R^{f} is CF₃.

Embodiments of this invention, including Embodiments 1-28b above as well as any other embodiments described herein, can be combined in any manner, and the descriptions of variables in the embodiments pertain not only to the compounds of Formula **1** but also to the starting compounds and intermediate compounds useful for preparing the compounds of Formula **1.** In addition, embodiments of this invention, including Embodiments 1-28b above as well as any other embodiments described herein, and any combination thereof, pertain to the compositions and methods of the present invention.

Combinations of Embodiments 1-28b are illustrated by:
Embodiment PA. A compound of Formula **1** as described in the Summary of the Disclosure wherein Q is O, S or CR¹¹R¹².
Embodiment A. A compound of Formula **1** as described in the Summary of the Disclosure wherein
   G is CONR⁵R⁶,
   R¹ is H, C₁-C₇ alkyl, halogen, C₂-C₆ alkenyl, C₂-C₆ alkynyl, C₃-C₇ cycloalkyl or C₁-C₇ haloalkyl;
   R² is H, C₁-C₇ alkyl, C₃-C₆ cycloalkyl, halogen or CN;
   R³ is H, C₁-C₇ alkyl, halogen, CN, C₁-C₇ alkoxy or C₁-C₇ haloalkyl;
   R⁴ is H, C(=O)R¹⁹, CO₂R¹⁹, C(=O)SR¹⁹, S(O)₂R¹⁹ or CH₂OCOR¹⁹;
   R⁵ is H, C₁-C₃ alkyl, C₂-C₃ alkenyl, C₂-C₃ alkynyl, C₃-C₆ cycloalkyl, C₄-C₇ cycloalkylalkyl, C₃-C₆ alkenylalkyl, C₃-C₆ alkynylalkyl or C₂-C₃ cyanoalkyl;
   R⁶ is H, C₁-C₃ alkyl, C₂-C₃ alkenyl, C₂-C₃ alkynyl, C₃-C₆ cycloalkyl, C₄-C₇ cycloalkylalkyl, C₃-C₆ alkenylalkyl, C₃-C₆ alkynylalkyl or C₂-C₃ cyanoalkyl; and
   R^{f} is C₁-C₃ haloalkyl.
Embodiment B. A compound of Embodiment A wherein
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁵ is H, methyl, ethyl, propyl, cyanomethyl, CH₂CCH or c-propylmethyl;
   R⁶ is H, methyl, ethyl, propyl, cyanomethyl, CH₂CCH or c-propylmethyl; and
   R^{f} is CF₃.
Embodiment C. A compound of Embodiment B wherein
   R¹ is Me or Cl;
   R³ is Me;
   R⁴ is H, CH₂OCO-*t*-Bu or SO₂CF₃;
   R⁵ is methyl;
   R⁶ is methyl.
Embodiment D. A compound of Formula **1** as described in the Summary of the Disclosure wherein
   Q is CONR⁵R⁶;
   R¹ is H, C₁-C₇ alkyl, halogen, C₂-C₆ alkenyl, C₂-C₆ alkynyl, C₃-C₇ cycloalkyl or C₁-C₇ haloalkyl;
   R² is H, C₁-C₇ alkyl, C₃-C₆ cycloalkyl, halogen or CN;
   R³ is H, C₁-C₇ alkyl, halogen, CN, C₁-C₇ alkoxy or C₁-C₇ haloalkyl;
   R⁴ is H, C(=O)R¹⁹, CO₂R¹⁹, C(=O)SR¹⁹, S(O)₂R¹⁹ or CH₂OCOR¹⁹;
   R⁵ and R⁶ are taken together with the nitrogen atom to which they are attached to form a 3- to 7-membered ring, containing carbon atoms and optionally 1 to 3 oxygen, sulfur or nitrogen atoms as ring members, wherein up to 2 carbon atom ring members are independently selected from C(=O) and C(=S), and the sulfur atom ring member is selected from S, S(O) or S(O)₂, said ring optionally substituted with up to 5 substituents independently selected from (R^{v})ᵣ and r is the number of the substituents;
   R^{v} is independently selected from the group consisting of H, methyl, ethyl, propyl, c-propylmethyl, propargyl or cyanomethyl; and
   r is 1 or 2.
Embodiment E. A compound of Embodiment D wherein
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁵ and R⁶ are taken together with the nitrogen atom to which they are attached to form a 3- to 7-membered ring, said ring is a 5-membered ring; and
   R^{f} is CF₃.
Embodiment F. A compound of Embodiment D wherein
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁵ and R⁶ are taken together with the nitrogen atom to which they are attached to form a 3- to 7-membered ring, said ring is a 6-membered ring; and
   R^{f} is CF₃.
Embodiment A1. A compound of Formula **1** as described in the Summary of the Disclosure wherein
   G is G-1;
   R¹ is H, C₁-C₇ alkyl, halogen, C₂-C₆ alkenyl, C₂-C₆ alkynyl, C₃-C₇ cycloalkyl or C₁-C₇ haloalkyl;
   R² is H, C₁-C₇ alkyl, C₃-C₆ cycloalkyl, halogen or CN;
   R³ is H, C₁-C₇ alkyl, halogen, CN, C₁-C₇ alkoxy or C₁-C₇ haloalkyl;
   R⁴ is H, C(=O)R¹⁹, CO₂R¹⁹, C(=O)SR¹⁹, S(O)₂R¹⁹ or CH₂OCOR¹⁹;
   R^{f} is C₁-C₃ haloalkyl.
Embodiment B1. A compound of Embodiment A1 wherein
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁷ is H, C₁-C₇ alkyl, halogen, -CN, C₁-C₇ haloalkyl or C₁-C₇ alkoxy;
   R⁸ is H or C₁-C₇ alkyl;
   R⁹ is H, C₁-C₇ alkyl, C₁-C₇ haloalkyl or C₂-C₇ alkoxyalkyl;
   R¹⁰ is H or C₁-C₇ alkyl; and
   R^{f} is CF₃.
Embodiment C1. A compound of Embodiment B1 wherein
   R¹ is Me or Cl;
   R³ is Me;
   R⁴ is H, CH₂OCO-*t*-Bu or SO₂CF₃.
   R⁷ is H;
   R⁸ is H;
   R⁹ is methy, ethyl, t-butyl, chloromethyl or methoxymethyl; and
   R¹⁰ is methyl or ethyl.
Embodiment D1. A compound of Embodiment A1 wherein
   R⁷ and R⁹ are taken together to form a fused 3- to 7-membered ring, containing carbon atoms and optionally 1-2 oxygen, sulfur or nitrogen atom members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
   R⁸ is H; and
   R¹⁰ is H;
Embodiment E1. A compound of Embodiment D1 wherein
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁷ and R⁹ are taken together to form a fused 3- to 7-membered ring, said 3- to 7-membered ring is a 5- or 6-membered ring.
Embodiment F1. A compound of Embodiment E1 wherein
   R⁷ and R⁹ are taken together to form a 5- or 6-membered ring of c-pentyl, c-hexyl or tetrahydropyran, said ring unsubstituted or substituted with at least one substituent independently selected from H, halogen or C₁-C₄ alkyl.
Embodiment G1. A compound of Embodiment A1 wherein
   R⁹ and R¹⁰ are taken together with the carbon atom to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂, said ring optionally substituted with up to 5 substituents independently selected from (R^{v})ᵣ and r is the number of the substituents; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂.
   R⁷ is H; and
   R⁸ is H;
Embodiment H1. A compound of Embodiment G1 wherein
   R¹ is H, C₁-C₃ alkyl, halogen or C₃-C₄ cycloalkyl.
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R⁹ and R¹⁰ are taken together with the carbon atom to which they are attached to form a 3- to 7-membered ring, said ring is a 5- or 6-membered ring.
   R^{v} is independently selected from the group consisting of H, methyl, ethyl, propyl, c-propylmethyl, propargyl or cyanomethyl.
   r is 1 or 2.
Embodiment I1. A compound of Embodiment H1 wherein
   R⁹ and R¹⁰ are taken together with the carbon atom to which they are attached to form a 3- to 7-membered ring, said ring is a 5- or 6-membered ring of cyclopentane, cyclohexane, tetrahydro-2H-pyran or tetrahydro-2H-thiopyran.
Embodiment A2. A compound of Formula 1 as described in the Summary of the Disclosure wherein
   G is G-2;
   R¹ is H, C₁-C₇ alkyl, halogen, C₂-C₆ alkenyl, C₂-C₆ alkynyl, C₃-C₇ cycloalkyl or C₁-C₇ haloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe; and
   R^{f} is C₁-C₃ haloalkyl.
Embodiment B2. A compound of Embodiment A2 wherein
   Q is O or S;
   R⁷ is H;
   R⁸ is H;
   R^{f} is CF₃.
   R⁹ and R¹⁰ are taken together with the carbon atom to which they are attached to form a 3- to 7-membered ring, said ring is a 5- or 6-membered ring;
   R^{v} is independently selected from the group consisting of H, methyl, ethyl, propyl, *c-*propylmethyl, propargyl or cyanomethyl.
   r is 1 or 2.
Embodiment C2. A compound of Embodiment B2 wherein
   R⁹ and R¹⁰ are taken together with the carbon atom to which they are attached to form a cyclopentane, cyclohexane, tetrahydro-2*H*-pyran or tetrahydro-2*H*-thiopyran; and
   R^{v} is H.
Embodiment D2. A compound of Embodiment A2 wherein
   Q is CR¹¹R¹²;
   R⁷ is H;
   R⁸ is H;
   R⁹ is H;
   R¹⁰ is H;
   R¹¹ and R¹² are taken together with the carbon atom to which they are attached to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy; and
   R^{f} is CF₃.
Embodiment E2. A compound of Embodiment D2 wherein
   R¹¹ and R¹² are taken together with the carbon atom to which they are attached to form a fused 3- to 7-membered ring, said ring is an unsubstituted 5- or 6-membered ring.
Embodiment A3. A compound of Formula **1** as described in the Summary of the Disclosure wherein
   G is G-3;
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R¹³ is C₁-C₇ alkyl;
   R¹⁴ is C₁-C₄ alkyl;
   R¹⁵ is H; and
   R^{f} is C₁-C₃ haloalkyl.
Embodiment A4. A compound of Formula **1** as described in the Summary of the Disclosure wherein
   G is G-4;
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R¹³ is C₁-C₇ alkyl;
   R^{f} is C₁-C₃ haloalkyl;
   R¹⁵ is H, C₁-C₃ alkyl or C₁-C₃ alkoxy; and
   R¹⁶ is H, cyano, C₁-C₄ alkyl, halogen, C₁-C₄ alkylthio, C₁-C₄ haloalkyl or C₁-C₄ alkoxy.
Embodiment A5. A compound of Formula **1** as described in the Summary of the Disclosure wherein
   G is G-5;
   R¹ is H, C₁-C₇ alkyl, halogen or C₃-C₇ cycloalkyl;
   R² is H, Me or F;
   R³ is H, Me, F, Cl, CN, OMe or CF₃;
   R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
   R^{f} is C₁-C₃ haloalkyl;
   R¹⁶ is H or C₁-C₄ alkyl;
   R¹⁷ is H, C₁-C₄ alkyl, halogen or C₁-C₄ alkoxy; and
   R¹⁸ is H or C₁-C₃ alkoxy.

Specific embodiments include compounds of Formula **1** selected from the group consisting of:
N-[2,4-Dimethyl-5-(1-piperidinylcarbonyl)phenyl]-1,1,1-trifluoromethanesulfonamide (Compound 260);
N-[2-Chloro-4-methyl-5-(4-morpholinylcarbonyl)phenyl]-1,1,1-trifluoromethanesulfonamide (Compound 16);
N-[2,4-Dimethyl-5-(4-morpholinylcarbonyl)phenyl]-1,1,1-trifluoromethanesulfonamide (Compound 6);
N-[2-Chloro-4-methyl-5-(1-piperidinylcarbonyl)phenyl]-1,1,1-trifluoromethanesulfonamide (Compound 18);
3-Fluoro-N,N,2,4-tetramethyl-5-[[(trifluoromethyl)sulfonyl]amino]benzamide (Compound 128);
1,1,1-Trifluoro-N-[3-fluoro-2,4-dimethyl-5-(4-morpholinylcarbonyl)phenyl]methanesulfonamide (Compound 190);
N-[2,4-Dimethyl-5-(1-oxa-2-azaspiro[4.4]non-2-en-3-yl)phenyl]-1,1,1-trifluoromethanesulfonamide (Compound 207);
N-[2,4-Dimethyl-5-[(3aR,6aR)-3a,5,6,6a-tetrahydro-4H-cyclopent[d]isoxazol-3-yl]phenyl]-1,1,1-trifluoromethanesulfonamide (Compound 103);
N-[2,4-Dimethyl-5-(1-oxa-2-azaspiro[4.5]dec-2-en-3-yl)phenyl]-1,1,1-trifluoromethanesulfonamide (Compound 197);
N-[2,4-Dimethyl-5-(3a,4,7,7a-tetrahydro-5H-pyrano[4,3-d]isoxazol-3-yl)phenyl]-1,1,1-trifluoromethanesulfonamide (Compound 121);
N-[2,4-Dimethyl-5-(3a,6,7,7a-tetrahydro-4H-pyrano[3,4-d]isoxazol-3-yl)phenyl]-1,1,1-trifluoromethanesulfonamide (Compound 120);
N-[2,4-Dimethyl-5-(1-oxo-2-azaspiro[4.5]dec-2-yl)phenyl]-1,1,1-trifluoro-N-[(trifluoromethyl)sulfonyl]methanesulfonamide (Compound 267);
[[2,4-Dimethyl-5-(1-oxa-2-azaspiro[4.4]non-2-en-3-yl)phenyl][(trifluoromethyl)sulfonyl]amino]methyl 2,2-dimethylpropanoate (Compound 140);
[[2,4-Dimethyl-5-(1-oxa-2-azaspiro[4.5]dec-2-en-3-yl)phenyl][(trifluoromethyl)sulfonyl]amino]methyl 2,2-dimethylpropanoate (Compound 159);
[[2,4-Dimethyl-5-[(3aR,6aR)-3a,5,6,6a-tetrahydro-4H-cyclopent[d]isoxazol-3-yl]phenyl][(trifluoromethyl)sulfonyl]amino]methyl 2,2-dimethylpropanoate (Compound 100);
[[2,4-Dimethyl-5-(1-oxo-2-azaspiro[4.5]dec-2-yl)phenyl][(trifluoromethyl)sulfonyl]amino]methyl 2,2-dimethylpropanoate (Compound 268);

Other specific embodiments include compounds of Formula **1** selected from the group consisting of:
Specific embodiments include compounds of Formula **1** selected from the group consisting of:
[[(Trifluoromethyl)sulfonyl][2,3,4-trimethyl-5-(4-morpholinylcarbonyl)phenyl]amino]methyl 2,2-dimethylpropanoate (Compound 324);
Ethyl N-[(trifluoromethyl)sulfonyl]-N-[2,3,4-trimethyl-5-(1-piperidinylcarbonyl)phenyl]carbamate (Compound 330);
[[(Trifluoromethyl)sulfonyl][2,3,4-trimethyl-5-(1-piperidinylcarbonyl)phenyl]amino]methyl 2,2-dimethylpropanoate (Compound 329);
1,1,1-Trifluoro-N-[2,3,4-trimethyl-5-(4-morpholinylcarbonyl)phenyl]methanesulfonamide (Compound 289); and
[[(Trifluoromethyl)sulfonyl][2,3,4-trimethyl-5-[(3aR,6aR)-3a,5,6,6a-tetrahydro-4H-cyclopent[d]isoxazol-3-yl]phenyl]amino]methyl 2,2-dimethylpropanoate (Compound 336);

Other specific embodiments include compounds of Formula **1** wherein
G is CONR⁵R⁶ and NR⁵R⁶ is J-3a, R¹ is Me, R² is Me, R³ is Me, R⁴ is CH₂OCO-*t-*Bu, and R^{f} is CF₃ (Compound 331);
G is CONR⁵R⁶ and NR⁵R⁶ is J-4, R¹ is Me, R² is Me, R³ is Me, R⁴ is CO₂Et and R^{f} is CF₃ (Compound 325)

This invention also relates to a method for controlling undesired vegetation comprising applying to the locus of the vegetation herbicidally effective amounts of the compounds of the invention (e.g., as a composition described herein). Of note as embodiments relating to methods of use are those involving the compounds of embodiments described above. Compounds of the invention are particularly useful for selective control of weeds in crops such as wheat, barley, maize, soybean, sunflower, cotton, oilseed rape and rice, and specialty crops such as sugarcane, citrus, fruit and nut crops.

Also noteworthy as embodiments are herbicidal compositions of the present invention comprising the compounds of embodiments described above.

This invention also includes a herbicidal mixture comprising (a) a compound selected from Formula **1,** *N*-oxides, and salts thereof, and (b) at least one additional active ingredient selected from (b1) photosystem II inhibitors, (b2) acetohydroxy acid synthase (AHAS) inhibitors, (b3) acetyl-CoA carboxylase (ACCase) inhibitors, (b4) auxin mimics, (b5) 5-enol-pyruvylshikimate-3-phosphate (EPSP) synthase inhibitors, (b6) photosystem I electron diverters, (b7) protoporphyrinogen oxidase (PPO) inhibitors, (b8) glutamine synthetase (GS) inhibitors, (b9) very long chain fatty acid (VLCFA) elongase inhibitors, (b10) auxin transport inhibitors, (b11) phytoene desaturase (PDS) inhibitors, (b12) 4-hydroxyphenyl-pyruvate dioxygenase (HPPD) inhibitors, (b13) homogentisate solanesyltransferase (HST) inhibitors, (b14) cellulose biosynthesis inhibitors, (b15) other herbicides including mitotic disruptors organic arsenicals, asulam, bromobutide, cinmethylin, cumyluron, dazomet, difenzoquat, dymron, etobenzanid, flurenol, fosamine, fosamine-ammonium, hydantocidin, metam, methyldymron, oleic acid, oxaziclomefone, pelargonic acid and pyributicarb, (b16) herbicide safeners, and salts of compounds of (b1) through (b16).

"Photosystem II inhibitors" (b1) are chemical compounds that bind to the D-1 protein at the Q_{B}-binding niche and thus block electron transport from Q_{A} to Q_{B} in the chloroplast thylakoid membranes. The electrons blocked from passing through photosystem II are transferred through a series of reactions to form toxic compounds that disrupt cell membranes and cause chloroplast swelling, membrane leakage, and ultimately cellular destruction. The Q_{B}-binding niche has three different binding sites: binding site A binds the triazines such as atrazine, triazinones such as hexazinone, and uracils such as bromacil, binding site B binds the phenylureas such as diuron, and binding site C binds benzothiadiazoles such as bentazon, nitriles such as bromoxynil and phenyl-pyridazines such as pyridate. Examples of photosystem II inhibitors include ametryn, amicarbazone, atrazine, bentazon, bromacil, bromofenoxim, bromoxynil, chlorbromuron, chloridazon, chlorotoluron, chloroxuron, cumyluron, cyanazine, daimuron, desmedipham, desmetryn, dimefuron, dimethametryn, diuron, ethidimuron, fenuron, fluometuron, hexazinone, ioxynil, isoproturon, isouron, lenacil, linuron, metamitron, methabenzthiazuron, metobromuron, metoxuron, metribuzin, monolinuron, neburon, pentanochlor, phenmedipham, prometon, prometryn, propanil, propazine, pyridafol, pyridate, siduron, simazine, simetryn, tebuthiuron, terbacil, terbumeton, terbuthylazine, terbutryn and trietazine.

"AHAS inhibitors" (b2) are chemical compounds that inhibit acetohydroxy acid synthase (AHAS), also known as acetolactate synthase (ALS), and thus kill plants by inhibiting the production of the branched-chain aliphatic amino acids such as valine, leucine and isoleucine, which are required for protein synthesis and cell growth. Examples of AHAS inhibitors include amidosulfuron, azimsulfuron, bensulfuron-methyl, bispyribac-sodium, cloransulam-methyl, chlorimuron-ethyl, chlorsulfuron, cinosulfuron, cyclosulfamuron, diclosulam, ethametsulfuron-methyl, ethoxysulfuron, flazasulfuron, florasulam, flucarbazone-sodium, flumetsulam, flupyrsulfuron-methyl, flupyrsulfuron-sodium, foramsulfuron, halosulfuron-methyl, imazamethabenz-methyl, imazamox, imazapic, imazapyr, imazaquin, imazethapyr, imazosulfuron, iodosulfuron-methyl (including sodium salt), iofensulfuron (2-iodo-*N*-[[(4-methoxy-6-methyl-1,3,5-triazin-2-yl)amino]carbonyl]benzenesulfonamide), mesosulfuron-methyl, metazosulfuron (3-chloro-4-(5,6-dihydro-5-methyl-1,4,2-dioxazin-3-yl)-*N*-[[(4,6-dimethoxy-2-pyrimidinyl)amino]carbonyl]-1-methyl-1*H*-pyrazole-5-sulfonamide), metosulam, metsulfuron-methyl, nicosulfuron, oxasulfuron, penoxsulam, primisulfuron-methyl, propoxycarbazone-sodium, propyrisulfuron (2-chloro-*N*-[[(4,6-dimethoxy-2-pyrimidinyl)amino]carbonyl]-6-propylimidazo[1,2-*b*]pyridazine-3-sulfonamide), prosulfuron, pyrazosulfuron-ethyl, pyribenzoxim, pyriftalid, pyriminobac-methyl, pyrithiobac-sodium, rimsulfuron, sulfometuron-methyl, sulfosulfuron, thiencarbazone, thifensulfuron-methyl, triafamone (*N*-[2-[(4,6-dimethoxy-1,3,5-triazin-2-yl)carbonyl]-6-fluorophenyl]-1,1-difluoro-*N*-methylmethanesulfonamide), triasulfuron, tribenuron-methyl, trifloxysulfuron (including sodium salt), triflusulfuron-methyl and tritosulfuron.

"ACCase inhibitors" (b3) are chemical compounds that inhibit the acetyl-CoA carboxylase enzyme, which is responsible for catalyzing an early step in lipid and fatty acid synthesis in plants. Lipids are essential components of cell membranes, and without them, new cells cannot be produced. The inhibition of acetyl CoA carboxylase and the subsequent lack of lipid production leads to losses in cell membrane integrity, especially in regions of active growth such as meristems. Eventually shoot and rhizome growth ceases, and shoot meristems and rhizome buds begin to die back. Examples of ACCase inhibitors include alloxydim, butroxydim, clethodim, clodinafop, cycloxydim, cyhalofop, diclofop, fenoxaprop, fluazifop, haloxyfop, pinoxaden, profoxydim, propaquizafop, quizalofop, sethoxydim, tepraloxydim and tralkoxydim, including resolved forms such as fenoxaprop-P, fluazifop-P, haloxyfop-P and quizalofop-P and ester forms such as clodinafop-propargyl, cyhalofop-butyl, diclofop-methyl and fenoxaprop-P-ethyl.

Auxin is a plant hormone that regulates growth in many plant tissues. "Auxin mimics" (b4) are chemical compounds mimicking the plant growth hormone auxin, thus causing uncontrolled and disorganized growth leading to plant death in susceptible species. Examples of auxin mimics include aminocyclopyrachlor (6-amino-5-chloro-2-cyclopropyl-4-pyrimidinecarboxylic acid) and its methyl and ethyl esters and its sodium and potassium salts, aminopyralid, benazolin-ethyl, chloramben, clacyfos, clomeprop, clopyralid, dicamba, 2,4-D, 2,4-DB, dichlorprop, fluroxypyr, halauxifen (4-amino-3-chloro-6-(4-chloro-2-fluoro-3-methoxyphenyl)-2-pyridinecarboxylic acid), halauxifen-methyl (methyl 4-amino-3-chloro-6-(4-chloro-2-fluoro-3-methoxyphenyl)-2-pyridinecarboxylate), MCPA, MCPB, mecoprop, picloram, quinclorac, quinmerac, 2,3,6-TBA, triclopyr, and methyl 4-amino-3-chloro-6-(4-chloro-2-fluoro-3-methoxyphenyl)-5-fluoro-2-pyridinecarboxylate.

"EPSP synthase inhibitors" (b5) are chemical compounds that inhibit the enzyme, 5-enol-pyruvylshikimate-3-phosphate synthase, which is involved in the synthesis of aromatic amino acids such as tyrosine, tryptophan and phenylalanine. EPSP inhibitor herbicides are readily absorbed through plant foliage and translocated in the phloem to the growing points. Glyphosate is a relatively nonselective postemergence herbicide that belongs to this group. Glyphosate includes esters and salts such as ammonium, isopropylammonium, potassium, sodium (including sesquisodium) and trimesium (alternatively named sulfosate).

"Photosystem I electron diverters" (b6) are chemical compounds that accept electrons from Photosystem I, and after several cycles, generate hydroxyl radicals. These radicals are extremely reactive and readily destroy unsaturated lipids, including membrane fatty acids and chlorophyll. This destroys cell membrane integrity, so that cells and organelles "leak", leading to rapid leaf wilting and desiccation, and eventually to plant death. Examples of this second type of photosynthesis inhibitor include diquat and paraquat.

"PPO inhibitors" (b7) are chemical compounds that inhibit the enzyme protoporphyrinogen oxidase, quickly resulting in formation of highly reactive compounds in plants that rupture cell membranes, causing cell fluids to leak out. Examples of PPO inhibitors include acifluorfen-sodium, azafenidin, benzfendizone, bifenox, butafenacil, carfentrazone, carfentrazone-ethyl, chlomethoxyfen, cinidon-ethyl, fluazolate, flufenpyr-ethyl, flumiclorac-pentyl, flumioxazin, fluoroglycofen-ethyl, fluthiacet-methyl, fomesafen, halosafen, lactofen, oxadiargyl, oxadiazon, oxyfluorfen, pentoxazone, profluazol, pyraclonil, pyraflufen-ethyl, saflufenacil, sulfentrazone, thidiazimin, trifludimoxazin (dihydro-1,5-dimehyl-6-thioxo-3-[2,2,7-trifluoro-3,4-dihydro-3-oxo-4-(2-propyn-1-yl)-2*H*-1,4-benzoxazin-6-yl]-1,3,5-triazine-2,4(1*H*,3*H*)-dione) and tiafenacil (methyl *N*-[2-[[2-chloro-5-[3,6-dihydro-3-methyl-2,6-dioxo-4-(trifluoromethyl)-1(2*H*)-pyrimidinyl]-4-fluorophenyl]thio]-1-oxopropyl]-β-alaninate).

"GS inhibitors" (b8) are chemical compounds that inhibit the activity of the glutamine synthetase enzyme, which plants use to convert ammonia into glutamine. Consequently, ammonia accumulates and glutamine levels decrease. Plant damage probably occurs due to the combined effects of ammonia toxicity and deficiency of amino acids required for other metabolic processes. The GS inhibitors include glufosinate and its esters and salts such as glufosinate-ammonium and other phosphinothricin derivatives, glufosinate-P ((2*S*)-2-amino-4-(hydroxymethylphosphinyl)butanoic acid) and bilanaphos.

"VLCFA elongase inhibitors" (b9) are herbicides having a wide variety of chemical structures, which inhibit the elongase. Elongase is one of the enzymes located in or near chloroplasts which are involved in biosynthesis of VLCFAs. In plants, very-long-chain fatty acids are the main constituents of hydrophobic polymers that prevent desiccation at the leaf surface and provide stability to pollen grains. Such herbicides include acetochlor, alachlor, anilofos, butachlor, cafenstrole, dimethachlor, dimethenamid, diphenamid, fenoxasulfone (3-[[(2,5-dichloro-4-ethoxyphenyl)methyl]sulfonyl]-4,5-dihydro-5,5-dimethylisoxazole), fentrazamide, flufenacet, indanofan, mefenacet, metazachlor, metolachlor, naproanilide, napropamide, napropamide-M ((2R)-*N*,*N*-diethyl-2-(1-naphthalenyloxy)propanamide), pethoxamid, piperophos, pretilachlor, propachlor, propisochlor, pyroxasulfone, and thenylchlor, including resolved forms such as S-metolachlor and chloroacetamides and oxyacetamides.

"Auxin transport inhibitors" (b10) are chemical substances that inhibit auxin transport in plants, such as by binding with an auxin-carrier protein. Examples of auxin transport inhibitors include diflufenzopyr, naptalam (also known as *N*-(1-naphthyl)phthalamic acid and 2-[(1-naphthalenylamino)carbonyl benzoic acid).

"PDS inhibitors" (b11) are chemical compounds that inhibit carotenoid biosynthesis pathway at the phytoene desaturase step. Examples of PDS inhibitors include beflubutamid, diflufenican, fluridone, flurochloridone, flurtamone norflurzon and picolinafen.

"HPPD inhibitors" (b12) are chemical substances that inhibit the biosynthesis of synthesis of 4-hydroxyphenyl-pyruvate dioxygenase. Examples of HPPD inhibitors include benzobicyclon, benzofenap, bicyclopyrone (4-hydroxy-3-[[2-[(2-methoxyethoxy)methyl]-6-(trifluoromethyl)-3-pyridinyl]carbonyl]bicyclo[3.2.1]oct-3-en-2-one), fenquinotrione (2-[[8-chloro-3,4-dihydro-4-(4-methoxyphenyl)-3-oxo-2-quinoxalinyl]carbonyl]-1,3-cyclohexanedione), isoxachlortole, isoxaflutole, mesotrione, pyrasulfotole, pyrazolynate, pyrazoxyfen, sulcotrione, tefuryltrione, tembotrione, tolpyralate (1-[[1-ethyl-4-[3-(2-methoxyethoxy)-2-methyl-4-(methylsulfonyl)benzoyl]-1H-pyrazol-5-yl]oxy]ethyl methyl carbonate), topramezone, 5-chloro-3-[(2-hydroxy-6-oxo-1-cyclohexen-1-yl)carbonyl]-1-(4-methoxyphenyl)-2(1*H*)-quinoxalinone, 4-(2,6-diethyl-4-methylphenyl)-5-hydroxy-2,6-dimethyl-3(2*H*)-pyridazinone, 4-(4-fluorophenyl)-6-[(2-hydroxy-6-oxo-1-cyclohexen-1-yl)carbonyl]-2-methyl-1,2,4-triazine-3,5(2*H*,4*H*)-dione, 5-[(2-hydroxy-6-oxo-1-cyclohexen-1-yl)carbonyl]-2-(3-methoxyphenyl)-3-(3-methoxypropyl)-4(3*H*)-pyrimidinone, 2-methyl-*N-*(4-methyl-1,2,5-oxadiazol-3-yl)-3-(methylsulfinyl)-4-(trifluoromethyl)benzamide and 2-methyl-3-(methylsulfonyl)-*N*-(1-methyl-1*H*-tetrazol-5-yl)-4-(trifluoromethyl)benzamide.

"HST inhibitors" (b13) disrupt a plant's ability to convert homogentisate to 2-methyl-6-solanyl-1,4-benzoquinone, thereby disrupting carotenoid biosynthesis. Examples of HST inhibitors include haloxydine, pyriclor, 3-(2-chloro-3,6-difluorophenyl)-4-hydroxy-1-methyl-1,5-naphthyridin-2(1*H*)-one, 7-(3,5-dichloro-4-pyridinyl)-5-(2,2-difluoroethyl)-8-hydroxypyrido[2,3-*b*]pyrazin-6(5*H*)-one and 4-(2,6-diethyl-4-methylphenyl)-5-hydroxy-2,6-dimethyl-3(2*H*)-pyridazinone.

HST inhibitors also include compounds of Formulae **A** and **B.** 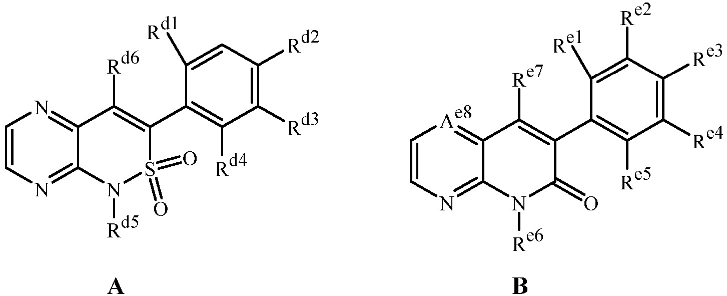 wherein R^{d1} is H, Cl or CF₃; R^{d2} is H, Cl or Br; R^{d3} is H or Cl; R^{d4} is H, Cl or CF₃; R^{d5} is CH₃, CH₂CH₃ or CH₂CHF₂; and R^{d6} is OH or -OC(=O)-*i*-Pr; and R^{e1} is H, F, Cl, CH₃ or CH₂CH₃; R^{e2} is H or CF₃; R^{e3} is H, CH₃ or CH₂CH₃; R^{e4} is H, F or Br; R^{e5} is Cl, CH₃, CF₃, OCF₃ or CH₂CH₃; R^{e6} is H, CH₃, CH₂CHF₂ or C≡CH; R^{e7} is OH, -OC(=O)Et, -OC(=O)-*i*-Pr or -OC(=O)-t-Bu; and A^{e8} is N or CH.

"Cellulose biosynthesis inhibitors" (b14) inhibit the biosynthesis of cellulose in certain plants. They are most effective when applied preemergence or early postemergence on young or rapidly growing plants. Examples of cellulose biosynthesis inhibitors include chlorthiamid, dichlobenil, flupoxam, indaziflam (*N*²-[(1*R*,2*S*)-2,3-dihydro-2,6-dimethyl-1*H*-inden-1-yl]-6-(1-fluoroethyl)-1,3,5-triazine-2,4-diamine), isoxaben and triaziflam.

"Other herbicides" (b15) include herbicides that act through a variety of different modes of action such as mitotic disruptors (e.g., flamprop-M-methyl and flamprop-M-isopropyl) organic arsenicals (e.g., DSMA, and MSMA), 7,8-dihydropteroate synthase inhibitors, chloroplast isoprenoid synthesis inhibitors and cell-wall biosynthesis inhibitors. Other herbicides include those herbicides having unknown modes of action or do not fall into a specific category listed in (b1) through (b14) or act through a combination of modes of action listed above. Examples of other herbicides include aclonifen, asulam, amitrole, bromobutide, cinmethylin, clomazone, cumyluron, cyclopyrimorate (6-chloro-3-(2-cyclopropyl-6-methylphenoxy)-4-pyridazinyl 4-morpholinecarboxylate), daimuron, difenzoquat, etobenzanid, fluometuron, flurenol, fosamine, fosamine-ammonium, dazomet, dymron, ipfencarbazone (1-(2,4-dichlorophenyl)-*N*-(2,4-difluorophenyl)-1,5-dihydro-*N*-(1-methylethyl)-5-oxo-4*H*-1,2,4-triazole-4-carboxamide), metam, methyldymron, oleic acid, oxaziclomefone, pelargonic acid, pyributicarb and 5-[[(2,6-difluorophenyl)methoxy]methyl]-4,5-dihydro-5-methyl-3-(3-methyl-2-thienyl)isoxazole.

"Other herbicides" (b15) also include a compound of Formula (b15A) 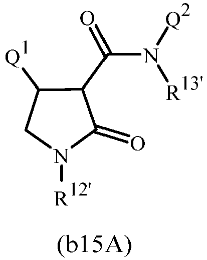 wherein
R^{12'} is H, C₁-C₆ alkyl, C₁-C₆ haloalkyl or C₄-C₈ cycloalkyl;
R^{13'} is H, C₁-C₆ alkyl or C₁-C₆ alkoxy;
Q¹ is an optionally substituted ring system selected from the group consisting of phenyl, thienyl, pyridinyl, benzodioxolyl, naphthalenyl, benzofuranyl, furanyl, benzothiophenyl and pyrazolyl, wherein when substituted said ring system is substituted with 1 to 3 R^{14'};
Q² is and optionally substituted ring system selected from the group consisting of phenyl, pyridinyl, benzodioxolyl, pyridinonyl, thiadiazolyl, thiazolyl, and oxazolyl, wherein when substituted said ring system is substituted with 1 to 3 R^{15'};
each R^{14'} is independently halogen, C₁-C₆ alkyl, C₁-C₆ haloalkyl, C₁-C₆ alkoxy, C₁-C₆ haloalkoxy, C₃-C₈ cyaloalkyl, cyano, C₁-C₆ alkylthio, C₁-C₆ alkylsulfinyl, C₁-C₆ alkylsulfonyl, SF₅, NHR¹⁷; or phenyl optionally substituted by 1 to 3 R¹⁶; or pyrazolyl optionally substituted by 1 to 3 R¹⁶;
each R^{15'} is independently halogen, C₁-C₆ alkyl, C₁-C₆ haloalkyl, C₁-C₆ alkoxy, C₁-C₆ haloalkoxy, cyano, nitro, C₁-C₆ alkylthio, C₁-C₆ alkylsulfinyl, C₁-C₆ alkylsulfonyl;
each R^{16'} is independently halogen, C₁-C₆ alkyl or C₁-C₆ haloalkyl; and
R^{17'} is C₁-C₄ alkoxycarbonyl.

In one Embodiment wherein "other herbicides" (b15) also include a compound of Formula (b15A), it is preferred that R^{12'} is H or C₁-C₆ alkyl; more preferably R^{12'} is H or methyl. Preferrably R^{13'} is H. Preferably Q¹ is either a phenyl ring or a pyridinyl ring, each ring substituted by 1 to 3 R^{14'}; more preferably Q¹ is a phenyl ring substituted by 1 to 2 R^{14'}. Preferably Q² is a phenyl ring substituted with 1 to 3 R^{15'}; more preferably Q² is a phenyl ring substituted by 1 to 2 R^{15'}. Preferably each R^{14'} is independently halogen, C₁-C₄ alkyl, C₁-C₃ haloalkyl, C₁-C₃ alkoxy or C₁-C₃ haloalkoxy; more preferably each R^{14'} is independently chloro, fluoro, bromo, C₁-C₂ haloalkyl, C₁-C₂ haloalkoxy or C₁-C₂ alkoxy. Preferrably each R^{15'} is independently halogen, C₁-C₄ alkyl, C₁-C₃ haloalkoxy; more preferably each R^{15'} is independently chloro, fluoro, bromo, C₁-C₂ haloalkyl, C₁-C₂ haloalkoxy or C₁-C₂ alkoxy. Specifically preferred as "other herbicides" (b15) include any one of the following (b15A-1) through (b15A-19): 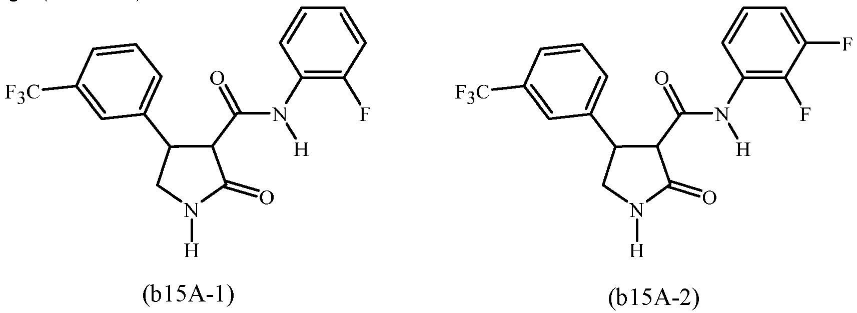 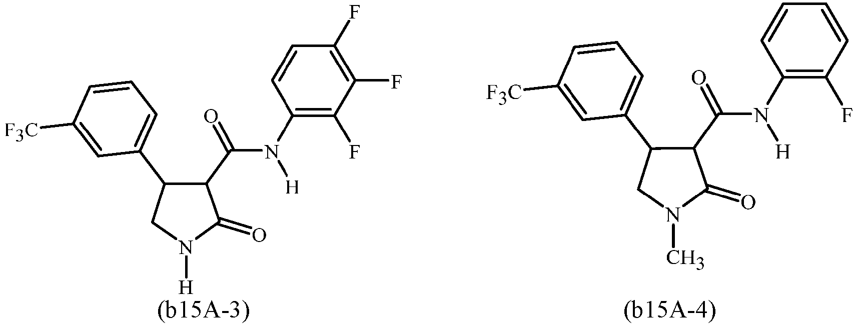 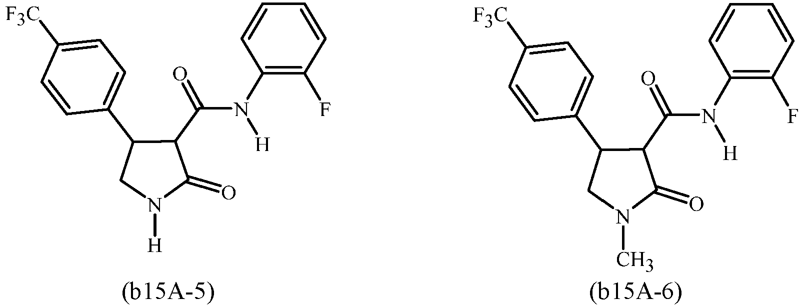 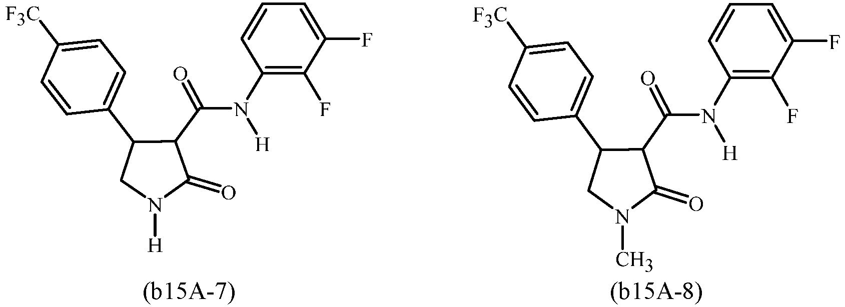 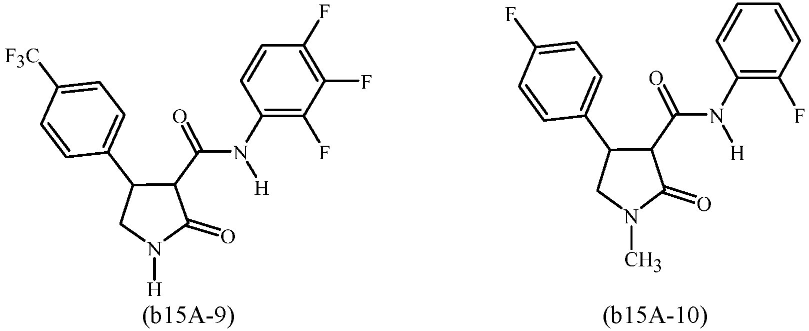 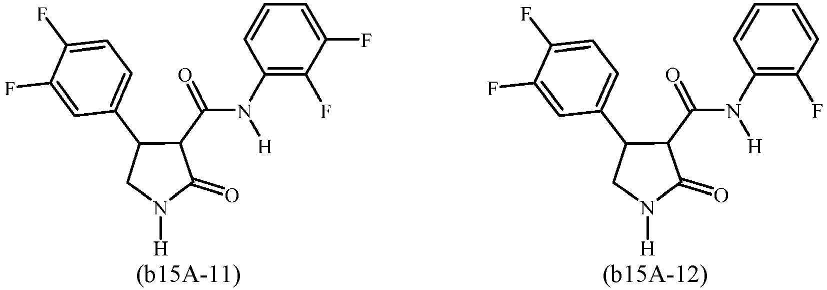 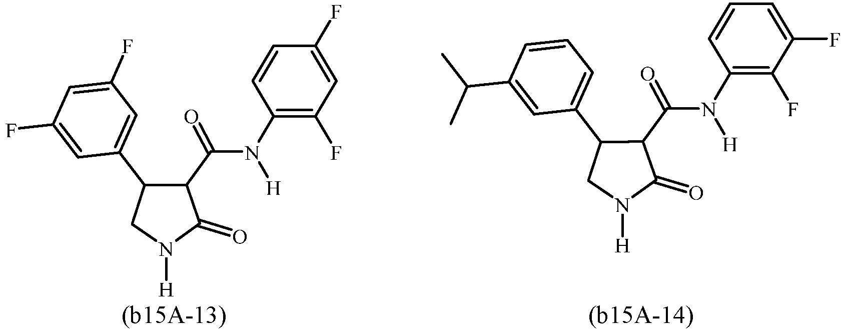 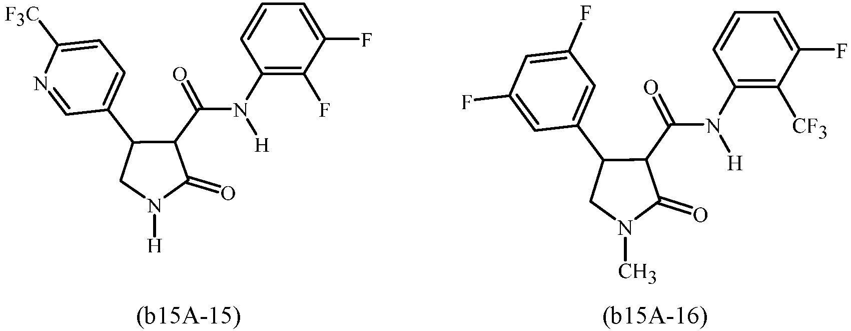 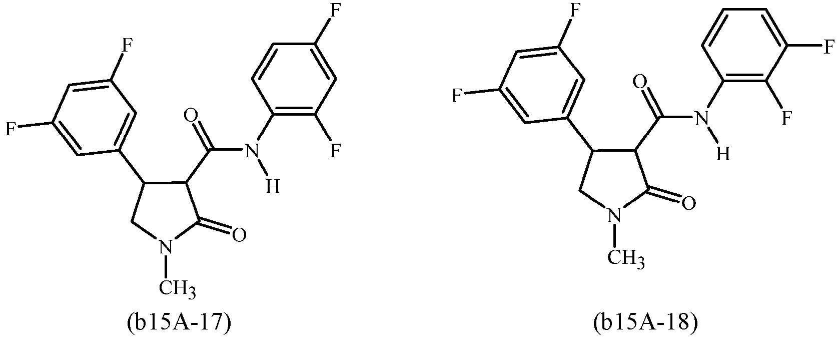 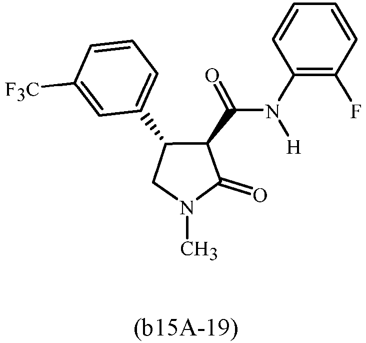 "Other herbicides" (b15) also include a compound of Formula (b15B) 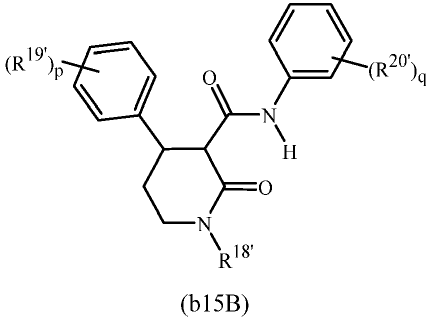 wherein
R^{18'} is H, C₁-C₆ alkyl, C₁-C₆ haloalkyl or C₄-C₈ cycloalkyl;
each R^{19'} is independently halogen, C₁-C₆ haloalkyl or C₁-C₆ haloalkoxy;
p is an integer of 0, 1, 2 or 3;
each R^{20'} is independently halogen, C₁-C₆ haloalkyl or C₁-C₆ haloalkoxy; and
q is an integer of 0, 1, 2 or 3.

In one Embodiment wherein "other herbicides" (b15) also include a compound of Formula (b15B), it is preferred that R¹⁸ is H, methyl, ethyl or propyl; more preferably R¹⁸ is H or methyl; most preferably R¹⁸ is H. Preferrably each R¹⁹ is independently chloro, fluoro, C₁-C₃ haloalkyl or C₁-C₃ haloalkoxy; more preferably each R¹⁹ is independently chloro, fluoro, C₁ fluoroalkyl (i.e. fluoromethyl, difluoromethyl or trifluoromethyl) or C₁ fluoroalkoxy (i.e. trifluoromethoxy, difluoromethoxy or fluoromethoxy). Preferably each R²⁰ is independently chloro, fluoro, C₁ haloalkyl or C₁ haloalkoxy; more preferably each R²⁰ is independently chloro, fluoro, C₁ fluoroalkyl (i.e. fluoromethyl, difluoromethyl or trifluromethyl) or C₁ fluoroalkoxy (i.e. trifluoromethoxy, difluoromethoxy or fluoromethoxy). Specifically preferred as "other herbicides" (b15) include any one of the following (b15B-1) through (b15B-19):

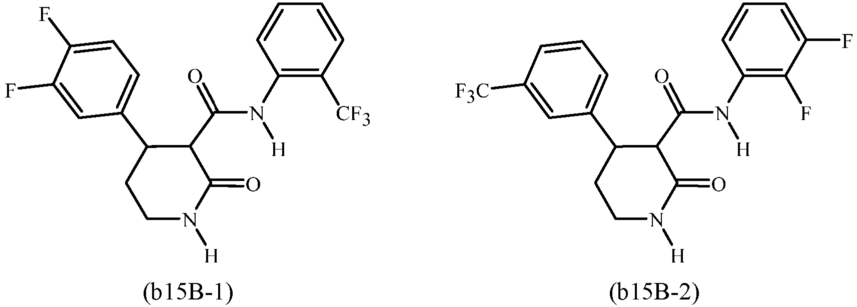

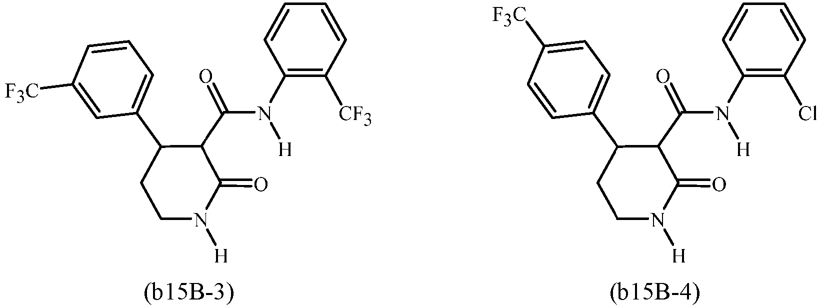

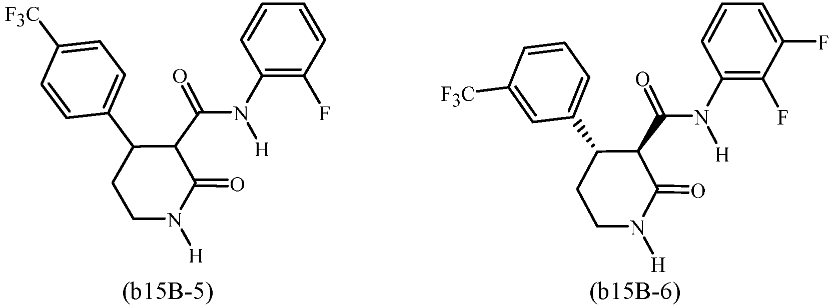

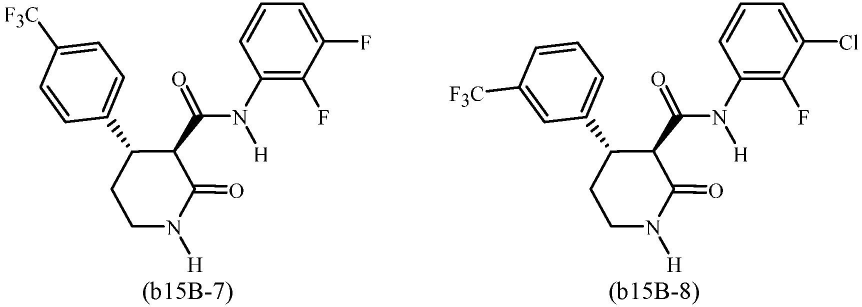

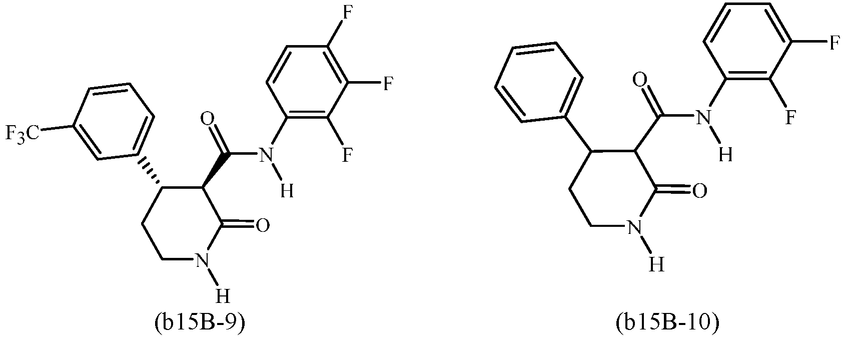

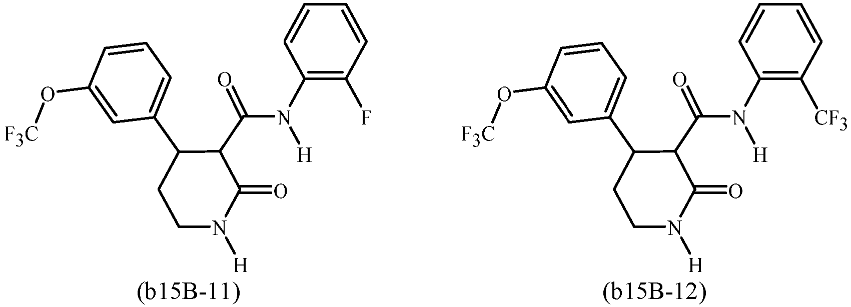

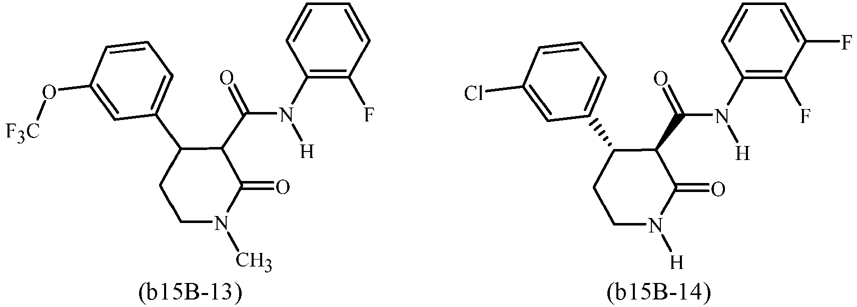

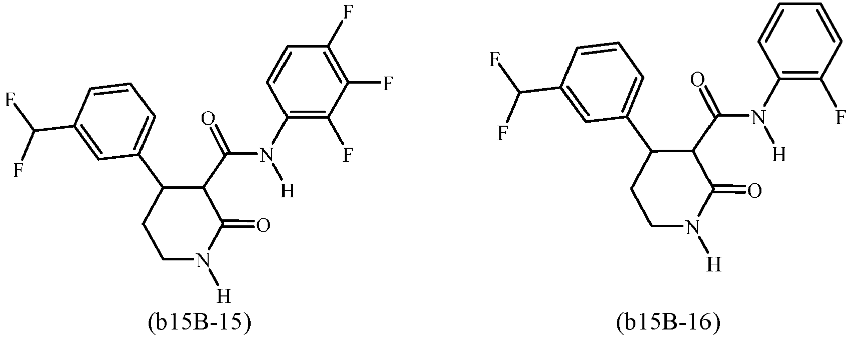

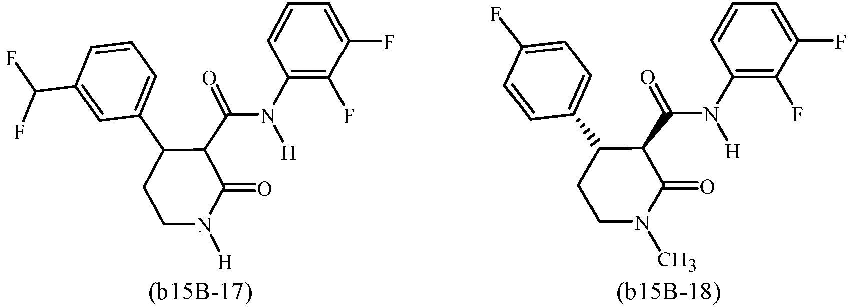

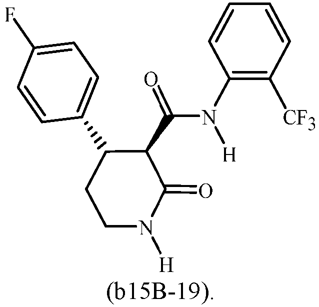

Another Embodiment wherein "other herbicides" (b15) also include a compound of Formula (b15C), 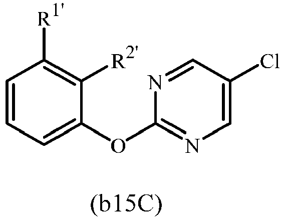 wherein R^{1'} is Cl, Br or CN; and R^{2'} is C(=O)CH₂CH₂CF₃, CH₂CH₂CH₂CH₂CF₃ or 3-CHF₂-isoxazol-5-yl.

"Herbicide safeners" (b16) are substances added to a herbicide formulation to eliminate or reduce phytotoxic effects of the herbicide to certain crops. These compounds protect crops from injury by herbicides but typically do not prevent the herbicide from controlling undesired vegetation. Examples of herbicide safeners include but are not limited to benoxacor, cloquintocet-mexyl, cumyluron, cyometrinil, cyprosulfamide, daimuron, dichlormid, dicyclonon, dietholate, dimepiperate, fenchlorazole-ethyl, fenclorim, flurazole, fluxofenim, furilazole, isoxadifen-ethyl, mefenpyr-diethyl, mephenate, methoxyphenone, naphthalic anhydride, oxabetrinil, N-(aminocarbonyl)-2-methylbenzenesulfonamide and *N-*(aminocarbonyl)-2-fluorobenzenesulfonamide, 1-bromo-4-[(chloromethyl)sulfonyl]benzene, 2-(dichloromethyl)-2-methyl-1,3-dioxolane (MG 191), 4-(dichloroacetyl)-1-oxa-4-azospiro[4.5]decane (MON 4660), 2,2-dichloro-1-(2,2,5-trimethyl-3-oxazolidinyl)-ethanone and 2-methoxy-*N*-[[4-[[(methylamino)carbonyl]amino]phenyl]sulfonyl]-benzamide.

The compounds of Formula **1** can be prepared by general methods known in the art of synthetic organic chemistry. One or more of the following methods and variations as described in Schemes 1-14 can be used to prepare the compounds of Formula **1.** The definitions of G, R¹-R¹⁹, R^{v} and R^{f} in the compounds of Formulae **1-15** below are as defined above in the Summary of the Disclosure unless otherwise noted. Compounds of Formulae **1a**, **1b, 3a, 3b, 3b', 3c, 3d, 3e** and **3f** are various subsets of the compounds of Formulae **1** and **3;** and all substituents for Formulae **1a**, **1b, 3a, 3b, 3b', 3c, 3d, 3e** and **3f** are as defined above for Formula **1** unless otherwise noted in the disclosure including the schemes.

As shown in Scheme 1, a compound of Formula **1a** (i.e. a compound of Formula **1** wherein R⁴ is H) can be prepared by reaction of an appropriately substituted aniline of Formula **2** with 1 equivalent (or a slight excess over 1 equivalent) of a compound of R^{f}SO₂Cl or a corresponding anhydride of R^{f}(SO₂)₂O in the presence of a suitable base like pyridine, triethylamine, diisopropylethylamine or potassium carbonate in a compatible solvent including but not limited to tetrahydrofuran, acetonitrile, toluene, diethyl ether, dioxane, dichloromethane or *N*,*N*-dimethylformamide at temperatures generally ranging from -78 °C to 0 °C. Alternatively, a compound of Formula **1b** (i.e. a compound of Formula **1** wherein R⁴ is SO₂R¹⁹ and R¹⁹ is R^{f}) are accessible by reacting an aniline of Formula **2** with 2 equivalents (or an excess over 2.0 equivalents) of a compound of Formula R^{f}SO₂Cl or corresponding anhydride of Formula R^{f}(SO₂)₂O under similar reaction conditions as described above. Treating *bis*-sulfonamides of Formula **1b** with an excess of aqueous base followed by neutralization or acidification with acid readily provides the corresponding mono-sulfonamide of Formula **1a**. Preferred conditions for this hydrolysis are usually aqueous sodium or potassium hydroxide, optionally used with a cosolvent such as methanol, ethanol, dioxane or tetrahydrofuran, followed by neutralization or acidification with concentrated or aqueous hydrochloric acid.

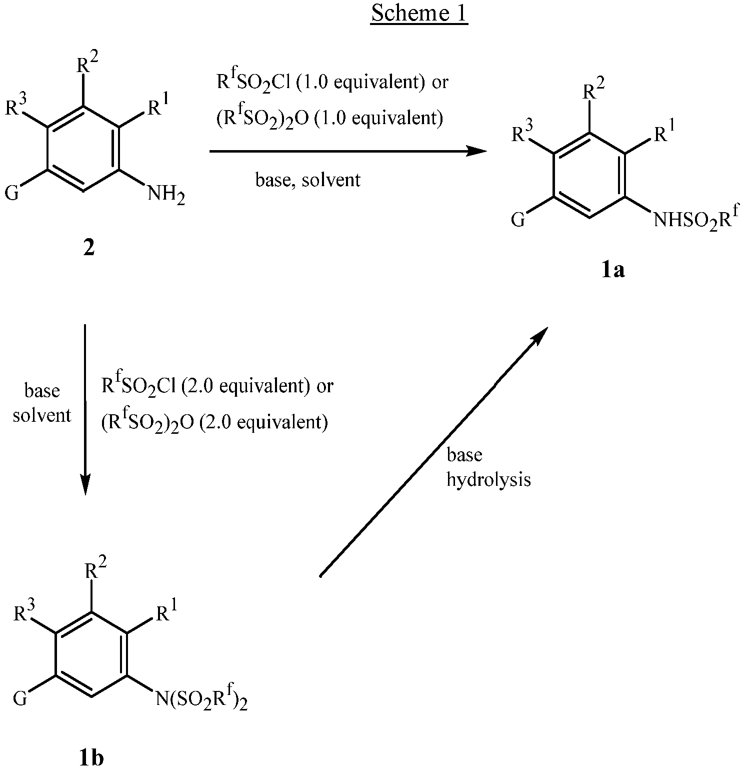

As shown in Scheme 2, substituted anilines of Formula **2** are readily accessed by hydrogenation of nitrobenzenes of Formula **3** under conditions that include but not limited to catalytic hydrogenation with 5-10% palladium metal on carbon or platinum oxide in solvents such methanol, ethanol or ethyl acetate under an atmosphere of hydrogen. This reaction can generally be done in a Parr Hydrogenator. Alternatively, reduction of the nitro group can be accomplished with activated zinc metal in acetic acid, with stannous chloride in aqueous hydrochloric acid, iron metal in acetic acid or in aqueous alcohol or in an aqueous ethyl acetate mixture with ammonium chloride (for example, Fe with 3 equivalents of ammonium chloride in aqueous ethanol) or with sodium borohydride in methanol in the presence of NiCl₂·6H₂O (see Journal the American Chemical Society, volume 127, p119 (2005)).

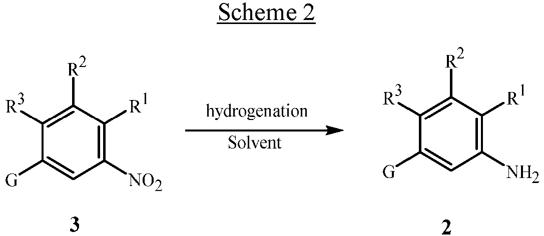

As shown in Scheme 3, an amide-substituted nitrobenzene of Formula **3a** (i.e. a compound of Formula **3** wherein G is CONR⁵R⁶) can be prepared from substituted nitrobenzene carboxylic acids of Formula **4** by initial formation of an acid chloride which is then allowed to react with an amine of Formula HNR⁵R⁶. Alternatively, a compound of Formula **3a** can be prepared by reacting a carboxylic acid of Formula **4** with an amine of Formula HNR⁵R⁶ directly in the presence of a dehydrative amide coupling reagent, optionally with base in an appropriate solvent. Acid chloride formation from **4** can be achieved with either oxalyl chloride or thionyl chloride with a catalytic amount of *N*,*N*-dimethylformamide in a suitable solvent such as dichloromethane, toluene or dichloroethane. Reaction of the generated acid chloride with amine of Formula HNR⁵R⁶ can be carried out in the presence of triethylamine, diisopropylethylamine (Hunig's Base) or pyridine in a solvent such as tetrahydrofuran, dioxane or dichloromethane. Dehydrative amide-coupling reagents suitable for direct coupling of the carboxylic acid **4** with amine HNR⁵R⁶ include *N,N'-*Dicyclohexylcarbodiimide (DCC), (Benzotriazol-1-yloxy)tris (dimethylamino) phosphonium hexafluoro phosphate (BOP reagent) or propanephosphonic acid anhydride (T3P reagent) or 1-Ethyl-3-(3-dimethylaminopropyl)carbodiimide (EDC). Generally a base such as triethylamine, diisopropylethylamine, pyridine or *N*,*N*-dimethylaminopyridine (DMAP) is added to this dehydrative coupling reaction in a solvent like *N,N-dimethylformamide,* acetonitrile or dichloromethane. The temperature for these reactions generally ranges from 0° Cto ambient temperature.

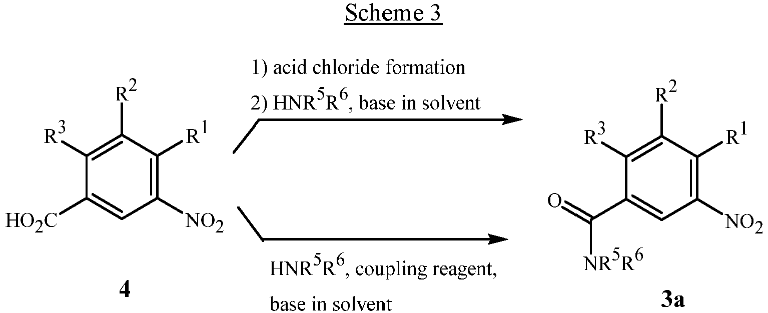

As shown in Scheme 4, isoxazoline-substituted nitrobenzene intermediates of Formula **3b** (i.e. a compound of Formula **3** wherein G is an isoxazolinyl ring) can be made by cycloaddition of a nitrobenzene chlorooxime of Formula **5** with an olefin of Formula R⁷R⁸C=CR⁹R¹⁰ in the presence of an appropriate base such as triethylamine, diisopropylethylamine or pyridine in a compatible solvent such as chloroform, dichloromethane, acetonitrile, tetrahydrofuran, toluene, dioxane or dichloroethane at temperatures generally ranging from 0 °C to ambient temperature. When an unsymmetrical olefin R⁷R⁸C=CR⁹R¹⁰ is used in this cycloaddition reaction, regioisomeric mixtures of isoxazoline nitrobenzenes of Formulae **3b** and **3b'** can be obtained where separation of the two regiosomers by silica gel chromatography may be required.

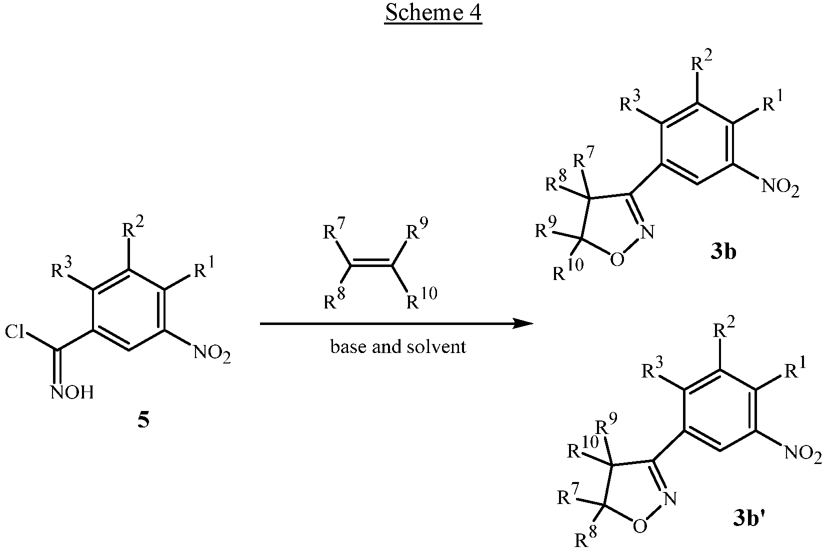

A chloro-oxime of Formula **5** can be prepared by chlorination of an oxime of Formula **6** with a chlorinating agent that generally includes but not limited to *N*-chlorosuccinimide, sodium hypochlorite or chlorine gas in a solvent such as *N*,*N*-dimethylformamide, acetonitrile, dichloromethane, dichloroethane or toluene at temperatures generally ranging from 0 °C to ambient temperature as shown in Scheme 5.

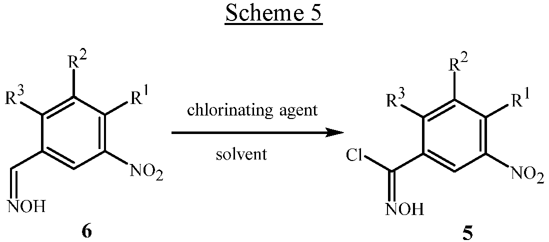

An oxime of Formula **6** can be accessed from a nitrobenzaldehyde of Formula **7** by reacting with a hydroxylamine as the free base or as the hydrochloride or acetate salt with a base such as sodium acetate, pyridine or potassium carbonate in a compatible solvent including but not limited to methanol, ethanol, acetonitrile, dichloromethane at temperatures generally ranging from 0 °C to ambient temperature as shown in Scheme 6.

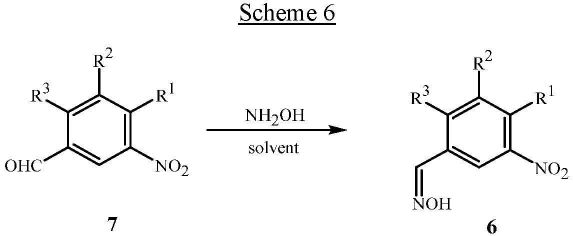

A compound of Fomula **7** can be prepared through oxidation of a nitrobenzyl alcohol of Formula **8** with a suitable oxidizing agent, e.g. pyridinium chlorochromate (PCC, optionally with Celite^{®} diatomaceous earth filter aid), chromic acid or manganese (IV) oxide in an appropriate solvent including but not limited to dichloromethane or dichloroethane as shown in Scheme 7. Alternatively, the oxidation can be done under Swern conditions using oxalyl chloride, dimethyl sulfoxide and triethylamine. Oxidation of benzylic alcohols to benzaldehydes are well documented in the synthetic organic chemistry art.

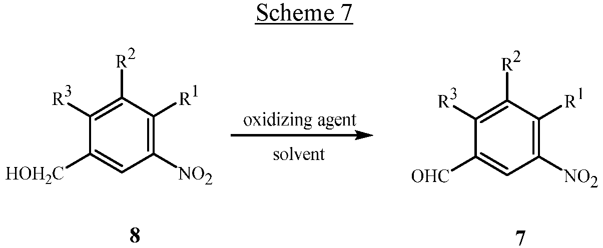

Nitrobenzylic alcohols of Formula **8** are readily obtained from nitrobenzoic acids **4** by reduction with 2-3 equivalents of borane (e.g. BH₃·THF) or lithium aluminum hydride in tetrahydrofuran, diethyl ether or dioxane at temperatures generally ranging from -78 °C to ambient temperature as shown in Scheme 8.

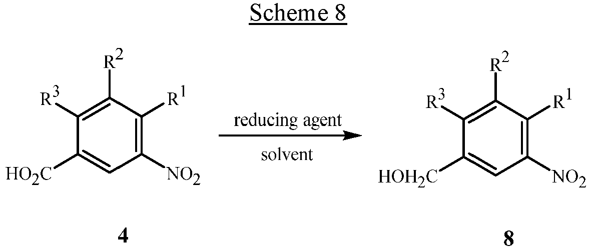

A benzoic acid of Formula **9** can be nitrated in a mixture of a nitric acid and a sulfuric acid at temperatures ranging from 0 °C to ambient temperature to afford a nitrobenzoic acid of Formula **4** as shown in Scheme 9. Other sources of nitronium ion for this nitration include nitronium tetrafluoroborate, acetyl nitrate, guanidinium nitrate, can also be used in an appropriate solvent such as tetramethylene sulfone to accomplish this reaction. Benzoic acids of Formula **9** are readily accessible commercially or readily prepared by established methods from the literature.

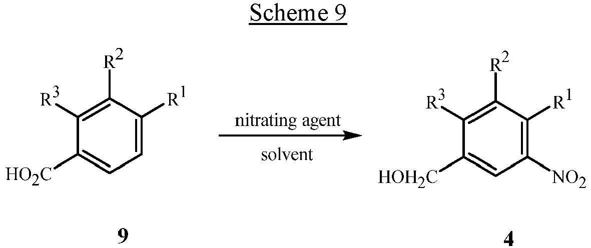

As shown in Scheme 10, a compound of Formula **3c** (i.e. a compound of Formula **3** wherein G is G-2) can be prepared by copper-mediated coupling of a *meta*-bromo or *meta-*iodo substituted nitrobenzene of Formula **10a** (i.e. a compound of Formula **10** wherein X is bromine or iodine) with a cyclic carbamate, cyclic thiocarbamate, cyclic lactam or cyclic urea of Formula **11** (wherein Q is O, S, CR¹¹R¹² or NR¹³) in the presence of copper (I) iodide with a diamine ligand such as trans-*N*,*N*'-Dimethylcyclohexane-1,2-diamine or tetramethylethylenediamine (TMEDA) and potassium phosphate (K₃PO₄) in an appropriate solvent such as *N*,*N*-dimethylformamide, acetonitrile, tetrahydrofuran or dioxane, optionally with water as a cosolvent. A similar copper-mediated coupling can also be carried out under Chan-Lam conditions where a boronic acid of Formula **10b** (i.e. a compound of Formula **10** wherein X is B(OH)₂) is coupled with a compound of Formula **11** in the presence of copper (II) acetate and pyridine in dichloromethane. This cross-coupling can also be carried out with **10a** and a compound of Formula **11** under the well-documented Buchwald-Hartwig amination protocol involving palladium-mediation with a suitable phosphine ligand, either as part of the pre-catalyst or as an additive in an appropriate solvent such as tetrahydrofuran, toluene or dichloromethane. For most of the substrates, an auxiliary base, e.g. sodium *tert*-butoxide or cesium carbonate, is used in the reaction. Examples of palladium catalysts suitable for this transformation include but are not limited to tetrakis(triphenylphosphine) palladium(0) [Pd(PPh₃)₄], bistriphenylphosphine) palladium chloride [PdCl₂(PPh₃)₂], palladium(II) chloride-tris(2-methylphenyl)phosphine [PdCl₂[P(o-Tol)₃]₂] or [1,1'bis(diphenylphosphino) ferrocene]dichloropalladium(II) [Pd(dppf)Cl₂]. Finally, this cross-coupling can also be accomplished with palladium acetate [Pd(OAc)₂] or tris(dibenzylideneacetone) dipalladium(0) [Pd₂(dba)₃] used in combination with a suitable dialkyl diarylphosphine ligand with a base such as sodium tert-butoxide in toluene or cesium carbonate in *N,N-*dimethylformamide.

A nitrobenzene boronic acid of Formula **10b** can also be prepared by Suzuki coupling.

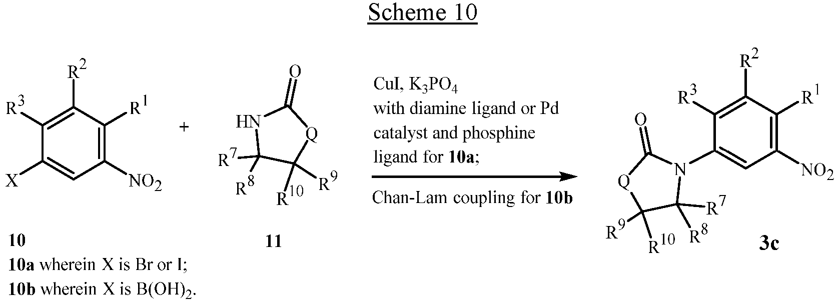

A uracil-substituted nitrobenzene of Formula **3d** (i.e. a compound of Formula 3 wherein G is G-3) and a pyridazinone-substituted nitrobenzene of Formula **3e** (i.e. a compound of Formula 3 wherein G is G-4) can be prepared by cross-coupling of a nitrobenzene boronic acid pinacole ester of Formula **12** with an appropriately substituted 5-bromo or 5-iodo substituted uracil of Formula **13** (wherein X is bromo or iodo) or a pyridazinone of Formula **14** (where X is bromo or iodo) with palladium mediation in an appropriate solvent such aqueous dioxane, aqueous tetrahydrofuran or N,N-dimethylformamide with a suitable base such as sodium carbonate, potassium carbonate or sodium bicarbonate, as outlined in Scheme 11. Examples of palladium catalysts useful for this transformation include but are not limited to tetrakis(triphenylphosphine) palladium(0) [Pd(PPh₃)₄] or bis(triphenylphosphine) palladium chloride [PdCl₂(PPh₃)₂].

A halogen substituted uracil of Formula **13** (wherein X is bromine or iodine) can be readily made by halogenation of a uracil of Formula **13a** (wherein X is hydrogen) with bromine, iodine, N-bromosuccinimide or N-iodosuccinimide in an appropriate solvent such as acetic acid, dichloromethane, carbon tetrachloride, chloroform, acetonitrile or *N,N-*dimethylformamide by established methods. Pyridazinones of Formula **14** where X is iodine can be made from pyridazinones of Formula **14a** where X is hydrogen by treating with 2,2,6,6-tetramethylpiperidylzincchloride-LiCl (TMPZnCl·LiCl) in tetrahydrofuran or dioxane followed by the addition of iodine.

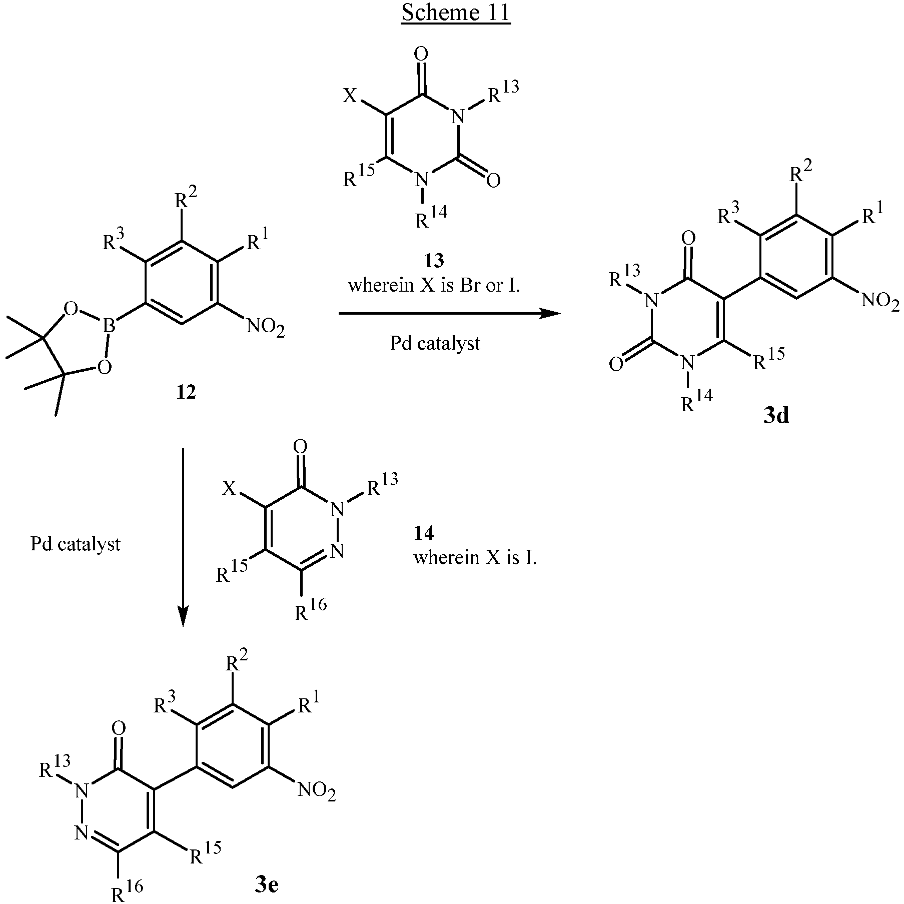

A nitrobenzene boronic acid pinacole ester of Formula **12** can be readily prepared from a nitrobenzyl bromide or iodide of Formula **15** by treating with bis(pinacolato)diboron (B₂pin₂) in the presence of a palladium catalyst such as bistriphenylphosphine palladium chloride [PdCl₂(PPh₃)₂] in a solvent, i.e. dioxane or tetrahydrofuran as shown in Scheme 12.

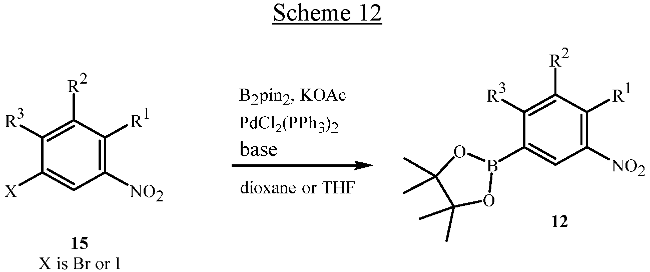

As shown in Scheme 13, an N-linked pyridazinone nitrobenzene of Formula **3f** (i.e. a compound of Formula 3 wherein G is G-5) can be prepared by cross-coupling of a bromo or iodo substituted nitrobenzene of Formula **10a** (wherein X is bromine or iodine) with a pyridazinone of Formula **15** in the presence of copper(I) iodide and K₃PO₄ and a diamine ligand, i.e. trans-*N*,*N*'-Dimethylcyclohexane-1,2-diamine or tetramethylethylenediamine (TMEDA) in an appropriate solvent such as N,N-dimethylformamide, acetonitrile, tetrahydrofuran or dioxane, optionally with water as a cosolvent. Alternatively, a similar coupling can be achieved by cross-coupling an aryl boronic acid of Formula **10b** (wherein X is B(OH)₂ with **15** under the Buchwald-Hartwig amination conditions).

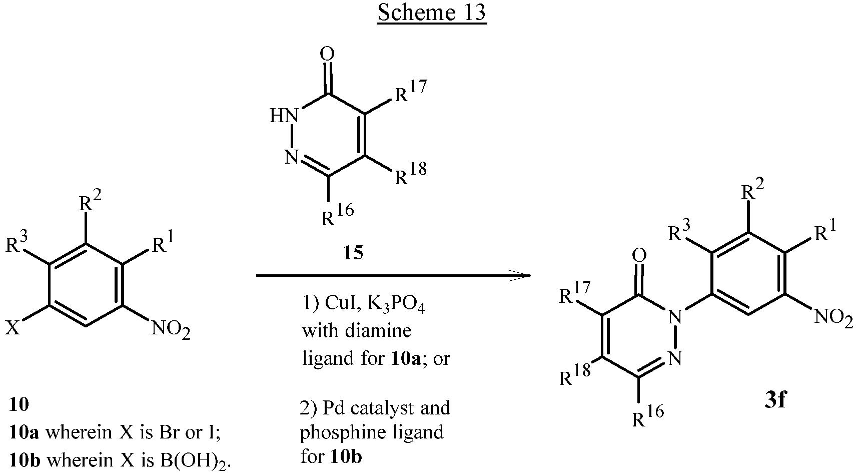

As shown in Scheme 14, compounds of Formula 1 wherein R⁴ is C(=O)R¹⁹, C(=S)R¹⁹, CO₂R⁹, C(=O)SR¹⁹, S(O)₂R¹⁹, CONR²⁰R¹⁹, S(O)₂NR²⁰R¹⁹, S(OH)₂NR²⁰R¹⁹ or CH₂OCOR¹⁹ can be made by reaction of a sulfonanilide of Formula **1** wherein R⁴ is hydrogen with an appropriately substituted acyl halide, thioacyl halide, carbamoyl halide, sulfonyl halide or sulfamoyl halide or an acyloxymethyl halide (i.e. ClCH₂O(C=O)R¹⁹) in the presence of base such as triethylamine, pyridine or diisopropylethyl amine (Hunig's Base) or potassium carbonate in a solvent including but not limited to tetrahydrofuran, dioxane, dichloromethane, acetonitrile or N,N-dimethylformamide.

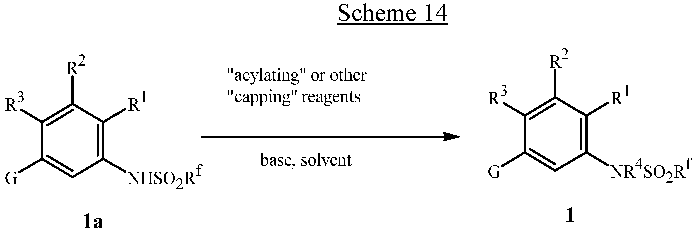

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd Ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula **1.** One skilled in the art will also recognize that it may be necessary to perform a combination of the steps illustrated in the above schemes in an order other than that implied by the particular sequence presented to prepare the compounds of Formula **1.**

One skilled in the art will also recognize that compounds of Formula **1** and the intermediates described herein can be subjected to various electrophilic, nucleophilic, radical organometallic, oxidation, and reduction reactions to add substituents or modify existing substituents.

Without further elaboration, it is believed that one skilled in the art using the preceding description can utilize the present invention to its fullest extent. The following non-limiting Examples are illustrative of the invention. Steps in the following Examples illustrate a procedure for each step in an overall synthetic transformation, and the starting material for each step may not have necessarily been prepared by a particular preparative run whose procedure is described in other Examples or Steps. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; "s" means singlet, "d" means doublet, "t" means triplet, "q" means quartet, "m" means multiplet, "dd" means doublet of doublets, "dt" means doublet of triplets, and "br s" means broad singlet. Mass spectra (MS) are reported as the molecular weight of the highest isotopic abundance parent ion (M+1) formed by addition of H+ (molecular weight of 1) to the molecule or (M-1) formed by the loss of H+ (molecular weight of 1) from the molecule, observed by using liquid chromatography coupled to a mass spectrometer (LCMS) using either atmospheric pressure chemical ionization (AP+) where "amu" stands for unified atomic mass units.

The following non-limiting Examples are meant to be illustrative of the present processes for preparing compounds of Formula 1 and corresponding intermediates. All NMR spectra are reported in CDCl₃ at 500 MHz downfield from tetramethyl silane unless otherwise indicated.

### SYNTHESIS EXAMPLE 1

### Preparation of N-[5-[(4,4-Difluoro-1-piperidinyl)carbonyl]-2,4-dimethylphenyl]-1,1,1-trifluoromethanesulfonamide (i.e. Compound 241)

### Step A: Preparation of (4,4-difluoropiperidin-1-yl)(2,4-dimethyl-5-nitrophenyl)methanone

To a stirred solution of 2,4-dimethyl-5-nitro-benzoic acid (0.30 g, 1.5 mmol), 4,4-difluoropiperidine (0.20 g, 1.7 mmol) and triethylamine (0.64 mL, 4.6 mmol) in dichloromethane (8 mL) was added propylphosphonic anhydride (50 wt% in ethyl acetate, 1.7 g, 2.7 mmol). The reaction mixture was stirred at room temperature overnight then the mixture was concentrated under reduced pressure. The mixture was diluted with 50% ethyl acetate in hexanes and filtered through a pad of silica. The filtrate was concentrated under reduced pressure to afford the title compound as a yellow solid (0.57 g), which was used without further purification in the next step.
¹H NMR (CDCl₃) δ 7.87 (s, 1H), 7.24 (s, 1H), 4.11-3.97 (m, 1H), 3.92-3.77 (m, 1H), 3.43-3.36 (m, 2H), 2.62 (s, 3H), 2.36 (s, 3H), 2.15-2.07 (m, 2H), 1.99-1.86 (m, 2H).

### Step B: Preparation of (5-amino-2,4-dimethylphenyl)4,4-difluoropiperidin-1-yl)methanone

To a stirred solution of (4,4-difluoropiperidin-1-yl)(2,4-dimethyl-5-nitrophenyl)methanone (i.e. the product of Step A) (0.57 g) in ethanol (9 mL) and water (1 mL) was added ammonium chloride (0.10 g, 1.9 mmol) and iron powder (0.32 g, 5.7 mmol). The reaction mixture was stirred at 80 °C for 2 h, then was cooled to room temperature, diluted with ethyl acetate and filtered through a pad of Celite^{®} diatomaceous earth filter aid followed by a pad of silica. The filtrate was concentrated under reduced pressure to afford the title compound as an orange oil (0.40 g), which was used without further purification in the next step.
¹H NMR (CDCl₃) δ 6.90 (s, 1H), 6.48 (s, 1H), 4.07-3.99 (m, 1H), 3.81-3.73 (m, 1H), 3.57 (br s, 2H), 3.41-3.38 (m, 2H), 2.15 (s x 2, 6H), 2.11-2.02 (m, 2H), 1.92-1.83 (m, 2H).

### Step C: Preparation of N-[5-[(4,4-Difluoro-1-piperidinyl)carbonyl]-2,4-dimethylphenyl]-1,1,1-trifluoromethanesulfonamide

To a stirred solution of (5-amino-2,4-dimethylphenyl)4,4-difluoropiperidin-1-yl)methanone (i.e. the product of Step B) (0.40 g, 1.5 mmol) in dichloromethane (8 mL) at - 40 °C was added triethylamine (0.27 mL, 1.9 mmol) followed by the dropwise addition of trifluoromethanesulfonic anhydride (0.27 mL, 1.6 mmol) over 5 minutes. The reaction mixture was stirred at -40 °C for 30 min then was poured into water. The layers were separated, the aqueous phase was extracted with ethyl acetate and the combined organic extracts were concentrated under reduced pressure. The crude material was purified by column chromatography eluting with ethyl acetate/hexanes (gradient of 0 to 60% ethyl acetate in hexanes) to afford the title compound, a compound of the present disclosure, as a white solid (0.26 g).
¹H NMR (CDCl₃) δ 9.61 (br s, 1H), 7.04 (s, 1H), 6.55 (s, 1H), 4.08-4.01 (m, 1H), 3.84-3.76 (m, 1H), 3.34-3.28 (m, 2H), 2.25 (s, 6H), 2.13-2.03 (m, 2H), 1.92-1.82 (m, 2H).

### SYNTHESIS EXAMPLE 2

### Preparation of N-[2-Chloro-5-[(4,4-difluoro-1-piperidinyl)carbonyl]-4-methylphenyl]-1,1,1-trifluoromethanesulfonamide (i.e. Compound 229)

### Step A: Preparation of (4-chloro-2-methyl-5-nitrophenyl)(4,4-difluoropiperidin-1-yl)methanone

To a stirred solution of 4-chloro-2-methyl-5-nitro-benzoic acid (0.30 g, 1.4 mmol), 4,4-difluoropiperidine (0.19 g, 1.6 mmol) and triethylamine (0.58 mL, 4.2 mmol) in dichloromethane (8 mL) was added propylphosphonic anhydride (50 wt% in ethyl acetate, 1.5 g, 2.4 mmol). The reaction mixture was stirred at room temperature overnight then the mixture was concentrated under reduced pressure. The mixture was diluted with 50% ethyl acetate in hexanes and filtered through a pad of silica. The filtrate was concentrated under reduced pressure to afford the title compound as an off-white solid (0.39 g), which was used without further purification in the next step.
¹H NMR (CDCl₃) δ 7.76 (s, 1H), 7.44 (s, 1H), 4.06-3.95 (m, 1H), 3.86-3.74 (m, 1H), 3.39-3.35 (m, 2H), 2.36 (s, 3H), 2.12-2.04 (m, 2H), 1.98-1.85 (m, 2H).

### Step B: Preparation of (5-amino-4-chloro-2-methylphenyl)4,4-difluoropiperidin-1-yl)methanone

To a stirred solution of (4-chloro-2-methyl-5-nitrophenyl)(4,4-difluoropiperidin-1-yl)methanone (i.e. the product of Step A) (0.39 g, 1.2 mmol) in ethanol (9 mL) and water (1 mL) was added ammonium chloride (65 mg, 1.2 mmol) and iron powder (0.21 g, 3.8 mmol). The reaction mixture was stirred at 80 °C for 2 h then was cooled to room temperature, diluted with ethyl acetate and filtered through a pad of Celite^{®} diatomaceous earth filter aid followed by a pad of silica. The filtrate was concentrated under reduced pressure to afford the title compound as an orange oil (0.40 g), which was used without further purification in the next step.
¹H NMR (CDCl₃) δ 7.07 (s, 1H), 6.53 (s, 1H), 4.13-3.93 (m, 3H), 3.76-3.68 (m, 1H), 3.35-3.31 (m, 2H), 2.11 (s, 3H), 2.06-1.97 (m, 2H), 1.87-1.79 (m, 2H).

### Step C: Preparation of N-[2-Chloro-5-[(4,4-difluoro-1-piperidinyl)carbonyl]-4-methylphenyl]-1,1,1-trifluoromethanesulfonamide

To a stirred solution of (5-amino-4-chloro-2-methylphenyl)(4,4-difluoropiperidin-1-yl)methanone (i.e. the product of Step B) (0.40 g) in dichloromethane (8 mL) at -10 °C was added triethylamine (0.25 mL, 1.8 mmol) followed by the dropwise addition of trifluoromethanesulfonic anhydride (0.25 mL, 1.5 mmol) over 5 minutes. The reaction mixture was stirred at -10 °C for 30 min then was poured into water. The layers were separated, the aqueous phase was extracted with ethyl acetate and the combined organic extracts were concentrated under reduced pressure. The crude material was purified by trituration with diethyl ether to afford the title compound, a compound of the disclosure, as a white solid (0.24 g).
¹H NMR (CDCl₃) δ 9.23 (br s, 1H), 7.27 (s, 1H), 6.97 (s, 1H), 4.15-4.08 (m, 1H), 3.80-3.72 (m, 1H), 3.37-3.27 (m, 2H), 2.30 (s, 3H), 2.14-2.06 (m, 2H), 1.96-1.86 (m, 2H).

### SYNTHESIS EXAMPLE 3

### Preparation of Preparation of 3-Fluoro-N,N,2,4-tetramethyl-5-[[(trifluoromethyl)sulfonyl]amino]benzamide (i.e. Compound 128)

### Step A: Preparation of 3-fluoro-2,4-dimethyl-benzoic acid

To a stirred solution of *n*-butyllithium (1.6 M solution in hexanes, 18 mL, 29 mmol) in anhydrous tetrahydrofuran (40 mL) at 0 °C was slowly added 2,2,6,6-tetramethylpiperidine (4.9 mL, 29 mmol). The mixture was stirred for 15 minutes then was cooled to -78 °C and a solution of 3-fluoro-4-methyl-benzoic acid (2.0 g, 13 mmol) in anhydrous tetrahydrofuran (10 mL) was added dropwise. The reaction mixture was stirred at -78 °C for 1.5 h, then warmed to -50 °C and stirred for an additional 45 min. Iodomethane (3.2 mL, 52 mmol) was then added slowly and the reaction mixture was allowed to warm to room temperature and stirred overnight. Water was added and the mixture was washed with diethyl ether. The aqueous phase was acidified with 6 N hydrochloric acid to pH 2 and extracted with diethyl ether (x2) then the combined organic extracts were washed with brine (x1), dried over sodium sulfate and concentrated under reduced pressure to afford the title compound as a pale yellow solid (2.18 g), which was used without further purification in the next step.
¹H NMR (CDCl₃) δ 7.75 (d, 1H), 7.10-7.07 (m, 1H), 2.55 (d, 3H), 2.33 (d, 3H).

### Step B: Preparation of 3-fluoro-2,4-dimethyl-5-nitro-benzoic acid

To a stirred mixture of 3-fluoro-2,4-dimethyl-benzoic acid (i.e. the product of Step A) (0.50 g, 3.0 mmol) in concentrated sulfuric acid (6 mL) at -20 °C was added concentrated nitric acid (0.5 mL) dropwise. The reaction mixture was stirred between -20 °C and 0 °C for 2 h then was poured onto ice. The resulting solid was collected by filtration, washed with water (x1) and dried under vacuum to afford a 1:1 mixture of the title compound and 3-fluoro-2,4-dimethyl-6-nitro-benzoic acid as a white solid (0.44 g), which was used without further purification in the next step.
¹H NMR (DMSO-d₆, mixture of regioisomers) δ 8.24 (d, 1H), 8.06 (d, 1H), 2.52 (d, 3H), 2.45 (d, 3H), 2.34 (d, 3H), 2.26 (d, 3H).

### Step C: Preparation of 3-fluoro-N,N,2,4-tetramethyl-5-nitro-benzamide

To a stirred solution of 3-fluoro-2,4-dimethyl-5-nitro-benzoic acid (i.e. the product of Step B) (0.86 g mixture of regioisomers including 2 mmol of required regioisomer), dimethylamine hydrochloride (0.20 g, 2.5 mmol) and triethylamine (0.97 mL, 7 mmol) in dichloromethane (10 mL) at 0 °C was slowly added propylphosphonic anhydride (50 wt% in ethyl acetate, 3.8 g, 6 mmol). The reaction mixture was stirred at room temperature overnight then the mixture was washed with 1 N sodium hydroxide (x1), the aqueous phase was extracted with dichloromethane (x1) and the combined organic extracts were washed with brine (x1), dried over sodium sulfate and concentrated under reduced pressure. The crude material was purified by column chromatography eluting with ethy acetate/hexanes (gradient of 20-50% ethyl acetate in hexanes) to afford the title compound as a pale-yellow oil (0.39 g). ¹H NMR (CDCl₃) δ 7.66 (d, 1H), 3.15 (s, 3H), 2.88 (s, 3H), 2.50 (d, 3H), 2.28 (d, 3H).

### Step D: Preparation of 5-amino-3-fluoro-N,N,2,4-tetramethylbenzamide

To a stirred solution of 3-fluoro-N,N,2,4-tetramethyl-5-nitrobenzamide (i.e. the product of Step C) (3.10 g, 12.9 mmol) in ethanol (40 mL) at 70 °C was added a solution of ammonium chloride (1.36 g, 25.4 mmol) in water (5 mL). Iron powder (2.17 g, 38.9 mmol) was then added portionwise. After stirring for 1 h, additional iron powder (0.30 g, 5.4 mmol) was added and the reaction mixture was stirred at 70 °C overnight. The mixture was cooled to room temperature, diluted with ethyl acetate and Celite^{®} diatomaceous earth filter aid was added. The mixture was filtered through a pad of Celite^{®} diatomaceous earth filter aid. Ethyl acetate and water were added to the filtrate, the layers were separated, and the aqueous phase was extracted with ethyl acetate (x1). The combined organic extracts were washed with saturated aqueous ammonium chloride solution (x1), dried over sodium sulfate, filtered through a pad of silica and concentrated under reduced pressure to afford the title compound as a pale orange solid (2.68 g), which was used without further purification in the next step. ¹H NMR (CDCl₃) δ 6.32 (d, 1H), 3.63 (br s, 2H), 3.10 (s, 3H), 2.84 (s, 3H), 2.07-2.06 (m, 6H).

### Step E: Preparation of 3-fluoro-N,N,2,4-tetramethyl-5-((1,1,1-trifluoro-N-((trifluoromethyl)sulfonyl)methyl)sulfonamido)benzamide

To a stirred solution of 5-amino-3-fluoro-*N*,*N*,2,4-tetramethyl-benzamide (i.e. the product of Step D) (2.68 g, 12.7 mmol) in dichloromethane (30 mL) at -78 °C was added triethylamine (5.3 mL, 38 mmol) followed by the dropwise addition of a solution of trifluoromethanesulfonic anhydride (5.1 mL, 30 mmol) in dichloromethane (10 mL) over 20 minutes. The reaction mixture was stirred at -20 °C for 1 h then was poured into water. The layers were separated, and the aqueous phase was extracted with dichloromethane (x1) then the combined organic extracts were dried over sodium sulfate, filtered through a pad of silica and concentrated under reduced pressure. The crude material was purified by column chromatography (gradient of 5 to 30% ethyl acetate in hexanes) to afford the title compound as a white solid (4.48 g).
¹H NMR (CDCl₃) δ 6.99 (s, 1H), 3.14 (s, 3H), 2.83 (s, 3H), 2.33 (d, 3H), 2.28 (d, 3H).

### Step F: Preparation of 3-Fluoro-N,N,2,4-tetramethyl-5-[[(trifluoromethyl)sulfonyl]amino]benzamide

To a stirred solution of 5-[bis(trifluoromethylsulfonyl)amino]-3-fluoro-*N*,*N*,2,4-tetramethyl benzamide (i.e. the product of Step E) (4.48 g, 9.4 mmol) in dioxane (70 mL) was slowly added 1 N sodium hydroxide (20 mL, 20 mmol). The reaction mixture was stirred overnight at room temperature then was concentrated under reduced pressure to remove most of the dioxane. The mixture was diluted with water and acidified with 1 N hydrochloric acid then the resulting precipitate was collected by filtration and washed with water (x2), diethyl ether (x1) and hexanes (x1). The obtained material was purified by crystallization from methanol/water to afford the title compound, a compound of the disclosure, as a white solid (2.32 g).
¹H NMR (CDCl₃) δ 10.50 (br s, 1H), 6.40 (s, 1H), 3.15 (s, 3H), 2.79 (s, 3H), 2.16 (m, 6H).

### SYNTHESIS EXAMPLE 4

### Preparation of N-[2,4-Dimethyl-5-(1-oxa-2-azaspiro[4.5]dec-2-en-3-yl)phenyl]-1,1,1-trifluoromethanesulfonamide (i.e. Compound 197)

### Step A: Preparation of (2,4-dimethyl-5-nitrophenyl)methanol

To a stirred solution of 2,4-dimethyl-5-nitrobenzoic acid (21.5 g, 0.11 mol) in anhydrous tetrahydrofuran (275 mL) at -5 °C was added borane tetrahydrofuran complex (1M solution in tetrahydrofuran, 200 mL, 0.2 mol). The reaction mixture was then allowed to warm to room temperature and stirred overnight. Methanol (12 mL) was slowly added, followed by saturated aqueous sodium bicarbonate solution (100 mL) and water (150 mL). The mixture was extracted with methyl *tert-butyl* ether (x2) then the combined organic extracts were washed with water (x1) and brine (x1), dried over magnesium sulfate and concentrated under reduced pressure to afford the title compound as a pale yellow solid (20.0 g), which was used without further purification in the next step.
¹H NMR (CDCl₃) δ 8.07 (s, 1H), 7.14 (s, 1H), 4.73 (d, 2H), 2.58 (s, 3H), 2.37 (s, 3H).

### Step B: Preparation of 2,4-dimethyl-5-nitro-benzaldehyde

To a stirred solution of (2,4-dimethyl-5-nitrophenyl)methanol (i.e. the product of Step A) (20.0 g, 0.11 mol) in dichloromethane (330 mL) was added Celite^{®} diatomaceous earth filter aid (~20 g) followed by portionwise addition of pyridinium chlorochromate (28 g, 0.13 mol) over 1 h. The reaction mixture was stirred at room temperature overnight then was filtered through a pad of silica. The filtrate was concentrated under reduced pressure to afford the title compound as a pale yellow solid (18.6 g), which was used without further purification in the next step.
¹H NMR (CDCl₃) δ 10.23 (s, 1H), 8.45 (s, 1H), 7.27 (s, 1H), 2.72 (s, 3H), 2.67 (s, 3H).

### Step C: Preparation of 2,4-dimethyl-5-nitro-benzaldehyde oxime

To a stirred solution of 2,4-dimethyl-5-nitro-benzaldehyde (i.e. the product of Step B) (29.0 g, 0.16 mol) in methanol (480 mL) was added dropwise over 25 min a solution of hydroxylamine (50 wt% in water, 13.2 g, 0.2 mol) in water (47 mL). The reaction mixture was stirred at room temperature overnight then was concentrated under reduced pressure to remove most of the methanol. Water was added, the mixture was stirred then the solid material collected by filtration, washed with water and dried under vacuum to afford the title compound as a white solid (30.5 g, 11:1 *E*/*Z*), which was used without further purification in the next step.
¹H NMR (CDCl₃, *E* isomer) δ 8.34 (s, 1H), 8.33 (s, 1H), 7.51 (m, 1H), 7.17 (s, 1H), 2.59 (s, 3H), 2.46 (s, 3H).

### Step D: Preparation of N-hydroxy-2,4-dimethyl-5-nitro-benzimidoyl chloride

To a stirred solution of 2,4-dimethyl-5-nitro-benzaldehyde oxime (i.e. the product of Step C) (30.5 g, 0.157 mol) in anhydrous *N*,*N*-dimethylformamide (160 mL) was added N-chlorosuccinimide (22.1 g, 0.166 mol) portionwise over 2 h, maintaining the internal reaction temperature at 30 °C. The reaction mixture was stirred for an additional 3 h at room temperature then was poured into iced water and diluted with methyl *tert-butyl* ether. The layers were then separated, and the aqueous phase was extracted with methyl *tert-butyl* ether (x2). The combined organic extracts were washed with water (x3), 1 N hydrochloric acid, saturated aqueous ammonium chloride solution , dried over magnesium sulfate and concentrated under reduced pressure to afford the title compound as a pale yellow solid (35.0 g), which was used without further purification in the next step. ¹H NMR (CDCl₃) δ 8.20 (s, 1H), 8.04-8.03 (m, 1H), 7.23 (s, 1H), 2.63 (s, 3H), 2.49 (s, 3H).

### Step E: Preparation of 3-(2,4-dimethyl-5-nitrophenyl)-1-oxa-2-azaspiro[4.5]dec-2-ene

To a stirred solution of *N*-hydroxy-2,4-dimethyl-5-nitro-benzimidoyl chloride (i.e. the product of Step D) (11.5 g, 50 mmol) and methylenecyclohexane (5.8 g, 60 mmol) in chloroform (200 mL) was added triethylamine (11.2 mL, 80 mmol) dropwise over 5 minutes. The reaction mixture was stirred at room temperature overnight then was poured into water and the layers were separated. The aqueous phase was extracted with dichloromethane then the combined organic extracts were washed with 1 N hydrochloric acid (x1), brine (x1), dried over magnesium sulfate and concentrated under reduced pressure to afford the title compound as a viscous yellow oil (16.0 g), which was used without further purification in the next step. ¹H NMR (CDCl₃) δ 7.98 (s, 1H), 7.24 (s, 1H), 3.13 (s, 2H), 2.64 (s, 3H), 2.63 (s, 3H), 1.87-1.79 (m, 4H), 1.71-1.67 (m, 2H), 1.55-1.47 (m, 4H).

### Step F: Preparation of 2,4-dimethyl-5-(1-oxa-2-azaspiro[4.5]dec-2-en-3-yl)aniline

To a stirred solution of 3-(2,4-dimethyl-5-nitro-phenyl)-1-oxa-2-azaspiro[4.5]dec-2-ene (i.e. the product of Step E) (16.0 g) in ethanol (180 mL) at 50 °C was added a solution of ammonium chloride (5.4 g, 0.10 mol) in water (20 mL). Iron powder (8.4 g, 0.15 mol) was then added portion-wise over 25 min as the reaction mixture was heated from 50 °C to 70 °C. After stirring at 70 °C for an additional 30 min, the mixture was cooled to room temperature and filtered through a pad of Celite^{®} diatomaceous earth filter aid. The filtrate was concentrated under reduced pressure then ethyl acetate and water were added. The layers were separated and the aqueous phase was extracted with ethyl acetate. The combined organic extracts were washed with saturated aqueous ammonium chloride solution, dried over magnesium sulfate and concentrated under reduced pressure to afford the title compound as a viscous amber oil (13.0 g), which was used without further purification in the next step.
¹H NMR (CDCl₃) δ 6.93 (s, 1H), 6.67 (s, 1H), 3.54 (br s, 2H), 3.05 (s, 2H), 2.41 (s, 3H), 2.16 (s, 3H), 1.85-1.78 (m, 4H), 1.68-1.63 (m, 2H), 1.51-1.42 (m, 4H).

Step G: Preparation of *N*-[2,4-dimethyl-5-(1-oxa-2-azaspiro[4.5]dec-2-en-3-yl)phenyl]-1,1,1-trifluoro-*N*-(trifluoromethylsulfonyl)methanesulfonamide

To a stirred solution of 2,4-dimethyl-5-(1-oxa-2-azaspiro[4.5]dec-2-en-3-yl)aniline (i.e. the product of Step F) (13.0 g, 50 mmol) in dichloromethane (180 mL) was added triethylamine (21 mL, 0.15 mol). The mixture was cooled to -22 °C then a solution of trifluoromethanesulfonic anhydride (20 mL, 0.12 mol) in dichloromethane (20 mL) was added dropwise over 25 minutes. The reaction mixture was stirred between 0 °C and 10 °C for 1 h then was poured into water. The layers were separated and the aqueous phase was extracted with dichloromethane. The combined organic extracts were washed with saturated aqueous sodium bicarbonate solution, saturated aqueous ammonium chloride solution, dried over magnesium sulfate and concentrated under reduced pressure. The crude material was purified by column chromatography (gradient of 0 to 10% methyl *tert-butyl* ether in hexanes) to afford the title compound as a white solid (18.8 g).
¹H NMR (CDCl₃) δ 7.27 (s, 1H), 7.19 (s, 1H), 3.02 (s, 2H), 2.59 (s, 3H), 2.42 (s, 3H), 1.89-1.78 (m, 4H), 1.69-1.65 (m, 2H), 1.55-1.42 (m, 4H).

### Step H: Preparation of N-[2,4-Dimethyl-5-(1-oxa-2-azaspiro[4.5]dec-2-en-3-yl)phenyl]-1,1,1-trifluoromethanesulfonamide

To a stirred solution of *N*-[2,4-dimethyl-5-(1-oxa-2-azaspiro[4.5]dec-2-en-3-yl)phenyl]-1,1,1-trifluoro-*N*-(trifluoromethylsulfonyl)methanesulfonamide (i.e. the product of Step G) (18.8 g, 36 mmol) in dioxane (250 mL) was added 1 N sodium hydroxide (75 mL, 75 mmol) dropwise. The reaction mixture was stirred overnight at room temperature then was concentrated under reduced pressure to remove most of the dioxane. Water was added and the mixture was acidified with 1 N hydrochloric acid then was extracted with dichloromethane (x2). The combined organic extracts were washed with saturated aqueous ammonium chloride solution, dried over magnesium sulfate and concentrated under reduced pressure. The crude material was purified by trituration with hot cyclohexane to afford the title compound, a compound of the disclosure, as a white solid (11.1 g).
¹H NMR (CDCl₃) δ 7.30 (s, 1H), 7.16 (s, 1H), 6.46 (s, 1H), 3.06 (s, 2H), 2.54 (s, 3H), 2.36 (s, 3H), 1.87-1.78 (m, 4H), 1.69-1.64 (m, 2H), 1.54-1.43 (m, 4H).

### SYNTHESIS EXAMPLE 5

Preparation of [[2,4-Dimethyl-5-(1-oxa-2-azaspiro[4.5]dec-2-en-3-yl)phenyl][(trifluoromethyl)sulfonyl]amino]methyl 2,2-dimethylpropanoate (i.e.

### Compound 159)

To a stirred solution of *N*-[2,4-dimethyl-5-(1-oxa-2-azaspiro[4.5]dec-2-en-3-yl)phenyl]-1,1,1-trifluoro-methanesulfonamide (i.e. the product of Synthesis Example 4) (0.20 g, 0.51 mmol) in dichloromethane (8 mL) was added triethylamine (0.14 mL, 1.0 mmol) followed by chloromethyl pivalate (0.11 mL, 0.76 mmol). The reaction mixture was stirred overnight at room temperature then additional chloromethyl pivalate (0.15 mL, 1.0 mmol) was added. The reaction mixture was stirred at 40 °C for 5 h then stirred at room temperature overnight. The mixture was concentrated under reduced pressure and the crude material was purified by column chromatography (gradient of 0 to 20% ethyl acetate in hexanes) to afford the title compound, a compound of the disclosure, as a clear colorless oil (0.19 g).
¹H NMR (CDCl₃) δ 7.22 (s, 2H), 5.75 (d, 1H), 5.41 (d, 1H), 3.00 (m, 2H), 2.54 (s, 3H), 2.38 (s, 3H), 1.86-1.76 (m, 4H), 1.68-1.61 (m, 2H), 1.53-1.42 (m, 4H), 1.20 (s, 9H).

### SYNTHESIS EXAMPLE 6

### Preparation of N-[2,4-Dimethyl-5-[(3aR,6aR)-3a,5,6,6a-tetrahydro-4H-cyclopent[d]isoxazol-3-yl]phenyl]-1,1,1-trifluoromethanesulfonamide (i.e. Compound 103)

### Step A: Preparation of 3-(2,4-dimethyl-5-nitro-phenyl)-4,5,6,6a-tetrahydro-3aH-cyclopenta[d]isoxazole

To a stirred solution of *N*-hydroxy-2,4-dimethyl-5-nitro-benzimidoyl chloride (i.e. the product of Step D in Synthesis Example 4) (0.50 g, 2.2 mmol) in chloroform (8 mL) was added triethylamine (0.76 mL, 5.4 mmol) followed by cyclopentene (0.29 mL, 3.3 mmol). The reaction mixture was stirred at room temperature overnight then was concentrated under reduced pressure and the crude material was purified by column chromatography (gradient of 0 to 20% ethyl acetate in hexanes) to afford the title compound as a white solid (0.41 g). ¹H NMR (CDCl₃) δ 8.05 (s, 1H), 7.25 (s, 1H), 5.23-5.20 (m, 1H), 4.16-4.12 (m, 1H), 2.62 (s, 3H), 2.58 (s, 3H), 2.23-2.19 (m, 1H), 1.92-1.74 (m, 4H), 1.58-1.47 (m, 1H).

### Step B: Preparation of 5-(4,5,6,6a-tetrahydro-3aH-cyclopenta[d]isoxazol-3-yl)-2,4-dimethyl-aniline

To a stirred solution of 3-(2,4-dimethyl-5-nitro-phenyl)-4,5,6,6a-tetrahydro-3a*H-*cyclopenta[d]isoxazole (i.e. the product of Step A) (0.38 g, 1.5 mmol) in ethanol (9 mL) and water (1 mL) was added ammonium chloride (0.15 g, 2.8 mmol) and iron powder (0.26 g, 4.7 mmol). The reaction mixture was stirred at 80 °C for 1 h then was cooled to room temperature, diluted with ethyl acetate and filtered through a pad of Celite^{®} diatomaceous earth filter aid followed by a pad of silica. The filtrate was concentrated under reduced pressure to afford the title compound as a brown oil (0.35 g), which was used without further purification in the next step.
¹H NMR (CDCl₃) δ 6.92 (s, 1H), 6.67 (s, 1H), 5.11-5.09 (m, 1H), 4.05-4.02 (m, 1H), 3.57 (br s, 2H), 2.35 (s, 3H), 2.16-2.12 (m, 4H), 1.82-1.66 (m, 4H), 1.54-1.44 (m, 1H).

### Step C: Preparation of N-[5-(4,5,6,6a-tetrahydro-3aH-cyclopenta[d]isoxazol-3-yl)-2,4-dimethyl-phenyl]-1,1,1-trifluoro-N-(trifluoromethylsulfonyl)methanesulfonamide

To a stirred solution of 5-(4,5,6,6a-tetrahydro-3a*H*-cyclopenta[*d*]isoxazol-3-yl)-2,4-dimethyl-aniline (i.e. the product of Step B) (0.33 g, 1.4 mmol) in dichloromethane (10 mL) was added triethylamine (0.59 mL, 4.2 mmol). The mixture was cooled to -10 °C then trifluoromethanesulfonic anhydride (0.59 mL, 3.5 mmol) was added dropwise over 5 minutes. The reaction mixture was stirred at -10 °C for 20 min then was poured into water. The layers were separated, the aqueous phase was extracted with dichloromethane and the combined organic extracts were concentrated under reduced pressure. The crude material was purified by column chromatography (gradient of 0 to 20% ethyl acetate in hexanes) to afford the title compound as a white solid (0.48 g).
¹H NMR (CDCl₃) δ 7.28 (s, 2H), 5.21-5.18 (m, 1H), 4.07-4.03 (m, 1H), 2.57 (s, 3H), 2.42 (s, 3H), 2.22-2.18 (m, 1H), 1.88-1.73 (m, 4H), 1.56-1.46 (m, 1H).

### Step D: Preparation of N-[2,4-Dimethyl-5-[(3aR,6aR)-3a,5,6,6a-tetrahydro-4H-cyclopent[d]isoxazol-3-yl]phenyl]-1,1,1-trifluoromethanesulfonamide

To a stirred solution of *N*-[5-(4,5,6,6a-tetrahydro-3a*H*-cyclopenta[*d*]isoxazol-3-yl)-2,4-dimethyl-phenyl]-1,1,1-trifluoro-N-(trifluoromethylsulfonyl)methanesulfonamide (i.e. the product of Step C) (0.48 g, 0.97 mmol) in dioxane (10 mL) was added 0.5 N sodium hydroxide (5 mL, 2.5 mmol). The reaction mixture was stirred at room temperature for 1 h then was concentrated under reduced pressure to remove most of the dioxane. The mixture was diluted with water, acidified with 1 N hydrochloric acid then was extracted with ethyl acetate (x2). The combined organic extracts were washed with water, brine, dried over magnesium sulfate and concentrated under reduced pressure to afford the title compound, a compound of the disclosure, as a white solid (0.33 g).
¹H NMR (CDCl₃) δ 7.55 (br s, 1H), 7.22 (s, 1H), 7.12 (s, 1H), 5.18-5.15 (m, 1H), 4.06-4.02 (m, 1H), 2.42 (s, 3H), 2.33 (s, 3H), 2.17-2.14 (m, 1H), 1.83-1.70 (m, 4H), 1.52-1.42 (m, 1H).

### SYNTHESIS EXAMPLE 7

### Preparation of [[2,4-Dimethyl-5-[(3aR,6aR)-3a,5,6,6a-tetrahydro-4H-cyclopent[d]isoxazol-3-yl]phenyl][(trifluoromethyl)sulfonyl]amino]methyl 2,2-dimethylpropanoate (i.e. Compound 100)

To a stirred solution of *N*-[5-(4,5,6,6a-tetrahydro-3a*H*-cyclopenta[*d*]isoxazol-3-yl)-2,4-dimethyl-phenyl]-1,1,1-trifluoro-methanesulfonamide (i.e. the product of Step D in Synthesis Example 6) (0.13 g, 0.36 mmol) in dichloromethane (8 mL) was added triethylamine (0.10 mL, 0.72 mmol) followed by chloromethyl pivalate (0.08 mL, 0.6 mmol). The reaction mixture was stirred overnight at room temperature then additional triethylamine (0.10 mL, 0.72 mmol) and chloromethyl pivalate (0.10 mL, 0.69 mmol) were added. The reaction mixture was stirred overnight at room temperature. The mixture was concentrated under reduced pressure and the crude material was purified by column chromatography (gradient of 0 to 20% ethyl acetate in hexanes) to afford the title compound, a compound of the dislcosure, as a clear colorless oil (79 mg).
¹H NMR (CDCl₃) δ 7.28-7.22 (m, 2H), 5.80-5.76 (m, 1H), 5.44-5.39 (m, 1H), 5.20-5.14 (m, 1H), 4.03-3.99 (m, 1H), 2.53-2.47 (m, 3H), 2.39 (m, 3H), 2.19-2.16 (m, 1H), 1.81-1.71 (m, 4H), 1.54-1.43 (m, 1H), 1.21-1.20 (m, 9H).

### SYNTHESIS EXAMPLE 8

### Preparation of N-[2,4-Dimethyl-5-(1-oxo-2-azaspiro[4.5]dec-2-yl)phenyl]-1,1,1-trifluoromethanesulfonamide (i.e. Compound 278)

### Step A: Preparation of 2-(2,4-dimethyl-5-nitro-phenyl)-2-azaspiro[4.5]decan-1-one

To a 25 mL scintillation vial with septum, copper iodide (0.148 g, 10.0 mmol), potassium phosphate tribasic (K₃PO₄) (3.5 g, 16.4 mmol) and 2-azaspiro[4.5]decan-1-one (1.0g, 6.5 mmol) were added. The reaction vial was evacuated and backfilled with nitrogen three times. Separately, trans-(1R,2R)N,N'-dimethyl-cyclohexane-1,2-diamine (0.246 mL, 20.0 mol%) and 1-bromo-2,4-dimethyl-5-nitro-benzene (1.8 g, 7.8 mmol) were combined in toluene (10 mL) and added to the reaction mixture vial via syringe. The reaction mixture was stirred under nitrogen at reflux overnight, then diluted with ethyl acetate and filtered through a pad of Celite^{®} diatomaceous earth filter aid. The resulting filtrate was dried over magnesium sulfate and concentrated under reduced pressure to a residue. The residue was purified by column chromatography (0 to 80% ethyl acetate in hexanes gradient, 40 g column) to afford the desired product as a yellow solid (1.85 g).
¹H NMR (CDCl₃) δ 7.85 (s, 1H), 7.23 (s, 1H), 3.65 (m, 2H), 2.59 (s, 3H), 2.23 (s, 3H), 2.14-2.18 (m, 2H), 1.65-1.81 (m, 6H), 1.31-1.44 (m, 4H).

### Step B: Preparation of 2-(5-amino-2,4-dimethyl-phenyl)-2-azaspiro[4.5]decan-1-one

To a stirred solution of 2-(2,4-dimethyl-5-nitro-phenyl)-2-azaspiro[4.5]decan-1-one (i.e. the product of Step A) (1.85 g, 6.8 mmol) in ethanol (20 mL) was added a solution of ammonium chloride (0.728 g, 13.6 mmol) in water (2 mL). Iron powder (1.13 g, 20.3 mmol) was then added and stirred at 80 °C under nitrogen for 2 h. Thin layer chromatography showed the reaction was partially complete after this time. A second equivalent of ammonium chloride and iron powder was added and stirring was continued at 80 °C overnight. The mixture was cooled to room temperature and filtered through a pad of Celite^{®} diatomaceous earth filter aid. Filtered a second time through Celite^{®} diatomaceous earth filter aid to remove cloudiness. The filtrate was dried over magnesium sulfate and concentrated under reduced pressure to a yellow residue. The residue was purified by column chromatography (20 to 100% ethyl acetate in hexanes gradient, 40 g column) to afford the title compound as yellow solid (1.36 g).
¹H NMR (CDCl₃) δ 6.92 (s, 1H), 6.46 (s, 1H), 3.53-3.58 (m, 2H), 2.12 (s, 3H), 2.08 (m, 2H), 2.05 (s, 3H), 1.65-1.81 (m, 6H), 1.29-1.44 (m, 4H).

### Step C: Preparation of N-[2,4-dimethyl-5-(1-oxo-2-azaspiro[4.5]decan-2-yl)phenyl]-1,1,1-trifluoro-N-(trifluoromethylsulfonyl)methanesulfonamide

To a stirred solution of 2-(5-amino-2,4-dimethyl-phenyl)-2-azaspiro[4.5]decan-1-one (i.e. the product of Step B) (0.500 g, 1.84 mmol) in dichloromethane (10 mL) was added triethylamine (0.562 mL, 4.03 mmol). The mixture was cooled to 0 °C then a solution of trifluoromethanesulfonic anhydride (0.677 mL, 4.03 mmol) in dichloromethane (10 mL) was added dropwise over 5 minutes. The reaction mixture was then stirred at room temperature overnight. The reaction mixture was concentrated *in vacuo.* The crude material was purified by column chromatography (gradient of 0 to 100% ethyl acetate in hexanes, 40 g column) to afford the title compound as an off white solid (0.650 g).
¹H NMR (CDCl₃) δ 7.25 (s, 1H), 7.07 (s, 1H), 3.61 (m, 2H), 2.39 (s, 3H), 2.21 (s, 3H), 2.14 (m, 2H), 1.58-1.81 (m, 6H), 1.56-1.60, 1.29-1.52 (m, 4H).

### Step D: Preparation of N-[2,4-Dimethyl-5-(1-oxo-2-azaspiro[4.5]dec-2-yl)phenyl]-1,1,1-trifluoromethanesulfonamide

To a stirred solution of *N*-[2,4-dimethyl-5-(1-oxo-2-azaspiro[4.5]decan-2-yl)phenyl]-1,1,1-trifluoro-N-(trifluoromethylsulfonyl)methanesulfonamide (i.e. the product of Step C) (0.510 g, 0.951 mmol) in dioxane (15 mL) was added 1.0 N aqueous sodium hydroxide solution (1 mL, 1.0 mmol) dropwise. The reaction mixture was stirred overnight at room temperature then was concentrated under reduced pressure to remove most of the dioxane and water. The mixture was acidified with a few drops of 6 N aqueous hydrochloric acid solution, forming a white precipitate. This precipitate was then filtered and dried under vacuum overnight to afford the title compound (0.335g).
¹H NMR (CDCl₃) δ 6.93 (s, 1H), 6.79 (s, 1H), 3.56 (m, 2H), 2.08-2.16 (m, 8H), 1.79 (m, 4H), 1.56 (m, 3H), 1.37 (m, 3H).

### SYNTHESIS EXAMPLE 9

### Preparation of [[2,4-Dimethyl-5-(1-oxo-2-azaspiro[4.5]dec-2-yl)phenyl][(trifluoromethyl)sulfonyl]amino]methyl 2,2-dimethylpropanoate (i.e. Compound 268)

To a stirred solution of *N*-[2,4-dimethyl-5-(1-oxo-2-azaspiro[4.5]decan-2-yl)phenyl]-1,1,1-trifluoro-methanesulfonamide (i.e. the product of Step D in Synthesis Example 8) (0.150 g, 0.370 mmol) in methylene chloride (15 mL) was added triethylamine (0.103 mL, 0.740 mmol) and chloromethyl pivalate (0.064 mL, 0.440 mmol). The reaction mixture was stirred overnight at room temperature. The reaction mixture was concentrated *in vacuo.* The residue was purified by column chromatography (0 to 100% ethyl acetate in hexanes gradient, 12 g column) to afford the title compound, a compound of the disclosure, as a clear oil (0.092 g).
¹H NMR (CDCl₃) δ 7.22 (s, 1H), 7.05 (s, 1H), 5.71 (d, 1H), 5.42 (d, 1H), 3.53-3.63 (m, 2H), 2.38 (s, 3H), 2.19 (s, 3H), 2.10-2.15 (m, 2H), 2.05 (s, 1H), 1.57-1.82 (m, 4H), 1.47-1.52 (m, 1H), 1.30-1.51 (m, 3H), 1.20 (s, 9H).

### SYNTHESIS EXAMPLE 10

### Preparation of N-[5-(6-Ethyl-2,3-dihydro-2-methyl-3-oxo-4-pyridazinyl)-2,4-dimethylphenyl]-1,1,1-trifluoromethanesulfonamide (i.e. Compound 34)

### Step A: Preparation of 2-(2,4-dimethyl-5-nitro-phenyl)-4,4,5,5-tetramethyl-1,3,2-dioxaborolane

To a stirred solution of 1-bromo-2,4-dimethyl-5-nitro-benzene (2.30 g, 10 mmol) in dioxane (20 mL) was added bis(pinacolato)diboron (3.4 g, 14 mmol) followed by potassium acetate (2.84 g, 30 mmol) and bis(triphenylphosphine)palladium(II) dichloride (0.35 g, 0.5 mmol). The reaction mixture was warmed to 110 °C and stirred overnight. The reaction mixture was then cooled to room temperature, diluted with ethyl acetate and filtered through a Celite^{®} diatomaceous earth filter aid pad. The reaction mixture was concentrated *in vacuo.* The crude material was purified by column chromatography to afford the title compound (4.10 g).
¹H NMR (CDCl₃) δ 8.40 (s, 1H), 7.10 (s, 1H), 2.57 (s, 3H), 2.56 (s, 3H), 1.35 (s, 12H).

### Step B: Preparation of 6-ethyl-4-iodo-2-methyl-pyridazin-3-one

To a stirred solution of 6-ethyl-2-methyl-pyridazin-3-one (0.95 g, 6.9 mmol) in tetrahydrofuran (10 mL) at 0 °C was added TMPZn•LiCl (14 mL, 9.6 mmol, 0.7 M in tetrahydrofuran). The reaction mixture was warmed to room temperature and stirred for 1 hour. Iodine (2.7 g, 10.3 mmol) was then added and the reaction mixture stirred overnight at room temperature. Saturated aqueous ammonium chloride solution was added and the aqueous phase extracted with ethyl acetate (x3). The combined organic layers were washed with Na₂SO₃ solution followed by brine. The combined organic extracts were dried (MgSO₄), filtered and concentrated *in vacuo.* Purification by column chromatography (gradient of 0 to 100% ethyl acetate in hexanes) afforded the title compound (290 mg).
¹H NMR (CDCl₃) δ 7.81 (d, 1H), 3.81 (s, 3H), 2.62 (m, 2H), 1.23 (m, 3H).

### Step C: Preparation of 4-(2,4-dimethyl-5-nitro-phenyl)-6-ethyl-2-methyl-pyridazin-3-one

To a stirred solution of 6-ethyl-4-iodo-2-methyl-pyridazin-3-one benzamide (i.e. the product of Step B) (0.26 g, 1 mmol) in dioxane (2 mL) was added 2-(2,4-dimethyl-5-nitrophenyl)-4,4,5,5-tetramethyl-1,3,2-dioxaborolane (i.e. the product of Step A) (0.39 g, 1.4 mmol), bis(triphenylphosphine)palladium(II) dichloride (0.035 mg, 0.05 mmol) and sodium carbonate (2 N in H₂O, 1 mL) and the reaction mixture was warmed to 80 °C for 3 hours. The reaction mixture was then cooled to room temperature, diluted with ethyl acetate and filtered through a Celite^{®} diatomaceous earth filter aid pad. The reaction mixture was concentrated *in vacuo.* The crude material was purified by column chromatography to afford the title compound (0.28 g).
¹H NMR (CDCl₃) δ 7.92 (s, 1H), 7.25 (s, 1H), 7.11 (s, 1H), 3.86 (s, 3H), 2.68 (m, 2H), 2.63 (s, 3H), 2.30 (s, 3H), 1.26 (m, 3H).

### Step D: Preparation of 4-(5-amino-2,4-dimethyl-phenyl)-6-ethyl-2-methyl-pyridazin-3-one

To a stirred solution of 4-(2,4-dimethyl-5-nitro-phenyl)-6-ethyl-2-methyl-pyridazin-3-one (i.e. the product of Step C) (0.28 g, 1 mmol) in ethanol (18 mL) at 70 °C was added a solution of ammonium chloride (0.16 g, 3 mmol) in water (2 mL). Iron powder (0.17 g, 3 mmol) was then added portionwise and the reaction mixture stirred for 3 hours. The mixture was cooled to room temperature, diluted with ethyl acetate and filtered through a pad of Celite^{®} diatomaceous earth filter aid. Ethyl acetate and water were added to the filtrate, the layers were separated, and the aqueous phase was extracted with ethyl acetate (x1). The combined organic extracts were washed with saturated aqueous ammonium chloride solution (x1), dried over sodium sulfate, filtered through a pad of silica and concentrated under reduced pressure to afford the title compound (0.21 g), which was used without further purification in the next step.
¹H NMR (CDCl₃) δ 7.05 (s, 1H), 6.97 (s, 1H), 6.61 (s, 1H), 3.81 (s, 3H), 2.66 (m, 2H), 2.19 (s, 3H), 2.11 (s, 3H), 1.25 (m, 3H).

### Step E: Preparation of N-[5-(6-ethyl-2-methyl-3-oxo-pyridazin-4-yl)-2,4-dimethylphenyl]-1,1,1-trifluoro-N-(trifluoromethylsulfonyl)methanesulfonamide

To a stirred solution of 4-(5-amino-2,4-dimethyl-phenyl)-6-ethyl-2-methyl-pyridazin-3-one (i.e. the product of Step D) (0.2 g, 0.7 mmol) in dichloromethane (3 mL) at -78 °C was added triethylamine (0.13 mL, 0.9 mmol) followed by the dropwise addition of a solution of trifluoromethanesulfonic anhydride (0.11 mL, 0.9 mmol) in dichloromethane (2 mL) over 20 minutes. Silica gel was added to the reaction mixture and the solvent removed *in vacuo.* The crude material was purified by column chromatography (gradient of 5 to 30% ethyl acetate in hexanes) to afford the title compound (0.08 g) as well as N-[5-(6-ethyl-2-methyl-3-oxo-pyridazin-4-yl)-2,4-dimethyl-phenyl]-1,1,1-trifluoro-methanesulfonamide (i.e. the product of Step F in this Synthesis Example)(0.13 g).
¹H NMR (CDCl₃) δ 7.28 (s, 1H), 7.20 (s, 1H), 7.06 (s, 1H), 3.83 (s, 3H), 2.71 (m, 2H), 2.43 (s, 3H), 2.29 (s, 3H), 1.27 (m, 3H).

### Step F: Preparation of N-[5-(6-Ethyl-2,3-dihydro-2-methyl-3-oxo-4-pyridazinyl)-2,4-dimethylphenyl]-1,1,1-trifluoromethanesulfonamide

To a stirred solution of *N*-[5-(6-ethyl-2-methyl-3-oxo-pyridazin-4-yl)-2,4-dimethylphenyl]-1,1,1-trifluoro-N-(trifluoromethylsulfonyl)methanesulfonamide (i.e. the product of Step E) (0.08 g) in dioxane (2 mL) was slowly added 1 N sodium hydroxide (0.5 mL, 0.5 mmol). The reaction mixture was stirred overnight at room temperature then was concentrated *in vacuo.* The crude material was acidified, worked up and purified by column chromatography to afford the title compound, a compound of the present disclosure, as a white solid (0.025 g).
¹H NMR (CDCl₃) δ 9.78 (s, 1H), 7.07 (s, 1H), 6.92 (s, 1H), 6.89 (s, 1H), 3.90 (s, 3H), 2.71 (m, 2H), 2.16 (s, 3H), 2.15 (s, 3H), 1.27 (m, 3H).

### SYNTHESIS EXAMPLE 11

### Preparation of N-[2,4-dimethyl-5-(1,2,3,4-tetrahydro-1,3-dimethyl-2,4-dioxo-5-pyrimidinyl)phenyl]-1,1,1-trifluoromethanesulfonamide (i.e. Compound 49)

### Step A: Preparation of 5-(2,4-dimethyl-5-nitro-phenyl)-1,3-dimethyl-pyrimidine-2,4-dione

To a stirred solution of 5-bromo-1,3-dimethyl-pyrimidine-2,4-dione (0.65 g, 3 mmol) in dioxane (6 mL) was added 2-(2,4-dimethyl-5-nitro-phenyl)-4,4,5,5-tetramethyl-1,3,2-dioxaborolane (i.e. the product of Step A in Synthesis Example 10)(1.1 g, 3.9 mmol), bis(triphenylphosphine)palladium(II) dichloride (0.11 g, 0.15 mmol) and sodium carbonate (2 N in H₂O, 3 mL) and the reaction mixture was warmed to 80 °C for 3 hours. The reaction mixture was then cooled to room temperature, diluted with ethyl acetate and filtered through a Celite^{®} diatomaceous earth filter aid pad. The reaction mixture was concentrated *in vacuo.* The crude material was purified by column chromatography to afford the title compound (0.72 g).
¹H NMR (CDCl₃) δ 7.84 (s, 1H), 7.29 (s, 1H), 7.25 (s, 1H), 3.49 (s, 3H), 3.42 (s, 3H), 2.62 (s, 3H), 2.29 (s, 3H).

### Step B: Preparation of 5-(5-amino-2,4-dimethyl-phenyl)-1,3-dimethyl-pyrimidine-2,4-dione

To a stirred solution of 5-(2,4-dimethyl-5-nitro-phenyl)-1,3-dimethyl-pyrimidine-2,4-dione (i.e. the product of Step A) (0.29 g, 1 mmol) in ethanol (18 mL) at 70 °C was added a solution of ammonium chloride (0.16 g, 3 mmol) in water (2 mL). Iron powder (0.17 g, 3 mmol) was then added portionwise and the reaction mixture stirred for 3 hours. The mixture was cooled to room temperature, diluted with ethyl acetate and filtered through a pad of Celite^{®} diatomaceous earth filter aid. Ethyl acetate and water were added to the filtrate, the layers were separated, and the aqueous phase was extracted with ethyl acetate (x1). The combined organic extracts were washed with saturated aqueous ammonium chloride solution (x1), dried over sodium sulfate, filtered through a pad of silica and concentrated under reduced pressure to afford the title compound (0.21 g), which was used without further purification in the next step.
¹H NMR (CDCl₃) δ 7.11 (s, 1H), 6.92 (s, 1H), 6.49 (s, 1H), 3.44 (s, 3H), 3.41 (s, 3H), 2.15 (s, 3H), 2.09 (s, 3H).

### Step C: Preparation of N-[2,4-dimethyl-5-(1,2,3,4-tetrahydro-1,3-dimethyl-2,4-dioxo-5-pyrimidinyl)phenyl]-1,1,1-trifluoromethanesulfonamide

To a stirred solution of 5-(5-amino-2,4-dimethyl-phenyl)-1,3-dimethyl-pyrimidine-2,4-dione (i.e. the product of Step B)(0.21 g, 0.81 mmol) in dichloromethane (3 mL) at -78 °C was added triethylamine (0.13 mL, 0.97 mmol) followed by the dropwise addition of a solution of trifluoromethanesulfonic anhydride (0.11 mL, 0.9 mmol) in dichloromethane (2 mL) over 20 minutes. Silica gel was added to the reaction mixture and the solvent removed *in vacuo.* The crude material was purified by column chromatography (gradient of 5 to 100% ethyl acetate in hexanes) to afford the title compound, a compound of the present disclosure, as a white solid (0.11 g).
¹H NMR (CDCl₃) δ 8.18 (s, 1H), 7.18 (s, 1H), 7.01 (s, 1H), 6.92 (s, 1H), 3.47 (s, 3H), 3.45 (s, 3H), 2.18 (s, 3H), 2.16 (s, 3H).

### SYNTHESIS EXAMPLE 12

### Preparation of 1,1,1-Trifluoro-N-[2,3,4-trimethyl-5-(4-morpholinylcarbonyl)phenyl]methanesulfonamide (i.e. Compound 289)

### Step A: Preparation of 2,3,4-trimethylbenzaldehyde

To a stirred solution of 1,2,3-trimethylbenzene (3 g, 25 mmol) in dichloromethane (35 mL) at 0 °C was added 1,1-dichlorodimethyl ether (3.6 mL, 40 mmol) followed by the dropwise addition of titanium tetrachloride (1 M solution in dichloromethane, 27.5 mL, 27.5 mmol). The reaction mixture was stirred at 0 °C for 2 h, then was poured into ice water (300 mL). Dichloromethane (100 mL) was added, the mixture was stirred vigorously for 10 min then the layers were separated. The aqueous phase was extracted with dichloromethane (x1) and the combined organic extracts were washed with water, brine and concentrated under reduced pressure to afford the title compound as a pale yellow oil (3.1 g), which was used without further purification in the next step.
¹H NMR (CDCl₃) δ 10.26 (s, 1H), 7.56 (d, 1H), 7.16 (d, 1H), 2.61 (s, 3H), 2.36 (s, 3H), 2.24 (s, 3H).

### Step B: Preparation of 2,3,4-trimethylbenzoic acid

To a stirred solution of 2,3,4-trimethylbenzaldehyde (i.e. the product of Step A) (3.1 g) in acetone (20 mL) and water (10 mL) at 0 °C was added potassium permanganate (6.61 g, 41.8 mmol) portionwise. The reaction mixture was then allowed to warm to room temperature and was stirred overnight. The mixture was filtered through a pad of Celite, rinsing with water and acetone then the filtrate was acidified with 1 N hydrochloric acid to pH~2 and extracted with ethyl acetate (x2). The combined organic extracts were washed with water and brine, dried over magnesium sulfate and concentrated under reduced pressure to afford the title compound as a white solid (2.6 g), which was used without further purification in the next step.
¹H NMR (CDCl₃) δ 7.74 (d, 1H), 7.08 (d, 1H), 2.57 (s, 3H), 2.35 (s, 3H), 2.25 (s, 3H).

### Step C: Preparation of 2,3,4-trimethyl-5-nitro-benzoic acid

Concentrated nitric acid (0.8 mL) was added dropwise to concentrated sulfuric acid (0.7 mL) at 0 °C and the mixture was stirred for 5 min. This mixture was then added dropwise to a stirred mixture of 2,3,4-trimethylbenzoic acid (i.e. the product of Step B) (1.5 g) in concentrated sulfuric acid (8 mL) at 5 °C. The reaction mixture was stirred between 0 °C and 5 °C for 2 h, then was poured into ice water (200 mL). The mixture was extracted with ethyl acetate (x2) and the combined organic extracts were washed with water and brine, dried over magnesium sulfate and concentrated under reduced pressure to afford the title compound as a tan solid (1.82 g), which was used without further purification in the next step.
¹H NMR (CDCl₃) δ 8.24 (s, 1H), 2.64 (s, 3H), 2.46 (s, 3H), 2.36 (s, 3H).

### Step D: Preparation of morpholino-(2,3,4-trimethyl-5-nitro-phenyl)methanone

To a stirred mixture of 2,3,4-trimethyl-5-nitro-benzoic acid (i.e. the product of Step C) (0.20 g) in chloroform (8 mL) was added triethylamine (0.4 mL, 2.9 mmol) and morpholine (0.1 mL, 1.1 mmol), followed by propylphosphonic anhydride (50 wt.% in ethyl acetate, 1.2 g, 1.9 mmol). The reaction mixture was stirred at 60 °C for 3 h, then the mixture was cooled to room temperature and concentrated under reduced pressure. The crude material was purified by column chromatography (gradient of 0 - 60% ethyl acetate in hexanes) to afford the title compound as a yellow oil (0.19 g).
¹H NMR (CDCl₃) δ 7.43 (s, 1H), 3.803.73 (m, 4H), 3.603.50 (m, 2H), 3.263.16 (m, 2H), 2.37 (s, 3H), 2.27 (s, 6H).

### Step E: Preparation of (5-amino-2,3,4-trimethyl-phenyl)-morpholino-methanone

To a stirred mixture of morpholino-(2,3,4-trimethyl-5-nitro-phenyl)methanone (i.e. the product of Step D) (0.19 g, 0.70 mmol) in ethanol (9 mL) and water (1 mL) was added ammonium chloride (75 mg, 1.4 mmol) and iron powder (0.12 g, 2.1 mmol). The reaction mixture was stirred at 80 °C for 2 h, then was cooled to room temperature, diluted with ethyl acetate and filtered through a pad of Celite^{®} followed by a pad of silica. The filtrate was concentrated under reduced pressure to afford the title compound as a yellow oil (0.14 g), which was used without further purification in the next step.
¹H NMR (CDCl₃) δ 6.26 (s, 1H), 3.793.55 (m, 6H), 3.533.51 (m, 2H), 3.233.20 (m, 2H), 2.15 (s, 3H), 2.09 (s, 3H), 2.06 (s, 3H).

### Step F: Preparation of 1,1,1-trifluoro-N-(trifluoromethylsulfonyl)-N-[2,3,4-trimethyl-5-(morpholine-4-carbonyl)phenyl]methanesulfonamide

To a stirred solution of (5-amino-2,3,4-trimethyl-phenyl)-morpholino-methanone (i.e. the product of Step E) (0.14 g) in dichloromethane (8 mL) at -10 °C was added triethylamine (0.24 mL, 1.7 mmol) followed by the dropwise addition of trifluoromethanesulfonic anhydride (0.19 mL, 1.1 mmol). The reaction mixture was stirred between 0 °C and 10 °C for 1 h then water was added. The layers were separated and the organic phase was concentrated under reduced pressure. The crude material was purified by column chromatography (gradient of 0 - 60% ethyl acetate in hexanes) to afford the title compound as a clear colorless oil (0.15 g). ¹H NMR (CDCl₃) δ 6.99 (s, 1H), 3.83-3.73 (m, 4H), 3.60-3.51 (m, 2H), 3.24-3.15 (m, 2H), 2.31 (s, 3H), 2.29 (s, 3H), 2.27 (s, 3H).

### Step G: Preparation of 1,1,1-trifluoro-N-[2,3,4-trimethyl-5-(morpholine-4-carbonyl)phenyl]methanesulfonamide

To a stirred solution of 1,1,1-trifluoro-*N*-(trifluoromethylsulfonyl)-*N*-[2,3,4-trimethyl-5-(morpholine-4-carbonyl)phenyl]methanesulfonamide (i.e. the product of Step F) (0.15 g, 0.30 mmol) in dioxane (8 mL) was added 0.5 N sodium hydroxide (3.6 mL, 1.8 mmol). The reaction mixture was stirred at room temperature for 2 h then was concentrated under reduced pressure to remove the dioxane. The mixture was diluted with water, acidified with 1 N hydrochloric acid to pH~2 then was extracted with ethyl acetate (x2). The combined organic extracts were washed with water and brine, dried over magnesium sulfate and concentrated under reduced pressure to afford the title compound, a compound of the present invention, as a white solid (0.10 g).
¹H NMR (CDCl₃) δ 10.16 (br s, 1H), 6.45 (s, 1H), 3.853.82 (s, 2H), 3.783.75 (s, 2H), 3.57 (m, 2H), 3.193.11 (m, 2H), 2.19 (s, 3H), 2.15 (s, 3H), 2.13 (s, 3H).

### SYNTHESIS EXAMPLE 13

### Preparation of [[(Trifluoromethyl)sulfonyl][2,3,4-trimethyl-5-(4-morpholinylcarbonyl)phenyl]amino]methyl 2,2-dimethylpropanoate (i.e. Compound 324)

To a stirred solution of 1,1,1-Trifluoro-N-[2,3,4-trimethyl-5-(4-morpholinylcarbonyl)phenyl]methanesulfonamide (i.e. the product of Synthesis Example 12, 0.10 g, 0.27 mmol) in acetonitrile (8 mL) was added sodium bicarbonate (80 mg, 0.95 mmol), tetrabutylammonium bromide (87 mg, 0.27 mmol) and chloromethyl pivalate (0.12 mL, 0.81 mmol). The reaction mixture was stirred at 80 °C for 3 h then was cooled to room temperature and concentrated under reduced pressure. The crude material was purified by column chromatography (gradient of 0~50% ethyl acetate in hexanes) to afford the title compound, a compound of the present invention, as a clear colorless oil (93 mg).
¹H NMR (CDCl₃) δ 6.97 - 6.93 (m, 1H), 5.795.67 (m, 1H), 5.455.37 (m, 1H), 3.933.68 (m, 4H), 3.593.50 (m, 2H), 3.253.16 (m, 2H), 2.312.29 (m, 3H), 2.25 (s, 6 H), 1.19 (s, 9H).

By the procedures described herein together with methods known in the art, the following compounds of Tables 1 to 11 can be prepared. The following abbreviations are used in the Tables which follow: *t* means tertiary, *s* means secondary, *n* means normal, *i* means iso, c means cyclo, Me means methyl, Et means ethyl, Pr means propyl, Bu means butyl, n-Pr means 1-Propyl, *i*-Pr means isopropyl, Bu means butyl, *c*-Pr cyclopropyl, *c*-Bu means cyclobutyl, *i*-Bu means isobutyl, Ph means phenyl, OMe means methoxy, OEt means ethoxy, SMe means methylthio, SEt means ethylthio, NHMe methylamino, -CN means cyano, Py means pyridinyl, -NO₂ means nitro, TMS means trimethylsilyl, S(O)Me means methylsulfinyl, and S(O)₂Me means methylsulfonyl.

**Table 1**

| 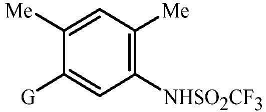 | | | | |
|---|---|---|---|---|
| G is CO-J and J is | | | | |
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | |
| J-3a* | J-3b* | | | |

| | | | | |
|---|---|---|---|---|
| See Exhibit 1 for J-1 through J-52. | | | | |

**Table 2**

| 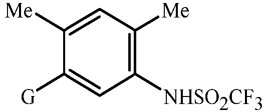 | | | | |
|---|---|---|---|---|
| G is CO-K and K is | | | | |
| K-1 | K-2 | K-3 | K-4 | K-5 |
| K-6 | K-7 | K-8 | K-9 | |

| | | | | |
|---|---|---|---|---|
| See Exhibit 2 for K-1 through K-9. | | | | |

**Table 3**

| 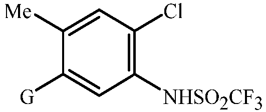 | | | | |
|---|---|---|---|---|
| G is CO-J or CO-K; and J or K is | | | | |
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | K-2 | K-3 | K-4 | K-5 |
| K-6 | K-7 | K-8 | K-9 | K-1 |

| | | | | |
|---|---|---|---|---|
| See Exhibit 1 for J-1 through J-52; and Exhibit 2 for K-1 through K-9. | | | | |

**Table 4**

| 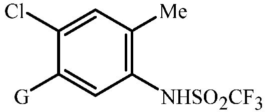 | | | | |
|---|---|---|---|---|
| G is CO-J and J is | | | | |
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | | | | |

| | | | | |
|---|---|---|---|---|
| See Exhibit 1 for J-1 through J-54. | | | | |

**Table 5**

| 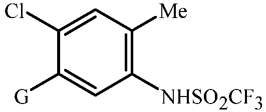 | | | | |
|---|---|---|---|---|
| G is CO-K and K is | | | | |
| K-1 | K-2 | K-3 | K-4 | K-5 |
| K-6 | K-7 | K-8 | K-9 | |

| | | | | |
|---|---|---|---|---|
| See Exhibit 2 for K-1 through K-9. | | | | |

**Table 6**

| 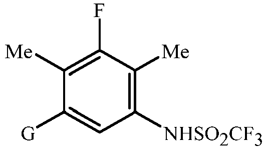 | | | | |
|---|---|---|---|---|
| G is CO-J and J is | | | | |
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | K-2 | K-3 | K-4 | K-5 |
| K-6 | K-7 | K-8 | K-9 | K-1 |

| | | | | |
|---|---|---|---|---|
| See Exhibit 1 for J-1 through J-54; and Exhibit 2 for K-1 through K-9. | | | | |

**Table 7**

| 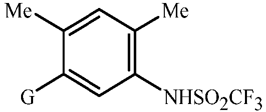 G is | | | | | | |
|---|---|---|---|---|---|---|
| G-1-1 | G-1-1a | G-1-1b | G-1-2 | G-1-3 | G-1-4 | G-1-5 |
| G-1-6 | G-1-7 | G-1-8 | G-1-8a | G-1-8b | G-1-9 | G-1-10 |
| G-1-11 | G-1-12 | G-1-13 | G-1-14 | G-1-15 | G-1-16 | G-1-17 |
| G-1-18 | G-1-19 | G-1-20 | G-1-21 | G-1-22 | G-1-23 | G-1-24 |
| G-1-25 | G-1-26 | G-1-27 | G-1-24a | G-1-24b | G-1-28a* | G-1-28b* |
| G-1-29 | G-1-30 | G-1-31 | G-1-32 | G-1-33 | G-1-33a* | G-1-33b* |
| G-1-34 | G-1-34a* | G-1-34b* | G-1-35 | G-1-35a* | G-1-35b* | G-1-36 |
| G-1-37 | G-1-38 | G-1-38a* | G-1-38b* | G-1-1aa | G-1-lab | G-1-8aa |
| G-1-8ab | | | | | | |

| | | | | | | |
|---|---|---|---|---|---|---|
| See Exhibit 3 for G-1-1 through G-1-27. | | | | | | |

**Table 8**

| 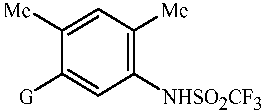 G is | | | | | |
|---|---|---|---|---|---|
| G-2-1 | G-2-2 | G-2-3 | G-2-4 | G-2-5 | G-2-6 |
| G-2-7 | G-2-8 | G-2-9 | G-2-10 | G-2-11 | G-2-12 |
| G-2-13 | G-2-14 | G-2-15 | G-2-16 | G-2-17 | |

| | | | | | |
|---|---|---|---|---|---|
| See Exhibit 4 for G-2-1 through G-2-12. | | | | | |

**Table 9**

| 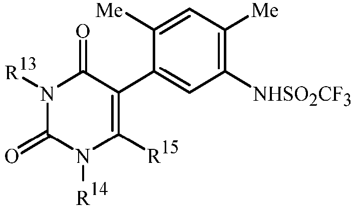 | | | | | | |
|---|---|---|---|---|---|---|
| R¹³ | R¹⁴ | R¹⁵ | | R¹³ | R¹⁴ | R¹⁵ |
| Me | Me | H | | *i*-Pr | Me | H |
| Me | Me | Me | | *i*-Pr | Me | Me |
| Me | Me | Cl | | *i*-Pr | Me | Cl |
| Me | Et | H | | *i*-Pr | Et | H |
| Me | Et | Me | | *i*-Pr | Et | Me |
| Me | Et | Cl | | *i*-Pr | Et | Cl |
| Et | Me | H | | CH₂OCH₃ | Me | H |
| Et | Me | Me | | CH₂OCH₃ | Me | Me |
| Et | Me | Cl | | CH₂OCH₃ | Me | Cl |
| Et | Et | H | | CH₂OCH₃ | Et | H |
| Et | Et | Me | | CH₂OCH₂CH₃ | Et | Me |
| Et | Et | Cl | | CH₂OCH₃ | Et | Cl |

**Table 10**

| 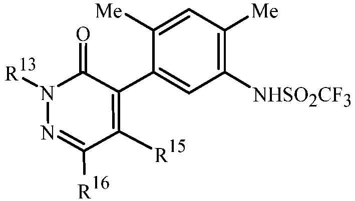 | | | | | | |
|---|---|---|---|---|---|---|
| R¹³ | R¹⁵ | R¹⁶ | | R¹³ | R¹⁵ | R¹⁶ |
| Me | H | H | | *i*-Pr | H | H |
| Me | H | Cl | | *i-* Pr | H | Cl |
| Me | H | Me | | *i*-Pr | H | Me |
| Me | Cl | H | | *i-*Pr | Cl | H |
| Me | Cl | Cl | | *i*-Pr | Cl | Cl |
| Me | Cl | Me | | *i*-Pr | Cl | Me |
| Me | Me | H | | *i*-Pr | Me | H |
| Me | Me | Cl | | *i*-Pr | Me | Cl |
| Me | Me | Me | | *i*-Pr | Me | Me |
| Et | H | H | | *c*-Pr | H | H |
| Et | H | Cl | | *c*-Pr | H | Cl |
| Et | H | Me | | *c*-Pr | H | Me |
| Et | Cl | H | | *c*-Pr | Cl | H |
| Et | Cl | Cl | | *c*-Pr | Cl | Cl |
| Et | Cl | Me | | *c*-Pr | Cl | Me |
| Et | Me | H | | *c*-Pr | Me | H |
| Et | Me | Cl | | *c*-Pr | Me | Cl |
| Et | Me | Me | | *c*-Pr | Me | Me |

**Table 11**

| 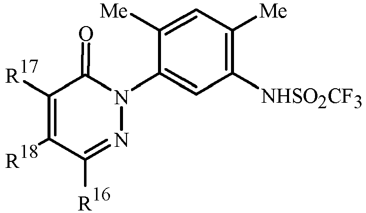 | | |
|---|---|---|
| R¹⁶ | R¹⁷ | R¹⁸ |
| H | Me | H |
| H | Me | Me |
| H | Et | H |

| R¹⁶ | R¹⁷ | R¹⁸ |
|---|---|---|
| H | Et | Me |
| Me | Me | H |
| Me | Me | Me |
| Me | Et | H |
| Me | Et | Me |
| Cl | Me | H |
| Cl | Me | Me |
| Cl | Et | H |
| Cl | Et | Me |

**Table 12**

| 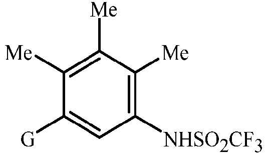 G is CO-J and J is | | | | |
|---|---|---|---|---|
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | | | | |

| | | | | |
|---|---|---|---|---|
| See Exhibit 1 for J-1 through J-54. | | | | |

**Table 13**

| | | | | |
|---|---|---|---|---|
| 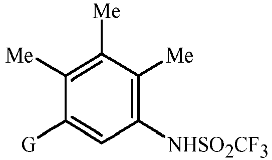 | | | | |
| | | | | |

| G is CO-K and K is | | | | |
|---|---|---|---|---|
| K-1 | K-2 | K-3 | K-4 | K-5 |
| K-6 | K-7 | K-8 | K-9 | |

| | | | | |
|---|---|---|---|---|
| See Exhibit 2 for K-1 through K-9. | | | | |

**Table 14**

| 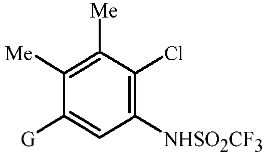 G is CO-J or CO-K; and J or K is | | | | |
|---|---|---|---|---|
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | K-2 | K-3 | K-4 | K-5 |
| K-6 | K-7 | K-8 | K-9 | K-1 |

| | | | | |
|---|---|---|---|---|
| See Exhibit 1 for J-1 through J-54; and Exhibit 2 for K-1 through K-9. | | | | |

**Table 15**

| 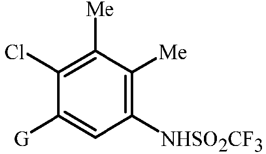 | | | | |
|---|---|---|---|---|
| G is CO-J and J is | | | | |
| J-1 | J-2 | J-3 | J-4 | J-5 |
| J-6 | J-7 | J-8 | J-9 | J-10 |
| J-11 | J-12 | J-13 | J-14 | J-15 |
| J-16 | J-17 | J-18 | J-19 | J-20 |
| J-21 | J-22 | J-23 | J-24 | J-25 |
| J-26 | J-27 | J-28 | J-29 | J-30 |
| J-31 | J-32 | J-33 | J-34 | J-35 |
| J-36 | J-37 | J-38 | J-39 | J-40 |
| J-41 | J-42 | J-43 | J-44 | J-45 |
| J-46 | J-47 | J-48 | J-49 | J-50 |
| J-51 | J-52 | J-53 | J-54 | J-3a* |
| J-3b* | | | | |

| | | | | |
|---|---|---|---|---|
| See Exhibit 1 for J-1 through J-54 | | | | |

**Table 16**

| 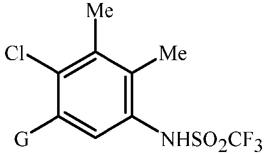 | | | | |
|---|---|---|---|---|
| G is CO-K and K is | | | | |
| K-1 | K-2 | K-3 | K-4 | K-5 |
| K-6 | K-7 | K-8 | K-9 | |

| | | | | |
|---|---|---|---|---|
| See Exhibit 2 for K-1 through K-9. | | | | |

**Table 17**

| 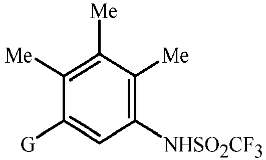 | | | | | | |
|---|---|---|---|---|---|---|
| G is | | | | | | |
| G-1-1 | G-1-1a | G-1-1b | G-1-2 | G-1-3 | G-1-4 | G-1-5 |
| G-1-6 | G-1-7 | G-1-8 | G-1-8a | G-1-8b | G-1-9 | G-1-10 |
| G-1-11 | G-1-12 | G-1-13 | G-1-14 | G-1-15 | G-1-16 | G-1-17 |
| G-1-18 | G-1-19 | G-1-20 | G-1-21 | G-1-22 | G-1-23 | G-1-24 |
| G-1-25 | G-1-26 | G-1-27 | G-1-24a | G-1-24b | G-1-28a* | G-1-28b* |
| G-1-29 | G-1-30 | G-1-31 | G-1-32 | G-1-33 | G-1-33a* | G-1-33b* |
| G-1-34 | G-1-34a* | G-1-34b* | G-1-35 | G-1-35a* | G-1-35b* | G-1-36 |
| G-1-37 | G-1-38 | G-1-38a* | G-1-38b* | G-1-1aa | G-1-lab | G-1-8aa |
| G-1-8ab | | | | | | |

| | | | | | | |
|---|---|---|---|---|---|---|
| See Exhibit 3 for G-1-1 through G-1-27. | | | | | | |

**Table 18**

| 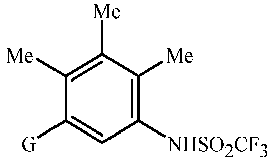 | | | | | |
|---|---|---|---|---|---|
| G is | | | | | |
| G-2-1 | G-2-2 | G-2-3 | G-2-4 | G-2-5 | G-2-6 |
| G-2-7 | G-2-8 | G-2-9 | G-2-10 | G-2-11 | G-2-12 |
| G-2-13 | G-2-14 | G-2-15 | G-2-16 | G-2-17 | |

| | | | | | |
|---|---|---|---|---|---|
| See Exhibit 4 for G-2-1 through G-2-12. | | | | | |

**Table 19**

| 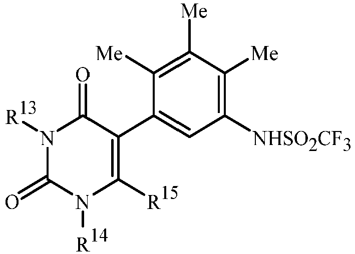 | | | | | | |
|---|---|---|---|---|---|---|
| R¹³ | R¹⁴ | R¹⁵ | | R¹³ | R¹⁴ | R¹⁵ |
| Me | Me | H | | *i*- Pr | Me | H |
| Me | Me | Me | | *i*- Pr | Me | Me |
| Me | Me | Cl | | *i*- Pr | Me | Cl |
| Me | Et | H | | *i*- Pr | Et | H |
| Me | Et | Me | | *i*-Pr | Et | Me |
| Me | Et | Cl | | *i*-Pr | Et | Cl |
| Et | Me | H | | CH₂OCH₃ | Me | H |
| Et | Me | Me | | CH₂OCH₃ | Me | Me |
| Et | Me | Cl | | CH₂OCH₃ | Me | Cl |
| Et | Et | H | | CH₂OCH₃ | Et | H |
| Et | Et | Me | | CH₂OCH₂CH₃ | Et | Me |
| Et | Et | Cl | | CH₂OCH₃ | Et | Cl |

**Table 20**

| 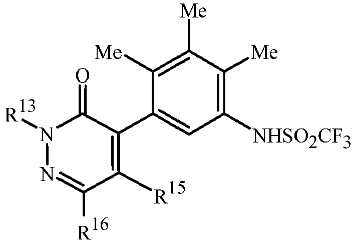 | | | | | | |
|---|---|---|---|---|---|---|
| R¹³ | R¹⁵ | R¹⁶ | | R¹³ | R¹⁵ | R¹⁶ |
| Me | H | H | | *i*-Pr | H | H |
| Me | H | Cl | | *i-* Pr | H | Cl |
| Me | H | Me | | *i*-Pr | H | Me |
| Me | Cl | H | | *i-* Pr | Cl | H |
| Me | Cl | Cl | | *i*-Pr | Cl | Cl |
| Me | Cl | Me | | *i*-Pr | Cl | Me |
| Me | Me | H | | *i*-Pr | Me | H |
| Me | Me | Cl | | *i*-Pr | Me | Cl |
| Me | Me | Me | | *i*-Pr | Me | Me |
| Et | H | H | | *c*-Pr | H | H |
| Et | H | Cl | | *c*-Pr | H | Cl |
| Et | H | Me | | *c*-Pr | H | Me |
| Et | Cl | H | | *c*-Pr | Cl | H |
| Et | Cl | Cl | | *c*-Pr | Cl | Cl |
| Et | Cl | Me | | *c*-Pr | Cl | Me |
| Et | Me | H | | *c*-Pr | Me | H |
| Et | Me | Cl | | *c*-Pr | Me | Cl |
| Et | Me | Me | | *c*-Pr | Me | Me |

**Table 21**

| | | | | | | |
|---|---|---|---|---|---|---|
| 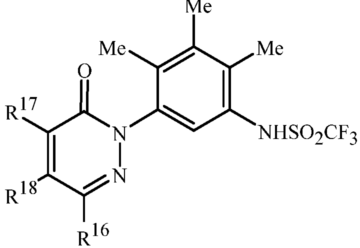 | | | | | | |
| | | | | | | |

| R¹⁶ | R¹⁷ | R¹⁸ | | R¹⁶ | R¹⁷ | R¹⁸ |
|---|---|---|---|---|---|---|
| H | Me | H | | Me | Et | H |
| H | Me | Me | | Me | Et | Me |
| H | Et | H | | Cl | Me | H |
| H | Et | Me | | Cl | Me | Me |
| Me | Me | H | | Cl | Et | H |
| Me | Me | Me | | Cl | Et | Me |

A compound of this invention will generally be used as a herbicidal active ingredient in a composition, i.e. formulation, with at least one additional component selected from the group consisting of surfactants, solid diluents and liquid diluents, which serves as a carrier. The formulation or composition ingredients are selected to be consistent with the physical properties of the active ingredient, mode of application and environmental factors such as soil type, moisture and temperature.

Useful formulations include both liquid and solid compositions. Liquid compositions include solutions (including emulsifiable concentrates), suspensions, emulsions (including microemulsions, oil-in -water emulsions, flowable concentrates and/or suspoemulsions) and the like, which optionally can be thickened into gels. The general types of aqueous liquid compositions are soluble concentrate, suspension concentrate, capsule suspension, concentrated emulsion, microemulsion, oil-in-water emulsion, flowable concentrate and suspo-emulsion. The general types of nonaqueous liquid compositions are emulsifiable concentrate, microemulsifiable concentrate, dispersible concentrate and oil dispersion.

The general types of solid compositions are dusts, powders, granules, pellets, prills, pastilles, tablets, filled films (including seed coatings) and the like, which can be water-dispersible ("wettable") or water-soluble. Films and coatings formed from film-forming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Sprayable formulations are typically extended in a suitable medium before spraying. Such liquid and solid formulations are formulated to be readily diluted in the spray medium, usually water, but occasionally another suitable medium like an aromatic or paraffinic hydrocarbon or vegetable oil. Spray volumes can range from about from about one to several thousand liters per hectare, but more typically are in the range from about ten to several hundred liters per hectare. Sprayable formulations can be tank mixed with water or another suitable medium for foliar treatment by aerial or ground application or for application to the growing medium of the plant. Liquid and dry formulations can be metered directly into drip irrigation systems or metered into the furrow during planting.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-99 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., Handbook of Insecticide Dust Diluents and Carriers, 2nd Ed., Dorland Books, Caldwell, New Jersey.

Liquid diluents include, for example, water, N,N-dimethylalkanamides (e.g., N,N-dimethylformamide), limonene, dimethyl sulfoxide, N-alkylpyrrolidones (e.g., N-methylpyrrolidinone), alkyl phosphates (e.g., triethyl phosphate), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters, alkyl and aryl benzoates and γ-butyrolactone, and alcohols, which can be linear, branched, saturated or unsaturated, such as methanol, ethanol, n-propanol, isopropyl alcohol, n-butanol, isobutyl alcohol, n-hexanol, 2-ethylhexanol, n-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol, cresol and benzyl alcohol. Liquid diluents also include glycerol esters of saturated and unsaturated fatty acids (typically C₆-C₂₂), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides.

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N,N-*alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as N-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquaternary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this invention may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic thickeners), in-container microbial growth (antimicrobials), product freezing (antifreezes), color (dyes/pigment dispersions), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon's Volume 2: Functional Materials, annual International and North American editions published by McCutcheon's Division, The Manufacturing Confectioner Publishing Co.; and PCT Publication WO 03/024222.

The compound of Formula 1 and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 µm can be wet milled using media mills to obtain particles with average diameters below 3 µm. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 µm range. Dusts and powders can be prepared by blending and usually grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Compound numbers refer to compounds in Index Tables A-G. Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except where otherwise indicated.

### Example A

### High Strength Concentrate

| | |
|---|---|
| Compound 260 | 98.5% |
| silica aerogel | 0.5% |
| synthetic amorphous fine silica | 1.0% |

### Example B

### Wettable Powder

| | |
|---|---|
| Compound 260 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

### Example C

### Granule

| | |
|---|---|
| Compound 260 | 10.0% |
| attapulgite granules (low volatile matter, 0.71/0.30 mm; U.S.S. No. 25-50 sieves) | 90.0% |

### Example D

### Extruded Pellet

| | |
|---|---|
| Compound 260 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

### Example E

### Emulsifiable Concentrate

| | |
|---|---|
| Compound 260 | 10.0% |
| polyoxyethylene sorbitol hexoleate | 20.0% |
| C₆-C₁₀ fatty acid methyl ester | 70.0% |

### Example F

### Microemulsion

| | |
|---|---|
| Compound 260 | 5.0% |
| polyvinylpyrrolidone-vinyl acetate copolymer | 30.0% |
| alkylpolyglycoside | 30.0% |
| glyceryl monooleate | 15.0% |
| water | 20.0% |

### Example G

### Suspension Concentrate

| | |
|---|---|
| Compound 260 | 35% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| water | 53.7% |

### Example H

### Emulsion in Water

| | |
|---|---|
| Compound 260 | 10.0% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| aromatic petroleum based hydrocarbon | 20.0 |
| water | 58.7% |

### Example I

### Oil Dispersion

| | |
|---|---|
| Compound 260 | 25% |
| polyoxyethylene sorbitol hexaoleate | 15% |
| organically modified bentonite clay | 2.5% |
| fatty acid methyl ester | 57.5% |

Additinonal Example Formulations include Examples A through I above wherein "Compound 260" is replaced in each of the Examples A through I with the respective compounds from Index Table A as shown below.

| Compound No. | Compound No. |
|---|---|
| Compound 16 | Compound 197 |
| Compound 6 | Compound 121 |
| Compound 18 | Compound 120 |
| Compound 128 | Compound 267 |

| Compound No. | Compound No. |
|---|---|
| Compound 190 | Compound 140 |
| Compound 207 | Compound 159 |
| Compound 103 | Compound 100 |
| Compound 268 | Compound 324 |
| Compound 330 | Compound 329 |
| Compound 289 | Compound 336 |
| Compound 331 | Compound 325 |

Test results indicate that the compounds of the present invention are highly active preemergent and/or postemergent herbicides and/or plant growth regulants. The compounds of the disclosure generally show highest activity for postemergence weed control (i.e. applied after weed seedlings emerge from the soil) and preemergence weed control (i.e. applied before weed seedlings emerge from the soil). Many of them have utility for broad-spectrum pre- and/or postemergence weed control in areas where complete control of all vegetation is desired such as around fuel storage tanks, industrial storage areas, parking lots, drive-in theaters, air fields, river banks, irrigation and other waterways, around billboards and highway and railroad structures. Many of the compounds of this invention, by virtue of selective metabolism in crops versus weeds or by selective activity at the locus of physiological inhibition in crops and weeds or by selective placement on or within the environment of a mixture of crops and weeds, are useful for the selective control of grass and broadleaf weeds within a crop/weed mixture. One skilled in the art will recognize that the preferred combination of these selectivity factors within a compound or group of compounds can readily be determined by performing routine biological and/or biochemical assays. Compounds of this invention may show tolerance to important agronomic crops including, but is not limited to, alfalfa, barley, cotton, wheat, rape, sugar beets, corn (maize), sorghum, soybeans, rice, oats, peanuts, vegetables, tomato, potato, perennial plantation crops including coffee, cocoa, oil palm, rubber, sugarcane, citrus, grapes, fruit trees, nut trees, banana, plantain, pineapple, hops, tea and forests such as eucalyptus and conifers (e.g., loblolly pine), and turf species (e.g., Kentucky bluegrass, St. Augustine grass, Kentucky fescue and Bermuda grass). Compounds of this invention can be used in crops genetically transformed or bred to incorporate resistance to herbicides, express proteins toxic to invertebrate pests (such as *Bacillus thuringiensis* toxin), and/or express other useful traits. Those skilled in the art will appreciate that not all compounds are equally effective against all weeds. Alternatively, the subject compounds are useful to modify plant growth.

As the compounds of the invention have both preemergent and postemergent herbicidal activity, to control undesired vegetation by killing or injuring the vegetation or reducing its growth, the compounds can be usefully applied by a variety of methods involving contacting a herbicidally effective amount of a compound of the disclosure or a composition comprising said compound and at least one of a surfactant, a solid diluent or a liquid diluent, to the foliage or other part of the undesired vegetation or to the environment of the undesired vegetation such as the soil or water in which the undesired vegetation is growing or which surrounds the seed or other propagule of the undesired vegetation.

A herbicidally effective amount of the compounds of this invention is determined by a number of factors. These factors include: formulation selected, method of application, amount and type of vegetation present, growing conditions, etc. In general, a herbicidally effective amount of compounds of this invention is about 0.001 to 20 kg/ha with a preferred range of about 0.004 to 1 kg/ha. One skilled in the art can easily determine the herbicidally effective amount necessary for the desired level of weed control.

In one common embodiment, a compound of the disclosure is applied, typically in a formulated composition, to a locus comprising desired vegetation (e.g., crops) and undesired vegetation (i.e. weeds), both of which may be seeds, seedlings and/or larger plants, in contact with a growth medium (e.g., soil). In this locus, a composition comprising a compound of the disclosure can be directly applied to a plant or a part thereof, particularly of the undesired vegetation, and/or to the growth medium in contact with the plant.

Plant varieties and cultivars of the desired vegetation in the locus treated with a compound of the disclosure can be obtained by conventional propagation and breeding methods or by genetic engineering methods. Genetically modified plants (transgenic plants) are those in which a heterologous gene (transgene) has been stably integrated into the plant's genome. A transgene that is defined by its particular location in the plant genome is called a transformation or transgenic event.

Genetically modified plant cultivars in the locus which can be treated according to the invention include those that are resistant against one or more biotic stresses (pests such as nematodes, insects, mites, fungi, etc.) or abiotic stresses (drought, cold temperature, soil salinity, etc.) or that contain other desirable characteristics. Plants can be genetically modified to exhibit traits of, for example, herbicide tolerance, insect-resistance, modified oil profiles or drought tolerance.

Although most typically, compounds of the invention are used to control undesired vegetation, contact of desired vegetation in the treated locus with compounds of the invention may result in super-additive or synergistic effects with genetic traits in the desired vegetation, including traits incorporated through genetic modification. For example, resistance to phytophagous insect pests or plant diseases, tolerance to biotic/abiotic stresses or storage stability may be greater than expected from the genetic traits in the desired vegetation.

Compounds of this invention can also be mixed with one or more other biologically active compounds or agents including herbicides, herbicide safeners, fungicides, insecticides, nematocides, bactericides, acaricides, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Mixtures of the compounds of the invention with other herbicides can broaden the spectrum of activity against additional weed species, and suppress the proliferation of any resistant biotypes. Thus the present invention also pertains to a composition comprising a compound of Formula 1 (in a herbicidally effective amount) and at least one additional biologically active compound or agent (in a biologically effective amount) and can further comprise at least one of a surfactant, a solid diluent or a liquid diluent. The other biologically active compounds or agents can be formulated in compositions comprising at least one of a surfactant, solid or liquid diluent. For mixtures of the present invention, one or more other biologically active compounds or agents can be formulated together with a compound of Formula **1,** to form a premix or one or more other biologically active compounds or agents can be formulated separately from the compound of Formula **1,** and the formulations combined together before application (e.g., in a spray tank) or, alternatively, applied in succession.

A mixture of one or more of the following herbicides with a compound of this invention may be particularly useful for weed control: acetochlor, acifluorfen and its sodium salt, aclonifen, acrolein (2-propenal), alachlor, alloxydim, ametryn, amicarbazone, amidosulfuron, aminocyclopyrachlor and its esters (e.g., methyl, ethyl) and salts (e.g., sodium, potassium), aminopyralid, amitrole, ammonium sulfamate, anilofos, asulam, atrazine, azimsulfuron, beflubutamid, benazolin, benazolin-ethyl, bencarbazone, benfluralin, benfuresate, bensulfuron-methyl, bensulide, bentazone, benzobicyclon, benzofenap, bicyclopyrone, bifenox, bilanafos, bispyribac and its sodium salt, bromacil, bromobutide, bromofenoxim, bromoxynil, bromoxynil octanoate, butachlor, butafenacil, butamifos, butralin, butroxydim, butylate, cafenstrole, carbetamide, carfentrazone-ethyl, catechin, chlomethoxyfen, chloramben, chlorbromuron, chlorflurenol-methyl, chloridazon, chlorimuron-ethyl, chlorotoluron, chlorpropham, chlorsulfuron, chlorthal-dimethyl, chlorthiamid, cinidon-ethyl, cinmethylin, cinosulfuron, clacyfos, clefoxydim, clethodim, clodinafop-propargyl, clomazone, clomeprop, clopyralid, clopyralid-olamine, cloransulam-methyl, cumyluron, cyanazine, cycloate, cyclopyrimorate, cyclosulfamuron, cycloxydim, cyhalofop-butyl, 2,4-D and its butotyl, butyl, isoctyl and isopropyl esters and its dimethylammonium, diolamine and trolamine salts, daimuron, dalapon, dalapon-sodium, dazomet, 2,4-DB and its dimethylammonium, potassium and sodium salts, desmedipham, desmetryn, dicamba and its diglycolammonium, dimethylammonium, potassium and sodium salts, dichlobenil, dichlorprop, diclofop-methyl, diclosulam, difenzoquat metilsulfate, diflufenican, diflufenzopyr, dimefuron, dimepiperate, dimethachlor, dimethametryn, dimethenamid, dimethenamid-P, dimethipin, dimethylarsinic acid and its sodium salt, dinitramine, dinoterb, diphenamid, diquat dibromide, dithiopyr, diuron, DNOC, endothal, EPTC, esprocarb, ethalfluralin, ethametsulfuron-methyl, ethiozin, ethofumesate, ethoxyfen, ethoxysulfuron, etobenzanid, fenoxaprop-ethyl, fenoxaprop-P-ethyl, fenoxasulfone, fenquinotrione, fentrazamide, fenuron, fenuron-TCA, flamprop-methyl, flamprop-M-isopropyl, flamprop-M-methyl, flazasulfuron, florasulam, fluazifop-butyl, fluazifop-P-butyl, fluazolate, flucarbazone, flucetosulfuron, fluchloralin, flufenacet, flufenpyr, flufenpyr-ethyl, flumetsulam, flumiclorac-pentyl, flumioxazin, fluometuron, fluoroglycofen-ethyl, flupoxam, flupyrsulfuron-methyl and its sodium salt, flurenol, flurenol-butyl, fluridone, flurochloridone, fluroxypyr, flurtamone, fluthiacet-methyl, fomesafen, foramsulfuron, fosamine-ammonium, glufosinate, glufosinate-ammonium, glufosinate-P, glyphosate and its salts such as ammonium, isopropylammonium, potassium, sodium (including sesquisodium) and trimesium (alternatively named sulfosate), halauxifen, halauxifen-methyl, halosulfuron-methyl, haloxyfop-etotyl, haloxyfop-methyl, hexazinone, hydantocidin, imazamethabenz-methyl, imazamox, imazapic, imazapyr, imazaquin, imazaquin-ammonium, imazethapyr, imazethapyr-ammonium, imazosulfuron, indanofan, indaziflam, iofensulfuron, iodosulfuron-methyl, ioxynil, ioxynil octanoate, ioxynil-sodium, ipfencarbazone, isoproturon, isouron, isoxaben, isoxaflutole, isoxachlortole, lactofen, lenacil, linuron, maleic hydrazide, MCPA and its salts (e.g., MCPA-dimethylammonium, MCPA-potassium and MCPA-sodium, esters (e.g., MCPA-2-ethylhexyl, MCPA-butotyl) and thioesters (e.g., MCPA-thioethyl), MCPB and its salts (e.g., MCPB-sodium) and esters (e.g., MCPB-ethyl), mecoprop, mecoprop-P, mefenacet, mefluidide, mesosulfuron-methyl, mesotrione, metam-sodium, metamifop, metamitron, metazachlor, metazosulfuron, methabenzthiazuron, methylarsonic acid and its calcium, monoammonium, monosodium and disodium salts, methyldymron, metobenzuron, metobromuron, metolachlor, S-metolachlor, metosulam, metoxuron, metribuzin, metsulfuron-methyl, molinate, monolinuron, naproanilide, napropamide, napropamide-M, naptalam, neburon, nicosulfuron, norflurazon orbencarb or thosulfamuron oryzalin, oxadiargyl, oxadiazon, oxasulfuron, oxaziclomefone, oxyfluorfen, paraquat dichloride, pebulate, pelargonic acid, pendimethalin, penoxsulam, pentanochlor, pentoxazone, perfluidone, pethoxamid, pethoxyamid, phenmedipham, picloram, picloram-potassium, picolinafen, pinoxaden, piperophos, pretilachlor, primisulfuron-methyl, prodiamine, profoxydim, prometon, prometryn, propachlor, propanil, propaquizafop, propazine, propham, propisochlor, propoxycarbazone, propyrisulfuron, propyzamide, prosulfocarb, prosulfuron, pyraclonil, pyraflufen-ethyl, pyrasulfotole, pyrazogyl, pyrazolynate, pyrazoxyfen, pyrazosulfuron-ethyl, pyribenzoxim, pyributicarb, pyridate, pyriftalid, pyriminobac-methyl, pyrimisulfan, pyrithiobac, pyrithiobac-sodium, pyroxasulfone, pyroxsulam, quinclorac, quinmerac, quinoclamine, quizalofop-ethyl, quizalofop-P-ethyl, quizalofop-P-tefuryl, rimsulfuron, saflufenacil, sethoxydim, siduron, simazine, simetryn, sulcotrione, sulfentrazone, sulfometuron-methyl, sulfosulfuron, 2,3,6-TBA, TCA, TCA-sodium, tebutam, tebuthiuron, tefuryltrione, tembotrione, tepraloxydim, terbacil, terbumeton, terbuthylazine, terbutryn, thenylchlor, thiazopyr, thiencarbazone, thifensulfuron-methyl, thiobencarb, tiafenacil, tiocarbazil, tolpyralate, topramezone, tralkoxydim, tri-allate, triafamone, triasulfuron, triaziflam, tribenuron-methyl, triclopyr, triclopyr-butotyl, triclopyr-triethylammonium, tridiphane, trietazine, trifloxysulfuron, trifludimoxazin, trifluralin, triflusulfuron-methyl, tritosulfuron, vernolate, 3-(2-chloro-3,6-difluorophenyl)-4-hydroxy-1-methyl-1,5-naphthyridin-2(1*H*)-one, 5-chloro-3-[(2-hydroxy-6-oxo-1-cyclohexen-1-yl)carbonyl]-1-(4-methoxyphenyl)-2(1*H*)-quinoxalinone, 2-chloro-*N*-(1-methyl-1*H*-tetrazol-5-yl)-6-(trifluoromethyl)-3-pyridinecarboxamide, 7-(3,5-dichloro-4-pyridinyl)-5-(2,2-difluoroethyl)-8-hydroxypyrido[2,3-b]pyrazin-6(5*H*)-one), 4-(2,6-diethyl-4-methylphenyl)-5-hydroxy-2,6-dimethyl-3(2*H*)-pyridazinone), 5-[[(2,6-difluorophenyl)methoxy]methyl]-4,5-dihydro-5-methyl-3-(3-methyl-2-thienyl)isoxazole (previously methioxolin), 4-(4-fluorophenyl)-6-[(2-hydroxy-6-oxo-1-cyclohexen-1-yl)carbonyl]-2-methyl-1,2,4-triazine-3,5(2*H*,4*H*)-dione, methyl 4-amino-3-chloro-6-(4-chloro-2-fluoro-3-methoxyphenyl)-5-fluoro-2-pyridinecarboxylate, 2-methyl-3-(methylsulfonyl)-*N*-(1-methyl-1*H*-tetrazol-5-yl)-4-(trifluoromethyl)benzamide and 2-methyl-*N*-(4-methyl-1,2,5-oxadiazol-3-yl)-3-(methylsulfinyl)-4-(trifluoromethyl)benzamide. Other herbicides also include bioherbicides such as *Alternaria destruens* Simmons, *Colletotrichum gloeosporiodes* (Penz.) Penz. & Sacc., *Drechsiera monoceras* (MTB-951), *Myrothecium verrucaria* (Albertini & Schweinitz) Ditmar: Fries, *Phytophthora palmivora* (Butl.) Butl. and *Puccinia thlaspeos* Schub.

Compounds of this invention can also be used in combination with plant growth regulators such as aviglycine, *N*-(phenylmethyl)-1*H*-purin-6-amine, epocholeone, gibberellic acid, gibberellin A₄ and A₇, harpin protein, mepiquat chloride, prohexadione calcium, prohydrojasmon, sodium nitrophenolate and trinexapac-methyl, and plant growth modifying organisms such as *Bacillus cereus* strain BP01.

General references for agricultural protectants (i.e. herbicides, herbicide safeners, insecticides, fungicides, nematocides, acaricides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

For embodiments where one or more of these various mixing partners are used, the mixing partners are typically used in the amounts similar to amounts customary when the mixture partners are used alone. More particularly in mixtures, active ingredients are often applied at an application rate between one-half and the full application rate specified on product labels for use of active ingredient alone. These amounts are listed in references such as *The Pesticide Manual* and *The BioPesticide Manual.* The weight ratio of these various mixing partners (in total) to the compound of Formula **1** is typically between about 1:3000 and about 3000:1. Of note are weight ratios between about 1:300 and about 300:1 (for example ratios between about 1:30 and about 30:1). One skilled in the art can easily determine through simple experimentation the biologically effective amounts of active ingredients necessary for the desired spectrum of biological activity. It will be evident that including these additional components may expand the spectrum of weeds controlled beyond the spectrum controlled by the compound of Formula 1 alone.

In certain instances, combinations of a compound of this invention with other biologically active (particularly herbicidal) compounds or agents (i.e. active ingredients) can result in a greater-than-additive (i.e. synergistic) effect on weeds and/or a less-than-additive effect (i.e. safening) on crops or other desirable plants. Reducing the quantity of active ingredients released in the environment while ensuring effective pest control is always desirable. Ability to use greater amounts of active ingredients to provide more effective weed control without excessive crop injury is also desirable. When synergism of herbicidal active ingredients occurs on weeds at application rates giving agronomically satisfactory levels of weed control, such combinations can be advantageous for reducing crop production cost and decreasing environmental load. When safening of herbicidal active ingredients occurs on crops, such combinations can be advantageous for increasing crop protection by reducing weed competition.

Of note is a combination of a compound of the disclosure with at least one other herbicidal active ingredient. Of particular note is such a combination where the other herbicidal active ingredient has different site of action from the compound of the invention. In certain instances, a combination with at least one other herbicidal active ingredient having a similar spectrum of control but a different site of action will be particularly advantageous for resistance management. Thus, a composition of the present invention can further comprise (in a herbicidally effective amount) at least one additional herbicidal active ingredient having a similar spectrum of control but a different site of action.

Compounds of this invention can also be used in combination with herbicide safeners such as allidochlor, benoxacor, cloquintocet-mexyl, cumyluron, cyometrinil, cyprosulfonamide, daimuron, dichlormid, dicyclonon, dietholate, dimepiperate, fenchlorazole-ethyl, fenclorim, flurazole, fluxofenim, furilazole, isoxadifen-ethyl, mefenpyr-diethyl, mephenate, methoxyphenone naphthalic anhydride (1,8-naphthalic anhydride), oxabetrinil, *N*-(aminocarbonyl)-2-methylbenzenesulfonamide, *N*-(aminocarbonyl)-2-fluorobenzenesulfonamide, 1-bromo-4-[(chloromethyl)sulfonyl]benzene (BCS), 4-(dichloroacetyl)-1-oxa-4-azospiro[4.5]decane (MON 4660), 2-(dichloromethyl)-2-methyl-1,3-dioxolane (MG 191), ethyl 1,6-dihydro-l-(2-methoxyphenyl)-6-oxo-2-phenyl-5-pyrimidinecarboxylate, 2-hydroxy-*N*,*N*-dimethyl-6-(trifluoromethyl)pyridine-3-carboxamide, and 3-oxo-1-cyclohexen-1-yl 1-(3,4-dimethylphenyl)-1,6-dihydro-6-oxo-2-phenyl-5-pyrimidinecarboxylate, 2,2-dichloro-1-(2,2,5-trimethyl-3-oxazolidinyl)-ethanone and 2-methoxy*-N*-[[4-[[(methylamino)carbonyl]amino]phenyl]sulfonyl]-benzamide to increase safety to certain crops. Antidotally effective amounts of the herbicide safeners can be applied at the same time as the compounds of this invention or applied as seed treatments. Therefore an aspect of the present invention relates to a herbicidal mixture comprising a compound of this invention and an antidotally effective amount of a herbicide safener. Seed treatment is particularly useful for selective weed control, because it physically restricts antidoting to the crop plants. Therefore a particularly useful embodiment of the present invention is a method for selectively controlling the growth of undesired vegetation in a crop comprising contacting the locus of the crop with a herbicidally effective amount of a compound of this invention wherein seed from which the crop is grown is treated with an antidotally effective amount of safener. Antidotally effective amounts of safeners can be easily determined by one skilled in the art through simple experimentation.

Compounds of the invention cans also be mixed with: (1) polynucleotides including but not limited to DNA, RNA, and/or chemically modified nucleotides influencing the amount of a particular target through down regulation, interference, suppression or silencing of the genetically derived transcript that render a herbicidal effect; or (2) polynucleotides including but not limited to DNA, RNA, and/or chemically modified nucleotides influencing the amount of a particular target through down regulation, interference, suppression or silencing of the genetically derived transcript that render a safening effect.

Of note is a composition comprising a compound of the disclosure (in a herbicidally effective amount), at least one additional active ingredient selected from the group consisting of other herbicides and herbicide safeners (in an effective amount), and at least one component selected from the group consisting of surfactants, solid diluents and liquid diluents.

Preferred for better control of undesired vegetation (e.g., lower use rate such as from synergism, broader spectrum of weeds controlled or enhanced crop safety) or for preventing the development of resistant weeds are mixtures of a compound of this invention with a herbicide selected from the group consisting of atrazine, azimsulfuron, beflubutamid, S-beflubutamid, benzisothiazolinone, carfentrazone-ethyl, chlorimuron-ethyl, chlorsulfuron-methyl, clomazone, clopyralid potassium, cloransulam-methyl, 2-[(2,4-dichlorophenyl)methyl]-4,4-dimethyl-3-isoxazolidinone (CA No. 81777-95-9) and 2-[(2,5-dichlorophenyl)methyl]-4,4-dimethyl-3-isoxazolidinone (CA No. 81778-66-7) ethametsulfuron-methyl, flumetsulam, 4-(4-fluorophenyl)-6-[(2-hydroxy-6-oxo-1-cyclohexen-1-yl)carbonyl]-2-methyl-1,2,4-triazine-3,5-(2*H,*4*H*)-dione, flupyrsulfuron-methyl, fluthiacet-methyl, fomesafen, imazethapyr, lenacil, mesotrione, metribuzin, metsulfuron-methyl, pethoxamid, picloram, pyroxasulfone, quinclorac, rimsulfuron, rinskor, S-metolachlor, sulfentrazone, thifensulfuron-methyl, triflusulfuron-methyl and tribenuron-methyl. Table A1 lists specific combinations of a Component (a) with Component (b) illustrative of the mixtures, compositions and methods of the present invention. Compound # in the Component (a) column is identified in Index Table A. The second column of Table A1 lists the specific Component (b) compound (e.g., "2,4-D" in the first line). The third, fourth and fifth columns of Table A1 lists ranges of weight ratios for rates at which the Component (a) compound is typically applied to a field-grown crop relative to Component (b) (i.e. (a):(b)). Thus, for example, the first line of Table A1 specifically discloses the combination of Component (a) (i.e. Compound 45 in Index Table A) with 2,4-D is typically applied in a weight ratio between 1:192 - 6:1. The remaining lines of Table A1 are to be construed similarly.

**TABLE A1**

| Component (a) (Compound #) | Component (b) | Typical Weight Ratio | More Typical Weight Ratio | Most Typical Weight Ratio |
|---|---|---|---|---|
| 260 | 2,4-D | 1:192 - 6:1 | 1:64 - 2:1 | 1:24 - 1:3 |
| 260 | Acetochlor | 1:768 - 2:1 | 1:256 - 1:2 | 1:96 - 1:11 |
| 260 | Acifluorfen | 1:96 - 12:1 | 1:32 - 4:1 | 1:12 - 1:2 |
| 260 | Aclonifen | 1:857 - 2:1 | 1:285 - 1:3 | 1:107 - 1:12 |
| 260 | Alachlor | 1:768 - 2:1 | 1:256 - 1:2 | 1:96 - 1:11 |
| 260 | Ametryn | 1:384 - 3:1 | 1:128 - 1:1 | 1:48 - 1:6 |
| 260 | Amicarbazone | 1:192 - 6:1 | 1:64 - 2:1 | 1:24 - 1:3 |
| 260 | Amidosulfuron | 1:6 - 168:1 | 1:2 - 56:1 | 1:1 - 11:1 |
| 260 | Aminocyclopyrachlor | 1:48 - 24:1 | 1:16 - 8:1 | 1:6 - 2:1 |
| 260 | Aminopyralid | 1:20 - 56:1 | 1:6 - 19:1 | 1:2 - 4:1 |
| 260 | Amitrole | 1:768 - 2:1 | 1:256 - 1:2 | 1:96 - 1:11 |
| 260 | Anilofos | 1:96 - 12:1 | 1:32 - 4:1 | 1:12 - 1:2 |
| 260 | Asulam | 1:960 - 2:1 | 1:320 - 1:3 | 1:120 - 1:14 |
| 260 | Atrazine | 1:192 - 6:1 | 1:64 - 2:1 | 1:24 - 1:3 |
| 260 | Azimsulfuron | 1:6 - 168:1 | 1:2 - 56:1 | 1:1 - 11:1 |
| 260 | Beflubutamid | 1:342 - 4:1 | 1:114 - 2:1 | 1:42 - 1:5 |
| 260 | Benfuresate | 1:617 - 2:1 | 1:205 - 1:2 | 1:77 - 1:9 |
| 260 | Bensulfuron-methyl | 1:25 - 45:1 | 1:8 - 15:1 | 1:3 - 3:1 |
| 260 | Bentazone | 1:192 - 6:1 | 1:64 - 2:1 | 1:24 - 1:3 |
| 260 | Benzobicyclon | 1:85 - 14:1 | 1:28 - 5:1 | 1:10 - 1:2 |
| 260 | Benzofenap | 1:257 - 5:1 | 1:85 - 2:1 | 1:32 - 1:4 |
| 260 | Bicyclopyrone | 1:42 - 27:1 | 1:14 -9:1 | 1:5 - 2:1 |
| 260 | Bifenox | 1:257 - 5:1 | 1:85 - 2:1 | 1:32 - 1:4 |
| 260 | Bispyribac-sodium | 1:10 - 112:1 | 1:3 - 38:1 | 1:1 - 7:1 |
| 260 | Bromacil | 1:384 - 3:1 | 1:128 - 1:1 | 1:48 - 1:6 |
| 260 | Bromobutide | 1:384 - 3:1 | 1:128 - 1:1 | 1:48 - 1:6 |
| 260 | Bromoxynil | 1:96 - 12:1 | 1:32 - 4:1 | 1:12 - 1:2 |
| 260 | Butachlor | 1:768 - 2:1 | 1:256 - 1:2 | 1:96 - 1:11 |
| 260 | Butafenacil | 1:42 - 27:1 | 1:14 - 9:1 | 1:5 - 2:1 |
| 260 | Butylate | 1:1542 - 1:2 | 1:514-1:5 | 1:192 - 1:22 |
| 260 | Carfenstrole | 1:192 - 6:1 | 1:64 - 2:1 | 1:24 - 1:3 |
| 260 | Carfentrazone-ethyl | 1:128 - 9:1 | 1:42 - 3:1 | 1:16 - 1:2 |
| 260 | Chlorimuron-ethyl | 1:8 - 135:1 | 1:2 - 45:1 | 1:1 - 9:1 |
| 260 | Chlorotoluron | 1:768 - 2:1 | 1:256 - 1:2 | 1:96 - 1:11 |
| 260 | Chlorsulfuron | 1:6 - 168:1 | 1:2 - 56:1 | 1:1 - 11:1 |
| 260 | Cincosulfuron | 1:17 - 68:1 | 1:5 - 23:1 | 1:2 - 5:1 |
| 260 | Cinidon-ethyl | 1:384 - 3:1 | 1:128 - 1:1 | 1:48 - 1:6 |
| 260 | Cinmethylin | 1:34 - 34:1 | 1:11 - 12:1 | 1:4 - 3:1 |
| 260 | Clacyfos | 1:34 - 34:1 | 1:11 - 12:1 | 1:4 - 3:1 |
| 260 | Clethodim | 1:48 - 24:1 | 1:16 - 8:1 | 1:6 - 2:1 |
| 260 | Clodinafop-propargyl | 1:20 - 56:1 | 1:6 - 19:1 | 1:2 - 4:1 |
| 260 | Clomazone | 1:384 - 3:1 | 1:128 - 1:1 | 1:48 - 1:6 |
| 260 | Clomeprop | 1:171-7:1 | 1:57 - 3:1 | 1:21-1:3 |
| 260 | Clopyralid | 1:192 - 6:1 | 1:64 - 2:1 | 1:24 - 1:3 |
| 260 | Cloransulam-methyl | 1:12 - 96:1 | 1:4 - 32:1 | 1:1 - 6:1 |
| 260 | Cumyluron | 1:384 - 3:1 | 1:128 - 1:1 | 1:48 - 1:6 |
| 260 | Cyanazine | 1:384 - 3:1 | 1:128 - 1:1 | 1:48 - 1:6 |
| 260 | Cyclopyrimorate | 1:17 - 68:1 | 1:5 - 23:1 | 1:2 - 5:1 |
| 260 | Cyclosulfamuron | 1:17 - 68:1 | 1:5 - 23:1 | 1:2 - 5:1 |
| 260 | Cycloxydim | 1:96 - 12:1 | 1:32 - 4:1 | 1:12 - 1:2 |
| 260 | Cyhalofop | 1:25 - 45:1 | 1:8 - 15:1 | 1:3 - 3:1 |
| 260 | Daimuron | 1:192 - 6:1 | 1:64 - 2:1 | 1:24 - 1:3 |
| 260 | Desmedipham | 1:322 - 4:1 | 1:107 - 2:1 | 1:40 - 1:5 |
| 260 | Dicamba | 1:192 - 6:1 | 1:64 - 2:1 | 1:24 - 1:3 |
| 260 | Dichlobenil | 1:1371 - 1:2 | 1:457 - 1:4 | 1:171 - 1:20 |
| 260 | Dichlorprop | 1:925 - 2:1 | 1:308 - 1:3 | 1:115 - 1:13 |
| 260 | Diclofop-methyl | 1:384 - 3:1 | 1:128 - 1:1 | 1:48 - 1:6 |
| 260 | Diclosulam | 1:10 - 112:1 | 1:3 - 38:1 | 1:1-7:1 |
| 260 | Difenzoquat | 1:288 - 4:1 | 1:96 - 2:1 | 1:36 - 1:4 |
| 260 | Diflufenican | 1:857 - 2:1 | 1:285 - 1:3 | 1:107 - 1:12 |
| 260 | Diflufenzopyr | 1:12 - 96:1 | 1:4 - 32:1 | 1:1 - 6:1 |
| 260 | Dimethachlor | 1:768 - 2:1 | 1:256 - 1:2 | 1:96 - 1:11 |
| 260 | Dimethametryn | 1:192 - 6:1 | 1:64 - 2:1 | 1:24 - 1:3 |
| 260 | Dimethenamid-P | 1:384 - 3:1 | 1:128 - 1:1 | 1:48 - 1:6 |
| 260 | Dithiopyr | 1:192 - 6:1 | 1:64 - 2:1 | 1:24 - 1:3 |
| 260 | Diuron | 1:384 - 3:1 | 1:128 - 1:1 | 1:48 - 1:6 |
| 260 | EPTC | 1:768 - 2:1 | 1:256 - 1:2 | 1:96 - 1:11 |
| 260 | Esprocarb | 1:1371 - 1:2 | 1:457 - 1:4 | 1:171 - 1:20 |
| 260 | Ethalfluralin | 1:384 - 3:1 | 1:128 - 1:1 | 1:48 - 1:6 |
| 260 | Ethametsulfuron-methyl | 1:17 - 68:1 | 1:5 - 23:1 | 1:2 - 5:1 |
| 260 | Ethoxyfen | 1:8 - 135:1 | 1:2 -45:1 | 1:1 - 9:1 |
| 260 | Ethoxysulfuron | 1:20 - 56:1 | 1:6 - 19:1 | 1:2 - 4:1 |
| 260 | Etobenzanid | 1:257 - 5:1 | 1:85 - 2:1 | 1:32 - 1:4 |
| 260 | Fenoxaprop-ethyl | 1:120 - 10:1 | 1:40 - 4:1 | 1:15 - 1:2 |
| 260 | Fenoxasulfone | 1:85 - 14:1 | 1:28 - 5:1 | 1:10 - 1:2 |
| 260 | Fenquinotrione | 1:17 - 68:1 | 1:5 - 23:1 | 1:2 - 5:1 |
| 260 | Fentrazamide | 1:17 - 68:1 | 1:5 - 23:1 | 1:2 - 5:1 |
| 260 | Flazasulfuron | 1:17 - 68:1 | 1:5 - 23:1 | 1:2 - 5:1 |
| 260 | Florasulam | 1:2 - 420:1 | 1:1 - 140:1 | 2:1 - 27:1 |
| 260 | Fluazifop-butyl | 1:192 - 6:1 | 1:64 - 2:1 | 1:24 - 1:3 |
| 260 | Flucarbazone | 1:8 - 135:1 | 1:2 - 45:1 | 1:1 - 9:1 |
| 260 | Flucetosulfuron | 1:8 - 135:1 | 1:2 - 45:1 | 1:1 - 9:1 |
| 260 | Flufenacet | 1:257 - 5:1 | 1:85 - 2:1 | 1:32 - 1:4 |
| 260 | Flumetsulam | 1:24 - 48:1 | 1:8- 16:1 | 1:3 - 3:1 |
| 260 | Flumiclorac-pentyl | 1:10 - 112:1 | 1:3 - 38:1 | 1:1 - 7:1 |
| 260 | Flumioxazin | 1:25 - 45:1 | 1:8 - 15:1 | 1:3 - 3:1 |
| 260 | Fluometuron | 1:384 - 3:1 | 1:128 - 1:1 | 1:48 - 1:6 |
| 260 | Flupyrsulfuron-methyl | 1:3 - 336:1 | 1:1 - 112:1 | 2:1 - 21:1 |
| 260 | Fluridone | 1:384 - 3:1 | 1:128 - 1:1 | 1:48 - 1:6 |
| 260 | Fluroxypyr | 1:96 - 12:1 | 1:32 - 4:1 | 1:12 - 1:2 |
| 260 | Flurtamone | 1:857 - 2:1 | 1:285 - 1:3 | 1:107 - 1:12 |
| 260 | Fluthiacet-methyl | 1:48 - 42:1 | 1:16 - 14:1 | 1:3 - 3:1 |
| 260 | Fomesafen | 1:96 - 12:1 | 1:32 -4:1 | 1:12 - 1:2 |
| 260 | Foramsulfuron | 1:13 - 84:1 | 1:4 - 28:1 | 1:1 - 6:1 |
| 260 | Glufosinate | 1:288 - 4:1 | 1:96 - 2:1 | 1:36 - 1:4 |
| 260 | Glyphosate | 1:288 - 4:1 | 1:96 - 2:1 | 1:36 - 1:4 |
| 260 | Halosulfuron-methyl | 1:17 - 68:1 | 1:5 - 23:1 | 1:2 - 5:1 |
| 260 | Halauxifen | 1:20 - 56:1 | 1:6 - 19:1 | 1:2 - 4:1 |
| 260 | Halauxifen methyl | 1:20 - 56:1 | 1:6 - 19:1 | 1:2 -4:1 |
| 260 | Haloxyfop-methyl | 1:34 - 34:1 | 1:11 - 12:1 | 1:4 - 3:1 |
| 260 | Hexazinone | 1:192 - 6:1 | 1:64 - 2:1 | 1:24 - 1:3 |
| 260 | Hydantocidin | 1:1100 - 16:1 | 1:385 - 8:1 | 1:144 - 4:1 |
| 260 | Imazamox | 1:13 - 84:1 | 1:4 - 28:1 | 1:1 - 6:1 |
| 260 | Imazapic | 1:20 - 56:1 | 1:6 - 19:1 | 1:2 - 4:1 |
| 260 | Imazapyr | 1:85 - 14:1 | 1:28 - 5:1 | 1:10 - 1:2 |
| 260 | Imazaquin | 1:34 - 34:1 | 1:11 - 12:1 | 1:4 - 3:1 |
| 260 | Imazethabenz-methyl | 1:171 -7:1 | 1:57 - 3:1 | 1:21 - 1:3 |
| 260 | Imazethapyr | 1:24 - 48:1 | 1:8- 16:1 | 1:3 - 3:1 |
| 260 | Imazosulfuron | 1:27 - 42:1 | 1:9 - 14:1 | 1:3 - 3:1 |
| 260 | Indanofan | 1:342 - 4:1 | 1:114 - 2:1 | 1:42 - 1:5 |
| 260 | Indaziflam | 1:25 - 45:1 | 1:8 - 15:1 | 1:3 - 3:1 |
| 260 | Iodosulfuron-methyl | 1:3 - 336:1 | 1:1 - 112:1 | 2:1 - 21:1 |
| 260 | Ioxynil | 1:192 - 6:1 | 1:64 - 2:1 | 1:24 - 1:3 |
| 260 | Ipfencarbazone | 1:85 - 14:1 | 1:28 - 5:1 | 1:10 - 1:2 |
| 260 | Isoproturon | 1:384 - 3:1 | 1:128 - 1:1 | 1:48 - 1:6 |
| 260 | Isoxaben | 1:288 - 4:1 | 1:96 - 2:1 | 1:36 - 1:4 |
| 260 | Isoxaflutole | 1:60 - 20:1 | 1:20 - 7:1 | 1:7 -2:1 |
| 260 | Lactofen | 1:42 - 27:1 | 1:14 -9:1 | 1:5 - 2:1 |
| 260 | Lenacil | 1:384 - 3:1 | 1:128 - 1:1 | 1:48 - 1:6 |
| 260 | Linuron | 1:384 - 3:1 | 1:128 - 1:1 | 1:48 - 1:6 |
| 260 | MCPA | 1:192 - 6:1 | 1:64 - 2:1 | 1:24 - 1:3 |
| 260 | MCPB | 1:288 - 4:1 | 1:96 - 2:1 | 1:36 - 1:4 |
| 260 | Mecoprop | 1:768 - 2:1 | 1:256 - 1:2 | 1:96 - 1:11 |
| 260 | Mefenacet | 1:384 - 3:1 | 1:128 - 1:1 | 1:48 - 1:6 |
| 260 | Mefluidide | 1:192 - 6:1 | 1:64 - 2:1 | 1:24 - 1:3 |
| 260 | Mesosulfuron-methyl | 1:5 - 224:1 | 1:1 - 75:1 | 1:1 - 14:1 |
| 260 | Mesotrione | 1:42 - 27:1 | 1:14 -9:1 | 1:5 - 2:1 |
| 260 | Metamifop | 1:42 - 27:1 | 1:14 -9:1 | 1:5 - 2:1 |
| 260 | Metazachlor | 1:384 - 3:1 | 1:128 - 1:1 | 1:48 - 1:6 |
| 260 | Metazosulfuron | 1:25 - 45:1 | 1:8 - 15:1 | 1:3 - 3:1 |
| 260 | Methabenzthiazuron | 1:768 - 2:1 | 1:256 - 1:2 | 1:96 - 1:11 |
| 260 | Metolachlor | 1:768 - 2:1 | 1:256 - 1:2 | 1:96 - 1:11 |
| 260 | Metosulam | 1:8 - 135:1 | 1:2 - 45:1 | 1:1 - 9:1 |
| 260 | Metribuzin | 1:192 - 6:1 | 1:64 - 2:1 | 1:24 - 1:3 |
| 260 | Metsulfuron-methyl | 1:2 - 560:1 | 1:1 - 187:1 | 3:1 - 35:1 |
| 260 | Molinate | 1:1028 - 2:1 | 1:342 - 1:3 | 1:128 - 1:15 |
| 260 | Napropamide | 1:384 - 3:1 | 1:128 - 1:1 | 1:48 - 1:6 |
| 260 | Napropamide-M | 1:192 - 6:1 | 1:64 - 2:1 | 1:24 - 1:3 |
| 260 | Naptalam | 1:192 - 6:1 | 1:64 - 2:1 | 1:24 - 1:3 |
| 260 | Nicosulfuron | 1:12 - 96:1 | 1:4 - 32:1 | 1:1 - 6:1 |
| 260 | Norflurazon | 1:1152 - 1:1 | 1:384 - 1:3 | 1:144 - 1:16 |
| 260 | Orbencarb | 1:1371 - 1:2 | 1:457 - 1:4 | 1:171 - 1:20 |
| 260 | Orthosulfamuron | 1:20 - 56:1 | 1:6 - 19:1 | 1:2 - 4:1 |
| 260 | Oryzalin | 1:514 - 3:1 | 1:171 - 1:2 | 1:64 - 1:8 |
| 260 | Oxadiargyl | 1:384 - 3:1 | 1:128 - 1:1 | 1:48 - 1:6 |
| 260 | Oxadiazon | 1:548 - 3:1 | 1:182 - 1:2 | 1:68 - 1:8 |
| 260 | Oxasulfuron | 1:27 - 42:1 | 1:9 - 14:1 | 1:3 - 3:1 |
| 260 | Oxaziclomefone | 1:42 - 27:1 | 1:14 -9:1 | 1:5 - 2:1 |
| 260 | Oxyfluorfen | 1:384 - 3:1 | 1:128 - 1:1 | 1:48 - 1:6 |
| 260 | Paraquat | 1:192 - 6:1 | 1:64 - 2:1 | 1:24 - 1:3 |
| 260 | Pendimethalin | 1:384 - 3:1 | 1:128 - 1:1 | 1:48 - 1:6 |
| 260 | Penoxsulam | 1:10 - 112:1 | 1:3 - 38:1 | 1:1-7:1 |
| 260 | Penthoxamid | 1:384 - 3:1 | 1:128 - 1:1 | 1:48 - 1:6 |
| 260 | Pentoxazone | 1:102 - 12:1 | 1:34 - 4:1 | 1:12 - 1:2 |
| 260 | Phenmedipham | 1:102 - 12:1 | 1:34 - 4:1 | 1:12 - 1:2 |
| 260 | Picloram | 1:96 - 12:1 | 1:32 - 4:1 | 1:12 - 1:2 |
| 260 | Picolinafen | 1:34 - 34:1 | 1:11 - 12:1 | 1:4 - 3:1 |
| 260 | Pinoxaden | 1:25 - 45:1 | 1:8 - 15:1 | 1:3 - 3:1 |
| 260 | Pretilachlor | 1:192 - 6:1 | 1:64 - 2:1 | 1:24 - 1:3 |
| 260 | Primisulfuron-methyl | 1:8 - 135:1 | 1:2 - 45:1 | 1:1 -9:1 |
| 260 | Prodiamine | 1:384 - 3:1 | 1:128 - 1:1 | 1:48 - 1:6 |
| 260 | Profoxydim | 1:42 - 27:1 | 1:14 -9:1 | 1:5 - 2:1 |
| 260 | Prometryn | 1:384 - 3:1 | 1:128 - 1:1 | 1:48 - 1:6 |
| 260 | Propachlor | 1:1152 - 1:1 | 1:384 - 1:3 | 1:144 - 1:16 |
| 260 | Propanil | 1:384 - 3:1 | 1:128 - 1:1 | 1:48 - 1:6 |
| 260 | Propaquizafop | 1:48 - 24:1 | 1:16 - 8:1 | 1:6 - 2:1 |
| 260 | Propoxycarbazone | 1:17 - 68:1 | 1:5 - 23:1 | 1:2 - 5:1 |
| 260 | Propyrisulfuron | 1:17 - 68:1 | 1:5 - 23:1 | 1:2 - 5:1 |
| 260 | Propyzamide | 1:384 - 3:1 | 1:128 - 1:1 | 1:48 - 1:6 |
| 260 | Prosulfocarb | 1:1200 - 1:2 | 1:400 - 1:4 | 1:150 - 1:17 |
| 260 | Prosulfuron | 1:6 - 168:1 | 1:2 - 56:1 | 1:1 - 11:1 |
| 260 | Pyraclonil | 1:42 - 27:1 | 1:14 -9:1 | 1:5 - 2:1 |
| 260 | Pyraflufen-ethyl | 1:5 - 224:1 | 1:1 - 75:1 | 1:1 - 14:1 |
| 260 | Pyrasulfotole | 1:13 - 84:1 | 1:4 - 28:1 | 1:1 - 6:1 |
| 260 | Pyrazolynate | 1:857 - 2:1 | 1:285 - 1:3 | 1:107 - 1:12 |
| 260 | Pyrazosulfuron-ethyl | 1:10 - 112:1 | 1:3 - 38:1 | 1:1-7:1 |
| 260 | Pyrazoxyfen | 1:5 - 224:1 | 1:1 - 75:1 | 1:1 - 14:1 |
| 260 | Pyribenzoxim | 1:10 - 112:1 | 1:3 - 38:1 | 1:1-7:1 |
| 260 | Pyributicarb | 1:384 - 3:1 | 1:128 - 1:1 | 1:48 - 1:6 |
| 260 | Pyridate | 1:288 - 4:1 | 1:96 - 2:1 | 1:36 - 1:4 |
| 260 | Pyriftalid | 1:10 - 112:1 | 1:3 - 38:1 | 1:1-7:1 |
| 260 | Pyriminobac-methyl | 1:20 - 56:1 | 1:6 - 19:1 | 1:2 -4:1 |
| 260 | Pyrimisulfan | 1:17 - 68:1 | 1:5 - 23:1 | 1:2 - 5:1 |
| 260 | Pyrithiobac | 1:24 - 48:1 | 1:8- 16:1 | 1:3 - 3:1 |
| 260 | Pyroxasulfone | 1:85 - 14:1 | 1:28 - 5:1 | 1:10 - 1:2 |
| 260 | Pyroxsulam | 1:5 - 224:1 | 1:1 - 75:1 | 1:1 - 14:1 |
| 260 | Quinclorac | 1:192 - 6:1 | 1:64 - 2:1 | 1:24 - 1:3 |
| 260 | Quizalofop-ethyl | 1:42 - 27:1 | 1:14 -9:1 | 1:5 - 2:1 |
| 260 | Rimsulfuron | 1:13 - 84:1 | 1:4 - 28:1 | 1:1 - 6:1 |
| 260 | Saflufenacil | 1:25 - 45:1 | 1:8 - 15:1 | 1:3 - 3:1 |
| 260 | Sethoxydim | 1:96 - 12:1 | 1:32 - 4:1 | 1:12 - 1:2 |
| 260 | Simazine | 1:384 - 3:1 | 1:128 - 1:1 | 1:48 - 1:6 |
| 260 | Sulcotrione | 1:120 - 10:1 | 1:40 - 4:1 | 1:15 - 1:2 |
| 260 | Sulfentrazone | 1:147 - 8:1 | 1:49 - 3:1 | 1:18 - 1:3 |
| 260 | Sulfometuron-methyl | 1:34 - 34:1 | 1:11 - 12:1 | 1:4 - 3:1 |
| 260 | Sulfosulfuron | 1:8 - 135:1 | 1:2 - 45:1 | 1:1 - 9:1 |
| 260 | Tebuthiuron | 1:384 - 3:1 | 1:128 - 1:1 | 1:48 - 1:6 |
| 260 | Tefuryltrione | 1:42 - 27:1 | 1:14 - 9:1 | 1:5 - 2:1 |
| 260 | Tembotrione | 1:31 - 37:1 | 1:10 - 13:1 | 1:3 - 3:1 |
| 260 | Tepraloxydim | 1:25 - 45:1 | 1:8 - 15:1 | 1:3 - 3:1 |
| 260 | Terbacil | 1:288 - 4:1 | 1:96 - 2:1 | 1:36 - 1:4 |
| 260 | Terbuthylazine | 1:857 - 2:1 | 1:285 - 1:3 | 1:107 - 1:12 |
| 260 | Terbutryn | 1:192 - 6:1 | 1:64 - 2:1 | 1:24 - 1:3 |
| 260 | Thenylchlor | 1:85 - 14:1 | 1:28 - 5:1 | 1:10 - 1:2 |
| 260 | Thiazopyr | 1:384 - 3:1 | 1:128 - 1:1 | 1:48 - 1:6 |
| 260 | Thiencarbazone | 1:3 - 336:1 | 1:1 - 112:1 | 2: 1- 21:1 |
| 260 | Thifensulfuron-methyl | 1:5 - 224:1 | 1:1 - 75:1 | 1:1 - 14:1 |
| 260 | Tiafenacil | 1:17 - 68:1 | 1:5 - 23:1 | 1:2 - 5:1 |
| 260 | Thiobencarb | 1:768 - 2:1 | 1:256 - 1:2 | 1:96 - 1:11 |
| 260 | Tolpyralate | 1:31 - 37:1 | 1:10 - 13:1 | 1:3 - 3:1 |
| 260 | Topramzone | 1:6 - 168:1 | 1:2 - 56:1 | 1:1 - 11:1 |
| 260 | Tralkoxydim | 1:68 - 17:1 | 1:22 - 6:1 | 1:8 - 2:1 |
| 260 | Triafamone | 1:2 - 420:1 | 1:1 - 140:1 | 2:1 - 27:1 |
| 260 | Triallate | 1:768 - 2:1 | 1:256 - 1:2 | 1:96 - 1:11 |
| 260 | Triasulfuron | 1:5 - 224:1 | 1:1 - 75:1 | 1:1 - 14:1 |
| 260 | Triaziflam | 1:171 -7:1 | 1:57 - 3:1 | 1:21 - 1:3 |
| 260 | Tribenuron-methyl | 1:3 - 336:1 | 1:1 - 112:1 | 2:1 - 21:1 |
| 260 | Triclopyr | 1:192 - 6:1 | 1:64 - 2:1 | 1:24 - 1:3 |
| 260 | Trifloxysulfuron | 1:2 - 420:1 | 1:1 - 140:1 | 2:1 - 27:1 |
| 260 | Trifludimoxazin | 1:25 - 45:1 | 1:8 - 15:1 | 1:3 - 3:1 |
| 260 | Trifluralin | 1:288 - 4:1 | 1:96 - 2:1 | 1:36 - 1:4 |
| 260 | Triflusulfuron-methyl | 1:17 - 68:1 | 1:5 - 23:1 | 1:2 - 5:1 |
| 260 | Tritosulfuron | 1:13 - 84:1 | 1:4 - 28:1 | 1:1 - 6:1 |

Table A2 is constructed the same as Table A1 above except that entries below the "Component (a)(compound #)" column heading are replaced with the respective Component (a) Column Entry shown below. Compound 16 in the Component (a) column is identified in Index Table A. Thus, for example, in Table A2 the entries below the "Component (a)" column heading all recite "Compound 16" (i.e. Compound 16 identified in Index Table A), and the first line below the column headings in Table A2 specifically discloses a mixture of Compound 16 with 2,4-D. Tables A3 through A16 are constructed similarly.

| Table Number | Component (a) Column Entries | Table Number | Component (a) Column Entries |
|---|---|---|---|
| A2 | 16 | A9 | 268 |
| A3 | 6 | A10 | 197 |
| A4 | 18 | A11 | 121 |
| A5 | 128 | A12 | 120 |
| A6 | 190 | A13 | 267 |
| A7 | 207 | A14 | 140 |
| A8 | 103 | A15 | 159 |

| Table Number | Component (a) Column Entries | | |
|---|---|---|---|
| A16 | 100 | | |

The following Tests demonstrate the control efficacy of the compounds of this invention against specific weeds. The weed control afforded by the compounds is not limited, however, to these species. See Index Tables A-G for compound descriptions. The following abbreviations are used in the Index Tables which follow: *t* is tertiary, *s* is secondary, *n* is normal, *i* is iso, *c* is cyclo, Me is methyl, Et is ethyl, Pr is propyl, *i*-Pr is isopropyl, Bu is butyl, *c*-Pr is cyclopropyl, *t*-Bu is *tert-*butyl*,* Ph is phenyl, OMe is methoxy, OEt is ethoxy, SMe is methylthio, SEt is ethylthio, -CN is cyano, -NO₂ is nitro, TMS is trimethylsilyl, and naphthyl means naphthalenyl. *(R)* or *(S)* denotes the absolute chirality of the asymmetric carbon center. The abbreviation "(d)" indicates that the compound appeared to decompose on melting. The abbreviation "Cmpd. #" stands for "Compound Number". The abbreviation "Ex." stands for "Example" and is followed by a number indicating in which example the compound is prepared. Mass spectra are reported with an estimated precision within ±0.5 Da as the molecular weight of the highest isotopic abundance parent ion (M+1) formed by addition of H⁺ (molecular weight of 1) to the molecule observed by using atmospheric pressure chemical ionization (AP+).

**INDEX TABLE A**

| 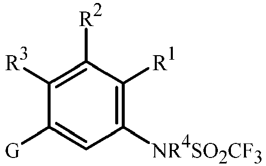 | | | | | | | |
|---|---|---|---|---|---|---|---|
| G is CONR⁵R⁶; and NR⁵R⁶ is J or K. | | | | | | | |
| Cmpd # | R¹ | R² | R³ | R⁴ | J or K@ | M.S. | M.P. (°C) |
| 1 | Cl | H | CH₃ | H | J-1 | 401 | |
| 2 | Cl | CH₃ | H | H | J-2 | 415 | |
| 3 | CH₃ | H | CH₃ | H | J-7 | 411 | |
| 4 | CF₃ | H | H | SO₂CF₃ | J-6 | 497 | |
| 5 | CH₃ | H | CH₃ | SO₂CF₃ | J-33 | 515 | |
| 6 | CH₃ | H | CH₃ | H | J-4 | 367 | |
| 7 | CH₃ | H | H | H | J-15 | | 182-186 |
| 8 | CH₃ | H | CH₃ | H | J-12 | 383 | |
| 9 | CH₃ | H | CH₃ | H | J-3 | 395 | |
| 10 | CH₃ | H | CH₃ | H | J-28 | 380 | |
| 11 | CH₃ | H | CH₃ | H | J-10 | 390 | |
| 12 | CH₃ | H | CH₃ | H | J-33 | 383 | |
| 13 | *c*-propyl | H | CH₃ | H | J-4 | 393 | |
| 14 | Br | H | CH₃ | H | J-4 | 432 | |
| 15 | Cl | H | H | H | J-4 | 373 | |
| 16 | Cl | H | CH₃ | H | J-4 | 387 | |
| 17 | Cl | H | CH₃ | H | J-10 | 410 | |
| 18 | Cl | H | CH₃ | H | J-6 | 385 | |
| 19 | CH₃ | H | CH₃ | H | J-9 | 395 | |
| 20 | Cl | H | CH₃ | H | J-3 | 415 | |
| 21 | Cl | H | CH₃ | H | K-2 | 373 | |
| 22 | Cl | H | CH₃ | H | K-11 | 373 | |
| 23 | Cl | H | CH₃ | H | K-1 | 345 | |
| 24 | Cl | H | CH₃ | H | J-4 | 519 | |
| 25 | Cl | H | CH₃ | H | J-6 | 517 | |
| 26 | Cl | H | CH₃ | H | J-33 | 403 | |
| 50 | CH₃ | H | CH₃ | H | J-46 | 423 | |
| 86 | CH₃ | H | CH₃ | H | J-8 (*S*) | 395 | |
| 87 | CH₃ | H | CH₃ | H | J-35 | 541 | |
| 88 | CH₃ | F | CH₃ | H | J-35 | 545 | |
| 89 | CH₃ | H | CH₃ | SO₂CF₃ | J-32 | 499 | |
| 90 | CH₃ | H | CH₃ | SO₂CF₃ | J-7 | 543 | |
| 91 | CH₃ | H | CH₃ | C(O)-*i*-propyl- | J-8 | 465 | |
| 92 | CH₃ | H | CH₃ | CH₂OCO-*i*-propyl | J-8 | 509 | |
| 93 | CH₃ | H | CH₃ | COCH₃ | J-8 | 437 | |
| 94 | CH₃ | H | CH₃ | H | J-8 (*R*) | 395 | |
| 95 | CH₃ | H | CH₃ | SO₂CF₃ | J-8 (*R*) | 527 | |
| 96 | CH₃ | H | CH₃ | CH₂OCOCH₃ | J-8 | 467 | |
| 97 | CH₃ | H | CH₃ | SO₂CF₃ | K-12 | 482 | |
| 98 | CH₃ | F | CH₃ | H | J-8 | 413 | |
| 101 | CH₃ | H | CH₃ | H | J-47 | 425 | |
| 105 | Cl | H | CH₃ | H | K-12 | 369 | |
| 107 | CH₃ | H | CH₃ | H | K-7 | 365 | |
| 109 | CH₃ | F | CH₃ | H | J-12 | | 250-254 |
| 110 | CH₃ | F | CH₃ | H | J-9 | | 194-198 |
| 111 | CH₃ | H | CH₃ | H | J-32 | 367 | |
| 112 | CH₃ | H | CH₃ | H | J-36 | 381 | |
| 113 | CH₃ | H | CH₃ | H | J-43 | 381 | |
| 115 | CH₃ | H | CH₃ | COOMe | J-8 | 453 | |
| 116 | CH₃ | F | CH₃ | H | J-3 | | 175-179 |
| 117 | CH₃ | H | CH₃ | SO₂CF₃ | J-8(*S*) | 527 | |
| 118 | CH₃ | H | CH₃ | SO₂CF₃ | J-14 | 515 | |
| 119 | CH₃ | H | CH₃ | SO₂CF₃ | J-8 | 527 | |
| 124 | CN | H | CH₃ | H | J-6 | 379 | |
| 126 | CH₃ | H | CN | H | J-6 | | 346-349 |
| 128 | CH₃ | F | CH₃ | H | K-1 | | 243-245 |
| 129 | CH₃ | F | CH₃ | H | J-41 | | 218-222 |
| 130 | Cl | H | CH₃ | SO₂CF₃ | K-12 | 501 | |
| 132 | CF₃ | H | CH₃ | H | K-1 | | 202-204 |
| 133 | CH₃ | H | CF₃ | H | K-1 | | 206-211 |
| 134 | CH₃ | H | CN | H | K-1 | | 230-233 |
| 137 | CH₃ | H | CH₃ | H | J-35 | 409 | |
| 138 | CH₃ | H | CH₃ | SO₂CF₃ | J-43 | 513 | |
| 141 | CH₃ | H | CH₃ | H | K-13 | 351 | |
| 142 | CH₃ | H | CH₃ | SO₂CF₃ | K-13 | 483 | |
| 145 | CH₃ | H | CH₃ | H | K-12 | 350 | |
| 146 | Cl | H | CH₃ | H | J-8 | 415 | |
| 147 | CH₃ | F | CH₃ | SO₂CF₃ | K-5 | 499 | |
| 153 | Cl | H | CH₃ | H | J-5 | 415 | |
| 154 | CH₃ | H | CH₃ | H | J-25 | 409 | |
| 155 | CH₃ | H | CH₃ | H | J-14 | 383 | |
| 160 | CH₃ | H | CH₃ | H | J-34 | 381 | |
| 161 | CH₃ | H | CH₃ | SO₂CF₃ | J-10 | 521 | |
| 162 | CH₃ | H | CH₃ | H | J-10 | 389 | |
| 165 | CF₃ | H | CH₃ | H | J-6 | | 105-108 |
| 169 | CH₃ | H | CH₃ | SO₂CF₃ | K-7 | 497 | |
| 174 | CH₃ | F | CH₃ | SO₂CF₃ | J-4 | 517 | |
| 175 | CH₃ | H | CH₃ | H | J-8 | 395 | |
| 179 | CH₃ | F | CH₃ | H | K-5 | 367 | |
| 180 | CH₃ | H | CF₃ | H | J-6 | | 211-214 |
| 181 | CH₃ | H | CH₃ | SO₂CF₃ | K-6 | 505 | |
| 182 | CH₃ | H | CH₃ | SO₂CF₃ | K-14 | 467 | |
| 187 | CH₃ | H | CH₃ | H | K-6 | 373 | |
| 188 | CH₃ | H | CH₃ | H | K-2 | 353 | |
| 189 | CH₃ | H | CH₃ | H | K-5 | 349 | |
| 190 | CH₃ | F | CH₃ | H | J-4 | | 236-240 |
| 191 | CH₃ | F | CH₃ | H | J-6 | | 263-267 |
| 192 | CH₃ | H | CH₃ | H | K-14 | 335 | |
| 193 | Cl | H | CH₃ | H | J-41 | 443 | |
| 194 | CH₃ | H | CH₃ | H | J-41 | 437 | |
| 195 | CH₃ | H | CH₃ | H | J-37 | 433 | |
| 199 | Cl | H | CH₃ | H | J-48 | 429 | |
| 200 | Cl | H | CH₃ | SO₂CF₃ | J-13 | 551 | |
| 201 | Cl | H | CH₃ | H | J-12 | 403 | |
| 202 | CH₃ | H | CH₃ | H | J-49 | 387 | |
| 205 | Cl | H | CH₃ | SO₂CF₃ | J-41 | 575 | |
| 210 | CH₃ | H | CH₃ | SO₂CF₃ | J-23 | 533 | |
| 211 | CH₃ | H | F | H | J-6 | 369 | |
| 212 | CH₃ | H | F | SO₂CF₃ | J-6 | 501 | |
| 213 | CH₃ | H | F | H | J-4 | 387 | |
| 214 | CH₃ | H | F | SO₂CF₃ | J-4 | 519 | |
| 215 | CH₃ | H | F | H | K-1 | 329 | |
| 216 | CH₃ | H | F | SO₂CF₃ | K-1 | 461 | |
| 223 | CH₃ | H | CH₃ | H | J-38 | 419 | |
| 224 | Cl | H | CH₃ | H | J-13 | 419 | |
| 225 | CH₃ | H | CH₃ | H | J-39 | 405 | |
| 229 | Cl | H | CH₃ | H | J-23 | 421 | |
| 230 | CH₃ | H | CH₃ | H | J-48 | 409 | |
| 231 | CH₃ | H | CH₃ | H | J-41 | 423 | |
| 232 | CH₃ | H | CH₃ | SO₂CF₃ | J-41 | 555 | |
| 233 | CH₃ | H | CH₃ | H | J-20 | 443 | |
| 234 | CH₃ | H | CH₃ | H | J-17 | 448 | |
| 235 | CH₃ | H | F | SO₂CF₃ | J-3 | 531 | |
| 236 | CH₃ | H | F | H | J-10 | 394 | |
| 237 | CH₃ | H | F | SO₂CF₃ | J-4 | 503 | |
| 238 | CH₃ | H | F | H | J-3 | 399 | |
| 239 | CH₃ | H | F | SO₂CF₃ | J-10 | 526 | |
| 240 | CH₃ | H | F | H | J-4 | 371 | |
| 241 | CH₃ | H | CH₃ | H | J-23 | 401 | |
| 242 | CH₃ | H | CH₃ | H | J-13 | 399 | |
| 243 | CH₃ | H | CH₃ | SO₂CF₃ | J-13 | 531 | |
| 244 | F | F | CH₃ | SO₂CF₃ | J-4 | 389 | |
| 245 | Cl | H | CH₃ | H | J-50 | 401 | |
| .246 | Cl | H | CH₃ | H | J-9 | 415 | |
| 247 | CH₃ | H | Cl | SO₂CF₃ | K-11 | 505 | |
| 248 | CH₃ | H | Cl | H | J-4 | 387 | |
| 249 | CH₃ | H | Cl | H | K-15 | 373 | |
| 250 | CH₃ | H | Cl | SO₂CF₃ | J-4 | 519 | |
| 251 | CH₃ | H | Cl | H | K-1 | 345 | |
| 252 | CH₃ | H | Cl | H | J-6 | 387 | |
| 253 | CH₃ | H | Cl | SO₂CF₃ | J-6 | 517 | |
| 254 | CH₃ | H | Cl | H | J-4 | 403 | |
| 255 | CH₃ | H | Cl | SO₂CF₃ | J-4 | 535 | |
| 256 | CH₃ | H | Cl | SO₂CF₃ | K-1 | 477 | |
| 257 | CH₃ | H | Cl | H | K-11 | 373 | |
| 258 | Cl | H | OMe | H | J-4 | | 212-216 |
| 259 | CH₃ | H | H | H | J-6 | | 213-217 |
| 260 | CH₃ | H | CH₃ | H | J-6 | | 262-266 |
| 261 | CH₃ | H | CH₃ | H | J-51 | | 244-247 |
| 262 | CH₃ | H | H | H | J-51 | | 173-177 |
| 263 | CH₃ | H | H | H | J-16 | | 187-191 |
| 264 | CH₃ | H | CH₃ | H | J-15 | | 228-232 |
| 276 | CH₃ | H | CH₃ | SO₂CF₃ | J-34 | 513 | |
| 283 | CH₃ | CH₃ | CH₃ | H | J-33 | 397 | |
| 284 | CH₃ | CH₃ | CH₃ | H | J-53 | 429 | |
| 285 | CH₃ | CH₃ | CH₃ | CH₂OCO-*t*-Bu | J-33 | 511 | |
| 286 | CH₃ | CH₃ | CH₃ | CO₂Et | J-33 | 469 | |
| 287 | CH₃ | Cl | CH₃ | H | K-1 | | 287-290 |
| 288 | CH₃ | CH₃ | CH₃ | H | K-1 | | 259-262 |
| 289 | CH₃ | CH₃ | CH₃ | H | J-4 | | 247-250 |
| 290 | CH₃ | Cl | CH₃ | H | J-4 | | 213-216 |
| 320 | Cl | F | CH₃ | H | K-1 | | 200-203 |
| 321 | Cl | F | CH₃ | H | J-4 | | 266-269 |
| 324 | CH₃ | CH₃ | CH₃ | CH₂OCO-*t*-Bu | J-4 | 495 | |
| 325 | CH₃ | CH₃ | CH₃ | CO₂Et | J-4 | 453 | |
| 326 | CH₃ | CH₃ | CH₃ | H | J-6 | 379 | |
| 327 | CH₃ | CH₃ | CH₃ | H | J-3a | 409 | |
| 328 | CH₃ | CH₃ | CH₃ | H | J-3b | 409 | |
| 329 | CH₃ | CH₃ | CH₃ | CH₂OCO-*t*-Bu | J-6 | 493 | |
| 330 | CH₃ | CH₃ | CH₃ | CO₂Et | J-6 | 451 | |
| 331 | CH₃ | CH₃ | CH₃ | CH₂OCO-*t*-Bu | J-3a | 523 | |
| 332 | CH₃ | CH₃ | CH₃ | CO₂Et | J-3a | 481 | |
| 333 | Cl | F | CH₃ | H | J-6 | | 315-318 |
| 334 | Cl | F | CH₃ | H | J-8 | | 72-75 |
| 337 | Cl | F | CH₃ | H | K-5 | | 189-192 |
| 350 | CH₃ | H | CH₃ | CH₂OCO-*t*-Bu | J-23 | 515 | |
| 351 | CH₃ | H | CH₃ | CO₂Et | J-23 | 473 | |
| 353 | CH₃ | H | CH₃ | CH₂OCO-*t*-Bu | J-15 | 493 | |
| 354 | CH₃ | H | CH₃ | CO₂Et | J-15 | 451 | |
| 356 | CH₃ | CH₃ | CH₃ | H | J-13 | 413 | |
| 357 | CH₃ | CH₃ | CH₃ | H | J-23 | 415 | |
| 358 | CH₃ | CH₃ | CH₃ | H | J-12 | 397 | |
| 359 | CH₃ | CH₃ | CH₃ | CH₂OCO-*t*-Bu | J-13 | 527 | |
| 360 | CH₃ | CH₃ | CH₃ | CH₂OCO-*t*-Bu | J-23 | 529 | |
| 361 | CH₃ | CH₃ | CH₃ | CH₂OCO-*t*-Bu | J-12 | 511 | |
| 362 | CH₃ | H | CH₃ | CH₂OCO-*t*-Bu | J-4 | 481 | |
| 363 | CH₃ | H | CH₃ | CO₂Et | J-4 | 439 | |
| 364 | CH₃ | H | CH₃ | CH₂OCO-*t*-Bu | J-54 | 555 | |
| 365 | CH₃ | H | CH₃ | CO₂Et | J-54 | 513 | |
| 366 | CH₃ | CH₃ | CH₃ | H | J-15 | 393 | |
| 367 | CH₃ | CH₃ | CH₃ | H | J-9 | 409 | |
| 368 | CH₃ | CH₃ | CH₃ | H | K-5 | 363 | |
| 369 | CH₃ | CH₃ | CH₃ | CH₂OCO-*t*-Bu | J-15 | 507 | |
| 370 | CH₃ | CH₃ | CH₃ | CH₂OCO-*t*-Bu | J-9 | 523 | |
| 371 | CH₃ | CH₃ | CH₃ | CH₂OCO-*t*-Bu | K-5 | 477 | |
| 373 | CH₃ | CH₃ | CH₃ | CH₂OCO*t*-Bu | J-3b | 523 | |
| 374 | CH₃ | CH₃ | CH₃ | CO₂Et | J-3b | 481 | |

| | | | | | | | |
|---|---|---|---|---|---|---|---|
| @See Exhibits 1 and 2 for J-1 through J-52 and K-1 through K-9 | | | | | | | |

**INDEX TABLE B**

| | | | | | | | |
|---|---|---|---|---|---|---|---|
| 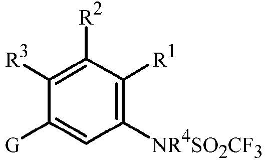 | | | | | | | |

| Cmpd # | R¹ | R² | R³ | R⁴ | G-1 | M.S. | M.P. (°C) |
|---|---|---|---|---|---|---|---|
| 99 | CH₃ | H | CH₃ | SO₂CF₃ | G-1-1 | | 121-125 |
| 100 | CH₃ | H | CH₃ | CH₂OCO-*t*-Bu | G-1-1a | 477 | |
| 102 | CH₃ | H | CH₃ | CH₂OCOMe | G-1-1a | 435 | |
| 103 | CH₃ | H | CH₃ | H | G-1-1a | 363 | |
| 104 | CH₃ | H | CH₃ | H | G-1-2 | 411 | |
| 108 | CH₃ | H | CH₃ | H | G-1-3 | | 79-82 |
| 114 | CH₃ | H | CH₃ | SO₂CF₃ | G-1-4 | 541 | |
| 120 | CH₃ | H | CH₃ | H | G-1-5 | 379 | |
| 121 | CH₃ | H | CH₃ | H | G-1-6 | | 117-120 |
| 122 | CH₃ | H | CH₃ | H | G-1-7 | | 170-173 |
| 123 | CH₃ | H | CH₃ | H | G-1-8aa | 377 | |
| 125 | CH₃ | H | CH₃ | H | G-1-8a | | 158-161 |
| 127 | CH₃ | H | CH₃ | SO₂CF₃ | G-1-3 | | 511 |
| 131 | CH₃ | H | CH₃ | H | G-1-8 | | 166-169 |
| 135 | CH₃ | H | CH₃ | H | G-1-9 | 449 | |
| 136 | CH₃ | H | CH₃ | SO₂CF₃ | G-1-9 | 581 | |
| 139 | CH₃ | H | CH₃ | SO₂CF₃ | G-1-10 | 551 | |
| 140 | CH₃ | H | CH₃ | CH₂OCO-*t*-butyl | G-1-11 | 491 | |
| 143 | CH₃ | H | CH₃ | CH₂OCO-*n*-butyl | G-1-11 | 491 | |
| 144 | CH₃ | H | CH₃ | CH₂OCOEt | G-1-11 | 463 | |
| 148 | CH₃ | H | CH₃ | CH₂OCO-*c*-hexyl | G-1-11 | 517 | |
| 149 | CH₃ | H | CH₃ | CH₂OCOPh | G-1-11 | 511 | |
| 150 | CH₃ | H | CH₃ | CH₂OCO-*c*-pentyl | G-1-11 | 503 | |
| 151 | CH₃ | H | CH₃ | CH₂OCO-*sec*-butyl | G-1-11 | 491 | |
| 152 | CH₃ | H | CH₃ | CH₂OCO-*i*-butyl | G-1-11 | 491 | |
| 158 | CH₃ | H | CH₃ | SO₂CF₃ | G-1-12 | 537 | |
| 159 | CH₃ | H | CH₃ | CH₂OCO-*t*-butyl | G-1-13 | 505 | |
| 163 | CH₃ | H | CH₃ | H | G-1-14 | 419 | |
| 164 | CH₃ | H | CH₃ | H | G-1-1ab | | 105-108 |
| 166 | CH₃ | H | CH₃ | H | G-1-1aa | | 109-111 |
| 167* | CH₃ | H | CH₃ | H | G-1-15 | | 140-143 |
| 168* | CH₃ | H | CH₃ | H | G-1-15 | | 164-167 |
| 170 | CH₃ | H | CH₃ | H | G-1-15 | 406 | |
| 171* | CH₃ | H | CH₃ | H | G-1-17* | | 136-140 |
| 172 | CH₃ | H | CH₃ | H | G-1-19 | | 148-152 |
| 173* | CH₃ | H | CH₃ | H | G-1-17* | | 168-171 |
| 176 | CH₃ | H | CH₃ | H | G-1-4 | 409 | |
| 177 | CH₃ | H | CH₃ | H | G-1-18 | 393 | |
| 178 | CH₃ | H | CH₃ | SO₂CF₃ | G-1-18 | 525 | |
| 183 | CH₃ | H | Cl | H | G-1-20 | 371 | |
| 184 | CH₃ | H | Cl | SO₂CF₃ | G-1-20 | 504 | |
| 185 | CH₃ | H | Cl | H | G-1-11 | 397 | |
| 186 | CH₃ | H | Cl | SO₂CF₃ | G-1-11 | 530 | |
| 196 | CH₃ | H | CH₃ | SO₂CF₃ | G-1-21 | 495 | |
| 197 | CH₃ | H | CH₃ | H | G-1-13 | 391 | |
| 198 | CH₃ | H | CH₃ | SO₂CF₃ | G-1-13 | 523 | |
| 203 | Cl | H | CH₃ | H | G-1-20 | 371 | |
| 204 | Cl | H | CH₃ | H | G-1-11 | 397 | |
| 206 | CH₃ | H | CH₃ | H | G-1-20 | 351 | |
| 207 | CH₃ | H | CH₃ | H | G-1-11 | 377 | |
| 208 | CH₃ | H | CH₃ | SO₂CF₃ | G-1-11 | 509 | |
| 209 | CH₃ | H | CH₃ | H | G-1-22 | 379 | |
| 217 | Cl | H | CH₃ | SO₂CF₃ | G-1-13 | 543 | |
| 218 | CH₃ | H | CH₃ | CH₂OCOMe | G-1-11 | 449 | |
| 219 | CH₃ | H | CH₃ | COSMe | G-1-11 | 451 | |
| 220 | CH₃ | H | CH₃ | COMe | G-1-11 | 419 | |
| 221 | Cl | H | CH₃ | H | G-1-13 | 411 | |
| 222 | Cl | H | CH₃ | SO₂CF₃ | G-1-20 | 503 | |
| 226 | CH₃ | H | CH₃ | H | G-1-23 | 385 | |
| 227 | CH₃ | H | CH₃ | SO₂CF₃ | G-1-23 | 517 | |
| 228 | CH₃ | H | CH₃ | H | G-1-21 | 363 | |
| 277 | CH₃ | H | CH₃ | H | G-1-12 | 405 | |
| 291 | CH₃ | H | CH₃ | SO₂CF₃ | G-1-28a | 523 | |
| 292 | CH₃ | H | CH₃ | H | G-1-28a | 391 | |
| 293 | CH₃ | H | CH₃ | CH₂OCO-*t*-Bu | G-1-28a** | 491 | |
| 294 | CH₃ | H | CH₃ | CH₂OCO-*t*-Bu | G-1-28a** | 505 | |
| 295 | CH₃ | H | CH₃ | CO₂-*i*-Pr | G-1-1a | 449 | |
| 296 | CH₃ | H | CH₃ | SO₂CF₃ | G-1-24a | 497 | |
| 297 | CH₃ | H | CH₃ | H | G-1-24a | 365 | |
| 298 | CH₃ | H | CH₃ | CO₂Et | G-1-1a | 365 | |
| 299 | CH₃ | H | CH₃ | CO₂-*i*-Bu | G-1-1a | 463 | |
| 300 | CH₃ | H | CH₃ | CO₂-*n*-Pr | G-1-1a | 463 | |
| 301 | CH₃ | H | CH₃ | CH₂OCO-*t*-Bu | G-1-24a | 479 | |
| 302 | CH₃ | H | Br | H | G-1-1a | | 160-163 |
| 303 | F | H | CH₃ | H | G-1-11 | | 115-118 |
| 304 | OMe | H | CH₃ | H | G-1-11 | 393 | |
| 305 | CH₃ | H | Et | H | G-1-1a | | 146-149 |
| 306 | CH₃ | H | CF₃ | H | G-1-1a | | 162v165 |
| 307 | CH₃ | H | c-Pr | H | G-1-1a | | 165-168 |
| 308 | CH₃ | H | CH₃ | CH₂OCO-*i*-Pr | G-1-1a | 463 | |
| 309 | Br | H | CH₃ | H | G-1-11 | | 150-153 |
| 310 | CH₃ | H | F | H | G-1-1a | | 135-138 |
| 311 | Et | H | CH₃ | H | G-1-11 | | 152-155 |
| 312 | CH₃ | H | OMe | H | G-1-1a | | 178-181 |
| 313 | c-Pr | H | CH₃ | H | G-1-11 | 403 | |
| 314 | CH₃ | H | CH₃ | CH₂OCOO-*i*-Pr | G-1-1a | 479 | |
| 315 | CH₃ | H | CH₃ | CH₂OCOOMe | G-1-1a | 451 | |
| 316 | CH₃ | H | CH₃ | CH₂OCOO-*t*-Bu | G-1-1a | 493 | |
| 317 | CH₃ | H | CH₃ | CH₂OCONMe₂ | G-1-1a | 464 | |
| 318 | CH₃ | H | CH₃ | H | G-1-29 | 367 | |
| 319 | CH₃ | H | CH₃ | H | G-1-30 | 367 | |
| 322 | CH₃ | H | CH₃ | H | G-1-31 | 395 | |
| 323 | CH₃ | H | CH₃ | H | G-1-32 | 395 | |
| 335 | CH₃ | CH₃ | CH₃ | H | G-1-1a | 377 | |
| 336 | CH₃ | CH₃ | CH₃ | CH₂OCO-*t*-Bu | G-1-1a | 491 | |
| 338 | CH₃ | H | CH₃ | CH₂OCO-*t*-Bu | G-1-30 | 481 | |
| 339 | CH₃ | H | CH₃ | CH₂OCO-*t*-Bu | G-1-29 | 481 | |
| 340 | CH₃ | H | CH₃ | H | G-1-38a | 377 | |
| 341 | CH₃ | H | CH₃ | CH₂OCO-*t*-Bu | G-1-38a | 491 | |
| 342 | CH₃ | H | CH₃ | CH₂OCOO-*t*-Bu | G-1-38a | 493 | |
| 343 | CH₃ | H | CH₃ | H | G-1-33a | 379 | |
| 344 | CH₃ | H | CH₃ | CH₂OCO-*t*-Bu | G-1-33a | 493 | |
| 345 | CH₃ | H | CH₃ | CO₂-*i*-Bu | G-1-33a | 479 | |
| 346 | CH₃ | H | CH₃ | H | G-1-33b | 379 | |
| 347 | CH₃ | H | CH₃ | CH₂OCO-*t*-Bu | G-1-33b | 493 | |
| 348 | CH₃ | H | CH₃ | CO₂-*i*-Bu | G-1-33b | 479 | |
| 349 | CH₃ | H | CH₃ | H | G-1-34a | 365 | |
| 352 | CH₃ | H | CH₃ | CH₂OCO-*t*-Bu | G-1-34a | 479 | |
| 355 | CH₃ | H | c-Pr | CH₂OCO-*t*-Bu | G-1-1a | 503 | |
| 372 | CH₃ | H | CH₃ | H | G-1-35a | 379 | |
| 375 | CH₃ | H | CH₃ | H | G-1-36 | 351 | |
| 376 | CH₃ | H | CH₃ | CH₂OCO-*t*-Bu | G-1-36 | 465 | |
| 377 | CH₃ | H | H | H | G-1-1a | 349 | |
| 378 | CH₃ | H | Et | CH₂OCO-*t*-Bu | G-1-1a | 491 | |
| 379 | CH₃ | H | Et | CO₂Et | G-1-1a | 449 | |
| 380 | CH₃ | CH₃ | CH₃ | H | G-1-11 | 391 | |
| 381 | CH₃ | H | CH₃ | H | G-1-37 | 365 | |
| 382 | CH₃ | CH₃ | CH₃ | CH₂OCO-*t*-Bu | G-1-11 | 505 | |
| 383 | CH₃ | H | CH₃ | CH₂OCO-*t*-Bu | G-1-37 | 479 | |
| 384 | CH₃ | H | n-Pr | H | G-1-1a | 391 | |
| 385 | CH₃ | H | n-Pr | CH₂OCO-*t*-Bu | G-1-1a | 505 | |
| 386 | CH₃ | CH₃ | CH₃ | CH₂OCO-*t*-Bu | G-1-33b | 507 | |
| 387 | CH₃ | CH₃ | CH₃ | H | G-1-24a | 379 | |
| 388 | CH₃ | CH₃ | CH₃ | H | G-1-34a | 379 | |
| 389 | CH₃ | CH₃ | CH₃ | CH₂OCO-*t*-Bu | G-1-24a | 493 | |
| 390 | CH₃ | CH₃ | CH₃ | CH₂OCO-*t*-Bu | G-1-34a | 493 | |
| 391 | CH₃ | CH₃ | CH₃ | CH₂OCO-*t*-Bu | G-1-33a | 507 | |

| | | | | | | | |
|---|---|---|---|---|---|---|---|
| See Exhibit 3 for G-1-1 through G-1-27. * indicates that the compound is either a *trans-isomer* or a *cis*-isomer concerning the two methyl groups on the six-membered ring. ** indicates the compounds are enantiomers. | | | | | | | |

**INDEX TABLE C**

| | | | | | | | |
|---|---|---|---|---|---|---|---|
| 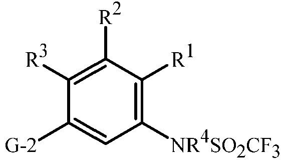 | | | | | | | |

| Cmpd # | R¹ | R² | R³ | R⁴ | G-2 | M.S. | |
|---|---|---|---|---|---|---|---|
| 156 | CH₃ | H | CH₃ | H | G-2-1 | 409 | |
| 157 | CH₃ | H | CH₃ | SO₂CF₃ | G-2-1 | ** | |
| 265 | CH₃ | H | CH₃ | COMe | G-2-2 | 447 | |
| 266 | CH₃ | H | CH₃ | COOMe | G-2-2 | 463 | |
| 267 | CH₃ | H | CH₃ | SO₂CF₃ | G-2-2 | 537 | |
| 268 | CH₃ | H | CH₃ | CH₂OCO-*t*-butyl | G-2-2 | 519 | |
| 269 | CH₃ | H | CH₃ | SO₂CF₃ | G-2-3 | 539 | |
| 270 | CH₃ | H | CH₃ | H | G-24 | 391 | |
| 273 | Cl | H | CH₃ | H | G-24 | 411 | |
| 274 | CH₃ | H | CH₃ | H | G-2-5 | 393 | |
| 275 | CH₃ | H | CH₃ | SO₂CF₃ | G-2-5 | ** | |
| 278 | CH₃ | H | CH₃ | H | G-2-2 | 405 | |
| 279 | CH₃ | H | CH₃ | H | G-2-3 | 407 | |
| 280 | CH₃ | H | CH₃ | SO₂CF₃ | G-24 | 523 | |
| 392 | CH₃ | H | CH₃ | H | G-2-13 | 405 | |
| 393 | Cl | H | CH₃ | H | G-2-2 | 425 | |
| 394 | Cl | H | CH₃ | H | G-2-3 | 427 | |
| 395 | CH₃ | H | CH₃ | SO₂CF₃ | G-2-14 | 573 | |
| 396 | CH₃ | H | CH₃ | H | G-2-14 | 441 | |
| 397 | CH₃ | H | CH₃ | CH₂OCO-*t*-Bu | G-2-14 | 555 | |
| 398 | CH₃ | H | CH₃ | H | G-2-15 | | 212-215 |
| 399 | CH₃ | H | CH₃ | SO₂CF₃ | G-2-16 | 605 | |
| 400 | CH₃ | H | CH₃ | H | G-2-16 | 473 | |
| 401 | CH₃ | H | CH₃ | CH₂OCO-*t*-Bu | G-2-17 | | 103-106 |
| 402 | CH₃ | H | CH₃ | H | G-2-17 | | 235-238 |
| 403 | CH₃ | H | CH₃ | CH₂OCO-*t*-Bu | G-2-16 | 587 | |

| | | | | | | | |
|---|---|---|---|---|---|---|---|
| ** See Index Table G for ¹H NMR data. See Exhibit 4 for G-2-1 through G-2-12. | | | | | | | |

**INDEX TABLE D**

| | | | | | | | | |
|---|---|---|---|---|---|---|---|---|
| 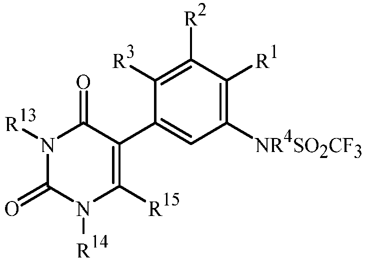 | | | | | | | | |

| Cmpd # | R¹ | R² | R³ | R⁴ | R¹³ | R¹⁴ | R¹⁵ | M.S. |
|---|---|---|---|---|---|---|---|---|
| 29 | Cl | H | CH₃ | H | CH₃ | CH₃ | H | 412 |
| 30 | CH₃ | H | CH₃ | SO₂CF₃ | CH₃ | Et | H | 538 |
| 32 | CH₃ | H | CH₃ | H | CH₃ | Et | H | 406 |
| 37 | CH₃ | H | CH₃ | H | Et | CH₃ | H | 406 |
| 38 | Cl | H | CH₃ | SO₂CF₃ | CH₃ | CH₃ | H | 545 |
| 42 | CH₃ | H | CH₃ | SO₂CF₃ | CH₃ | CH₃ | H | 524 |
| 43 | CH₃ | H | CH₃ | SO₂CF₃ | Et | Et | H | 552 |
| 44 | CH₃ | H | CH₃ | H | Et | Et | H | 420 |
| 49 | CH₃ | H | CH₃ | H | CH₃ | CH₃ | H | 392 |

**INDEX TABLE E**

| | | | | | | | | | |
|---|---|---|---|---|---|---|---|---|---|
| 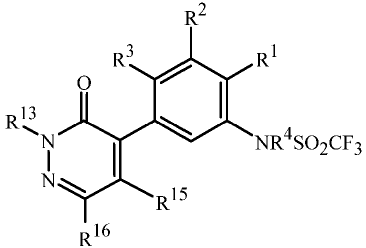 | | | | | | | | | |

| Cmpd # | R¹ | R² | R³ | R⁴ | R¹³ | R¹⁵ | R¹⁶ | M.S. | M.P. (°C) |
|---|---|---|---|---|---|---|---|---|---|
| 27 | CH₃ | H | CH₃ | H | CH₃ | H | CN | | 220-223 |
| 28 | CH₃ | H | CH₃ | SO₂CF₃ | CH₃ | H | Et | 522 | |
| 31 | CH₃ | H | CH₃ | H | CH₃ | H | F | | 188-191 |
| 34 | CH₃ | H | CH₃ | H | CH₃ | H | Et | 390 | |
| 35 | CH₃ | H | CH₃ | SO₂CF₃ | CH₃ | H | OMe | 524 | |
| 36 | CH₃ | H | CH₃ | H | CH₃ | H | SMe | | 212-215 |
| 39 | CH₃ | H | CH₃ | SO₂Me | CH₃ | H | *i*-propyl | 481 | |
| 41 | CH₃ | H | CH₃ | H | CH₃ | H | *i*-propyl | 404 | |
| 45 | CH₃ | H | CH₃ | SO₂Me | Et | H | Cl | 488 | |
| 46 | CH₃ | H | CH₃ | SO₂Me | CH₃ | H | Cl | 474 | |
| 47 | CH₃ | H | CH₃ | SO₂CF₃ | CH₃ | H | *i*-propyl | 536 | |
| 48 | CH₃ | H | CH₃ | SO₂Me | Et | H | H | 440 | |
| 51 | CH₃ | H | CH₃ | H | Et | H | Cl | 410 | |
| 52 | CH₃ | H | CH₃ | SO₂CF₃ | Et | H | CH₃ | 522 | |
| 53 | CH₃ | H | CH₃ | COOMe | CH₃ | H | H | 420 | |
| 55 | CH₃ | H | F | H | Et | H | H | 380 | |
| 56 | CH₃ | H | CH₃ | COMe | CH₃ | H | H | 404 | |
| 57 | CH₃ | H | CH₃ | H | CH₂CF₃ | H | H | 430 | |
| 58 | CH₃ | H | CH₃ | H | *n*-propyl | H | H | 390 | |
| 59 | CH₃ | H | H | H | CH₃ | H | H | 348 | |
| 61 | CH₃ | H | CH₃ | SO₂CF₃ | CH₃ | H | CF₃ | 562 | |
| 62 | CH₃ | H | CH₃ | H | CH₃ | H | CH₃ | 376 | |
| 63 | CH₃ | H | F | H | CH₃ | H | H | 366 | |
| 64 | CH₃ | H | CH₃ | H | CH₃ | OMe | Cl | 425 | |
| 65 | CH₃ | H | CH₃ | H | CH₃ | OMe | H | 391 | |
| 66 | CH₃ | H | H | SO₂CF₃ | CH₃ | H | H | 480 | |
| 67 | CH₃ | H | CH₃ | SO₂CF₃ | CH₃ | H | Br | 572 | |
| 68 | CH₃ | H | CH₃ | H | Et | H | CH₃ | 390 | |
| 69 | CH₃ | H | CH₃ | H | CH₃ | H | Br | 440 | |
| 70 | CH₃ | H | CH₃ | H | CH₃ | H | OMe | 392 | |
| 71 | CH₃ | H | CH₃ | H | CH₃ | H | CF₃ | 430 | |
| 72 | CH₃ | H | CH₃ | H | CH₃ | H | Cl | 396 | |
| 73 | CH₃ | H | CH₃ | SO₂Me | CH₃ | H | H | 439 | |
| 74 | CH₃ | H | CH₃ | SO₂CF₃ | CH₃ | H | Cl | 527 | |
| 75 | CH₃ | H | CH₃ | SO₂CF₃ | CH₃ | H | CH₃ | 508 | |
| 76 | CH₃ | H | CH₃ | SO₂CF₃ | CH₃ | H | H | 494 | |
| 77 | CH₃ | H | CH₃ | H | *i*-butyl | H | H | 404 | |
| 78 | CH₃ | H | CH₃ | H | *i*-propyl | H | H | 390 | |
| 79 | CH₃ | H | CH₃ | H | Et | H | H | 376 | |
| 80 | CH₃ | H | CH₃ | H | CH₃ | H | H | | 172-175 |
| 81 | CH₃ | H | OMe | H | CH₃ | H | H | 378 | |

**INDEX TABLE F**

| 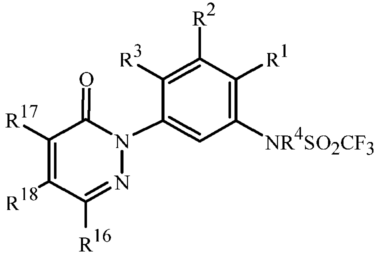 | | | | | | | | |
|---|---|---|---|---|---|---|---|---|
| Cmpd # | R¹ | R² | R³ | R⁴ | R¹⁶ | R¹⁷ | R¹⁸ | M.S. |
| 40 | CH₃ | H | CH₃ | S(O)₂CF₃ | H | OMe | OMe | 541 |
| 54 | CH₃ | H | CH₃ | H | H | H | OMe | 378 |
| 60 | CH₃ | H | CH₃ | H | H | OMe | H | 378 |
| 82 | CH₃ | H | CH₃ | H | H | Cl | H | 382 |
| 83 | CH₃ | H | CH₃ | H | H | H | H | 348 |
| 84 | CH₃ | H | CH₃ | H | H | Me | H | 362 |
| 85 | CH₃ | H | CH₃ | H | Me | H | H | 361 |
| 281 | CH₃ | H | CH₃ | H | H | Me | H | 259-262 |
| 282 | CH₃ | H | CH₃ | H | H | OMe | OMe | 230-233 |

**INDEX TABLE G**

| 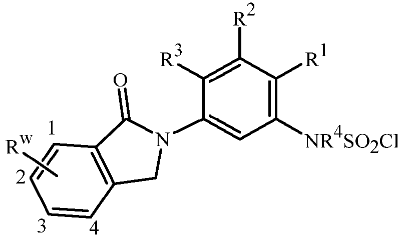 | | | | | | | |
|---|---|---|---|---|---|---|---|
| Cmpd # | R¹ | R² | R³ | R⁴ | R^{W} | M.S. | M.P. (°C) |
| 404 | CH₃ | H | CH₃ | CH₂OCO-*t*-Bu | H | 379 | |
| 405 | CH₃ | H | CH₃ | H | H | 507 | |
| 406 | CH₃ | H | CH₃ | H | 2-CH₃ | 505 | |
| 407 | CH₃ | H | CH₃ | CH₂OCO-*t*-Bu | 2-CH₃ | 391 | |
| 408 | CH₃ | H | CH₃ | H | 1-CH₃ | | 64-67 |
| 409 | CH₃ | H | CH₃ | H | 2-Cl | 513 | |
| 410 | CH₃ | H | CH₃ | CH₂OCO-*t*-Bu | 1-CH₃ | 555 | |
| 411 | CH₃ | H | CH₃ | CH₂OCO-*t*-Bu | 2-Cl | 439 | |
| 412 | CH₃ | H | CH₃ | H | 1-OMe | 481 | |
| 413 | CH₃ | H | CH₃ | CH₂OCO-*t*-Bu | 1-OMe | | 189-192 |
| 414 | CH₃ | H | CH₃ | CH₂OCO-*t*-Bu | 1-OEt | | 287-290 |
| 415 | CH₃ | H | CH₃ | H | 1-Br | | 211-214 |
| 416 | CH₃ | H | CH₃ | H | 1-Cl | 513 | |
| 417 | CH₃ | H | CH₃ | CH₂OCO-*t*-Bu | 1-Cl | | 91-94 |
| 418 | CH₃ | H | CH₃ | CH₂OCO-*t*-Bu | 1-Br | | 214-218 |

**INDEX TABLE H**

| Cmpd. No. | ¹H NMR Data (CDCl₃ solution at 500MHz unless indicated otherwise)^{a} |
|---|---|
| 157 | δ 7.29 (s, 1H), 7.15 (s, 1H), 3.81-3.95(m, 4H), 3.67 (s, 2H), 2.40 (s, 3H), 2.33 (s, 3H), 2.05-2.09 (m, 2H), 1.89-1.98 (m, 2H). |
| 275 | δ 7.29 (s, 1H), 7.17 (s, 1H), 3.83 (s, 2H), 2.40 (s, 3H), 2.34 (s, 3H), 2.23-2.29 (m, 2H), 1.90-1.99 (m, 2H), 1.77-1.84 (m, 4H). |
| ^{a 1}HNMR data are in ppm downfield from tetramethylsilane. Couplings are designated by (s)-singlet and (m)-multiplet. | |

### BIOLOGICAL EXAMPLES OF THE INVENTION

### TEST A

Seeds of plant species selected from barnyardgrass *(Echinochloa crus-galli*), kochia *(Bassia scoparia),* ragweed (common ragweed, *Ambrosia artemisiifolia),* Ryegrass, Italian (Italian ryegrass, *Lolium multiflorum),* Foxtail, Giant (giant foxtail, *Setaria faberi),* and pigweed *(Amaranthus retroflexus)* were planted into a blend of loam soil and sand and treated preemergence with a directed soil spray using test chemicals formulated in a non-phytotoxic solvent mixture which included a surfactant.

At the same time, plants selected from these weed species and also wheat *(Triticum aestivum),* corn *(Zea mays),* blackgrass *(Alopecurus myosuroides),* and galium (catchweed bedstraw, *Galium aparine)* were planted in pots containing the same blend of loam soil and sand and treated with postemergence applications of test chemicals formulated in the same manner. Plants ranged in height from 2 to 10 cm and were in the one- to two-leaf stage for the postemergence treatment. Treated plants and untreated controls were maintained in a greenhouse for approximately 10 days, after which time all treated plants were compared to untreated controls and visually evaluated for injury. Plant response ratings, summarized in Table A, are based on a 0 to 100 scale where 0 is no effect and 100 is complete control. A dash (-) response means no test result.

| Table A | Compounds | | | | | | | | | | | | | |
|---|---|---|---|---|---|---|---|---|---|---|---|---|---|---|
| 1000 g ai/ha | 4 | 5 | 6 | 7 | 8 | 10 | 11 | 12 | 19 | 50 | 242 | 258 | 259 | 260 |
| Preemergence | | | | | | | | | | | | | | |
| Barnyardgrass | 100 | 100 | 100 | 80 | 100 | 40 | 100 | 100 | 100 | 100 | 100 | 80 | 90 | 100 |
| Foxtail, Giant | 100 | 100 | 100 | 90 | 100 | 80 | 100 | 100 | 100 | 100 | 100 | 90 | 90 | 100 |
| Kochia | 80 | 90 | 100 | 30 | 100 | 60 | 90 | 90 | 90 | 90 | 90 | 90 | 40 | 40 |
| Pigweed | 100 | 100 | 100 | 80 | 100 | 100 | 100 | 100 | 100 | 100 | 100 | 100 | 80 | 100 |
| Ragweed | - | - | - | 80 | 90 | 90 | 80 | - | 90 | 80 | 90 | 90 | 70 | 90 |
| Ryegrass, Italian | 100 | 100 | 100 | 60 | 100 | 90 | 0 | 100 | 90 | 70 | 100 | 70 | 80 | 100 |

| Table A | Compounds | | | |
|---|---|---|---|---|
| 1000 g ai/ha | 261 | 262 | 263 | 264 |
| Preemergence | | | | |
| Barnyardgrass | 60 | 20 | 30 | 100 |
| Foxtail, Giant | 90 | 70 | 70 | 100 |
| Kochia | 0 | 0 | 20 | 70 |
| Pigweed | 20 | 0 | 60 | 80 |
| Ragweed | 0 | 0 | - | 90 |
| Ryegrass, Italian | 60 | 20 | 50 | 100 |

| Table A | Compounds | | | | | | | | | | | | | |
|---|---|---|---|---|---|---|---|---|---|---|---|---|---|---|
| 500 g ai/ha | 9 | 13 | 14 | 15 | 16 | 17 | 18 | 20 | 21 | 22 | 23 | 24 | 25 | 26 |
| Preemergence | | | | | | | | | | | | | | |
| Barnyardgrass | 100 | 100 | 90 | 0 | 100 | 100 | 100 | 100 | 100 | 100 | 100 | 100 | 100 | 100 |
| Foxtail, Giant | 100 | 10 | 80 | 70 | 100 | 100 | 100 | 100 | 100 | 80 | 100 | 100 | 100 | 100 |
| Kochia | 90 | 50 | 90 | 80 | 90 | 90 | 60 | 90 | 0 | 50 | 80 | 90 | 80 | 50 |
| Pigweed | 80 | 60 | 90 | 70 | 100 | 100 | 100 | 80 | 60 | 100 | 100 | 90 | 100 | 90 |
| Ragweed | 90 | - | - | - | 100 | 80 | 30 | 100 | - | - | - | - | - | 50 |
| Ryegrass, Italian | 100 | 30 | 100 | 30 | 100 | 100 | 90 | 100 | 100 | 100 | 100 | 100 | 100 | 100 |

| Table A | Compounds | | | | | | | | | | | | |
|---|---|---|---|---|---|---|---|---|---|---|---|---|---|
| 500 g ai/ha | 28 | 29 | 30 | 32 | 34 | 35 | 37 | 38 | 39 | 40 | 41 | 42 | 43 |
| Preemergence | | | | | | | | | | | | | |
| Barnyardgrass | 100 | 100 | 90 | 100 | 0 | 90 | 90 | 100 | 90 | 90 | 90 | 100 | 100 |
| Foxtail, Giant | 90 | 100 | 100 | 100 | 30 | 90 | 100 | 100 | 90 | 100 | 80 | 100 | 100 |
| Kochia | 90 | 90 | 90 | 90 | 70 | 20 | 90 | 90 | 0 | 80 | 0 | 70 | 90 |
| Pigweed | | 90 | 60 | 60 | | 80 | 90 | 80 | 0 | 100 | 0 | 80 | 60 |
| Ragweed | 30 | 90 | 40 | 50 | 20 | 80 | 80 | 90 | 30 | 50 | 80 | 80 | 0 |
| Ryegrass, Italian | 90 | 100 | 90 | 100 | 70 | 50 | 100 | 100 | 70 | 80 | 80 | 100 | 100 |

| Table A | Compounds | | | | | | | | | | | | | |
|---|---|---|---|---|---|---|---|---|---|---|---|---|---|---|
| 500 g ai/ha | 44 | 45 | 46 | 47 | 48 | 51 | 52 | 53 | 54 | 55 | 56 | 57 | 58 | 59 |
| Preemergence | | | | | | | | | | | | | | |
| Barnyardgrass | 100 | 100 | 70 | 90 | 100 | 100 | 100 | 0 | 0 | 100 | 100 | 100 | 100 | 90 |
| Foxtail, Giant | 100 | 100 | 90 | 90 | 100 | 100 | 100 | 0 | 10 | 100 | 20 | 100 | 100 | 70 |
| Kochia | 60 | 100 | 0 | 0 | 30 | 90 | 80 | 0 | 90 | 80 | 100 | 60 | 80 | 90 |
| Pigweed | 100 | 90 | 50 | 0 | 80 | 90 | 80 | 0 | 90 | 90 | 90 | 80 | 50 | 60 |
| Ragweed | 10 | 80 | 0 | 60 | 90 | 90 | 60 | 40 | 90 | 90 | 90 | 70 | 0 | 30 |
| Ryegrass, Italian | 100 | 100 | 80 | 100 | 100 | 100 | 90 | 0 | 20 | 90 | 100 | 90 | 100 | 60 |

| Table A | Compounds | | | | | | | | | | | | | |
|---|---|---|---|---|---|---|---|---|---|---|---|---|---|---|
| 500 g ai/ha | 60 | 61 | 62 | 63 | 64 | 65 | 66 | 67 | 68 | 69 | 70 | 71 | 72 | 73 |
| Preemergence | | | | | | | | | | | | | | |
| Barnyardgrass | 90 | 100 | 100 | 90 | 90 | 90 | 90 | 100 | 100 | 90 | 100 | 100 | 100 | 90 |
| Foxtail, Giant | 90 | 100 | 100 | 100 | 90 | 100 | 60 | 100 | 100 | 100 | 90 | 100 | 100 | 100 |
| Kochia | 90 | 90 | 90 | 90 | 90 | 60 | 40 | 70 | 90 | 90 | 0 | 40 | 100 | 90 |
| Pigweed | 100 | 80 | 90 | 90 | 50 | 30 | 30 | 80 | 90 | 100 | 80 | 60 | 100 | 100 |
| Ragweed | 70 | 0 | 80 | 80 | 40 | 0 | 10 | 60 | 90 | 90 | 70 | 60 | 90 | 80 |
| Ryegrass, Italian | 90 | 100 | 100 | 100 | 100 | 60 | 0 | 90 | 100 | 100 | 60 | 100 | 100 | 100 |

| Table A | Compounds | | | | | | | | | | | | | |
|---|---|---|---|---|---|---|---|---|---|---|---|---|---|---|
| 500 g ai/ha | 74 | 75 | 76 | 77 | 78 | 79 | 80 | 81 | 82 | 83 | 84 | 85 | 99 | 100 |
| Preemergence | | | | | | | | | | | | | | |
| Barnyardgrass | 100 | 100 | 90 | 90 | 100 | 100 | 90 | 90 | 80 | 80 | 80 | 70 | 100 | 90 |
| Foxtail, Giant | 100 | 100 | 100 | 100 | 100 | 100 | 100 | 100 | 60 | 80 | 90 | 90 | 80 | 90 |
| Kochia | 100 | 100 | 90 | 0 | 60 | 90 | 80 | 80 | 90 | 70 | 70 | 70 | 100 | 80 |
| Pigweed | 100 | 100 | 100 | 30 | 90 | 100 | 90 | 100 | 80 | 90 | 70 | 100 | 100 | 80 |
| Ragweed | 90 | 80 | 90 | 20 | 90 | 90 | 90 | 40 | 20 | 20 | 0 | 0 | 90 | 80 |
| Ryegrass, Italian | 100 | 100 | 100 | 90 | 100 | 100 | 90 | 50 | 40 | 50 | 90 | 30 | 90 | 70 |

| Table A | Compounds | | | | | | | | | | | | |
|---|---|---|---|---|---|---|---|---|---|---|---|---|---|
| 500 g ai/ha | 101 | 102 | 103 | 104 | 105 | 107 | 108 | 109 | 110 | 114 | 116 | 119 | 120 |
| Preemergence | | | | | | | | | | | | | |
| Barnyardgrass | 100 | 100 | 90 | 100 | 100 | 100 | 100 | 100 | 90 | 80 | 90 | 100 | 100 |
| Foxtail, Giant | 100 | 90 | 100 | 100 | 100 | 100 | 100 | 100 | 100 | 100 | 90 | 100 | 100 |
| Kochia | 90 | 90 | 90 | 50 | 100 | 50 | 90 | 100 | 100 | 70 | 100 | 100 | 100 |
| Pigweed | 90 | 100 | 100 | 90 | 100 | 90 | - | 100 | 100 | - | | 100 | - |
| Ragweed | 80 | 70 | 90 | 60 | 80 | 80 | 60 | 90 | 90 | 10 | 100 | 100 | 100 |
| Ryegrass, Italian | 100 | 90 | 90 | 100 | 100 | 90 | 100 | 80 | 90 | 90 | 90 | 100 | 100 |

| Table A | Compounds | | | | | | | | | | | | | |
|---|---|---|---|---|---|---|---|---|---|---|---|---|---|---|
| 500 g ai/ha | 121 | 122 | 123 | 124 | 125 | 126 | 127 | 128 | 129 | 130 | 132 | 133 | 134 | 135 |
| Preemergence | | | | | | | | | | | | | | |
| Barnyardgrass | 90 | 70 | 100 | 0 | 100 | 40 | 90 | 100 | 90 | 100 | 100 | 90 | 40 | 100 |
| Foxtail, Giant | 100 | 100 | 100 | 20 | 100 | 90 | 90 | 100 | 100 | 100 | 90 | 90 | 100 | 100 |
| Kochia | 90 | 30 | 80 | 0 | 100 | 30 | 80 | 80 | 90 | 100 | 70 | 0 | | 100 |
| Pigweed | - | 30 | 100 | 80 | 100 | 100 | 40 | 100 | 100 | 100 | 100 | 90 | 90 | 100 |
| Ragweed | 100 | 0 | 80 | 30 | 80 | 30 | 60 | 90 | 90 | 90 | 90 | 90 | 30 | 80 |
| Ryegrass, Italian | 100 | 80 | 100 | 0 | 100 | 40 | 80 | 100 | 100 | 100 | 100 | 90 | 90 | 100 |

| Table A | Compounds | | | | | | | | | | | | | |
|---|---|---|---|---|---|---|---|---|---|---|---|---|---|---|
| 500 g ai/ha | 136 | 139 | 140 | 141 | 142 | 146 | 147 | 158 | 159 | 160 | 163 | 164 | 165 | 166 |
| Preemergence | | | | | | | | | | | | | | |
| Barnyardgrass | 100 | 30 | 100 | 100 | 100 | 100 | 100 | 100 | 60 | 100 | 100 | 100 | 30 | 100 |
| Foxtail, Giant | 100 | 20 | 90 | 100 | 100 | 100 | 100 | 100 | 70 | 100 | 90 | 100 | 90 | 100 |
| Kochia | 90 | 60 | 70 | 50 | 0 | 80 | 90 | 80 | 70 | 90 | 80 | 100 | 100 | 100 |
| Pigweed | 70 | 70 | 50 | 90 | 100 | - | - | 100 | - | 100 | 100 | - | - | - |
| Ragweed | 80 | 30 | 60 | 90 | 70 | 70 | 90 | 40 | 80 | 90 | 60 | 100 | 100 | 80 |
| Ryegrass, Italian | 100 | 70 | 100 | 100 | 100 | 100 | 100 | 100 | 30 | 60 | 100 | 100 | 70 | 100 |

| Table A | Compounds | | | | | | | | | | | | | |
|---|---|---|---|---|---|---|---|---|---|---|---|---|---|---|
| 500 g ai/ha | 169 | 170 | 171 | 172 | 173 | 175 | 176 | 177 | 178 | 179 | 180 | 181 | 182 | 183 |
| Preemergence | | | | | | | | | | | | | | |
| Barnyardgrass | 100 | 0 | 100 | 90 | 90 | 100 | 90 | 100 | 100 | 90 | 80 | 100 | 50 | 100 |
| Foxtail, Giant | 90 | 20 | 100 | 100 | 100 | 100 | 100 | 100 | 100 | 100 | 90 | 100 | 10 | 70 |
| Kochia | 30 | 0 | 90 | 90 | 90 | 100 | 90 | 100 | 100 | 90 | 0 | 100 | 70 | 60 |
| Pigweed | 80 | 90 | - | - | - | 100 | - | 100 | 100 | - | 70 | 80 | 70 | 80 |
| Ragweed | 80 | 0 | 90 | 90 | 90 | 100 | 90 | 90 | 90 | 90 | 70 | 90 | 60 | 90 |
| Ryegrass, Italian | 100 | 40 | 100 | 100 | 100 | 100 | 100 | 100 | 100 | 100 | 50 | 90 | 80 | 100 |

| Table A | Compounds | | | | | | | | | | | | | |
|---|---|---|---|---|---|---|---|---|---|---|---|---|---|---|
| 500 g ai/ha | 184 | 185 | 186 | 187 | 188 | 189 | 190 | 191 | 192 | 193 | 194 | 195 | 196 | 197 |
| Preemergence | | | | | | | | | | | | | | |
| Barnyardgrass | 100 | 100 | 100 | 100 | 90 | 100 | 100 | 100 | 70 | 90 | 90 | 90 | 90 | 100 |
| Foxtail, Giant | 90 | 100 | 100 | 100 | 100 | 100 | 100 | 100 | 90 | 100 | 100 | 90 | 100 | 100 |
| Kochia | 0 | 0 | 70 | 80 | 60 | 100 | 100 | 90 | 80 | 40 | 90 | 0 | 80 | 90 |
| Pigweed | 100 | 80 | 80 | 80 | 90 | 100 | 100 | 100 | 100 | 90 | 100 | 80 | 100 | 100 |
| Ragweed | 90 | 70 | 50 | 90 | 50 | 90 | 90 | 100 | 90 | 90 | 90 | 0 | 0 | 90 |
| Ryegrass, Italian | 50 | 100 | 100 | 100 | 100 | 100 | 100 | 100 | 100 | 100 | 100 | 90 | 70 | 100 |

| Table A | Compounds | | | | | | | | | | | | | |
|---|---|---|---|---|---|---|---|---|---|---|---|---|---|---|
| 500 g ai/ha | 198 | 199 | 200 | 201 | 202 | 203 | 204 | 205 | 206 | 207 | 208 | 209 | 210 | 211 |
| Preemergence | | | | | | | | | | | | | | |
| Barnyardgrass | 100 | 100 | 100 | 100 | 90 | 90 | 90 | 90 | 90 | 90 | 90 | 90 | 100 | 90 |
| Foxtail, Giant | 100 | 100 | 100 | 100 | 70 | 100 | 100 | 100 | 90 | 100 | 100 | 70 | 100 | 90 |
| Kochia | 80 | 90 | 90 | 100 | 80 | 90 | 90 | 80 | 90 | 90 | 90 | 80 | 90 | 90 |
| Pigweed | 100 | 100 | 100 | 100 | 100 | 100 | 100 | 90 | 100 | 100 | 70 | 50 | 100 | 90 |
| Ragweed | 80 | 90 | 90 | 100 | 90 | 90 | 90 | 80 | 80 | 70 | 0 | 60 | 90 | 80 |
| Ryegrass, Italian | 90 | 80 | 90 | 90 | 70 | 100 | 100 | 100 | 90 | 100 | 80 | 90 | 100 | 70 |

| Table A | Compounds | | | | | | | | | | | | | |
|---|---|---|---|---|---|---|---|---|---|---|---|---|---|---|
| 500 g ai/ha | 212 | 213 | 214 | 215 | 216 | 217 | 218 | 219 | 220 | 221 | 222 | 223 | 224 | 225 |
| Preemergence | | | | | | | | | | | | | | |
| Barnyardgrass | 90 | 50 | 0 | 90 | 80 | 100 | 100 | 100 | 100 | 100 | 90 | 50 | 100 | 90 |
| Foxtail, Giant | 90 | 80 | 60 | 70 | 90 | 100 | 100 | 90 | 100 | 100 | 50 | 100 | 100 | 100 |
| Kochia | 90 | 90 | 90 | 90 | 90 | 90 | 60 | 40 | 70 | 90 | 40 | 20 | 90 | 40 |
| Pigweed | 90 | 100 | 60 | 90 | 100 | 100 | 90 | 60 | 100 | 100 | 100 | 90 | 100 | 100 |
| Ragweed | 30 | 20 | 60 | 20 | 30 | 90 | 90 | 0 | 60 | 90 | 90 | 50 | 80 | 90 |
| Ryegrass, Italian | 70 | 90 | 90 | 70 | 90 | 100 | 100 | 60 | 100 | 100 | 80 | 80 | 100 | 100 |

| Table A | Compounds | | | | | | | | | | | | | |
|---|---|---|---|---|---|---|---|---|---|---|---|---|---|---|
| 500 g ai/ha | 226 | 227 | 228 | 229 | 230 | 231 | 232 | 233 | 234 | 235 | 236 | 237 | 238 | 239 |
| Preemergence | | | | | | | | | | | | | | |
| Barnyardgrass | 100 | 90 | 100 | 90 | 100 | 100 | 100 | 30 | 80 | 0 | 0 | 30 | 0 | 20 |
| Foxtail, Giant | 100 | 100 | 100 | 100 | 100 | 100 | 100 | 100 | 50 | 10 | 60 | 40 | 60 | 60 |
| Kochia | 90 | 90 | 90 | 100 | 80 | 90 | 80 | 80 | 30 | 50 | 70 | 90 | 60 | 80 |
| Pigweed | 100 | 80 | 100 | 100 | 100 | 100 | 100 | 90 | 100 | 80 | 90 | 100 | 80 | 90 |
| Ragweed | 90 | 80 | 70 | 100 | 70 | 100 | 90 | 30 | 90 | 80 | 80 | 80 | 90 | 70 |
| Ryegrass, Italian | 100 | 100 | 100 | 90 | 100 | 100 | 100 | 70 | 50 | 90 | 60 | 50 | 90 | 30 |

| Table A | Compounds | | | | | | | | | | | | | |
|---|---|---|---|---|---|---|---|---|---|---|---|---|---|---|
| 500 g ai/ha | 240 | 241 | 243 | 244 | 245 | 246 | 247 | 248 | 249 | 250 | 251 | 252 | 253 | 254 |
| Preemergence | | | | | | | | | | | | | | |
| Barnyardgrass | 50 | 100 | 90 | 90 | 100 | 100 | 50 | 90 | 60 | 90 | 90 | 100 | 70 | 100 |
| Foxtail, Giant | 80 | 100 | 100 | 100 | 100 | 100 | 90 | 80 | 100 | 100 | 90 | 100 | 100 | 100 |
| Kochia | 90 | 100 | 80 | 90 | 80 | 90 | 80 | 90 | 70 | 100 | 90 | 80 | 80 | 80 |
| Pigweed | 80 | 100 | 100 | - | - | 100 | 90 | 90 | 80 | 100 | 100 | 100 | 100 | 90 |
| Ragweed | 50 | 90 | 80 | 90 | 90 | 90 | 30 | 90 | 0 | 90 | 80 | 90 | 40 | 60 |
| Ryegrass, Italian | 70 | 100 | 100 | 80 | 100 | 100 | 90 | 90 | 80 | 90 | 100 | 100 | 100 | 90 |

| Table A | Compounds | | | | | | | | | | | | | |
|---|---|---|---|---|---|---|---|---|---|---|---|---|---|---|
| 500 g ai/ha | 255 | 256 | 257 | 265 | 266 | 267 | 268 | 269 | 270 | 276 | 277 | 278 | 279 | 280 |
| Preemergence | | | | | | | | | | | | | | |
| Barnyardgrass | 90 | 100 | 70 | 100 | 40 | 100 | 50 | 100 | 90 | 100 | 100 | 100 | 100 | 90 |
| Foxtail, Giant | 100 | 90 | 90 | 100 | 20 | 100 | 70 | 100 | 100 | 100 | 100 | 100 | 100 | 100 |
| Kochia | 90 | 90 | 80 | 90 | 0 | 100 | 70 | 100 | 0 | 0 | 100 | 100 | 90 | 70 |
| Pigweed | 100 | 100 | 90 | - | - | - | - | - | 100 | 100 | 100 | - | 100 | 100 |
| Ragweed | 90 | 80 | 80 | 90 | 0 | 100 | 90 | 90 | 80 | 70 | 80 | 100 | 100 | 70 |
| Ryegrass, Italian | 90 | 100 | 90 | 100 | 0 | 100 | 60 | 100 | 100 | 80 | 100 | 100 | 100 | 100 |

| Table A | Compounds | | | | | | | | | | | | | |
|---|---|---|---|---|---|---|---|---|---|---|---|---|---|---|
| 125 g ai/ha | 1 | 2 | 3 | 9 | 13 | 14 | 15 | 16 | 17 | 18 | 20 | 21 | 22 | 23 |
| Preemergence | | | | | | | | | | | | | | |
| Barnyardgrass | 80 | 60 | 50 | 100 | 60 | - | 0 | 90 | 100 | 100 | 100 | 80 | 90 | 90 |
| Foxtail, Giant | 90 | 80 | 50 | 80 | 0 | 70 | 10 | 30 | 100 | 100 | 80 | 20 | 50 | 70 |
| Kochia | 70 | 50 | 80 | 70 | 20 | 70 | 0 | 90 | 70 | 10 | 40 | 0 | 20 | 80 |
| Pigweed | 100 | 100 | 100 | 30 | 20 | 80 | 60 | 100 | 90 | 100 | 20 | 40 | 30 | 80 |
| Ragweed | 50 | 90 | 70 | 90 | - | - | - | 80 | 80 | 0 | 90 | - | - | - |
| Ryegrass, Italian | 90 | 50 | 70 | 90 | 20 | 60 | 30 | 90 | 80 | 90 | 50 | 30 | 60 | 40 |

| Table A | Compounds | | | | | | | | | | | | |
|---|---|---|---|---|---|---|---|---|---|---|---|---|---|
| 125 g ai/ha | 24 | 25 | 26 | 27 | 28 | 29 | 30 | 32 | 34 | 35 | 36 | 37 | 38 |
| Preemergence | | | | | | | | | | | | | |
| Barnyardgrass | 100 | 100 | 70 | 0 | 60 | 40 | 90 | 100 | 0 | 70 | 50 | 90 | 90 |
| Foxtail, Giant | 30 | 100 | 100 | 60 | 30 | 100 | 80 | 100 | 0 | 60 | 80 | 100 | 100 |
| Kochia | 90 | 70 | 0 | 0 | 70 | 40 | 70 | 60 | 30 | 0 | 0 | 80 | 80 |
| Pigweed | 60 | 80 | 80 | 20 | - | 70 | 20 | 20 | - | 50 | 10 | 50 | 40 |
| Ragweed | - | - | 20 | 0 | 0 | 60 | 20 | 0 | 0 | 20 | 0 | 40 | 70 |
| Ryegrass, Italian | 70 | 70 | 80 | 0 | 50 | 90 | 90 | 100 | 30 | 40 | 60 | 100 | 100 |

| Table A | Compounds | | | | | | | | | | | | | |
|---|---|---|---|---|---|---|---|---|---|---|---|---|---|---|
| 125 g ai/ha | 39 | 40 | 41 | 42 | 43 | 44 | 45 | 46 | 47 | 48 | 51 | 52 | 53 | 54 |
| Preemergence | | | | | | | | | | | | | | |
| Barnyardgrass | 40 | 90 | 50 | 90 | 90 | 100 | 90 | 0 | 50 | 90 | 100 | 100 | 0 | 0 |
| Foxtail, Giant | 10 | 90 | 10 | 100 | 100 | 100 | 100 | 90 | 10 | 100 | 100 | 100 | 0 | 0 |
| Kochia | 0 | 80 | 0 | 40 | 0 | 0 | 70 | 0 | 0 | 30 | 80 | 70 | 0 | 60 |
| Pigweed | 0 | 100 | 0 | 40 | 20 | 40 | 60 | 0 | 0 | 70 | 80 | 50 | 0 | 40 |
| Ragweed | 0 | 50 | 30 | 30 | 0 | 0 | 60 | 0 | 20 | 80 | 60 | 10 | 0 | 50 |
| Ryegrass, Italian | 30 | 30 | 60 | 100 | 100 | 100 | 80 | 50 | 50 | 60 | 90 | 90 | 0 | 0 |

| Table A | Compounds | | | | | | | | | | | | | |
|---|---|---|---|---|---|---|---|---|---|---|---|---|---|---|
| 125 g ai/ha | 55 | 56 | 57 | 58 | 59 | 60 | 61 | 62 | 63 | 64 | 65 | 66 | 67 | 68 |
| Preemergence | | | | | | | | | | | | | | |
| Barnyardgrass | 0 | 100 | 100 | 100 | 0 | 90 | 100 | 100 | 70 | 50 | 0 | 0 | 70 | 100 |
| Foxtail, Giant | 60 | 0 | 100 | 100 | 20 | 80 | 100 | 100 | 90 | 90 | 80 | 0 | 90 | 100 |
| Kochia | 80 | 40 | 60 | 0 | 40 | 90 | 50 | 70 | 40 | 40 | 0 | 0 | 0 | 70 |
| Pigweed | 70 | 40 | 50 | 0 | 30 | 100 | 50 | 50 | 70 | 40 | 0 | 0 | 50 | 70 |
| Ragweed | 20 | 70 | 40 | 0 | 0 | 30 | 0 | 20 | 30 | 0 | 0 | 0 | 0 | 30 |
| Ryegrass, Italian | 70 | 100 | 90 | 70 | 0 | 80 | 70 | 40 | 90 | 70 | 30 | 0 | 80 | 90 |

| Table A | Compounds | | | | | | | | | | | | | |
|---|---|---|---|---|---|---|---|---|---|---|---|---|---|---|
| 125 g ai/ha | 69 | 70 | 71 | 72 | 73 | 74 | 75 | 76 | 77 | 78 | 79 | 80 | 81 | 82 |
| Preemergence | | | | | | | | | | | | | | |
| Barnyardgrass | 40 | 90 | 0 | 90 | 90 | 90 | 90 | 90 | 90 | 90 | 100 | 90 | 90 | 40 |
| Foxtail, Giant | 100 | 10 | 80 | 100 | 100 | 100 | 100 | 100 | 90 | 100 | 100 | 90 | 100 | 0 |
| Kochia | 70 | 0 | 80 | 100 | 80 | 70 | 80 | 60 | 0 | 0 | 70 | 80 | 0 | 70 |
| Pigweed | 80 | 80 | 40 | 80 | 90 | 60 | 80 | 50 | 20 | 30 | 90 | 60 | 30 | 90 |
| Ragweed | 50 | 10 | 10 | 80 | 80 | 70 | 20 | 70 | 0 | 80 | 80 | 70 | 30 | 0 |
| Ryegrass, Italian | 100 | 50 | 70 | 90 | 90 | 80 | 80 | 60 | 0 | 100 | 90 | 90 | 30 | 20 |

| Table A | Compounds | | | | | | | | | | | | | |
|---|---|---|---|---|---|---|---|---|---|---|---|---|---|---|
| 125 g ai/ha | 83 | 85 | 88 | 91 | 92 | 93 | 94 | 95 | 96 | 97 | 98 | 99 | 100 | 101 |
| Preemergence | | | | | | | | | | | | | | |
| Barnyardgrass | 70 | 70 | 30 | 90 | 60 | 80 | 90 | 80 | 70 | 90 | 90 | 90 | 70 | 100 |
| Foxtail, Giant | 10 | 10 | 90 | 100 | 70 | 80 | 100 | 100 | 80 | 50 | 100 | 70 | 40 | 90 |
| Kochia | 60 | 60 | 40 | 60 | 0 | 50 | 60 | 10 | 0 | 0 | 40 | 70 | 60 | 60 |
| Pigweed | 50 | 100 | 90 | 100 | 20 | 100 | 100 | 90 | 90 | 100 | 100 | 100 | 70 | 90 |
| Ragweed | 0 | 0 | 20 | 20 | 20 | 70 | 30 | 60 | 30 | 0 | 80 | 60 | 20 | 60 |
| Ryegrass, Italian | 30 | 0 | - | 50 | 30 | 80 | 90 | 90 | 80 | 0 | 50 | 50 | 40 | 80 |

| Table A | Compounds | | | | | | | | | | | | |
|---|---|---|---|---|---|---|---|---|---|---|---|---|---|
| 125 g ai/ha | 102 | 103 | 104 | 105 | 107 | 108 | 109 | 110 | 111 | 112 | 113 | 114 | 115 |
| Preemergence | | | | | | | | | | | | | |
| Barnyardgrass | 80 | 90 | 90 | 90 | 90 | 70 | 90 | 40 | 30 | 30 | 20 | 50 | 100 |
| Foxtail, Giant | 80 | 90 | 90 | 90 | 60 | 80 | 100 | 90 | 60 | 50 | 60 | 90 | 100 |
| Kochia | 70 | 80 | 0 | 20 | 0 | 80 | 90 | 80 | 50 | 60 | 50 | 30 | 40 |
| Pigweed | 100 | 100 | 70 | 100 | 80 | - | 100 | 80 | 90 | 100 | 90 | - | 90 |
| Ragweed | 20 | 70 | 30 | 70 | 50 | 20 | 90 | 80 | 40 | 90 | 60 | 0 | 20 |
| Ryegrass, Italian | 90 | 90 | 50 | 100 | 60 | 90 | 70 | 20 | 70 | 30 | 80 | 70 | 90 |

| Table A | Compounds | | | | | | | | | | | | | |
|---|---|---|---|---|---|---|---|---|---|---|---|---|---|---|
| 125 g ai/ha | 116 | 118 | 119 | 120 | 121 | 122 | 123 | 124 | 125 | 126 | 127 | 128 | 129 | 130 |
| Preemergence | | | | | | | | | | | | | | |
| Barnyardgrass | 30 | 90 | 100 | 90 | 90 | 0 | 100 | 0 | 100 | 0 | 50 | 100 | 100 | 100 |
| Foxtail, Giant | 60 | 100 | 100 | 100 | 100 | 50 | 100 | 0 | 100 | 20 | 20 | 50 | 100 | 100 |
| Kochia | 90 | 0 | 50 | 90 | 90 | 70 | 40 | 0 | 90 | 0 | 80 | 80 | 80 | 60 |
| Pigweed | - | 100 | 100 | - | - | 0 | 90 | 20 | 100 | 70 | 20 | 100 | 100 | 100 |
| Ragweed | 10 | 100 | 80 | 90 | 80 | 0 | 50 | 0 | 60 | 0 | 0 | 90 | 70 | 80 |
| Ryegrass, Italian | 40 | 80 | 80 | 100 | 100 | 30 | 90 | 0 | 100 | 0 | 60 | 100 | 80 | 90 |

| Table A | Compounds | | | | | | | | | | | | | |
|---|---|---|---|---|---|---|---|---|---|---|---|---|---|---|
| 125 g ai/ha | 131 | 132 | 133 | 134 | 135 | 136 | 137 | 139 | 140 | 141 | 142 | 143 | 144 | 145 |
| Preemergence | | | | | | | | | | | | | | |
| Barnyardgrass | 90 | 60 | 30 | 0 | 100 | 90 | 100 | 0 | 40 | 100 | 90 | 90 | 100 | 50 |
| Foxtail, Giant | 100 | 30 | 10 | 40 | 100 | 100 | 100 | 0 | 20 | 100 | 100 | 100 | 100 | 10 |
| Kochia | 40 | 0 | 0 | 0 | 80 | 80 | 30 | 0 | 0 | 0 | 0 | 20 | 30 | 0 |
| Pigweed | 90 | 100 | 30 | 80 | 60 | 20 | 100 | 20 | 0 | 90 | 90 | 100 | 50 | 70 |
| Ragweed | 40 | 80 | 20 | 10 | 10 | 50 | 60 | 0 | 30 | 50 | 30 | 0 | 30 | 40 |
| Ryegrass, Italian | 80 | 80 | 20 | 20 | 100 | 90 | 100 | 50 | 0 | 90 | 90 | 90 | 90 | 40 |

| Table A | Compounds | | | | | | | | | | | | | |
|---|---|---|---|---|---|---|---|---|---|---|---|---|---|---|
| 125 g ai/ha | 146 | 147 | 148 | 149 | 150 | 151 | 152 | 153 | 154 | 155 | 156 | 157 | 158 | 159 |
| Preemergence | | | | | | | | | | | | | | |
| Barnyardgrass | 90 | 90 | 80 | 0 | 90 | 90 | 70 | 70 | 50 | 90 | 100 | 100 | 30 | 40 |
| Foxtail, Giant | 100 | 90 | 100 | 80 | 100 | 100 | 80 | 90 | 90 | 80 | 100 | 100 | 90 | 30 |
| Kochia | 80 | 70 | 30 | 0 | 0 | 0 | 0 | 80 | 40 | 40 | 50 | 90 | 70 | 40 |
| Pigweed | - | - | 100 | 70 | 100 | 90 | 100 | 90 | 100 | 100 | 100 | 100 | 50 | - |
| Ragweed | 60 | 90 | 20 | 0 | 0 | 30 | 30 | 60 | 80 | 80 | 90 | 90 | 30 | 80 |
| Ryegrass, Italian | 90 | 90 | 100 | 50 | 90 | 90 | 40 | 70 | 70 | 80 | 70 | 60 | 30 | 0 |

| Table A | Compounds | | | | | | | | | | | | | |
|---|---|---|---|---|---|---|---|---|---|---|---|---|---|---|
| 125 g ai/ha | 160 | 161 | 162 | 163 | 164 | 165 | 166 | 167 | 168 | 169 | 170 | 171 | 172 | 173 |
| Preemergence | | | | | | | | | | | | | | |
| Barnyardgrass | 80 | 100 | 60 | 90 | 90 | 20 | 100 | 20 | 60 | 30 | 0 | 90 | 90 | 90 |
| Foxtail, Giant | 90 | 80 | 90 | 50 | 90 | 80 | 80 | 10 | 0 | 20 | 0 | 100 | 90 | 100 |
| Kochia | 20 | 90 | 40 | 0 | 100 | 90 | 90 | 40 | 20 | 0 | 0 | 70 | 60 | 60 |
| Pigweed | 90 | 100 | 100 | 50 | - | - | - | 20 | 10 | 60 | 20 | - | - | - |
| Ragweed | 10 | 90 | 30 | 20 | 90 | 50 | 20 | 0 | 0 | 40 | 0 | 80 | 40 | 90 |
| Ryegrass, Italian | 50 | 100 | 70 | 80 | 80 | 50 | 70 | 60 | 40 | 50 | 20 | 100 | 100 | 90 |

| Table A | Compounds | | | | | | | | | | | | | |
|---|---|---|---|---|---|---|---|---|---|---|---|---|---|---|
| 125 g ai/ha | 174 | 175 | 176 | 177 | 178 | 179 | 180 | 181 | 182 | 183 | 184 | 185 | 186 | 187 |
| Preemergence | | | | | | | | | | | | | | |
| Barnyardgrass | 80 | 90 | 70 | 80 | 90 | 90 | 0 | 90 | 0 | 90 | 70 | 70 | 60 | 100 |
| Foxtail, Giant | 90 | 100 | 100 | 70 | 90 | 80 | 0 | 100 | 10 | 50 | 30 | 50 | 60 | 100 |
| Kochia | 80 | 90 | 70 | 100 | 90 | 90 | 0 | 60 | 30 | 20 | 0 | 0 | 20 | 80 |
| Pigweed | 100 | 90 | - | 80 | 100 | - | 20 | 40 | 50 | 70 | 70 | 50 | 20 | 70 |
| Ragweed | 90 | 80 | 40 | 70 | 80 | 90 | 20 | 50 | 30 | 20 | 80 | 20 | 20 | 70 |
| Ryegrass, Italian | 60 | 90 | 90 | 100 | 90 | 100 | 0 | 80 | 30 | 70 | 30 | 50 | 60 | 100 |

| Table A | Compounds | | | | | | | | | | | | | |
|---|---|---|---|---|---|---|---|---|---|---|---|---|---|---|
| 125 g ai/ha | 188 | 189 | 190 | 191 | 192 | 193 | 194 | 195 | 196 | 197 | 198 | 199 | 200 | 201 |
| Preemergence | | | | | | | | | | | | | | |
| Barnyardgrass | 60 | 80 | 90 | 100 | 30 | - | 90 | 60 | 0 | 90 | 80 | 30 | 30 | 50 |
| Foxtail, Giant | 0 | 100 | 90 | 100 | 10 | 70 | 80 | 70 | 30 | 100 | 100 | 90 | 90 | 50 |
| Kochia | 20 | 80 | 90 | 90 | 20 | 0 | 40 | 0 | 70 | 90 | 60 | 0 | 60 | 90 |
| Pigweed | 50 | 90 | 100 | 100 | 70 | 60 | 90 | 60 | 50 | 80 | 70 | 80 | 100 | 100 |
| Ragweed | 40 | 80 | 70 | 60 | 30 | 70 | 80 | 0 | 0 | 80 | 70 | 50 | 40 | 70 |
| Ryegrass, Italian | 50 | 90 | 70 | 70 | 50 | 60 | 30 | 20 | 20 | 100 | 60 | 50 | 30 | 60 |

| Table A | Compounds | | | | | | | | | | | | | |
|---|---|---|---|---|---|---|---|---|---|---|---|---|---|---|
| 125 g ai/ha | 202 | 203 | 204 | 205 | 206 | 207 | 208 | 209 | 210 | 211 | 212 | 213 | 214 | 215 |
| Preemergence | | | | | | | | | | | | | | |
| Barnyardgrass | 30 | 90 | 90 | 50 | 90 | 90 | 90 | 40 | 90 | 60 | 0 | 20 | 0 | 60 |
| Foxtail, Giant | 60 | 30 | 80 | 50 | 80 | 90 | 90 | 20 | 90 | 60 | 20 | 30 | 40 | 40 |
| Kochia | 40 | 60 | 40 | 0 | 80 | 90 | 90 | 0 | 30 | 80 | 30 | 80 | 70 | 80 |
| Pigweed | 100 | 60 | 90 | 70 | 90 | 100 | 60 | 0 | 100 | 20 | 90 | 50 | 30 | 100 |
| Ragweed | 70 | 80 | 10 | 40 | 40 | 20 | 0 | 0 | 80 | 30 | 0 | 0 | 0 | 0 |
| Ryegrass, Italian | 40 | 50 | 90 | 60 | 80 | 90 | 70 | 50 | 50 | 50 | 30 | 60 | 60 | 20 |

| Table A | Compounds | | | | | | | | | | | | | |
|---|---|---|---|---|---|---|---|---|---|---|---|---|---|---|
| 125 g ai/ha | 216 | 217 | 218 | 219 | 220 | 221 | 222 | 223 | 224 2 | 225 | 226 | 227 | 228 | 229 |
| Preemergence | | | | | | | | | | | | | | |
| Barnyardgrass | 40 | 90 | 100 | 0 | 100 | 100 | - | 30 | 70 | 70 | 90 | 60 | 100 | 70 |
| Foxtail, Giant | 20 | 90 | 100 | 50 | 100 | 100 | 40 | 60 | 90 | 90 | 90 | 90 | 90 | 60 |
| Kochia | 100 | 70 | 30 | 20 | 20 | 70 | 0 | 0 | 0 | 0 | 40 | 60 | 60 | 80 |
| Pigweed | 80 | 70 | 80 | 40 | 70 | 60 | 50 | 10 | 100 | 70 | 80 | 50 | 100 | 100 |
| Ragweed | 0 | 60 | 20 | 0 | 30 | 90 | 60 | 0 | 50 | 30 | 30 | 30 | 10 | 90 |
| Ryegrass, Italian | 30 | 80 | 100 | 50 | 100 | 100 | 30 | 30 | 50 | 70 | 90 | 40 | 100 | 60 |

| Table A | Compounds | | | | | | | | | | | | | |
|---|---|---|---|---|---|---|---|---|---|---|---|---|---|---|
| 125 g ai/ha | 230 | 231 | 232 | 233 | 234 | 235 | 236 | 237 | 238 | 239 | 240 | 241 | 243 | 244 |
| Preemergence | | | | | | | | | | | | | | |
| Barnyardgrass | 70 | 90 | 100 | 0 | 80 | 0 | 0 | 40 | 0 | 0 | 0 | 100 | 80 | 70 |
| Foxtail, Giant | 100 | 100 | 100 | 40 | 50 | 10 | 30 | 20 | 30 | 20 | 30 | 80 | 100 | 50 |
| Kochia | 60 | 0 | 0 | 0 | 0 | 30 | 20 | 90 | 80 | 30 | 70 | 70 | 20 | 80 |
| Pigweed | 100 | 100 | 100 | 60 | 50 | 30 | 60 | 80 | 20 | 60 | 0 | 100 | 100 | - |
| Ragweed | 0 | 80 | 70 | 0 | 80 | 40 | 50 | 30 | 20 | 20 | 30 | 70 | 70 | 90 |
| Ryegrass, Italian | 20 | 80 | 100 | 20 | 20 | 50 | 20 | 20 | 60 | 0 | 40 | 70 | 90 | 50 |

| Table A | Compounds | | | | | | | | | | | | | |
|---|---|---|---|---|---|---|---|---|---|---|---|---|---|---|
| 125 g ai/ha | 245 | 246 | 247 | 248 | 249 | 250 | 251 | 252 | 253 | 254 | 255 | 256 | 257 | 265 |
| Preemergence | | | | | | | | | | | | | | |
| Barnyardgrass | 90 | 40 | 0 | 80 | 60 | 40 | 20 | 40 | 60 | 30 | 40 | 90 | 40 | 90 |
| Foxtail, Giant | 100 | 90 | 50 | 40 | 50 | 90 | 50 | 100 | 100 | 20 | 90 | 30 | 70 | 100 |
| Kochia | 80 | 40 | 0 | 90 | 0 | 80 | 90 | 60 | 70 | 70 | 70 | 60 | 60 | 90 |
| Pigweed | - | 90 | 90 | 90 | 80 | 100 | 100 | 100 | 100 | 70 | 100 | 100 | 100 | - |
| Ragweed | 90 | 80 | 0 | 80 | 0 | 80 | 80 | 80 | 40 | 10 | 20 | 60 | 30 | 90 |
| Ryegrass, Italian | 80 | 70 | 20 | 70 | 40 | 60 | 90 | 60 | 60 | 80 | 80 | 80 | 60 | 90 |

| Table A | Compounds | | | | | | | | | |
|---|---|---|---|---|---|---|---|---|---|---|
| 125 g ai/ha | 266 | 267 | 268 | 269 | 270 | 276 | 277 | 278 | 279 | 280 |
| Preemergence | | | | | | | | | | |
| Barnyardgrass | 0 | 100 | 40 | 100 | 90 | 70 | 100 | 60 | 100 | 90 |
| Foxtail, Giant | 0 | 100 | 50 | 100 | 100 | 100 | 100 | 60 | 100 | 100 |
| Kochia | 0 | 80 | 70 | 70 | 0 | 0 | 90 | 90 | 80 | 40 |
| Pigweed | - | - | - | - | 70 | 80 | 60 | - | 90 | 70 |
| Ragweed | 0 | 100 | 40 | 80 | 0 | 40 | 10 | 90 | 100 | 30 |
| Ryegrass, Italian | 0 | 90 | 0 | 80 | 100 | 30 | 100 | 100 | 90 | 100 |

| Table A | Compounds | | | | | | | | | | | | | |
|---|---|---|---|---|---|---|---|---|---|---|---|---|---|---|
| 31 g ai/ha | 1 | 2 | 3 | 27 | 36 | 88 | 91 | 92 | 93 | 94 | 95 | 96 | 97 | 98 |
| Preemergence | | | | | | | | | | | | | | |
| Barnyardgrass | 0 | 0 | 0 | 0 | 0 | 20 | 0 | 0 | 0 | 10 | 10 | 10 | 0 | 0 |
| Foxtail, Giant | 20 | 80 | 20 | 10 | 20 | 60 | 20 | 0 | 60 | 50 | 90 | 40 | 0 | 80 |
| Kochia | 40 | 20 | 30 | 0 | 0 | 30 | 0 | 0 | 0 | 0 | 0 | 0 | 0 | 40 |
| Pigweed | 70 | 100 | 90 | 0 | 0 | 0 | 60 | 0 | 50 | 100 | 70 | 20 | 10 | 70 |
| Ragweed | 30 | 30 | 0 | 0 | 0 | 0 | 0 | 0 | 0 | 0 | 0 | 0 | 0 | 30 |
| Ryegrass, Italian | 40 | 30 | 20 | 0 | 0 | 0 | 30 | 0 | 0 | 30 | 20 | 0 | 0 | 30 |

| Table A | Compounds | | | | | | | | | | | | | |
|---|---|---|---|---|---|---|---|---|---|---|---|---|---|---|
| 31 g ai/ha | 111 | 112 | 113 | 115 | 118 | 131 | 137 | 143 | 144 | 145 | 148 | 149 | 150 | 151 |
| Preemergence | | | | | | | | | | | | | | |
| Barnyardgrass | 10 | 20 | 0 | 50 | 0 | 70 | 20 | 80 | 20 | 0 | 20 | 0 | 0 | 0 |
| Foxtail, Giant | 0 | - | 10 | 100 | 80 | 90 | 50 | 50 | 90 | 0 | 40 | 30 | 20 | 60 |
| Kochia | 0 | 10 | 10 | 0 | 0 | 30 | 30 | 0 | 0 | 0 | 20 | 0 | 0 | 0 |
| Pigweed | 10 | 50 | 30 | 100 | 100 | 30 | 80 | 60 | 70 | 20 | 30 | 30 | 40 | 50 |
| Ragweed | 10 | 30 | 0 | 0 | 50 | 0 | 20 | 0 | 0 | 0 | 0 | 0 | 0 | 0 |
| Ryegrass, Italian | 0 | 0 | 0 | 70 | 40 | 30 | 60 | 90 | 70 | 0 | 30 | 50 | 40 | 30 |

| Table A | Compounds | | | | | | | | | | |
|---|---|---|---|---|---|---|---|---|---|---|---|
| 31 g ai/ha | 152 | 153 | 154 | 155 | 156 | 157 | 161 | 162 | 167 | 168 | 174 |
| Preemergence | | | | | | | | | | | |
| Barnyardgrass | 0 | 0 | 0 | 50 | 70 | 30 | 20 | 0 | 0 | 0 | 10 |
| Foxtail, Giant | 40 | 40 | 60 | 50 | 50 | 80 | 40 | 100 | 0 | 0 | 30 |
| Kochia | 0 | 60 | 0 | 30 | 30 | 50 | 30 | 30 | 30 | 0 | 40 |
| Pigweed | 30 | 80 | 20 | 90 | 80 | 100 | 70 | 100 | 0 | 0 | 90 |
| Ragweed | 0 | 40 | 50 | 90 | 60 | 40 | 90 | 0 | 0 | 0 | 30 |
| Ryegrass, Italian | 0 | 30 | 20 | 80 | 50 | 40 | 70 | 40 | 30 | 0 | 40 |

| Table A | Compounds | | | | | | | | | | | | | |
|---|---|---|---|---|---|---|---|---|---|---|---|---|---|---|
| 1000 g ai/ha | 4 | 5 | 6 | 7 | 8 | 10 | 11 | 12 | 19 | 50 | 242 | 258 | 259 | 260 |
| Postemergence | | | | | | | | | | | | | | |
| Barnyardgrass | 90 | 80 | 90 | 20 | 80 | 10 | 90 | 80 | 80 | 60 | 90 | 70 | 0 | 90 |
| Blackgrass | 40 | 0 | 60 | 20 | 70 | 0 | 50 | 30 | 60 | 20 | 40 | 50 | 50 | 50 |
| Corn | 40 | 10 | 60 | 0 | 30 | 10 | 60 | 20 | 50 | 10 | 70 | 10 | 10 | 10 |
| Foxtail, Giant | 90 | 90 | 90 | 0 | 90 | 20 | 90 | 90 | 90 | 90 | 90 | 50 | 0 | 50 |
| Galium | 90 | 90 | 90 | 40 | 20 | 80 | 90 | 90 | 40 | 90 | 90 | 80 | 60 | 70 |
| Kochia | 80 | 80 | 80 | 40 | 80 | 40 | 60 | 70 | 70 | 60 | 80 | 90 | 40 | 0 |
| Pigweed | 80 | 80 | 100 | 20 | 80 | 80 | 100 | 80 | 80 | 90 | 90 | 80 | 40 | 80 |
| Ragweed | 80 | 80 | 50 | 20 | 90 | 80 | 80 | 60 | 80 | 50 | 90 | 80 | 10 | 50 |
| Ryegrass, Italian | 80 | 30 | 80 | 90 | 80 | 60 | 70 | 90 | 50 | 90 | 0 | 70 | 10 | 60 |
| Wheat | 70 | 60 | 60 | 0 | 40 | 0 | 80 | 60 | 50 | 50 | 60 | 30 | 0 | 60 |

| Table A | Compounds | | | |
|---|---|---|---|---|
| 1000 g ai/ha | 261 | 262 | 263 | 264 |
| Postemergence | | | | |
| Barnyardgrass | 0 | 0 | 30 | 50 |
| Blackgrass | 0 | 20 | 20 | 60 |
| Corn | 10 | 0 | 20 | 20 |
| Foxtail, Giant | 0 | 0 | 0 | 40 |
| Galium | 50 | 40 | 60 | 70 |
| Kochia | 0 | 0 | 10 | 20 |
| Pigweed | 30 | 20 | 0 | 60 |
| Ragweed | 0 | 0 | 0 | 60 |
| Ryegrass, Italian | 20 | 20 | 80 | 50 |
| Wheat | 0 | 0 | 0 | 70 |

| Table A | Compounds | | | | | | | | | | | | | |
|---|---|---|---|---|---|---|---|---|---|---|---|---|---|---|
| 500 g ai/ha | 9 | 13 | 14 | 15 | 16 | 17 | 18 | 20 | 21 | 22 | 23 | 24 | 25 | 26 |
| Postemergence | | | | | | | | | | | | | | |
| Barnyardgrass | 90 | 40 | 80 | 50 | 90 | 90 | 70 | 80 | 40 | 50 | 70 | 90 | 80 | 70 |
| Blackgrass | 30 | 0 | 40 | 30 | 40 | 30 | 0 | 30 | 10 | 0 | 30 | 30 | 20 | 30 |
| Corn | 70 | 0 | 20 | 0 | 50 | 20 | 0 | 70 | 30 | 30 | 70 | 40 | 0 | 20 |
| Foxtail, Giant | 90 | 10 | 70 | 50 | 90 | 90 | 70 | 90 | 20 | 60 | 90 | 60 | 80 | 40 |
| Galium | 90 | 30 | 80 | 60 | 90 | 90 | 80 | 90 | 60 | 90 | 90 | 90 | 80 | 90 |
| Kochia | 30 | 10 | 70 | 70 | 80 | 70 | 40 | 30 | 30 | 40 | 70 | 80 | 60 | 10 |
| Pigweed | 80 | 20 | 90 | 30 | 90 | 70 | 90 | 80 | 40 | - | 80 | 100 | 70 | 90 |
| Ragweed | 80 | 30 | 70 | 80 | 90 | 90 | 80 | 80 | 90 | 50 | 80 | 80 | 90 | 100 |
| Ryegrass, Italian | 80 | 0 | 30 | 0 | 70 | 70 | 40 | 70 | 50 | 60 | 80 | 40 | 60 | 0 |
| Wheat | 70 | 0 | 70 | 0 | 70 | 60 | 70 | 70 | 30 | 60 | 70 | 80 | 70 | 60 |

| Table A | Compounds | | | | | | | | | | | | |
|---|---|---|---|---|---|---|---|---|---|---|---|---|---|
| 500 g ai/ha | 28 | 29 | 30 | 32 | 34 | 35 | 37 | 38 | 39 | 40 | 41 | 42 | 43 |
| Postemergence | | | | | | | | | | | | | |
| Barnyardgrass | 80 | 90 | 90 | 90 | 0 | 80 | 90 | 80 | 30 | 40 | 80 | 80 | 90 |
| Blackgrass | 30 | 90 | 80 | 90 | 0 | 30 | 90 | 90 | 0 | 10 | 40 | 90 | 90 |
| Corn | 0 | 10 | 40 | 80 | 0 | 0 | 80 | 70 | 0 | 0 | 0 | 60 | 80 |
| Foxtail, Giant | 10 | 90 | 90 | 90 | 0 | 10 | 90 | 90 | 0 | 10 | 30 | 90 | 90 |
| Galium | 70 | 90 | 90 | 90 | 0 | 70 | 100 | 90 | 0 | 70 | 30 | 60 | 90 |
| Kochia | 30 | 80 | 50 | 40 | 50 | 10 | 70 | 70 | 0 | 20 | 0 | 30 | 40 |
| Pigweed | 40 | 50 | 60 | 20 | 0 | 60 | 50 | 90 | 0 | 30 | 0 | 100 | 80 |
| Ragweed | 80 | 90 | 40 | 60 | 0 | 40 | 70 | 90 | 70 | 40 | 70 | 80 | 60 |
| Ryegrass, Italian | 0 | 90 | 70 | 90 | 0 | 40 | 90 | 80 | 40 | 60 | 60 | 80 | 50 |
| Wheat | 50 | 70 | 70 | 70 | 0 | 40 | 80 | 70 | 60 | 40 | 60 | 70 | 70 |

| Table A | Compounds | | | | | | | | | | | | | |
|---|---|---|---|---|---|---|---|---|---|---|---|---|---|---|
| 500 g ai/ha | 44 | 45 | 46 | 47 | 48 | 51 | 52 | 53 | 54 | 55 | 56 | 57 | 58 | 59 |
| Postemergence | | | | | | | | | | | | | | |
| Barnyardgrass | 90 | 70 | 0 | 80 | 80 | 80 | 80 | 0 | 10 | 40 | 90 | 90 | 90 | 80 |
| Blackgrass | 90 | 60 | 0 | 50 | 40 | 70 | 80 | 0 | 0 | 20 | 50 | 70 | 30 | 70 |
| Corn | 70 | 30 | 0 | 0 | 80 | 20 | 0 | 0 | 10 | 50 | 80 | 80 | 50 | 40 |
| Foxtail, Giant | 90 | 60 | 0 | 80 | 90 | 80 | 80 | 0 80 | 0 | 90 | 90 | 90 | 90 | 70 |
| Galium | 90 | 40 | 0 | 40 | 90 | 60 | 80 | | 90 | 90 | 90 | 90 | 90 | 90 |
| Kochia | 30 | 50 | 0 | 0 | 10 | 60 | 80 | 0 | 80 | 80 | 60 | 20 | 20 | 70 |
| Pigweed | 90 | 70 | 0 | 0 | 60 | 80 | 50 | 0 | 70 | 70 | 40 | 50 | 0 | 60 |
| Ragweed | 50 | 70 | 0 | 80 | 90 | 80 | 60 | 20 | 90 | 40 | 90 | 30 | 20 | 60 |
| Ryegrass, Italian | 30 | 90 | 0 | 80 | 90 | 80 | 40 | 0 | 40 | 60 | 80 | 90 | 60 | 60 |
| Wheat | 70 | 70 | 50 | 60 | 70 | 60 | 70 | 0 | 0 | 70 | 70 | 70 | 70 | 40 |

| Table A | Compounds | | | | | | | | | | | | | |
|---|---|---|---|---|---|---|---|---|---|---|---|---|---|---|
| 500 g ai/ha | 60 | 61 | 62 | 63 | 64 | 65 | 66 | 67 | 68 | 69 | 70 | 71 | 72 | 73 |
| Postemergence | | | | | | | | | | | | | | |
| Barnyardgrass | 70 | 80 | 70 | 90 | 20 | 70 | 30 | 40 | 80 | 80 | 50 | 80 | - | - |
| Blackgrass | 70 | 30 | 50 | 40 | 30 | 0 | 0 | 60 | 70 | 50 | 30 | 70 | 80 | 80 |
| Corn | 60 | 10 | 0 | 50 | 0 | 0 | 10 | 0 | 20 | 20 | 0 | 30 | 50 | 70 |
| Foxtail, Giant | 90 | 70 | 80 | 90 | 60 | 40 | 30 | 70 | 90 | 90 | 20 | 90 | 90 | 90 |
| Galium | 90 | 60 | 80 | 90 | 30 | 30 | 70 | 60 | 80 | 70 | 80 | 80 | 90 | 90 |
| Kochia | 80 | 60 | 40 | 90 | 60 | 0 | 10 | 40 | 50 | 60 | 0 | 40 | 90 | 70 |
| Pigweed | 90 | 80 | 40 | 80 | 30 | 0 | 50 | 80 | 50 | 90 | 20 | 60 | 80 | 80 |
| Ragweed | 70 | 30 | 20 | 60 | 0 | 0 | 0 | 10 | 80 | 80 | 0 | 0 | 70 | 80 |
| Ryegrass, Italian | 80 | 80 | 20 | 70 | 80 | 0 | 0 | 60 | 80 | 70 | 0 | 80 | 80 | 90 |
| Wheat | 70 | 70 | 60 | 80 | 60 | 50 | 40 | 60 | 80 | 70 | 30 | 70 | 70 | 70 |

| Table A | Compounds | | | | | | | | | | | | | |
|---|---|---|---|---|---|---|---|---|---|---|---|---|---|---|
| 500 g ai/ha | 74 | 75 | 76 | 77 | 78 | 79 | 80 | 81 | 82 | 83 | 84 | 85 | 99 | 100 |
| Postemergence | | | | | | | | | | | | | | |
| Barnyardgrass | - | - | - | 20 | 70 | 90 | 90 | 80 | 20 | 70 | 80 | 20 | 90 | 90 |
| Blackgrass | 50 | 70 | 50 | 0 | 30 | 30 | 100 | 70 | 0 | 0 | 20 | 0 | 20 | 0 |
| Corn | 10 | 0 | 70 | 60 | 50 | 70 | 80 | 20 | 40 | 30 | 60 | 0 | 0 | 30 |
| Foxtail, Giant | 80 | 90 | 90 | 40 | 90 | 90 | 90 | 90 | 50 | 40 | 50 | 0 | 10 | 40 |
| Galium | 90 | 90 | 100 | 40 | 90 | 90 | 90 | 90 | 10 | 80 | 50 | 0 | 90 | 90 |
| Kochia | 90 | 80 | 90 | 10 | 20 | 70 | 70 | 50 | 0 | 20 | 0 | 0 | 90 | 90 |
| Pigweed | 90 | 80 | 90 | 0 | 50 | 80 | 70 | 60 | 40 | 50 | 30 | 40 | 100 | 90 |
| Ragweed | 30 | 50 | 90 | 10 | 40 | 80 | 90 | 60 | 30 | 60 | 40 | 30 | 80 | 80 |
| Ryegrass, Italian | 70 | 60 | 80 | 0 | 60 | 80 | 80 | 60 | 0 | 0 | 50 | 30 | 60 | 40 |
| Wheat | 70 | 70 | 80 | 70 | 70 | 80 | 80 | 60 | 0 | 0 | 70 | 0 | 50 | 50 |

| Table A | Compounds | | | | | | | | | | | | |
|---|---|---|---|---|---|---|---|---|---|---|---|---|---|
| 500 g ai/ha | 101 | 102 | 103 | 104 | 105 | 107 | 108 | 109 | 110 | 114 | 116 | 119 | 120 |
| Postemergence | | | | | | | | | | | | | |
| Barnyardgrass | 90 | 90 | 90 | 90 | 90 | 90 | 90 | 70 | 80 | 70 | 90 | 90 | 90 |
| Blackgrass | 20 | 0 | 30 | 20 | 50 | 30 | 50 | 10 | 0 | 50 | 40 | 40 | 70 |
| Corn | 30 | 30 | 70 | 80 | 90 | 80 | 50 | 40 | 10 | 50 | 80 | 30 | 70 |
| Foxtail, Giant | 80 | 70 | 70 | 70 | 90 | 50 | 30 | 40 | 90 | 80 | 80 | 80 | 90 |
| Galium | 90 | 100 | 100 | 90 | 90 | 90 | 90 | 90 | 80 | 90 | 90 | 90 | 90 |
| Kochia | 30 | 90 | 80 | 40 | 80 | 40 | 80 | 40 | 50 | 40 | 70 | 60 | 90 |
| Pigweed | 90 | 80 | 80 | 80 | 80 | 60 | 60 | 80 | 100 | 80 | 80 | 70 | 90 |
| Ragweed | 70 | 90 | 70 | 90 | 90 | 90 | 80 | 80 | 90 | 30 | 90 | 90 | 90 |
| Ryegrass, Italian | 70 | 90 | 90 | 40 | 90 | 30 | 70 | 60 | 50 | 40 | 80 | 90 | 80 |
| Wheat | 60 | 80 | 80 | 60 | 70 | 40 | 70 | 70 | 60 | 60 | 80 | 70 | 70 |

| Table A | Compounds | | | | | | | | | | | | | |
|---|---|---|---|---|---|---|---|---|---|---|---|---|---|---|
| 500 g ai/ha | 121 | 122 | 123 | 124 | 125 | 126 | 127 | 128 | 129 | 130 | 132 | 133 | 134 | 135 |
| Postemergence | | | | | | | | | | | | | | |
| Barnyardgrass | 90 | 70 | 90 | 0 | 90 | 0 | 70 | 90 | 80 | 90 | 40 | 10 | 10 | 90 |
| Blackgrass | 80 | 10 | 50 | 20 | 60 | 30 | 20 | 30 | 30 | 10 | 0 | 0 | 0 | 60 |
| Corn | 80 | 0 | 70 | 10 | 60 | 20 | 20 | 60 | 20 | 90 | 20 | 10 | 0 | 80 |
| Foxtail, Giant | 90 | 20 | 80 | 0 | 50 | 0 | 0 | 50 | 30 | 90 | 40 | 10 | 10 | 90 |
| Galium | 90 | 90 | 90 | 80 | 90 | 90 | 90 | 90 | 80 | 90 | 90 | 90 | 80 | 90 |
| Kochia | 80 | 10 | 70 | 20 | 80 | 30 | 40 | 70 | 50 | 70 | 20 | 20 | 70 | 70 |
| Pigweed | 90 | 50 | 80 | 50 | 90 | 90 | 40 | 90 | 90 | 90 | 90 | 90 | 90 | 70 |
| Ragweed | 90 | 0 | 70 | 0 | 80 | 30 | 50 | 90 | 90 | 90 | 90 | 80 | 40 | 80 |
| Ryegrass, Italian | 80 | 0 | 50 | 0 | 70 | 0 | 50 | 100 | 70 | 90 | 40 | 40 | 0 | 80 |
| Wheat | 70 | 0 | 60 | 0 | 70 | 0 | 0 | 60 | 60 | 70 | 0 | 50 | 0 | 70 |

| Table A | Compounds | | | | | | | | | | | | | |
|---|---|---|---|---|---|---|---|---|---|---|---|---|---|---|
| 500 g ai/ha | 136 | 139 | 140 | 141 | 142 | 146 | 147 | 158 | 159 | 160 | 163 | 164 | 165 | 166 |
| Postemergence | | | | | | | | | | | | | | |
| Barnyardgrass | 80 | 0 | 90 | 90 | 90 | 80 | 90 | 20 | 80 | 80 | 80 | 90 | 30 | 90 |
| Blackgrass | 20 | 0 | 30 | 20 | 20 | 20 | 30 | 0 | 20 | 10 | 30 | 40 | 10 | 20 |
| Corn | 30 | 0 | 30 | 90 | 90 | 20 | 80 | 20 | 30 | 10 | 60 | 80 | 0 | 50 |
| Foxtail, Giant | 50 | 0 | 90 | 50 | 90 | 90 | 90 | 20 | 30 | 60 | 10 | 40 | 0 | 60 |
| Galium | 90 | 80 | 70 | 90 | 90 | 90 | 90 | 90 | 90 | 90 | 90 | 90 | 90 | 90 |
| Kochia | 40 | 50 | 60 | 50 | 40 | 50 | 70 | 30 | 40 | 30 | 60 | 90 | 60 | 80 |
| Pigweed | 90 | 80 | 80 | 90 | 80 | 80 | 90 | 80 | 70 | 90 | 80 | 90 | 90 | 90 |
| Ragweed | 50 | 0 | 50 | 90 | 90 | 90 | 90 | 20 | 90 | 80 | 30 | 90 | 60 | 80 |
| Ryegrass, Italian | 0 | 0 | 20 | 90 | 80 | 70 | 70 | 0 | 0 | 0 | 30 | 90 | 30 | 90 |
| Wheat | 60 | 0 | 60 | 70 | 70 | 60 | 60 | 0 | 50 | 20 | 0 | 70 | 0 | 70 |

| Table A | Compounds | | | | | | | | | | | | | |
|---|---|---|---|---|---|---|---|---|---|---|---|---|---|---|
| 500 g ai/ha | 169 | 170 | 171 | 172 | 173 | 175 | 176 | 177 | 178 | 179 | 180 | 181 | 182 | 183 |
| Postemergence | | | | | | | | | | | | | | |
| Barnyardgrass | 90 | 20 | 90 | 80 | 90 | 90 | 90 | 90 | 90 | 90 | 10 | 90 | 30 | 70 |
| Blackgrass | 20 | 0 | 80 | 40 | 60 | 20 | 70 | 30 | 0 | 40 | 20 | 30 | 0 | 10 |
| Corn | 90 | 30 | 80 | 70 | 80 | 10 | 80 | 50 | 10 | 80 | 0 | 80 | 60 | 30 |
| Foxtail, Giant | 40 | 80 | 90 | 80 | 90 | 30 | 90 | 60 | 20 | 90 | 10 | 90 | 60 | 10 |
| Galium | 90 | 90 | 90 | 90 | 90 | 90 | 90 | 90 | 90 | 90 | 90 | 70 | 40 | 90 |
| Kochia | 50 | 40 | 70 | 80 | 80 | 50 | 50 | 70 | 70 | 70 | 40 | 80 | 20 | 50 |
| Pigweed | 50 | 80 | 80 | 90 | 90 | 90 | 90 | 90 | 90 | 90 | 70 | 80 | 70 | 30 |
| Ragweed | 90 | 30 | 90 | 80 | 90 | 90 | 90 | 90 | 80 | 90 | 70 | 90 | 90 | 80 |
| Ryegrass, Italian | 30 | 0 | 80 | 80 | 80 | 80 | 80 | 90 | 30 | 80 | 0 | 90 | 80 | 0 |
| Wheat | 0 | 30 | 60 | 70 | 70 | 80 | 60 | 80 | 30 | 60 | 0 | 80 | 70 | 0 |

| Table A | Compounds | | | | | | | | | | | | | |
|---|---|---|---|---|---|---|---|---|---|---|---|---|---|---|
| 500 g ai/ha | 184 | 185 | 186 | 187 | 188 | 189 | 190 | 191 | 192 | 193 | 194 | 195 | 196 | 197 |
| Postemergence | | | | | | | | | | | | | | |
| Barnyardgrass | 50 | 20 | 20 | 90 | 70 | 90 | 90 | 30 | 60 | 80 | 80 | 0 | - | - |
| Blackgrass | 20 | 10 | 20 | 20 | 0 | 20 | 40 | 0 | 0 | 60 | 0 | 0 | 0 | 60 |
| Corn | 0 | 0 | 30 | 90 | 30 | 80 | 30 | 10 | 70 | 10 | 0 | 0 | 0 | 80 |
| Foxtail, Giant | 0 | 0 | 20 | 90 | 20 | 90 | 80 | 20 | 70 | 90 | 80 | 0 | 0 | 90 |
| Galium | 90 | 70 | 80 | 80 | 50 | 90 | 90 | 90 | 50 | 90 | 90 | 20 | 80 | 90 |
| Kochia | 30 | 10 | 40 | 70 | 0 | 80 | 70 | 20 | 20 | 60 | 40 | 0 | 20 | 80 |
| Pigweed | 40 | 40 | 50 | 60 | 80 | 90 | 100 | 70 | 80 | 90 | 50 | 0 | 50 | 90 |
| Ragweed | 70 | 40 | 60 | 90 | 70 | 90 | 90 | 60 | 80 | 90 | 90 | 50 | 30 | 80 |
| Ryegrass, Italian | 0 | 0 | 20 | 80 | 40 | 80 | 90 | 60 | 90 | 80 | 50 | 0 | 30 | 60 |
| Wheat | 0 | 0 | 30 | 70 | 70 | 70 | 70 | 60 | 80 | 70 | 60 | 0 | 0 | 70 |

| Table A | Compounds | | | | | | | | | | | | | |
|---|---|---|---|---|---|---|---|---|---|---|---|---|---|---|
| 500 g ai/ha | 198 | 199 | 200 | 201 | 202 | 203 | 204 | 205 | 206 | 207 | 208 | 209 | 210 | 211 |
| Postemergence | | | | | | | | | | | | | | |
| Barnyardgrass | - | - | - | - | - | 80 | 80 | 20 | 80 | 80 | 30 | 70 | 90 | 30 |
| Blackgrass | 0 | 50 | 0 | 30 | 0 | 30 | 40 | 0 | 50 | 60 | 20 | 30 | 0 | 0 |
| Corn | 20 | 30 | 20 | 50 | 20 | 60 | 50 | 0 | 70 | 70 | 20 | 30 | 40 | 0 |
| Foxtail, Giant | 30 | 90 | 20 | 90 | 70 | 50 | 60 | 20 | 60 | 90 | 40 | 10 | 30 | 50 |
| Galium | 90 | 90 | 90 | 90 | 80 | 90 | 80 | 70 | 80 | 70 | 70 | 80 | 90 | 70 |
| Kochia | 60 | 80 | 40 | 90 | 50 | 80 | 70 | 10 | 70 | 60 | 30 | 40 | 90 | 50 |
| Pigweed | 70 | 90 | 90 | 100 | 100 | 60 | 70 | 30 | 70 | 70 | 50 | 50 | 100 | 60 |
| Ragweed | 80 | 90 | 30 | 90 | 90 | 90 | 80 | 20 | 90 | 70 | 20 | 60 | 90 | 60 |
| Ryegrass, Italian | 40 | 70 | 20 | 70 | 70 | 60 | 50 | 0 | 90 | 90 | 40 | 90 | 40 | 30 |
| Wheat | 40 | 70 | 70 | 70 | 60 | 70 | 70 | 30 | 80 | 70 | 50 | 0 | 60 | 0 |

| Table A | Compounds | | | | | | | | | | | | | |
|---|---|---|---|---|---|---|---|---|---|---|---|---|---|---|
| 500 g ai/ha | 212 | 213 | 214 | 215 | 216 | 217 | 218 | 219 | 220 | 221 | 222 | 223 | 224 | 225 |
| Postemergence | | | | | | | | | | | | | | |
| Barnyardgrass | 0 | 70 | 0 | 40 | 50 | 30 | 90 | 70 | 70 | 80 | 70 | 20 | - | 10 |
| Blackgrass | 0 | 20 | 0 | 0 | 30 | 20 | 40 | 0 | 30 | 30 | 20 | 0 | 40 | 20 |
| Corn | 10 | 20 | 10 | 60 | 40 | 20 | 70 | 0 | 30 | 40 | 0 | 0 | 60 | 0 |
| Foxtail, Giant | 20 | 20 | 30 | 50 | 20 | 0 | 90 | 20 | 50 | 90 | 10 | 0 | 70 | 0 |
| Galium | 70 | 70 | 60 | 70 | 70 | 90 | 80 | 70 | 80 | 100 | 90 | 40 | 90 | 80 |
| Kochia | 70 | 60 | 20 | 70 | 30 | 70 | 80 | 60 | 60 | 70 | 50 | 0 | 70 | 10 |
| Pigweed | 60 | 80 | 80 | 80 | 70 | 80 | 80 | 70 | 70 | 80 | 40 | 30 | 100 | 30 |
| Ragweed | 20 | 0 | 0 | 80 | 60 | 70 | 80 | 50 | 40 | 90 | 90 | 20 | 90 | 40 |
| Ryegrass, Italian | 50 | 60 | 30 | 60 | 70 | 0 | 80 | 50 | 20 | 60 | 0 | 0 | 60 | 40 |
| Wheat | 0 | 70 | 40 | 70 | 70 | 30 | 70 | 0 | 60 | 70 | 70 | 0 | 70 | 0 |

| Table A | Compounds | | | | | | | | | | | | | |
|---|---|---|---|---|---|---|---|---|---|---|---|---|---|---|
| 500 g ai/ha | 226 | 227 | 228 | 229 | 230 | 231 | 232 | 233 | 234 | 235 | 236 | 237 | 238 | 239 |
| Postemergence | | | | | | | | | | | | | | |
| Barnyardgrass | - | - | - | - | - | 80 | 70 | 0 | 70 | 60 | 60 | 40 | 60 | 0 |
| Blackgrass | 50 | 20 | 0 | 70 | 40 | 0 | 40 | 40 | 0 | 20 | 20 | 0 | 30 | 0 |
| Corn | 70 | 0 | 70 | 70 | 20 | 0 | 0 | 0 | 0 | 30 | 20 | 20 | 0 | 10 |
| Foxtail, Giant | 60 | 20 | 40 | 90 | 70 | 80 | 50 | 0 | 50 | 30 | 80 | 40 | 70 | 30 |
| Galium | 80 | 90 | 90 | 90 | 90 | 80 | 90 | 90 | 80 | 70 | 70 | 50 | 80 | 40 |
| Kochia | 70 | 60 | 80 | 90 | 90 | 70 | 70 | 30 | 0 | 30 | 50 | 90 | 30 | 10 |
| Pigweed | 80 | 50 | 70 | 100 | 90 | 100 | 100 | 60 | 60 | 30 | 60 | 60 | 80 | 50 |
| Ragweed | 90 | 10 | 30 | 90 | 60 | 90 | 80 | 0 | 50 | 90 | 80 | 80 | 80 | 0 |
| Ryegrass, Italian | 60 | 0 | 60 | 70 | 70 | 70 | 70 | 0 | 0 | 60 | 60 | 40 | 70 | 0 |
| Wheat | 60 | 40 | 70 | 70 | 50 | 60 | 0 | 40 | 0 | 60 | 0 | 0 | 70 | 0 |

| Table A | Compounds | | | | | | | | | | | | | |
|---|---|---|---|---|---|---|---|---|---|---|---|---|---|---|
| 500 g ai/ha | 240 | 241 | 243 | 244 | 245 | 246 | 247 | 248 | 249 | 250 | 251 | 252 | 253 | 254 |
| Postemergence | | | | | | | | | | | | | | |
| Barnyardgrass | 70 | 90 | 70 | 90 | 90 | 90 | 0 | 90 | 0 | 40 | 70 | 30 | 20 | 30 |
| Blackgrass | 10 | 80 | 10 | 40 | 60 | 0 | 0 | 60 | 0 | 50 | 60 | 0 | 0 | 0 |
| Corn | 10 | 20 | 0 | 20 | 60 | 40 | 0 | 0 | 0 | 30 | 30 | 0 | 0 | 20 |
| Foxtail, Giant | 60 | 30 | 40 | 90 | 90 | 90 | 40 | 50 | 20 | 10 | 80 | 40 | 0 | 10 |
| Galium | 70 | 90 | 90 | 90 | 90 | 90 | 80 | 90 | 50 | 80 | 80 | 80 | 90 | 80 |
| Kochia | 80 | 90 | 70 | 70 | 60 | 80 | 50 | 90 | 40 | 90 | 60 | 60 | 60 | 50 |
| Pigweed | 50 | 80 | 100 | 90 | 90 | 90 | 80 | 80 | 50 | 90 | 80 | 80 | 80 | 80 |
| Ragweed | 90 | 80 | 90 | 90 | 90 | 90 | 40 | 90 | 10 | 90 | 80 | 90 | 50 | 30 |
| Ryegrass, Italian | 60 | 70 | 30 | 70 | 80 | 10 | 0 | 70 | 20 | 20 | 80 | 70 | 0 | 0 |
| Wheat | 30 | 70 | 30 | 60 | 60 | 60 | 60 | 60 | 70 | 30 | 70 | 40 | 0 | 60 |

| Table A | Compounds | | | | | | | | | | | | | |
|---|---|---|---|---|---|---|---|---|---|---|---|---|---|---|
| 500 g ai/ha | 255 | 256 | 257 | 265 | 266 | 267 | 268 | 269 | 270 | 276 | 277 | 278 | 279 | 280 |
| Postemergence | | | | | | | | | | | | | | |
| Barnyardgrass | 20 | 50 | 50 | 80 | 20 | 20 | 80 | 20 | 70 | 90 | 90 | 80 | 80 | 90 |
| Blackgrass | 20 | 0 | 0 | 20 | 0 | 20 | 40 | 20 | 70 | 0 | 60 | 50 | 20 | 0 |
| Corn | 10 | 20 | 0 | 60 | 0 | 0 | 70 | 10 | 50 | 20 | 70 | 30 | 20 | 10 |
| Foxtail, Giant | 10 | 0 | 50 | 20 | 0 | 0 | 30 | 0 | 20 | 90 | 90 | 20 | 30 | 20 |
| Galium | 80 | 80 | 80 | 90 | 90 | 90 | 90 | 90 | 90 | 90 | 100 | 90 | 90 | 90 |
| Kochia | 70 | 70 | 60 | 50 | 10 | 60 | 40 | 50 | 50 | 60 | 40 | 70 | 50 | 50 |
| Pigweed | 90 | 80 | 80 | 90 | 0 | 80 | 80 | 70 | 90 | 80 | 70 | 90 | 80 | 90 |
| Ragweed | 70 | 80 | 60 | 80 | 10 | 40 | 90 | 90 | 60 | 80 | 60 | 60 | 90 | 50 |
| Ryegrass, Italian | 0 | 50 | 0 | 70 | 0 | 30 | 60 | 20 | 70 | 10 | 60 | 80 | 60 | 70 |
| Wheat | 70 | 60 | 60 | 60 | 0 | 60 | 70 | 20 | 70 | 20 | 70 | 70 | 70 | 60 |

| Table A | Compounds | | | | | | | | | | | | | |
|---|---|---|---|---|---|---|---|---|---|---|---|---|---|---|
| 125 g ai/ha | 1 | 2 | 3 | 9 | 13 | 14 | 15 | 16 | 17 | 18 | 20 | 21 | 22 | 23 |
| Postemergence | | | | | | | | | | | | | | |
| Barnyardgrass | 90 | 80 | 30 | 80 | 0 | 40 | 0 | 60 | 30 | 0 | 60 | 20 | 0 | 50 |
| Blackgrass | 50 | 0 | 0 | 30 | 0 | 10 | 0 | 50 | 0 | 0 | 30 | 10 | 0 | 30 |
| Corn | 30 | 30 | 30 | 0 | 0 | 0 | 0 | 0 | 10 | 0 | 10 | 0 | 10 | 20 |
| Foxtail, Giant | 70 | 50 | 0 | 20 | 0 | 40 | 0 | 40 | 90 | 20 | 60 | 0 | 10 | 50 |
| Galium | 100 | 90 | 90 | 80 | 20 | 70 | 50 | 80 | 80 | 80 | 90 | 50 | 70 | 80 |
| Kochia | 60 | 20 | 40 | 10 | 0 | 40 | 40 | 40 | 60 | 10 | 10 | 10 | 30 | 40 |
| Pigweed | 80 | 70 | 90 | 60 | 0 | 60 | 0 | 70 | 60 | 40 | 30 | 0 | 0 | 70 |
| Ragweed | 90 | 80 | 80 | 70 | 10 | 60 | 70 | 80 | 80 | 0 | 80 | 50 | 20 | 80 |
| Ryegrass, Italian | 40 | 40 | 0 | 70 | 0 | 0 | 30 | 50 | 0 | 10 | 70 | 0 | 0 | 30 |
| Wheat | 50 | 60 | 50 | 60 | 0 | 30 | 0 | 50 | 70 | 0 | 60 | 0 | 0 | 50 |

| Table A | Compounds | | | | | | | | | | | | |
|---|---|---|---|---|---|---|---|---|---|---|---|---|---|
| 125 g ai/ha | 24 | 25 | 26 | 27 | 28 | 29 | 30 | 32 | 34 | 35 | 36 | 37 | 38 |
| Postemergence | | | | | | | | | | | | | |
| Barnyardgrass | 40 | 0 | 20 | 0 | 0 | 50 | 90 | 90 | 0 | 0 | 0 | 90 | 60 |
| Blackgrass | 20 | 10 | 0 | 0 | 0 | 80 | 60 | 90 | 0 | 20 | 0 | 80 | 80 |
| Corn | 20 | 0 | 0 | 0 | 0 | 0 | 0 | 10 | 0 | 0 | 0 | 20 | 20 |
| Foxtail, Giant | 0 | 20 | 10 | 0 | 0 | 90 | 70 | 90 | 0 | 0 | 0 | 90 | 90 |
| Galium | 60 | 60 | 70 | 0 | 20 | 90 | 70 | 70 | 10 | 60 | 40 | 90 | 80 |
| Kochia | 70 | 30 | 0 | 0 | 0 | 30 | 10 | 20 | 30 | 0 | 20 | 60 | 60 |
| Pigweed | 80 | 30 | 50 | 0 | 0 | 40 | 40 | 10 | 0 | 30 | 30 | 50 | 70 |
| Ragweed | 60 | 20 | 50 | 0 | 20 | 80 | 40 | 30 | 0 | 0 | 0 | 30 | 80 |
| Ryegrass, Italian | 70 | 40 | 0 | 0 | 0 | 70 | 40 | 80 | 0 | 0 | 10 | 90 | 60 |
| Wheat | 40 | 0 | 40 | 0 | 30 | 70 | 60 | 70 | 0 | 0 | 50 | 80 | 70 |

| Table A | Compounds | | | | | | | | | | | | | |
|---|---|---|---|---|---|---|---|---|---|---|---|---|---|---|
| 125 g ai/ha | 39 | 40 | 41 | 42 | 43 | 44 | 45 | 46 | 47 | 48 | 51 | 52 | 53 | 54 |
| Postemergence | | | | | | | | | | | | | | |
| Barnyardgrass | 0 | 10 | 0 | 70 | 90 | 90 | 20 | 0 | 50 | 70 | 30 | 0 | 0 | 0 |
| Blackgrass | 0 | 10 | 0 | 40 | 70 | 80 | 0 | 0 | 10 | 50 | 20 | 0 | 0 | 0 |
| Corn | 0 | 0 | 0 | 0 | 10 | 0 | 10 | 0 | 10 | 10 | 0 | 0 | 0 | 0 |
| Foxtail, Giant | 0 | 0 | 0 | 90 | 90 | 90 | 0 | 0 | 40 | 80 | 30 | 0 | 0 | 0 |
| Galium | 0 | 20 | 10 | 50 | 70 | 80 | 10 | 0 | 30 | 90 | 40 | 60 | 50 | 60 |
| Kochia | 0 | 10 | 0 | 20 | 20 | 10 | 10 | 0 | 0 | 10 | 10 | 30 | 0 | 60 |
| Pigweed | 0 | 0 | 0 | 40 | 80 | 80 | 30 | 0 | 0 | 50 | 60 | 30 | 0 | 40 |
| Ragweed | 20 | 40 | 40 | 40 | 60 | 20 | 20 | 0 | 70 | 70 | 50 | 30 | 20 | 80 |
| Ryegrass, Italian | 0 | 30 | 60 | 70 | 20 | 30 | 50 | 0 | 40 | 60 | 70 | 20 | 0 | 40 |
| Wheat | 60 | 0 | 50 | 70 | 70 | 70 | 40 | 0 | 50 | 60 | 50 | 60 | 0 | 0 |

| Table A | Compounds | | | | | | | | | | | | | |
|---|---|---|---|---|---|---|---|---|---|---|---|---|---|---|
| 125 g ai/ha | 55 | 56 | 57 | 58 | 59 | 60 | 61 | 62 | 63 | 64 | 65 | 66 | 67 | 68 |
| Postemergence | | | | | | | | | | | | | | |
| Barnyardgrass | 30 | 90 | 40 | 20 | 0 | 70 | 20 | 0 | 10 | 20 | 0 | 0 | 0 | 60 |
| Blackgrass | 0 | 50 | 0 | 0 | 0 | 40 | 0 | 0 | 0 | 0 | 0 | 0 | 0 | 70 |
| Corn | 0 | 10 | 30 | 20 | 10 | 10 | 20 | 0 | 0 | 0 | 0 | 0 | 0 | 10 |
| Foxtail, Giant | 20 | 90 | 30 | 30 | 10 | 60 | 0 | 0 | 70 | 0 | 0 | 0 | 10 | 20 |
| Galium | 90 | 90 | 80 | 80 | 80 | 90 | 50 | 70 | 90 | 0 | 0 | 50 | 50 | 50 |
| Kochia | 60 | 50 | 0 | 0 | 50 | 40 | 40 | 20 | 70 | 20 | 0 | 0 | 10 | 10 |
| Pigweed | 20 | 40 | 0 | 0 | 0 | 80 | 20 | 20 | 30 | 20 | 0 | 0 | 30 | 20 |
| Ragweed | 20 | 60 | 0 | 0 | 20 | 50 | 0 | 0 | 10 | 0 | 0 | 0 | 0 | 50 |
| Ryegrass, Italian | 50 | 0 | 30 | 0 | 0 | 70 | 40 | 0 | 30 | 40 | 0 | 0 | 0 | 40 |
| Wheat | 20 | 50 | 60 | 20 | 0 | 50 | 40 | 0 | 0 | 40 | 0 | 0 | 30 | 70 |

| Table A | Compounds | | | | | | | | | | | | | |
|---|---|---|---|---|---|---|---|---|---|---|---|---|---|---|
| 125 g ai/ha | 69 | 70 | 71 | 72 | 73 | 74 | 75 | 76 | 77 | 78 | 79 | 80 | 81 | 82 |
| Postemergence | | | | | | | | | | | | | | |
| Barnyardgrass | 0 | 0 | 0 | - | - | - | - | - | 0 | 40 | 80 | 70 | 70 | 60 |
| Blackgrass | 0 | 30 | 0 | 50 | 40 | 40 | 30 | 50 | 0 | 0 | 30 | 60 | 0 | 0 |
| Corn | 10 | 0 | 0 | 0 | 10 | 0 | 0 | 0 | 0 | 0 | 10 | 20 | 0 | 0 |
| Foxtail, Giant | 80 | 0 | 0 | 90 | 80 | 40 | 30 | 90 | 0 | 70 | 90 | 90 | 90 | 20 |
| Galium | 60 | 60 | 50 | 70 | 90 | 50 | 70 | 90 | 0 | 60 | 90 | 90 | 80 | 20 |
| Kochia | 40 | 0 | 20 | 40 | 10 | 30 | 20 | 50 | 0 | 0 | 40 | 40 | 40 | 0 |
| Pigweed | 70 | 0 | 0 | 60 | 60 | 80 | 40 | 70 | 0 | 0 | 30 | 70 | 20 | 0 |
| Ragweed | 60 | 0 | 0 | 20 | 20 | 0 | 0 | 60 | 0 | 0 | 30 | 90 | 20 | 0 |
| Ryegrass, Italian | 40 | 0 | 70 | 60 | 60 | 20 | 20 | 70 | 0 | 40 | 40 | 70 | 0 | 0 |
| Wheat | 60 | 0 | 40 | 60 | 60 | 60 | 40 | 40 | 0 | 0 | 70 | 60 | 40 | 0 |

| Table A | Compounds | | | | | | | | | | | | | |
|---|---|---|---|---|---|---|---|---|---|---|---|---|---|---|
| 125 g ai/ha | 83 | 85 | 88 | 91 | 92 | 93 | 94 | 95 | 96 | 97 | 98 | 99 | 100 | 101 |
| Postemergence | | | | | | | | | | | | | | |
| Barnyardgrass | 20 | 30 | 10 | 0 | 10 | 60 | 60 | 30 | 30 | 20 | 50 | 70 | 80 | 80 |
| Blackgrass | 0 | 0 | 30 | 0 | 0 | 70 | 20 | 0 | 0 | 0 | 0 | 0 | 0 | 20 |
| Corn | 0 | 0 | 10 | 0 | 20 | 20 | 20 | 20 | 10 | 10 | 10 | 0 | 10 | 20 |
| Foxtail, Giant | 0 | 0 | 0 | 10 | 0 | 10 | 30 | 0 | 10 | 0 | 20 | 0 | 10 | 0 |
| Galium | 60 | 60 | 90 | 70 | 90 | 80 | 90 | 80 | 80 | 80 | 90 | 90 | 80 | 80 |
| Kochia | 0 | 0 | 30 | 10 | 30 | 30 | 50 | 30 | 20 | 30 | 20 | 40 | 60 | 10 |
| Pigweed | 40 | 20 | 50 | 40 | 40 | 50 | 80 | 80 | 30 | 70 | 80 | 70 | 60 | 70 |
| Ragweed | 10 | 20 | 40 | 40 | 60 | 30 | 40 | 0 | 40 | 90 | 60 | 40 | 40 | 50 |
| Ryegrass, Italian | 0 | 40 | 0 | 10 | 0 | 20 | 50 | 0 | 20 | 0 | 30 | 0 | 20 | 60 |
| Wheat | 0 | 0 | 30 | 60 | 50 | 60 | 50 | 10 | 40 | 0 | 40 | 0 | 20 | 0 |

| Table A | Compounds | | | | | | | | | | | | |
|---|---|---|---|---|---|---|---|---|---|---|---|---|---|
| 125 g ai/ha | 102 | 103 | 104 | 105 | 107 | 108 | 109 | 110 | 111 | 112 | 113 | 114 | 115 |
| Postemergence | | | | | | | | | | | | | |
| Barnyardgrass | 90 | 90 | 80 | 90 | 70 | 20 | 0 | 20 | 80 | 70 | 80 | 20 | 50 |
| Blackgrass | 0 | 0 | 0 | 0 | 0 | 0 | 10 | 0 | 0 | 30 | 0 | 30 | 0 |
| Corn | 10 | 20 | 30 | 50 | 0 | 0 | 0 | 0 | 40 | 20 | 0 | 20 | 0 |
| Foxtail, Giant | 30 | 30 | 20 | 90 | 10 | 0 | 0 | 0 | 60 | 50 | 30 | 50 | 30 |
| Galium | 100 | 90 | 90 | 90 | 90 | 70 | 80 | 70 | 70 | 90 | 80 | 90 | 90 |
| Kochia | 70 | 70 | 30 | 70 | 20 | 30 | 10 | 10 | 60 | 30 | 50 | 10 | 30 |
| Pigweed | 70 | 80 | 40 | 60 | 50 | 0 | 50 | 70 | 90 | 60 | 90 | 60 | 60 |
| Ragweed | 70 | 60 | 70 | 90 | 70 | 40 | 70 | 90 | 90 | 90 | 90 | 0 | 50 |
| Ryegrass, Italian | 80 | 90 | 10 | 30 | 0 | 0 | 40 | 0 | 30 | 60 | 50 | 40 | 40 |
| Wheat | 40 | 60 | 20 | 50 | 0 | 30 | 30 | 20 | 50 | 40 | 20 | 60 | 60 |

| Table A | Compounds | | | | | | | | | | | | | |
|---|---|---|---|---|---|---|---|---|---|---|---|---|---|---|
| 125 g ai/ha | 116 | 118 | 119 | 120 | 121 | 122 | 123 | 124 | 125 | 126 | 127 | 128 | 129 | 130 |
| Postemergence | | | | | | | | | | | | | | |
| Barnyardgrass | 50 | 90 | 40 | 90 | 90 | 10 | 90 | 0 | 80 | 0 | 0 | 50 | 20 | 90 |
| Blackgrass | 0 | 10 | 0 | 20 | 40 | 0 | 20 | 0 | 10 | 20 | 20 | 10 | 20 | 30 |
| Corn | 0 | 0 | 10 | 0 | 50 | 0 | 10 | 0 | 0 | 0 | 0 | 10 | 10 | 10 |
| Foxtail, Giant | 0 | 30 | 30 | 40 | 50 | 0 | 30 | 0 | 20 | 0 | 0 | 10 | 0 | 70 |
| Galium | 90 | 90 | 80 | 90 | 90 | 60 | 80 | 60 | 90 | 80 | 20 | 80 | 60 | 90 |
| Kochia | 30 | 40 | 20 | 70 | 50 | 0 | 50 | 0 | 70 | 10 | 10 | 30 | 30 | 50 |
| Pigweed | 60 | 90 | 40 | 60 | 70 | 20 | 60 | 30 | 60 | 70 | 0 | 60 | 30 | 90 |
| Ragweed | 90 | 80 | 70 | 80 | 90 | 0 | 50 | 0 | 60 | 0 | 20 | 80 | 60 | 90 |
| Ryegrass, Italian | 60 | 0 | 20 | 90 | 90 | 0 | 50 | 0 | 0 | 0 | 40 | 80 | 0 | 90 |
| Wheat | 60 | 40 | 10 | 70 | 60 | 0 | 50 | 0 | 30 | 0 | 0 | 30 | 30 | 40 |

| Table A | Compounds | | | | | | | | | | | | | |
|---|---|---|---|---|---|---|---|---|---|---|---|---|---|---|
| 125 g ai/ha | 131 | 132 | 133 | 134 1 | 135 | 136 | 137 | 139 | 140 | 141 | 142 | 143 | 144 | 145 |
| Postemergence | | | | | | | | | | | | | | |
| Barnyardgrass | 80 | 0 | 0 | 0 | 80 | 20 | 30 | 0 | 70 | 80 | 80 | 50 | 20 | 40 |
| Blackgrass | 0 | 0 | 0 | 0 | 20 | 10 | 0 | 0 | 30 | 0 | 20 | 0 | 0 | 0 |
| Corn | 20 | 0 | 10 | 0 | 0 | 0 | 30 | 0 | 0 | 0 | 80 | 20 | 30 | 30 |
| Foxtail, Giant | 10 | 20 | 0 | 0 | 90 | 0 | 0 | 0 | 30 | 20 | 20 | 30 | 30 | 10 |
| Galium | 90 | 70 | 80 | 50 | 90 | 70 | 80 | 50 | 50 | 90 | 80 | 40 | 60 | 80 |
| Kochia | 60 | 20 | 20 | 40 | 50 | 30 | 20 | 0 | 10 | 30 | 30 | 20 | 10 | 30 |
| Pigweed | 70 | 60 | 50 | 80 | 40 | 30 | 90 | 40 | 10 | 60 | 60 | 40 | 40 | 80 |
| Ragweed | 60 | 90 | 70 | 20 | 60 | 10 | 30 | 0 | 0 | 80 | 80 | 10 | 30 | 80 |
| Ryegrass, Italian | 0 | 30 | 0 | 0 | 80 | 0 | 0 | 0 | 0 | 20 | 10 | 20 | 0 | 0 |
| Wheat | 40 | 0 | 0 | 0 | 70 | 0 | 30 | 0 | 0 | 20 | 30 | 50 | 60 | 0 |

| Table A | Compounds | | | | | | | | | | | | | |
|---|---|---|---|---|---|---|---|---|---|---|---|---|---|---|
| 125 g ai/ha | 146 | 147 | 148 | 149 | 150 | 151 | 152 | 153 | 154 | 155 | 156 | 157 | 158 | 159 |
| Postemergence | | | | | | | | | | | | | | |
| Barnyardgrass | 40 | 90 | 10 | 40 | 50 | 30 | 10 | 50 | 70 | 90 | 90 | 60 | 0 | 30 |
| Blackgrass | 0 | 0 | 0 | 20 | 0 | 0 | 0 | 50 | 20 | 30 | 40 | 30 | 20 | 0 |
| Corn | 10 | 10 | 0 | 0 | 0 | 10 | 0 | 20 | 10 | 90 | 20 | 20 | 0 | 0 |
| Foxtail, Giant | 20 | 70 | 0 | 30 | 20 | 30 | 10 | 20 | 20 | 30 | 70 | 30 | 0 | 0 |
| Galium | 90 | 80 | 20 | 60 | 60 | 70 | 60 | 70 | 80 | 90 | 100 | 100 | 70 | 80 |
| Kochia | 40 | 50 | 20 | 10 | 10 | 10 | 10 | 10 | 30 | 60 | 70 | 70 | 20 | 20 |
| Pigweed | 50 | 70 | 40 | 40 | 40 | 30 | 60 | 30 | 60 | 90 | 90 | 100 | 30 | 40 |
| Ragweed | 60 | 90 | 0 | 20 | 0 | 40 | 40 | 70 | 20 | 80 | 90 | 90 | 10 | 60 |
| Ryegrass, Italian | 30 | 30 | 0 | 0 | 0 | 0 | 0 | 80 | 0 | 90 | 10 | 30 | 0 | 0 |
| Wheat | 50 | 30 | 0 | 0 | 60 | 50 | 30 | 30 | 70 | 60 | 40 | 0 | 0 | 30 |

| Table A | Compounds | | | | | | | | | | | | | |
|---|---|---|---|---|---|---|---|---|---|---|---|---|---|---|
| 125 g ai/ha | 160 | 161 | 162 | 163 | 164 | 165 | 166 | 167 | 168 | 169 | 170 | 171 | 172 | 173 |
| Postemergence | | | | | | | | | | | | | | |
| Barnyardgrass | 30 | 80 | 80 | 0 | 80 | 0 | 90 | 60 | 70 | 40 | 0 | 90 | 60 | 80 |
| Blackgrass | 0 | 0 | 20 | 20 | 0 | 0 | 10 | 20 | 10 | 0 | 30 | 40 | 0 | 50 |
| Corn | 0 | 50 | 20 | 0 | 0 | 0 | 10 | 10 | 0 | 50 | 0 | 80 | 40 | 70 |
| Foxtail, Giant | 0 | 20 | 30 | 0 | 0 | 0 | 50 | 10 | 10 | 10 | 0 | 60 | 20 | 90 |
| Galium | 80 | 90 | 100 | 70 | 80 | 80 | 80 | 80 | 90 | 80 | 70 | 60 | 90 | 90 |
| Kochia | 10 | 50 | 10 | 10 | 60 | 10 | 50 | 40 | 10 | 20 | 10 | 30 | 40 | 50 |
| Pigweed | 70 | 80 | 60 | 60 | 70 | 60 | 40 | 70 | 80 | 40 | 60 | 60 | 70 | 80 |
| Ragweed | 70 | 70 | 60 | 0 | 70 | 0 | 40 | 20 | 60 | 60 | 30 | 40 | 60 | 80 |
| Ryegrass, Italian | 0 | 20 | 30 | 0 | 80 | 0 | 90 | 20 | 30 | 10 | 0 | 80 | 60 | 70 |
| Wheat | 0 | 0 | 50 | 0 | 60 | 0 | 70 | 30 | 0 | 0 | 0 | 60 | 30 | 70 |

| Table A | Compounds | | | | | | | | | | | | | |
|---|---|---|---|---|---|---|---|---|---|---|---|---|---|---|
| 125 g ai/ha | 174 | 175 | 176 | 177 | 178 | 179 | 180 | 181 | 182 | 183 | 184 | 185 | 186 | 187 |
| Postemergence | | | | | | | | | | | | | | |
| Barnyardgrass | 60 | 20 | 80 | 70 | 40 | 90 | 0 | 80 | 10 | 20 | 20 | 0 | 10 | 90 |
| Blackgrass | 0 | 0 | 40 | 0 | 0 | 20 | 0 | 10 | 0 | 10 | 0 | 0 | 0 | 20 |
| Corn | 20 | 0 | 70 | 0 | 0 | 20 | 0 | 10 | 0 | 0 | 0 | 0 | 0 | 80 |
| Foxtail, Giant | 0 | 0 | 90 | 10 | 0 | 70 | 10 | 60 | 20 | 0 | 0 | 0 | 0 | 80 |
| Galium | 80 | 80 | 90 | 90 | 80 | 90 | 70 | 60 | 40 | 70 | 80 | 30 | 60 | 80 |
| Kochia | 30 | 10 | 40 | 20 | 20 | 60 | 10 | 30 | 0 | 10 | 0 | 0 | 10 | 50 |
| Pigweed | 90 | 50 | 60 | 60 | 60 | 80 | 50 | 40 | 40 | 30 | 20 | 0 | 0 | 30 |
| Ragweed | 90 | 50 | 60 | 90 | 40 | 90 | 20 | 80 | 70 | 60 | 40 | 0 | 40 | 80 |
| Ryegrass, Italian | 0 | 30 | 60 | 40 | 0 | 40 | 0 | 70 | 20 | 0 | 0 | 0 | 0 | 90 |
| Wheat | 0 | 10 | 60 | 20 | 10 | 0 | 0 | 50 | 0 | 0 | 0 | 0 | 0 | 70 |

| Table A | Compounds | | | | | | | | | | | | | |
|---|---|---|---|---|---|---|---|---|---|---|---|---|---|---|
| 125 g ai/ha | 188 | 189 | 190 | 191 | 192 | 193 | 194 | 195 | 196 | 197 | 198 | 199 | 200 | 201 |
| Postemergence | | | | | | | | | | | | | | |
| Barnyardgrass | 10 | 80 | 80 | 0 | 30 | 10 | 0 | 0 | - | - | - | - | - | - |
| Blackgrass | 0 | 30 | 10 | 0 | 0 | 0 | 0 | 0 | 0 | 40 | 10 | 0 | 0 | 20 |
| Corn | 10 | 20 | 0 | 0 | 30 | 0 | 0 | 20 | 0 | 20 | 20 | 0 | 0 | 20 |
| Foxtail, Giant | 0 | 60 | 20 | 0 | 50 | 30 | 10 | 0 | 0 | 80 | 0 | 30 | 0 | 50 |
| Galium | 40 | 90 | 70 | 70 | 40 | 70 | 70 | 10 | 60 | 90 | 70 | 90 | 60 | 90 |
| Kochia | 0 | 20 | 30 | 0 | 20 | 30 | 20 | 0 | 10 | 50 | 20 | 50 | 10 | 60 |
| Pigweed | 20 | 60 | 70 | 20 | 60 | 80 | 0 | 0 | 30 | 60 | 50 | 60 | 60 | 60 |
| Ragweed | 20 | 90 | 80 | 20 | 70 | 70 | 60 | 0 | 0 | 80 | 20 | 70 | 0 | 80 |
| Ryegrass, Italian | 0 | 80 | 80 | 30 | 60 | 0 | 0 | 0 | 0 | 50 | 30 | 40 | 20 | 30 |
| Wheat | 40 | 50 | 40 | 30 | 60 | 0 | 0 | 0 | 0 | 60 | 20 | 30 | 20 | 60 |

| Table A | Compounds | | | | | | | | | | | | | |
|---|---|---|---|---|---|---|---|---|---|---|---|---|---|---|
| 125 g ai/ha | 202 | 203 | 204 | 205 | 206 | 207 | 208 | 209 | 210 | 211 | 212 | 213 | 214 | 215 |
| Postemergence | | | | | | | | | | | | | | |
| Barnyardgrass | - | 60 | 0 | 0 | 60 | 60 | 0 | 20 | 20 | 0 | 0 | 20 | 0 | 0 |
| Blackgrass | 0 | 20 | 0 | 0 | 50 | 20 | 0 | 20 | 0 | 0 | 0 | 0 | 0 | 0 |
| Corn | 0 | 10 | 10 | 0 | 0 | 20 | 0 | 0 | 10 | 0 | 0 | 10 | 10 | 0 |
| Foxtail, Giant | 0 | 0 | 10 | 0 | 50 | 50 | 0 | 0 | 0 | 0 | 0 | 0 | 10 | 20 |
| Galium | 50 | 80 | 50 | 40 | 70 | 50 | 30 | 70 | 80 | 60 | 50 | 40 | 20 | 50 |
| Kochia | 20 | 40 | 30 | 0 | 20 | 20 | 10 | 20 | 50 | 10 | 20 | 0 | 0 | 30 |
| Pigweed | 70 | 20 | 20 | 0 | 60 | 50 | 20 | 30 | 60 | 40 | 20 | 40 | 10 | 50 |
| Ragweed | 80 | 70 | 20 | 40 | 60 | 20 | 20 | 40 | 80 | 30 | 20 | 0 | 0 | 60 |
| Ryegrass, Italian | 20 | 0 | 40 | 0 | 50 | 40 | 0 | 40 | 20 | 40 | 0 | 0 | 30 | 0 |
| Wheat | 30 | 40 | 50 | 30 | 60 | 60 | 0 | 0 | 0 | 0 | 0 | 60 | 0 | 30 |

| Table A | Compounds | | | | | | | | | | | | | |
|---|---|---|---|---|---|---|---|---|---|---|---|---|---|---|
| 125 g ai/ha | 216 | 217 | 218 | 219 | 220 | 221 | 222 | 223 | 224 | 225 | 226 | 227 | 228 | 229 |
| Postemergence | | | | | | | | | | | | | | |
| Barnyardgrass | 0 | 0 | 80 | 0 | 20 | 30 | 10 | 10 | - | 0 | - | - | - | - |
| Blackgrass | 0 | 0 | 10 | 0 | 0 | 10 | 20 | 20 | 0 | 0 | 20 | 0 | 0 | 20 |
| Corn | 30 | 0 | 10 | 0 | 0 | 10 | 0 | 0 | 10 | 0 | 0 | 10 | 10 | 20 |
| Foxtail, Giant | 0 | 0 | 50 | 0 | 0 | 0 | 0 | 0 | 30 | 0 | 20 | 0 | 0 | 50 |
| Galium | 50 | 80 | 60 | 50 | 60 | 80 | 90 | 40 | 90 | 50 | 60 | 40 | 80 | 90 |
| Kochia | 20 | 50 | 60 | 30 | 20 | 60 | 10 | 0 | 50 | 0 | 10 | 10 | 20 | 60 |
| Pigweed | 30 | 40 | 50 | 40 | 0 | 20 | 20 | 40 | 60 | 90 | 30 | 20 | 30 | 80 |
| Ragweed | 10 | 60 | 40 | 20 | 0 | 70 | 50 | 0 | 50 | 20 | 30 | 0 | 20 | 90 |
| Ryegrass, Italian | 0 | 0 | 30 | 20 | 0 | 0 | 0 | 0 | 30 | 60 | 30 | 0 | 0 | 30 |
| Wheat | 0 | 0 | 10 | 0 | 20 | 20 | 0 | 0 | 30 | 0 | 0 | 70 | 0 | 60 |

| Table A | Compounds | | | | | | | | | | | | | |
|---|---|---|---|---|---|---|---|---|---|---|---|---|---|---|
| 125 g ai/ha | 230 | 231 | 232 | 233 | 234 | 235 | 236 | 237 | 238 | 239 | 240 | 241 | 243 | 244 |
| Postemergence | | | | | | | | | | | | | | |
| Barnyardgrass | - | 20 | 20 | 0 | 60 | 0 | 50 | 20 | 0 | 0 | 40 | 60 | 50 | 50 |
| Blackgrass | 0 | 0 | 0 | 0 | 0 | 0 | 0 | 0 | 0 | 0 | 0 | 0 | 0 | 40 |
| Corn | 0 | 0 | 0 | 0 | 0 | 0 | 0 | 0 | 0 | 0 | 20 | 10 | 0 | 0 |
| Foxtail, Giant | 10 | 0 | 20 | 0 | 0 | 20 | 20 | 20 | 30 | 0 | 20 | 0 | 0 | 10 |
| Galium | 80 | 80 | 50 | 80 | 10 | 70 | 40 | 0 | 80 | 0 | 50 | 80 | 70 | 90 |
| Kochia | 0 | 20 | 0 | 0 | 0 | 10 | 30 | 70 | 20 | 0 | 50 | 60 | 10 | 50 |
| Pigweed | 80 | 50 | 60 | 30 | 30 | 30 | 40 | 30 | 30 | 20 | 30 | 60 | 90 | 60 |
| Ragweed | 40 | 80 | 70 | 0 | 30 | 60 | 40 | 50 | 60 | 0 | 70 | 40 | 30 | 90 |
| Ryegrass, Italian | 10 | 20 | 60 | 0 | 0 | 30 | 30 | 40 | 30 | 0 | 30 | 0 | 0 | 40 |
| Wheat | 30 | 50 | 0 | 0 | 0 | 20 | 0 | 0 | 60 | 0 | 0 | 30 | 0 | 20 |

| Table A | Compounds | | | | | | | | | | | | | |
|---|---|---|---|---|---|---|---|---|---|---|---|---|---|---|
| 125 g ai/ha | 245 | 246 | 247 | 248 | 249 | 250 | 251 | 252 | 253 | 254 | 255 | 256 | 257 | 265 |
| Postemergence | | | | | | | | | | | | | | |
| Barnyardgrass | 90 | 70 | 20 | 60 | 30 | 20 | 70 | 0 | 0 | 0 | 0 | 60 | 0 | 60 |
| Blackgrass | 40 | 20 | 0 | 0 | 0 | 0 | 20 | 0 | 0 | 20 | 20 | 0 | 0 | 30 |
| Corn | 20 | 20 | 20 | 0 | 40 | 0 | 0 | 10 | 0 | 0 | 0 | 0 | 0 | 20 |
| Foxtail, Giant | 50 | 60 | 0 | 20 | 10 | 0 | 10 | 0 | 0 | 0 | 0 | 10 | 0 | 0 |
| Galium | 90 | 90 | 70 | 70 | 60 | 50 | 80 | 80 | 70 | 60 | 60 | 60 | 80 | 90 |
| Kochia | 40 | 20 | 20 | 60 | 30 | 70 | 40 | 30 | 30 | 10 | 20 | 50 | 30 | 40 |
| Pigweed | 80 | 90 | 50 | 80 | 20 | 80 | 80 | 70 | 50 | 70 | 60 | 60 | 60 | 60 |
| Ragweed | 90 | 90 | 30 | 90 | 0 | 80 | 70 | 10 | 30 | 0 | 0 | 40 | 50 | 60 |
| Ryegrass, Italian | 70 | 0 | 0 | 0 | 0 | 0 | 30 | 30 | 0 | 0 | 0 | 0 | 0 | 20 |
| Wheat | 60 | 20 | 0 | 40 | 0 | 20 | 40 | 0 | 0 | 0 | 20 | 20 | 0 | 30 |

| Table A | Compounds | | | | | | | | | |
|---|---|---|---|---|---|---|---|---|---|---|
| 125 g ai/ha | 266 | 267 | 268 | 269 | 270 | 276 | 277 | 278 | 279 | 280 |
| Postemergence | | | | | | | | | | |
| Barnyardgrass | 0 | 20 | 50 | 0 | 0 | 60 | 20 | 20 | 20 | 20 |
| Blackgrass | 30 | 0 | 20 | 20 | 20 | 0 | 10 | 0 | 0 | 0 |
| Corn | 0 | 0 | 50 | 0 | 0 | 0 | 10 | 0 | 20 | 0 |
| Foxtail, Giant | 0 | 0 | 0 | 0 | 0 | 10 | 30 | 0 | 10 | 0 |
| Galium | 30 | 80 | 90 | 70 | 80 | 80 | 100 | 90 | 90 | 80 |
| Kochia | 0 | 40 | 30 | 30 | 10 | 20 | 10 | 30 | 40 | 20 |
| Pigweed | 0 | 50 | 60 | 30 | 60 | 60 | 30 | 60 | 50 | 60 |
| Ragweed | 0 | 10 | 70 | 40 | 30 | 50 | 20 | 40 | 80 | 30 |
| Ryegrass, Italian | 0 | 0 | 40 | 0 | 0 | 0 | 0 | 30 | 0 | 40 |
| Wheat | 0 | 40 | 50 | 0 | 40 | 0 | 0 | 60 | 0 | 40 |

| Table A | Compounds | | | | | | | | | | | | | |
|---|---|---|---|---|---|---|---|---|---|---|---|---|---|---|
| 31 g ai/ha | 1 | 2 | 3 | 27 | 36 | 88 | 91 | 92 | 93 | 94 | 95 | 96 | 97 | 98 |
| Postemergence | | | | | | | | | | | | | | |
| Barnyardgrass | 70 | 30 | 0 | 0 | 0 | 0 | 0 | 0 | 0 | 20 | 0 | 0 | 0 | 0 |
| Blackgrass | 0 | 0 | 0 | 0 | 0 | 0 | 0 | 0 | 20 | 0 | 0 | 0 | 0 | 20 |
| Corn | 20 | 20 | 20 | 0 | 0 | 0 | 0 | 0 | 0 | 0 | 0 | 10 | 0 | 0 |
| Foxtail, Giant | 20 | 20 | 0 | 0 | 0 | 0 | 0 | 0 | 0 | 0 | 0 | 0 | 0 | 0 |
| Galium | 90 | 90 | 90 | 0 | 20 | 60 | 70 | 50 | 80 | 70 | 40 | 80 | 50 | 60 |
| Kochia | 20 | 10 | 10 | 0 | 10 | 0 | 10 | 0 | 10 | 10 | 10 | 10 | 30 | 10 |
| Pigweed | 50 | 60 | 70 | 0 | 0 | 30 | 0 | 0 | 30 | 50 | 40 | 30 | 50 | 30 |
| Ragweed | 80 | 30 | 40 | 0 | 0 | 30 | 0 | 10 | 30 | 10 | 0 | 30 | 70 | 50 |
| Ryegrass, Italian | 0 | 0 | 0 | 0 | 20 | 0 | 0 | 0 | 0 | 20 | 0 | 0 | 0 | 30 |
| Wheat | 30 | 40 | 30 | 0 | 0 | 0 | 10 | 0 | 30 | 10 | 0 | 0 | 0 | 0 |

| Table A | Compounds | | | | | | | | | | | | | |
|---|---|---|---|---|---|---|---|---|---|---|---|---|---|---|
| 31 g ai/ha | 111 | 112 | 113 | 115 | 118 | 131 | 137 | 143 | 144 | 145 | 148 | 149 | 150 | 151 |
| Postemergence | | | | | | | | | | | | | | |
| Barnyardgrass | 40 | 40 | 20 | 10 | 10 | 20 | 10 | 0 | 0 | 0 | 0 | 10 | 0 | 0 |
| Blackgrass | 0 | 0 | 0 | 0 | 20 | 20 | 0 | 60 | 0 | 0 | 0 | 0 | 0 | 0 |
| Corn | 0 | 0 | 0 | 0 | 0 | 0 | 30 | 0 | 0 | 10 | 0 | 0 | 0 | 0 |
| Foxtail, Giant | 0 | 10 | 0 | 0 | 0 | 0 | 0 | 0 | 0 | 0 | 0 | 0 | 0 | 0 |
| Galium | 50 | 90 | 70 | 80 | 40 | 60 | 30 | 50 | 30 | 60 | 0 | 0 | 40 | 60 |
| Kochia | 20 | 20 | 10 | 20 | 10 | 40 | 0 | 10 | 10 | 10 | 0 | 0 | 10 | 10 |
| Pigweed | 20 | 50 | 80 | 30 | 30 | 50 | 50 | 20 | 20 | 30 | 40 | 0 | 20 | 30 |
| Ragweed | 70 | 80 | 80 | 30 | 20 | 30 | 0 | 0 | 0 | 80 | 0 | 0 | 0 | 0 |
| Ryegrass, Italian | 30 | 0 | 20 | 0 | 0 | 0 | 0 | 0 | 0 | 0 | 0 | 0 | 0 | 0 |
| Wheat | 0 | 0 | 0 | 40 | 0 | 0 | 0 | 20 | 0 | 0 | 0 | 0 | 0 | 0 |

| Table A | Compounds | | | | | | | | | | |
|---|---|---|---|---|---|---|---|---|---|---|---|
| 31 g ai/ha | 152 | 153 | 154 | 155 | 156 | 157 | 161 | 162 | 167 | 168 | 174 |
| Postemergence | | | | | | | | | | | |
| Barnyardgrass | 0 | 10 | 30 | 90 | 90 | 20 | 40 | 40 | 10 | 30 | 0 |
| Blackgrass | 0 | 0 | 0 | 10 | 0 | 30 | 0 | 20 | 0 | 0 | 0 |
| Corn | 10 | 0 | 0 | 50 | 0 | 20 | 10 | 20 | 0 | 0 | 0 |
| Foxtail, Giant | 10 | 10 | 20 | 20 | 30 | 10 | 0 | 10 | 0 | 0 | 0 |
| Galium | 20 | 60 | 50 | 80 | 100 | 90 | 30 | 80 | 0 | 70 | 50 |
| Kochia | 10 | 0 | 10 | 20 | 40 | 10 | 10 | 10 | 10 | 0 | 10 |
| Pigweed | 30 | 0 | 40 | 80 | 60 | 90 | 70 | 50 | 20 | 60 | 70 |
| Ragweed | 0 | 30 | 0 | 60 | 90 | 70 | 40 | 60 | 0 | 0 | 70 |
| Ryegrass, Italian | 0 | 30 | 0 | 40 | 0 | 0 | 20 | 0 | 0 | 0 | 0 |
| Wheat | 40 | 0 | 30 | 30 | 30 | 0 | 0 | 30 | 0 | 0 | 0 |

### TEST Al

Seeds of plant species selected from, blackgrass *(Alopecurus myosuroides),* corn *(Zea mays),* Foxtail, Giant (giant foxtail, *Setaria faberi),* goosegrass *(Eleusine indica),* kochia *(Bassia scoparia),* Oat, Wild (wild oat, *Avena fatua*), Pigweed, Palmer (palmer amaranth, palmer pigweed, *Amaranthus palmeri),* ragweed (common ragweed, *Ambrosia artemisiifolia),* Ryegrass, Italian (Italian ryegrass, *Lolium multiflorum),* soybean *(Glycine max),* and wheat *(Triticum aestivum)* were planted into a blend of loam soil and sand and treated preemergence with a directed soil spray using test chemicals formulated in a non-phytotoxic solvent mixture which included a surfactant.

At the same time, plants selected from these crop and weed species and also galium (catchweed bedstraw, *Galium aparine*) and horseweed *(Erigeron canadensis)* were planted in pots containing the same blend of loam soil and sand and treated with postemergence applications of test chemicals formulated in the same manner. Plants ranged in height from 2 to 10 cm and were in the one- to two-leaf stage for the postemergence treatment. Treated plants and untreated controls were maintained in a greenhouse for 10 days, after which time all treated plants were compared to untreated controls and visually evaluated for injury. Plant response ratings, summarized in Table A, are based on a 0 to 100 scale where 0 is no effect and 100 is complete control. A dash (-) response means no test result.

| Table A1 | Compound | Table A1 | Compound |
|---|---|---|---|
| 500 g ai/ha | 397 | 500 g ai/ha | 397 |
| Preemergence | | Preemergence | |
| Blackgrass | 90 | Pigweed, Palmer | 100 |
| Corn | 60 | Ragweed | 90 |
| Foxtail, Giant | 100 | Ryegrass, Italian | 100 |
| Goosegrass | 100 | Soybean | 80 |
| Kochia | 100 | Wheat | 100 |
| Oat, Wild | 90 | | |

| Table A1 | Compounds | | | | | | | | | | | | | |
|---|---|---|---|---|---|---|---|---|---|---|---|---|---|---|
| 125 g ai/ha | 281 | 282 | 283 | 284 | 285 | 286 | 287 | 288 | 289 | 290 | 292 | 293 | 294 | 295 |
| Preemergence | | | | | | | | | | | | | | |
| Blackgrass | 0 | 60 | - | - | - | - | 30 | 0 | 60 | 70 | 30 | 0 | 0 | 0 |
| Corn | 0 | 50 | 10 | 20 | 10 | 0 | 0 | 0 | 30 | 20 | 40 | 0 | 0 | 0 |
| Foxtail, Giant | 100 | 30 | 90 | 100 | 90 | 100 | 30 | 90 | 100 | 90 | 100 | 30 | 20 | 40 |
| Goosegrass | 90 | 90 | 60 | 60 | 70 | 90 | 90 | 90 | 90 | 90 | 100 | 100 | 90 | 90 |
| Kochia | 20 | 80 | 90 | 90 | 70 | 80 | 60 | 40 | 80 | 30 | 80 | 30 | 60 | 30 |
| Oat, Wild | 0 | 30 | 0 | 50 | 20 | 0 | 10 | 0 | 50 | 20 | 50 | - | 20 | 0 |
| Pigweed, Palmer | 40 | 80 | 50 | 50 | 20 | 70 | 90 | 70 | 100 | 50 | 60 | 30 | 30 | 80 |
| Ragweed | 0 | 20 | 40 | 70 | 10 | 10 | 80 | 40 | 60 | 60 | 80 | 0 | 30 | 0 |
| Ryegrass, Italian | 40 | 40 | 0 | 40 | 0 | 0 | 90 | 0 | 70 | 60 | 100 | - | 90 | 70 |
| Soybean | 80 | 20 | 50 | 60 | 0 | 40 | 30 | 40 | 60 | 0 | 40 | 0 | 0 | 0 |
| Wheat | 20 | 80 | 50 | 60 | 20 | 30 | 30 | 0 | 80 | 40 | 90 | 0 | 0 | 30 |

| Table A1 | Compounds | | | | | | | | | | | | | |
|---|---|---|---|---|---|---|---|---|---|---|---|---|---|---|
| 125 g ai/ha | 296 | 297 | 298 | 299 | 300 | 301 | 302 | 303 | 304 | 305 | 306 | 307 | 308 | 309 |
| Preemergence | | | | | | | | | | | | | | |
| Blackgrass | 60 | 40 | 10 | 30 | 50 | 40 | 0 | 40 | 40 | 40 | 0 | 50 | 50 | 60 |
| Corn | 70 | 20 | 0 | 0 | 90 | 10 | 0 | 90 | 0 | 90 | 0 | 0 | 60 | 0 |
| Foxtail, Giant | 100 | 100 | 30 | 40 | 60 | 90 | 80 | 100 | 80 | 90 | 20 | 100 | 80 | 90 |
| Goosegrass | 90 | 90 | 90 | 90 | 90 | 100 | 90 | 100 | 90 | 90 | 100 | 100 | 90 | 100 |
| Kochia | 90 | 90 | 60 | 50 | 70 | 80 | 80 | 20 | 0 | 40 | 30 | 10 | 70 | 50 |
| Oat, Wild | 10 | 20 | 0 | 20 | 0 | 0 | 20 | 60 | 0 | 60 | 10 | 40 | 50 | 60 |
| Pigweed, Palmer | 80 | 90 | 60 | 70 | 50 | 40 | 50 | 20 | 20 | 60 | 100 | 80 | 70 | 40 |
| Ragweed | 90 | 60 | 0 | 30 | 40 | 80 | 30 | 0 | 50 | 20 | 10 | 60 | 50 | 20 |
| Ryegrass, Italian | 90 | 90 | 90 | 60 | 90 | 90 | 90 | 100 | 50 | 90 | 80 | 100 | 90 | 90 |
| Soybean | 30 | 50 | 0 | 0 | 0 | 20 | 20 | 0 | 0 | 40 | 0 | 30 | 30 | 0 |
| Wheat | 90 | 100 | 70 | 70 | 90 | 40 | 20 | 90 | 20 | 40 | 0 | 80 | 80 | 60 |

| Table A1 | Compounds | | | | | | | | | | | | | |
|---|---|---|---|---|---|---|---|---|---|---|---|---|---|---|
| 125 g ai/ha | 310 | 311 | 312 | 313 | 314 | 315 | 316 | 317 | 318 | 319 | 320 | 321 | 322 | 323 |
| Preemergence | | | | | | | | | | | | | | |
| Blackgrass | 0 | 40 | 0 | 0 | 20 | 60 | 0 | 0 | 70 | 50 | 0 | 70 | 70 | 30 |
| Corn | 0 | 0 | 10 | 0 | 40 | 20 | 0 | 0 | 70 | 90 | 30 | 30 | 30 | 10 |
| Foxtail, Giant | 20 | 10 | 30 | 40 | 90 | 90 | 30 | 0 | 90 | 100 | 100 | 100 | 80 | 90 |
| Goosegrass | 0 | 60 | 50 | 20 | 90 | 100 | 90 | 0 | 100 | 90 | 100 | 100 | 90 | 90 |
| Kochia | 40 | 0 | 0 | 0 | 70 | 60 | 50 | 0 | 70 | 40 | 50 | 90 | 20 | 90 |
| Oat, Wild | 0 | 0 | 0 | 0 | 0 | 20 | 0 | 0 | 10 | 70 | 0 | 50 | 30 | 30 |
| Pigweed, Palmer | 70 | 30 | 0 | 20 | 70 | 90 | 50 | 0 | 100 | 80 | 100 | 100 | 90 | 100 |
| Ragweed | 20 | 0 | 30 | 0 | 60 | 60 | 20 | 0 | 70 | 70 | 70 | 90 | 30 | 30 |
| Ryegrass, Italian | 50 | 60 | 40 | 50 | 90 | 100 | 50 | 0 | 90 | 90 | 90 | 60 | 90 | 90 |
| Soybean | 0 | 0 | 50 | 0 | 40 | 50 | 0 | 0 | 30 | 30 | 40 | 60 | 0 | 0 |
| Wheat | 0 | 0 | 0 | 0 | 50 | 70 | 0 | 0 | 50 | 100 | 50 | 80 | 50 | 50 |

| Table A1 | Compounds | | | | | | | | | | | | | |
|---|---|---|---|---|---|---|---|---|---|---|---|---|---|---|
| 125 g ai/ha | 324 | 325 | 326 | 327 | 328 | 329 | 330 | 331 | 332 | 333 | 334 | 335 | 336 | 337 |
| Preemergence | | | | | | | | | | | | | | |
| Blackgrass | 30 | 40 | 0 | 30 | 30 | 0 | 30 | 0 | 0 | 0 | 10 | 0 | 0 | 60 |
| Corn | 40 | 40 | 90 | 60 | 60 | 0 | 0 | 0 | 10 | 0 | 0 | 60 | 0 | 70 |
| Foxtail, Giant | 100 | 100 | 100 | 100 | 100 | 50 | 100 | 70 | 100 | 100 | 70 | 100 | 80 | 100 |
| Goosegrass | 100 | 100 | 90 | 90 | 80 | 100 | 100 | 90 | 90 | 80 | 90 | 70 | 70 | 100 |
| Kochia | 90 | 70 | 80 | 70 | 90 | 30 | 40 | 20 | 60 | 80 | 20 | 60 | 0 | 50 |
| Oat, Wild | 40 | 70 | 10 | 70 | 30 | 0 | 20 | 0 | 20 | 40 | 20 | 30 | 20 | 20 |
| Pigweed, Palmer | 100 | 100 | 100 | 90 | 100 | 50 | 80 | 30 | 90 | 100 | 70 | 100 | 100 | 100 |
| Ragweed | 80 | 90 | 90 | 90 | 80 | 70 | 70 | 90 | 80 | 70 | 50 | 90 | 50 | 90 |
| Ryegrass, Italian | 100 | 90 | 70 | 50 | 100 | 0 | 90 | 0 | 20 | 50 | 50 | 60 | 30 | 100 |
| Soybean | 60 | 60 | 90 | 90 | 90 | 0 | 30 | 10 | 70 | 60 | 30 | 40 | 0 | 90 |
| Wheat | 70 | 60 | 50 | 30 | 30 | 0 | 30 | 0 | 40 | 50 | 0 | 0 | 0 | 60 |

| Table A1 | Compounds | | | | | | | | | | | | | |
|---|---|---|---|---|---|---|---|---|---|---|---|---|---|---|
| 125 g ai/ha | 338 | 339 | 340 | 341 | 342 | 343 | 344 | 345 | 346 | 347 | 348 | 349 | 350 | 351 |
| Preemergence | | | | | | | | | | | | | | |
| Blackgrass | 0 | 0 | 90 | 30 | 40 | 90 | 30 | 70 | 90 | 30 | 70 | 80 | 0 | 50 |
| Corn | 50 | 0 | 90 | 0 | 0 | 60 | 10 | 10 | 20 | 0 | 10 | 100 | 0 | 0 |
| Foxtail, Giant | 90 | 20 | 100 | 50 | 80 | 100 | 100 | 100 | 100 | 60 | 90 | 100 | 80 | 100 |
| Goosegrass | 100 | 90 | 100 | 100 | 100 | 100 | 90 | 90 | 100 | 60 | 90 | 100 | 90 | 100 |
| Kochia | 10 | 90 | 60 | 40 | 50 | 70 | 30 | 30 | 30 | 50 | 30 | 100 | 70 | 90 |
| Oat, Wild | 0 | 0 | 90 | 0 | 10 | 70 | 10 | 10 | 80 | 0 | 10 | 50 | 10 | 30 |
| Pigweed, Palmer | 40 | 100 | 100 | 20 | 40 | 90 | 80 | 100 | 100 | 50 | 90 | 100 | 60 | 90 |
| Ragweed | 40 | 50 | 90 | 0 | 40 | 60 | 0 | 0 | 10 | 0 | 10 | 90 | 80 | 90 |
| Ryegrass, Italian | 30 | 50 | 100 | 40 | 50 | 100 | 40 | 80 | 90 | 0 | 70 | 80 | 30 | 90 |
| Soybean | 20 | 0 | 90 | 0 | 50 | 60 | 50 | 0 | 50 | 0 | 40 | 60 | 0 | 30 |
| Wheat | 80 | 0 | 90 | 0 | 0 | 90 | 0 | 10 | 70 | 20 | 30 | 70 | 10 | 50 |

| Table A1 | Compounds | | | | | | | | | | | | | |
|---|---|---|---|---|---|---|---|---|---|---|---|---|---|---|
| 125 g ai/ha | 352 | 353 | 354 | 355 | 356 | 357 | 358 | 359 | 360 | 361 | 362 | 363 | 364 | 365 |
| Preemergence | | | | | | | | | | | | | | |
| Blackgrass | 40 | 0 | 0 | 0 | 10 | 10 | 10 | 0 | 0 | 0 | 50 | 90 | 0 | 0 |
| Corn | 50 | 30 | 0 | 0 | 90 | 90 | 50 | 0 | 0 | 0 | 30 | 60 | 0 | 0 |
| Foxtail, Giant | 100 | 80 1 | 100 | 60 | 100 | 90 | 100 | 90 | 40 | 90 | 90 | 100 | 0 | 30 |
| Goosegrass | 100 | 70 1 | 100 | 90 | 90 | 100 | 100 | 90 | 90 | 100 | 100 | 100 | 0 | 90 |
| Kochia | 100 | 20 | 20 | 0 | 30 | 40 | 30 | 0 | 30 | 30 | 90 | 100 | 0 | 0 |
| Oat, Wild | 20 | 0 | 0 | 0 | 20 | 0 | 10 | 0 | 0 | 0 | 30 | 70 | 0 | 0 |
| Pigweed, Palmer | 100 | 40 | 50 | 70 | 100 | 100 | 90 | 20 | 40 | 20 | 80 | 100 | 0 | 30 |
| Ragweed | 90 | 20 | 50 | 40 | 0 | 80 | 80 | 80 | 80 | 90 | 50 | 80 | 0 | 20 |
| Ryegrass, Italian | 100 | 0 | 70 | 30 | 40 | 30 | 30 | 0 | 30 | 0 | 70 | 100 | 0 | 30 |
| Soybean | 40 | 30 | 70 | 30 | 80 | 20 | 70 | 30 | 0 | 20 | 40 | 30 | 0 | 0 |
| Wheat | 20 | 0 | 30 | 10 | 30 | 10 | 30 | 0 | 10 | 0 | 70 | 90 | 0 | 0 |

| Table A1 | Compounds | | | | | | | | | | | | | |
|---|---|---|---|---|---|---|---|---|---|---|---|---|---|---|
| 125 g ai/ha | 366 | 367 | 368 | 369 | 370 | 371 | 372 | 373 | 374 | 375 | 376 | 377 | 378 | 379 |
| Preemergence | | | | | | | | | | | | | | |
| Blackgrass | 50 | 0 | 40 | 0 | 0 | 20 | 40 | 30 | 0 | 50 | 20 | - | - | - |
| Corn | 10 | 90 | 90 | 0 | 0 | 90 | 10 | 20 | 40 | 70 | 0 | 0 | 0 | 0 |
| Foxtail, Giant | 100 | 100 | 100 | 20 | 90 | 100 | 100 | 90 | 100 | 90 | 30 | 0 | 20 | 70 |
| Goosegrass | 80 | 80 | 90 | 50 | 90 | 90 | 100 | 100 | 100 | 100 | 100 | 20 | 50 | 90 |
| Kochia | 30 | 20 | 90 | 0 | 20 | 70 | 70 | 0 | 0 | 70 | 80 | 20 | 20 | 10 |
| Oat, Wild | 30 | 30 | 20 | 20 | 0 | 0 | 20 | 0 | 30 | 20 | 10 | 0 | 40 | 20 |
| Pigweed, Palmer | 100 | 100 | 100 | 50 | 70 | 80 | 90 | 40 | 80 | 80 | 40 | 0 | 20 | - |
| Ragweed | 70 | 90 | 80 | 90 | 70 | 90 | 70 | 50 | 70 | 20 | 0 | 0 | 0 | 0 |
| Ryegrass, Italian | 40 | 20 | 100 | 40 | 30 | 80 | 90 | 0 | 90 | 80 | 30 | 40 | 40 | 40 |
| Soybean | 50 | 90 | 100 | 30 | 0 | 60 | 50 | 10 | 70 | 30 | 0 | 0 | 0 | 0 |
| Wheat | 10 | 40 | 100 | 0 | 10 | 10 | 30 | 20 | 30 | 100 | 20 | 20 | 20 | 30 |

| Table A1 | Compounds | | | | | | | | | | | | | |
|---|---|---|---|---|---|---|---|---|---|---|---|---|---|---|
| 125 g ai/ha | 380 | 381 | 382 | 383 | 392 | 393 | 394 | 397 | 398 | 399 | 400 | 401 | 402 | 403 |
| Preemergence | | | | | | | | | | | | | | |
| Blackgrass | - | - | - | - | 40 | 90 | 70 | 40 | 90 | 60 | 80 | 0 | 80 | 0 |
| Corn | 30 | 70 | 0 | 0 | 10 | 80 | 20 | 10 | 60 | 0 | 20 | 20 | 80 | 10 |
| Foxtail, Giant | 100 | 100 | 30 | 20 | 100 | 100 | 100 | 100 | 100 | 90 | 90 | 0 | 100 | 10 |
| Goosegrass | 100 | 100 | 30 | 80 | 100 | 100 | 100 | 100 | 100 | 90 | 100 | 30 | 100 | 0 |
| Kochia | 60 | 60 | 0 | 0 | 20 | 80 | 60 | 90 | 50 | 50 | 20 | 0 | 30 | 0 |
| Oat, Wild | 90 | 80 | 40 | 30 | 60 | 70 | 90 | 20 | 70 | 40 | 90 | 0 | 70 | 10 |
| Pigweed, Palmer | 40 | 90 | 20 | 0 | 20 | 60 | 90 | 90 | 100 | 60 | 100 | 20 | 80 | 0 |
| Ragweed | 40 | 0 | 0 | 0 | 0 | 90 | 90 | 90 | 80 | 60 | 90 | 10 | 50 | 20 |
| Ryegrass, Italian | 100 | 90 | 50 | 40 | 60 | 100 | 100 | 100 | 100 | 90 | 90 | 30 | 100 | 30 |
| Soybean | 50 | 50 | 0 | 0 | 0 | 80 | 60 | 40 | 90 | 40 | 50 | 0 | 80 | 0 |
| Wheat | 90 | 90 | 20 | 20 | 50 | 60 | 70 | 20 | 90 | 20 | 80 | 0 | 50 | 0 |

| Table A1 | Compounds | | | | | | | | | | | |
|---|---|---|---|---|---|---|---|---|---|---|---|---|
| 125 g ai/ha | 404 | 405 | 406 | 407 | 408 | 409 | 410 | 411 | 412 | 413 | 414 | 415 |
| Preemergence | | | | | | | | | | | | |
| Blackgrass | 0 | 40 | 90 | 40 | 100 | 90 | 80 | 50 | 20 | 20 | - | - |
| Corn | 0 | 30 | 30 | 0 | 90 | 60 | 0 | 0 | 70 | 10 | 10 | 80 |
| Foxtail, Giant | 90 | 90 | 100 | 80 | 100 | 100 | 10 | 30 | 90 | 10 | 0 | 90 |
| Goosegrass | 90 | 90 | 100 | 90 | 100 | 100 | 90 | 90 | 90 | 80 | 0 | 100 |
| Kochia | 60 | 70 | 80 | 40 | 70 | 90 | 0 | 80 | 100 | 90 | 80 | 60 |
| Oat, Wild | 0 | 30 | 30 | 40 | 100 | 70 | 70 | 30 | 20 | 0 | 30 | 80 |
| Pigweed, Palmer | 30 | 60 | 70 | 40 | 60 | 90 | 0 | 60 | 90 | 90 | 40 | 60 |
| Ragweed | 90 | 100 | 90 | 90 | 50 | 90 | 70 | 90 | 100 | 90 | 20 | 90 |
| Ryegrass, Italian | 50 | 90 | 100 | 60 | 100 | 100 | 70 | 40 | 100 | 20 | 30 | 70 |
| Soybean | 40 | 70 | 80 | 30 | 50 | 90 | 20 | 40 | 90 | 50 | 0 | 0 |
| Wheat | 10 | 30 | 30 | 0 | 100 | 90 | 0 | 0 | 10 | 0 | 0 | 50 |

| Table A1 | Compounds | | | | | | | | | | | | | |
|---|---|---|---|---|---|---|---|---|---|---|---|---|---|---|
| 31 g ai/ha | 281 | 282 | 283 | 284 | 285 | 286 | 287 | 288 | 289 | 290 | 292 | 293 | 294 | 295 |
| Preemergence | | | | | | | | | | | | | | |
| Blackgrass | 0 | 30 | - | - | - | - | 0 | 0 | 0 | 20 | 0 | 0 | 10 | 0 |
| Corn | 0 | 0 | 0 | 0 | 0 | 0 | 0 | 0 | 0 | 0 | 0 | 0 | 0 | 0 |
| Foxtail, Giant | 0 | 0 | 10 | 0 | 40 | 80 | 0 | 20 | 30 | 0 | 50 | 0 | 0 | 0 |
| Goosegrass | 10 | 0 | 30 | 40 | 0 | 30 | 70 | 30 | 80 | 70 | 80 | 70 | 0 | 80 |
| Kochia | 0 | 0 | 30 | 70 | 20 | 0 | 20 | 0 | 20 | 20 | 30 | 0 | 0 | 0 |
| Oat, Wild | 50 | 20 | - | 0 | 0 | 0 | 0 | 0 | 20 | 0 | 0 | 0 | 0 | 0 |
| Pigweed, Palmer | 0 | 0 | 10 | 30 | 10 | 50 | 0 | 50 | 90 | 0 | 60 | 0 | 10 | 0 |
| Ragweed | 0 | 0 | 0 | 0 | 0 | 0 | 0 | 0 | 0 | 20 | 10 | 0 | 0 | 0 |
| Ryegrass, Italian | 0 | 0 | 0 | 0 | 0 | 0 | 70 | 0 | 20 | 30 | 60 | 30 | 40 | 40 |
| Soybean | 0 | 20 | 0 | 40 | 0 | 0 | 0 | 30 | 20 | 0 | 0 | 0 | 0 | 0 |
| Wheat | 0 | 0 | 0 | 0 | 0 | 0 | 0 | 0 | 30 | 0 | 20 | 0 | 0 | 0 |

| Table A1 | Compounds | | | | | | | | | | | | | |
|---|---|---|---|---|---|---|---|---|---|---|---|---|---|---|
| 31 g ai/ha | 296 | 297 | 298 2 | 299 | 300 | 301 | 302 | 303 | 304 | 305 | 306 | 307 | 308 | 309 |
| Preemergence | | | | | | | | | | | | | | |
| Blackgrass | 10 | 20 | 10 | 0 | 0 | 30 | 0 | 0 | 0 | 10 | 0 | 0 | 0 | 30 |
| Corn | 0 | 0 | 0 | 0 | 0 | 0 | 0 | 0 | 0 | 10 | 0 | 0 | 0 | 0 |
| Foxtail, Giant | 30 | 80 | 10 | 0 | 20 | 10 | 20 | 80 | 0 | 20 | 0 | 50 | 50 | 60 |
| Goosegrass | 40 | 70 | 70 | 60 | 60 | 80 | 0 | 50 | 30 | 90 | 50 | 80 | 80 | 50 |
| Kochia | 60 | 80 | 20 | 0 | 0 | 30 | 30 | 20 | 0 | 0 | 0 | 0 | 20 | 0 |
| Oat, Wild | 0 | 0 | 0 | 0 | 0 | 0 | 0 | 20 | 50 | 20 | 0 | 0 | 0 | 20 |
| Pigweed, Palmer | 10 | 30 | 50 | 0 | 10 | 10 | 20 | 20 | 0 | 40 | 50 | 30 | 40 | 40 |
| Ragweed | 50 | 20 | 0 | 0 | 0 | 0 | 0 | 0 | 0 | 0 | 0 | 0 | 0 | 0 |
| Ryegrass, Italian | 0 | 0 | 60 | 50 | 40 | 20 | 50 | 50 | 40 | 40 | 0 | 50 | 60 | 60 |
| Soybean | 20 | 0 | 0 | 0 | 0 | 0 | 0 | 0 | 0 | 0 | 0 | 0 | 0 | 0 |
| Wheat | 10 | 20 | 0 | 0 | 10 | 0 | 0 | 30 | 0 | 20 | 0 | 10 | 20 | 0 |

| Table A1 | Compounds | | | | | | | | | | | | | |
|---|---|---|---|---|---|---|---|---|---|---|---|---|---|---|
| 31 g ai/ha | 310 | 311 | 312 | 313 | 314 | 315 | 316 | 317 | 318 | 319 | 320 | 321 | 322 | 323 |
| Preemergence | | | | | | | | | | | | | | |
| Blackgrass | 0 | 20 | 0 | 0 | 0 | 20 | 0 | 0 | 30 | 30 | 0 | 0 | 0 | 0 |
| Corn | 0 | 0 | 0 | 0 | 10 | 10 | 0 | 0 | 0 | 40 | 0 | 0 | 0 | 0 |
| Foxtail, Giant | 0 | 0 | 20 | 0 | 30 | 70 | 0 | 0 | 30 | 90 | 10 | 30 | 40 | 50 |
| Goosegrass | 0 | 10 | 0 | 0 | 80 | 90 | 40 | 0 | 80 | 90 | 80 | 70 | 0 | 50 |
| Kochia | 0 | 0 | 0 | 0 | 0 | 0 | 0 | 0 | 60 | 0 | 20 | 80 | 0 | 20 |
| Oat, Wild | 0 | 0 | 0 | 0 | 0 | 0 | 0 | 0 | 0 | 60 | 0 | 0 | 0 | 20 |
| Pigweed, Palmer | 0 | 30 | 0 | 0 | 70 | 100 | 40 | 0 | 80 | 70 | 50 | 90 | 40 | 90 |
| Ragweed | 0 | 0 | 0 | 0 | 0 | 20 | 0 | 0 | 50 | 0 | 60 | 50 | 20 | 0 |
| Ryegrass, Italian | 40 | 0 | 0 | 30 | 40 | 70 | 30 | 0 | 50 | 50 | 90 | 60 | 60 | 40 |
| Soybean | 0 | 0 | 0 | 0 | 20 | 40 | 0 | 0 | 0 | 20 | 20 | 60 | 0 | 0 |
| Wheat | 0 | 0 | 0 | 0 | 0 | 20 | 0 | 0 | 0 | 80 | 10 | 0 | 0 | 0 |

| Table A1 | Compounds | | | | | | | | | | | | | |
|---|---|---|---|---|---|---|---|---|---|---|---|---|---|---|
| 31 g ai/ha | 324 | 325 | 326 | 327 | 328 | 329 | 330 | 331 | 332 | 333 | 334 | 335 | 336 | 337 |
| Preemergence | | | | | | | | | | | | | | |
| Blackgrass | 20 | 0 | 0 | 0 | 20 | 0 | 0 | 0 | 0 | 0 | 10 | 0 | 0 | 40 |
| Corn | 20 | 0 | 0 | 0 | 10 | 0 | 0 | 0 | 0 | 0 | 0 | 0 | 0 | 0 |
| Foxtail, Giant | 90 | 30 | 100 | 100 | 20 | 30 | 80 | 0 | 100 | 30 | 40 | 60 | 20 | 20 |
| Goosegrass | 80 | 100 | 90 | 50 | 40 | 20 | 40 | 0 | 50 | 80 | 70 | 30 | 30 | 50 |
| Kochia | 60 | 20 | 80 | 0 | 20 | 0 | 10 | 0 | 20 | 20 | 0 | 20 | 0 | 20 |
| Oat, Wild | 20 | 0 | 10 | 0 | 0 | 0 | 0 | 0 | 0 | 20 | 10 | 0 | 0 | 0 |
| Pigweed, Palmer | 100 | 80 | 90 | 90 | 100 | 10 | 70 | 0 | 60 | 90 | 20 | 100 | 60 | 40 |
| Ragweed | 20 | 50 | 90 | 90 | 10 | 60 | 40 | 0 | 50 | 0 | 0 | 20 | 0 | 70 |
| Ryegrass, Italian | 20 | 40 | 20 | 40 | 30 | 0 | 0 | 0 | 0 | 30 | 30 | 0 | 30 | 30 |
| Soybean | 20 | 0 | 40 | 30 | 50 | 0 | 0 | 0 | 20 | 40 | 0 | 30 | 0 | 40 |
| Wheat | 0 | 20 | 0 | 0 | 0 | 0 | 0 | 0 | 10 | 0 | 0 | 0 | 0 | 10 |

| Table A1 | Compounds | | | | | | | | | | | | | |
|---|---|---|---|---|---|---|---|---|---|---|---|---|---|---|
| 31 g ai/ha | 338 | 339 | 340 | 341 | 342 | 343 | 344 | 345 | 346 | 347 3 | 348 | 349 | 350 | 351 |
| Preemergence | | | | | | | | | | | | | | |
| Blackgrass | 0 | 0 | 60 | 0 | 0 | 60 | 0 | 40 | 80 | 10 | 30 | 50 | 0 | 10 |
| Corn | 10 | 0 | 0 | 0 | 0 | 10 | 10 | 10 | 10 | 0 | 0 | 20 | 0 | 0 |
| Foxtail, Giant | 10 | 0 | 50 | 0 | 0 | 90 | 30 | 40 | 60 | 0 | 30 | 90 | 0 | 30 |
| Goosegrass | 40 | 0 | 100 | 30 | 40 | 90 | 20 | 30 | 90 | 10 | 30 | 90 | 30 | 40 |
| Kochia | 0 | 0 | - | 0 | 0 | 30 | 0 | 20 | 30 | 0 | 0 | 80 | 20 | 20 |
| Oat, Wild | 0 | 0 | 0 | 0 | 0 | 0 | 0 | 20 | 30 | 0 | 0 | 10 | 0 | 10 |
| Pigweed, Palmer | 0 | 30 | 60 | 0 | 0 | 40 | 50 | 80 | 100 | 20 | 10 | 100 | 20 | 80 |
| Ragweed | 0 | 20 | 10 | 0 | 0 | 10 | 0 | 0 | 0 | 0 | 0 | 30 | 20 | 50 |
| Ryegrass, Italian | 30 | 30 | 50 | 0 | 40 | 60 | 20 | 30 | 50 | 0 | 0 | 70 | 30 | 40 |
| Soybean | 0 | 0 | 40 | 0 | 0 | 50 | 50 | 0 | 50 | 0 | 30 | 20 | 0 | 20 |
| Wheat | 0 | 0 | 20 | 0 | 0 | 10 | 0 | 30 | 20 | 0 | 0 | 20 | 0 | 10 |

| Table A1 | Compounds | | | | | | | | | | | | | |
|---|---|---|---|---|---|---|---|---|---|---|---|---|---|---|
| 31 g ai/ha | 352 | 353 | 354 | 355 | 356 | 357 | 358 | 359 | 360 | 361 | 362 | 363 | 364 | 365 |
| Preemergence | | | | | | | | | | | | | | |
| Blackgrass | 20 | 0 | 0 | 0 | 0 | 0 | 0 | 0 | 0 | 0 | 10 | 50 | 0 | 0 |
| Corn | 10 | 10 | 0 | 0 | 0 | 10 | 0 | 0 | 0 | 0 | 0 | 0 | 0 | 0 |
| Foxtail, Giant | 30 | 10 | 80 | 0 | 90 | 10 | 80 | 0 | 0 | 0 | 20 | 90 | 0 | 0 |
| Goosegrass | 90 | 20 | 70 | 20 | 90 | 80 | 30 | 0 | 0 | 10 | 90 | 100 | 0 | 10 |
| Kochia | 30 | 0 | 0 | 0 | 0 | 20 | 20 | 0 | 0 | 0 | 50 | 80 | 0 | 0 |
| Oat, Wild | 0 | 0 | 0 | 0 | 0 | 0 | 0 | 0 | 0 | 0 | 0 | 30 | 0 | 0 |
| Pigweed, Palmer | 70 | 30 | 10 | 0 | 50 | 40 | 90 | 0 | 0 | 0 | 10 | 90 | 0 | 30 |
| Ragweed | 60 | 20 | 20 | 0 | 0 | 0 | 40 | 0 | 60 | 20 | 0 | 20 | 0 | 0 |
| Ryegrass, Italian | 20 | 0 | 20 | 20 | 0 | 0 | 0 | 0 | 0 | 0 | 30 | 80 | 0 | 0 |
| Soybean | 30 | 10 | 30 | 20 | 0 | 0 | 50 | 0 | 0 | 0 | 10 | 10 | 0 | 0 |
| Wheat | 10 | 0 | 0 | 10 | 0 | 0 | 0 | 0 | 0 | 0 | 20 | 40 | 0 | 0 |

| Table A1 | Compounds | | | | | | | | | | | | | |
|---|---|---|---|---|---|---|---|---|---|---|---|---|---|---|
| 31 g ai/ha | 366 | 367 | 368 | 369 | 370 | 371 | 372 | 373 | 374 | 375 | 376 | 377 | 378 | 379 |
| Preemergence | | | | | | | | | | | | | | |
| Blackgrass | 0 | 0 | 10 | 0 | 0 | 0 | 0 | 30 | 0 | 20 | 0 | - | - | - |
| Corn | 0 | 10 | 0 | 0 | 0 | 0 | 0 | 0 | 20 | 0 | 0 | 0 | 0 | 0 |
| Foxtail, Giant | 100 | 90 | 90 | 0 | 10 | 10 | 90 | 0 | 50 | 10 | 0 | 0 | 0 | 0 |
| Goosegrass | 10 | 60 | 70 | 0 | 30 | 0 | 90 | 30 | 60 | 70 | 50 | 0 | 20 | 20 |
| Kochia | 0 | 0 | 20 | 0 | 0 | 0 | 0 | 0 | 0 | 0 | 0 | 0 | 0 | 0 |
| Oat, Wild | 10 | 20 | 10 | 0 | 0 | 0 | 0 | 0 | 0 | 0 | 0 | 0 | 10 | 0 |
| Pigweed, Palmer | 100 | 80 | 100 | 0 | 30 | 10 | 40 | 20 | 60 | 0 | 0 | 0 | 0 | - |
| Ragweed | 30 | 20 | 50 | 0 | 0 | 40 | 0 | 50 | 0 | 0 | 0 | 0 | 0 | 0 |
| Ryegrass, Italian | 0 | 0 | 50 | 40 | 30 | 0 | 40 | 0 | 0 | 30 | 0 | 0 | 0 | 0 |
| Soybean | 0 | 0 | 90 | 0 | 0 | 0 | 0 | 0 | 0 | 0 | 0 | 0 | 0 | 0 |
| Wheat | 0 | 10 | 20 | 0 | 0 | 0 | 10 | 0 | 0 | 0 | 0 | 0 | 10 | 0 |

| Table A1 | Compounds | | | | | | | | | | | | | |
|---|---|---|---|---|---|---|---|---|---|---|---|---|---|---|
| 31 g ai/ha | 380 | 381 | 382 | 383 | 392 | 393 | 394 | 398 | 399 | 400 | 401 | 402 | 403 | 404 |
| Preemergence | | | | | | | | | | | | | | |
| Blackgrass | - | - | - | - | 10 | 70 | 0 | 60 | 20 | 20 | 0 | 70 | 0 | 0 |
| Corn | 0 | 0 | 0 | 0 | 10 | 0 | 0 | 0 | 0 | 0 | 0 | 0 | 0 | 0 |
| Foxtail, Giant | 80 | 20 | 0 | 0 | 0 | 70 | 90 | 90 | 10 | 20 | 0 | 90 | 0 | 0 |
| Goosegrass | 50 | 30 | 0 | 0 | 40 | 100 | 80 | 100 | 90 | 90 | 0 | 90 | 0 | 20 |
| Kochia | 0 | 0 | 0 | 0 | 20 | 60 | 30 | 40 | 0 | 0 | 0 | 0 | 0 | 40 |
| Oat, Wild | 20 | 50 | 20 | 0 | 20 | 10 | 20 | 20 | 20 | 50 | 0 | 50 | 0 | 30 |
| Pigweed, Palmer | 30 | 30 | 0 | 0 | 0 | 30 | 20 | 80 | 30 | 50 | 0 | 60 | 0 | 10 |
| Ragweed | 0 | 0 | 0 | 0 | 0 | 40 | 40 | 20 | 0 | 70 | 0 | 20 | 0 | 0 |
| Ryegrass, Italian | 60 | 50 | 30 | 0 | 0 | 90 | 40 | 60 | 40 | 60 | 0 | 90 | 30 | 0 |
| Soybean | 20 | 0 | 0 | 0 | 0 | 60 | 50 | 70 | 30 | 50 | 0 | 10 | 0 | 0 |
| Wheat | 30 | 40 | 20 | 20 | 0 | 40 | 30 | 20 | 0 | 10 | 0 | 30 | 0 | 20 |

| Table A1 | Compounds | | | | | | | | | | |
|---|---|---|---|---|---|---|---|---|---|---|---|
| 31 g ai/ha | 405 | 406 | 407 | 408 | 409 | 410 | 411 | 412 | 413 | 414 | 415 |
| Preemergence | | | | | | | | | | | |
| Blackgrass | 30 | 80 | 0 | 90 | 50 | 0 | 0 | 0 | 20 | - | - |
| Corn | 0 | 0 | 0 | 40 | 0 | 0 | 0 | 20 | 0 | 0 | 0 |
| Foxtail, Giant | 80 | 90 | 0 | 100 | 60 | 0 | 0 | 20 | 0 | 0 | 80 |
| Goosegrass | 70 | 90 | 40 | 100 | 90 | 40 | 30 | 20 | 50 | 0 | 90 |
| Kochia | 30 | 40 | 0 | 0 | 50 | 0 | 0 | 90 | 70 | 20 | 10 |
| Oat, Wild | 30 | 20 | 0 | 90 | 30 | 0 | 0 | 0 | 0 | 20 | 30 |
| Pigweed, Palmer | 20 | 70 | 0 | 30 | 60 | 0 | 0 | 90 | 30 | 10 | 0 |
| Ragweed | 90 | 40 | 20 | 40 | 40 | 0 | 60 | 50 | 30 | 0 | 0 |
| Ryegrass, Italian | 60 | 90 | 0 | 100 | 80 | 20 | 30 | 30 | 0 | 0 | 50 |
| Soybean | 30 | 30 | 0 | 40 | 50 | 0 | 20 | 50 | 0 | 0 | 0 |
| Wheat | 30 | 0 | 0 | 90 | 30 | 0 | 0 | 0 | 0 | 0 | 20 |

| Table A1 | Compound | Table A1 | Compound |
|---|---|---|---|
| 500 g ai/ha | 397 | 500 g ai/ha | 397 |
| Postemergence | | Postemergence | |
| Blackgrass | 90 | Oat, Wild | 0 |
| Corn | 10 | Pigweed, Palmer | 100 |
| Foxtail, Giant | 70 | Ragweed | 80 |
| Galium | 100 | Ryegrass, Italian | 1 80 |
| Goosegrass | 90 | Soybean | 60 |
| Kochia | 90 | Wheat | 70 |

| Table A1 | Compounds | | | | | | | | | | | | | |
|---|---|---|---|---|---|---|---|---|---|---|---|---|---|---|
| 125 g ai/ha | 281 | 282 | 283 | 284 | 285 | 286 | 287 | 288 | 289 | 290 | 292 | 293 | 294 | 295 |
| Postemergence | | | | | | | | | | | | | | |
| Blackgrass | 0 | 20 | 10 | 10 | 30 | 40 | 0 | 0 | 10 | 0 | 0 | 0 | 0 | 0 |
| Corn | 0 | 80 | 0 | 0 | 0 | 0 | 0 | 10 | 20 | 20 | 0 | 10 | 20 | 0 |
| Foxtail, Giant | 30 | 20 | 90 | 70 | 80 | 80 | 0 | 70 | 90 | 70 | 30 | 40 | 20 | 10 |
| Galium | 0 | 0 | 70 | 70 | 80 | 80 | 0 | 0 | 80 | 90 | 80 | 70 | 70 | 60 |
| Goosegrass | 40 | 80 | 30 | 20 | 50 | 40 | 60 | 70 | 70 | 70 | 80 | 60 | 30 | 70 |
| Horseweed | 70 | 80 | 80 | 80 | 80 | 60 | 40 | 30 | 90 | 90 | 30 | 50 | 90 | 50 |
| Kochia | 10 | 30 | 60 | 40 | 90 | 50 | 10 | 10 | 60 | 20 | 50 | 60 | 20 | 30 |
| Oat, Wild | 0 | 50 | 0 | 0 | 10 | 0 | 0 | 0 | 20 | 10 | 30 | 20 | 20 | 0 |
| Pigweed, Palmer | 40 | 0 | 50 | 40 | 20 | 30 | 40 | 30 | 60 | 20 | 30 | 40 | 30 | 30 |
| Ragweed | 70 | 70 | 80 | 90 | 90 | 40 | 70 | 80 | 80 | 70 | 40 | 30 | 80 | 70 |
| Ryegrass, Italian | 0 | 60 | 40 | 0 | 0 | 0 | 60 | 0 | 60 | 50 | 30 | 40 | 40 | 30 |
| Soybean | 60 | 60 | 60 | 60 | 60 | 60 | 50 | 40 | 50 | 20 | 80 | 70 | 50 | 50 |
| Wheat | 20 | 80 | 50 | 30 | 20 | 20 | 10 | 10 | 50 | 60 | 70 | 50 | 20 | 70 |

| Table A1 | Compounds | | | | | | | | | | | | | |
|---|---|---|---|---|---|---|---|---|---|---|---|---|---|---|
| 125 g ai/ha | 296 | 297 | 298 | 299 | 300 | 301 | 302 | 303 | 304 | 305 | 306 | 307 | 308 | 309 |
| Postemergence | | | | | | | | | | | | | | |
| Blackgrass | 0 | 0 | 0 | 0 | 0 | 0 | 0 | - | - | 0 | 0 | 10 | 0 | - |
| Corn | 0 | 0 | 0 | 0 | 0 | 30 | 0 | 40 | 20 | 0 | 0 | 0 | 30 | 30 |
| Foxtail, Giant | 10 | 60 | 30 | 30 | 20 | 60 | 0 | 80 | 50 | 60 | 30 | 40 | 40 | 60 |
| Galium | 80 | 90 | 60 | 60 | 60 | 90 | 0 | 90 | 0 | 60 | 80 | 60 | 90 | 80 |
| Goosegrass | 60 | 70 | 70 | 40 | 70 | 60 | 10 | 70 | 0 | 80 | 80 | 80 | 80 | 30 |
| Horseweed | 80 | 80 | 70 | 30 | 70 | 80 | 40 | 70 | 0 | - | 40 | 80 | 60 | 20 |
| Kochia | 40 | 60 | 10 | 20 | 20 | 30 | 50 | 60 | 20 | 10 | 20 | 30 | 70 | 60 |
| Oat, Wild | 10 | 30 | 0 | 0 | 0 | 10 | 0 | 20 | 0 | 20 | 20 | 20 | 0 | 20 |
| Pigweed, Palmer | 20 | 20 | 30 | 10 | 10 | 30 | 40 | 20 | 0 | 40 | 40 | 40 | 0 | 20 |
| Ragweed | 30 | 70 | 60 | 70 | 40 | 70 | 70 | 80 | 30 | 60 | 70 | 80 | 90 | 50 |
| Ryegrass, Italian | 40 | 60 | 70 | 50 | 40 | 60 | 0 | 60 | 0 | 40 | 10 | 80 | 30 | 30 |
| Soybean | 40 | 50 | 50 | 50 | 40 | 40 | 40 | 50 | 50 | 50 | 40 | 50 | 60 | 30 |
| Wheat | 30 | 80 | 70 | 70 | 60 | 60 | 10 | 50 | 20 | 60 | 20 | 40 | 50 | 40 |

| Table A1 | Com poun ds | | | | | | | | | | | | | |
|---|---|---|---|---|---|---|---|---|---|---|---|---|---|---|
| 125 g ai/ha | 310 | 311 | 312 | 313 | 314 | 315 | 316 | 317 | 318 | 319 | 320 | 321 | 322 | 323 |
| Postemergence | | | | | | | | | | | | | | |
| Blackgrass | 0 | 0 | 0 | 0 | 10 | 0 | 0 | 0 | 50 | 10 | 0 | 10 | 50 | 50 |
| Corn | 0 | 0 | 0 | 0 | 30 | 20 | 0 | 0 | 60 | 80 | 0 | 20 | 0 | 0 |
| Foxtail, Giant | 0 | 20 | 10 | 0 | 30 | 20 | 0 | 0 | 80 | 90 | 40 | 0 | 40 | 50 |
| Galium | 0 | 0 | 30 | 30 | 70 | 90 | 50 | 0 | 60 | 70 | 90 | 100 | 70 | 80 |
| Goosegrass | 0 | 0 | 0 | 0 | 80 | 80 | 90 | 0 | 30 | 90 | 0 | 80 | 50 | 50 |
| Horseweed | 30 | - | 20 | 0 | 50 | 60 | 40 | 0 | 50 | 40 | 70 | 80 | 40 | 50 |
| Kochia | 20 | 0 | 0 | 20 | 70 | 80 | 70 | 0 | 90 | 80 | 70 | 80 | 30 | 60 |
| Oat, Wild | 10 | 0 | 20 | 0 | 10 | 20 | 10 | 0 | 10 | 10 | 0 | 10 | 0 | 0 |
| Pigweed, Palmer | 0 | 0 | 0 | 10 | 60 | 50 | 70 | 0 | 50 | 50 | 80 | 70 | 50 | 60 |
| Ragweed | 30 | 0 | 40 | 0 | 70 | 80 | 50 | 0 | 80 | 60 | 80 | 90 | 80 | 50 |
| Ryegrass, Italian | 30 | 0 | 20 | 0 | 70 | 70 | 30 | 0 | 70 | 50 | 40 | 40 | 40 | 20 |
| Soybean | 30 | 40 | 20 | 20 | 50 | 60 | 50 | 0 | 40 | 50 | 70 | 40 | 30 | 30 |
| Wheat | 20 | 0 | 0 | 0 | 70 | 70 | 40 | 0 | 70 | 70 | 30 | 10 | 30 | 50 |

| Table A1 | Compounds | | | | | | | | | | | | | |
|---|---|---|---|---|---|---|---|---|---|---|---|---|---|---|
| 125 g ai/ha | 324 | 325 | 326 | 327 | 328 | 329 | 330 | 331 | 332 | 333 | 334 | 335 | 336 | 337 |
| Postemergence | | | | | | | | | | | | | | |
| Blackgrass | 0 | 90 | 0 | 0 | 0 | 30 | 40 | 50 | 90 | 30 | 0 | 10 | 0 | 30 |
| Corn | 30 | 30 | 0 | 40 | 50 | 0 | 10 | 50 | 50 | 0 | 0 | 0 | 0 | 40 |
| Foxtail, Giant | 90 | 90 | 30 | 90 | 70 | 70 | 80 | 80 | 90 | 30 | 0 | 0 | 40 | 90 |
| Galium | 80 | 90 | 100 | 100 | 100 | 80 | 60 | 90 | 90 | 90 | 60 | 80 | 100 | 100 |
| Goosegrass | 80 | 90 | 80 | 70 | 80 | 30 | 40 | 50 | 90 | 30 | 0 | 10 | 0 | 90 |
| Horseweed | - | 90 | - | - | - | 90 | 90 | 80 | 80 | 30 | 30 | 70 | 60 | - |
| Kochia | 50 | 50 | 70 | 30 | 60 | 20 | 50 | 10 | 10 | 70 | 40 | 60 | 80 | 70 |
| Oat, Wild | 0 | 10 | 0 | 0 | 0 | 0 | 10 | 20 | 30 | 10 | 0 | 0 | 0 | 20 |
| Pigweed, Palmer | 70 | 90 | 80 | 50 | 40 | 10 | 50 | 20 | 30 | 60 | 50 | 30 | 50 | 50 |
| Ragweed | 70 | 70 | 80 | 90 | 70 | 70 | 80 | 80 | 80 | 80 | 50 | 80 | 80 | 90 |
| Ryegrass, Italian | 30 | 60 | 30 | 30 | 60 | 0 | 0 | 50 | 50 | 0 | 0 | 30 | 0 | 20 |
| Soybean | 60 | 80 | 70 | 60 | 60 | 60 | 80 | 70 | 100 | 80 | 40 | 30 | 50 | 70 |
| Wheat | 30 | 50 | 0 | 50 | 50 | 0 | 30 | 50 | 50 | 30 | 0 | 0 | 0 | 30 |

| Table A1 | Compounds | | | | | | | | | | | | | |
|---|---|---|---|---|---|---|---|---|---|---|---|---|---|---|
| 125 g ai/ha | 338 | 339 | 340 | 341 | 342 | 343 | 344 | 345 | 346 | 347 | 348 | 349 | 350 | 351 |
| Postemergence | | | | | | | | | | | | | | |
| Blackgrass | 0 | 30 | 20 | 0 | 0 | 30 | 50 | 40 | 60 | 30 | 90 | 70 | 0 | 80 |
| Corn | 30 | 30 | 50 | 0 | 30 | 40 | 50 | 50 | 60 | 10 | 20 | 90 | 0 | 70 |
| Foxtail, Giant | 80 | 50 | 50 | 20 | 30 | 30 | 30 | 50 | 50 | 30 | 30 | 90 | 30 | 50 |
| Galium | 50 | 50 | 80 | 80 | 80 | 100 | 100 | 100 | 100 | 100 | 80 | 100 | 90 | 100 |
| Goosegrass | 70 | 30 | 90 | 60 | 80 | 90 | 30 | 70 | 90 | 40 | 30 | 90 | 80 | 90 |
| Horseweed | - | 100 | - | - | - | - | - | - | - | - | - | 100 | 100 | 90 |
| Kochia | 60 | 70 | 50 | 30 | 30 | 60 | 60 | 80 | 60 | 60 | 70 | 80 | 60 | 30 |
| Oat, Wild | 10 | 0 | 20 | 20 | 20 | 50 | 30 | 40 | 40 | 20 | 20 | 30 | 0 | 10 |
| Pigweed, Palmer | 30 | 20 | 30 | 20 | 30 | 60 | 50 | 60 | 50 | 50 | 70 | 70 | 40 | 20 |
| Ragweed | 70 | 70 | 80 | 80 | 80 | 80 | 60 | 70 | 70 | 70 | 60 | 90 | 90 | 90 |
| Ryegrass, Italian | 10 | 50 | 20 | 0 | 10 | 80 | 40 | 60 | 80 | 60 | 40 | 90 | 30 | 50 |
| Soybean | 40 | 10 | 60 | 50 | 50 | 60 | 50 | 50 | 60 | 50 | 90 | 70 | 40 | 50 |
| Wheat | 70 | 40 | 70 | 70 | 70 | 60 | 70 | 60 | 70 | 40 | 60 | 70 | 10 | 60 |

| Table A1 | Compounds | | | | | | | | | | | | | |
|---|---|---|---|---|---|---|---|---|---|---|---|---|---|---|
| 125 g ai/ha | 352 | 353 | 354 | 355 | 356 | 357 | 358 | 359 | 360 | 361 | 362 | 363 | 364 | 365 |
| Postemergence | | | | | | | | | | | | | | |
| Blackgrass | 20 | 0 | 0 | 0 | 0 | 0 | 0 | 0 | 0 | 0 | 50 | 50 | 0 | 30 |
| Corn | 80 | 0 | 0 | 20 | 40 | 0 | 0 | 0 | 0 | 40 | 40 | 60 | 0 | 0 |
| Foxtail, Giant | 70 | 50 | 70 | 60 | 40 | 20 | 40 | 60 | 0 | 50 | 90 | 90 | 20 | 0 |
| Galium | 100 | 70 | 80 | 60 | 100 | 100 | 100 | 80 | 100 | 90 | 80 | 90 | 30 | 80 |
| Goosegrass | 90 | 30 | 100 | 70 | 70 | 70 | 80 | 20 | 40 | 30 | 90 | 90 | 0 | 0 |
| Horseweed | 100 | 100 | 90 | 70 | 80 | 80 | 70 | 80 | 70 | 90 | 90 | 90 | 0 | 20 |
| Kochia | 70 | 50 | 50 | 50 | 50 | 30 | 70 | 40 | 50 | 50 | 80 | 70 | 30 | 40 |
| Oat, Wild | 20 | 10 | 10 | 10 | 10 | 0 | 0 | 0 | 0 | 20 | 10 | 50 | 0 | 0 |
| Pigweed, Palmer | 60 | 30 | 10 | 30 | 10 | 50 | 40 | 20 | 40 | 20 | 30 | 10 | 0 | 0 |
| Ragweed | 90 | 60 | 50 | 60 | 80 | 80 | 80 | 70 | 90 | 80 | 80 | 70 | 0 | 30 |
| Ryegrass, Italian | 90 | 0 | 40 | 40 | 30 | 20 | 20 | 0 | 0 | 0 | 90 | 90 | 0 | 10 |
| Soybean | 60 | 60 | 60 | 40 | 80 | 90 | 70 | 60 | 40 | 70 | 70 | 60 | 10 | 60 |
| Wheat | 50 | 50 | 40 | 50 | 30 | 0 | 0 | 30 | 0 | 20 | 60 | 60 | 0 | 20 |

| Table A1 | Compounds | | | | | | | | | | | | | |
|---|---|---|---|---|---|---|---|---|---|---|---|---|---|---|
| 125 g ai/ha | 366 | 367 | 368 | 369 | 370 | 371 | 372 | 373 | 374 | 375 | 376 | 377 | 378 | 379 |
| Postemergence | | | | | | | | | | | | | | |
| Blackgrass | 0 | 0 | 10 | 0 | 0 | 10 | 30 | 10 | 10 | 0 | 50 | 0 | 30 | 30 |
| Corn | 0 | 70 | 90 | 50 | 90 | 90 | 50 | 40 | 70 | 0 | 0 | 0 | 30 | 0 |
| Foxtail, Giant | 80 | 90 | 90 | 90 | 80 | 90 | 40 | 60 | 80 | 10 | 20 | 0 | 10 | 10 |
| Galium | 100 | 100 | 100 | 80 | 100 | 100 | 80 | 70 | 80 | 20 | 30 | 0 | 40 | 40 |
| Goosegrass | 80 | 70 | 90 | 0 | 60 | 80 | 80 | 70 | 90 | 90 | 50 | 0 | 30 | 60 |
| Horseweed | - | - | - | - | - | - | - | - | - | 50 | 0 | 80 | 90 | 50 |
| Kochia | 30 | 30 | 80 | 50 | 20 | 70 | 0 | 10 | 10 | 60 | 50 | 30 | 60 | 40 |
| Oat, Wild | 0 | 0 | 10 | 0 | 0 | 0 | 10 | 10 | 10 | 0 | 0 | 0 | 30 | 20 |
| Pigweed, Palmer | 20 | 40 | 40 | 40 | 20 | 30 | 40 | 10 | 30 | 20 | 20 | 0 | 10 | 10 |
| Ragweed | 80 | 70 | 80 | 80 | 70 | 70 | 80 | 80 | 80 | 50 | 40 | 30 | 30 | 40 |
| Ryegrass, Italian | 20 | 0 | 90 | 20 | 0 | 80 | 50 | 30 | 50 | 40 | 0 | 0 | 0 | 0 |
| Soybean | 60 | 80 | 90 | 90 | 80 | 90 | 60 | 70 | 90 | 60 | 100 | 30 | 20 | 20 |
| Wheat | 20 | 20 | 60 | 20 | 20 | 60 | 70 | 70 | 50 | 70 | 40 | 20 | 60 | 70 |

| Table A1 | Compounds | | | | | | | | | | | | | |
|---|---|---|---|---|---|---|---|---|---|---|---|---|---|---|
| 125 g ai/ha | 380 | 381 | 382 | 383 | 392 | 393 | 394 | 397 | 398 | 399 | 400 | 401 | 402 | 403 |
| Postemergence | | | | | | | | | | | | | | |
| Blackgrass | 20 | 70 | 0 | 50 | 0 | - | - | 40 | 30 | 90 | 30 | 30 | 0 | 0 |
| Corn | 50 | 20 | 0 | 0 | 0 | 30 | 40 | 10 | 60 | 10 | 0 | 60 | 0 | 0 |
| Foxtail, Giant | 80 | 50 | 50 | 60 | 0 | 40 | 50 | 20 | 60 | 60 | 0 | 60 | 20 | 0 |
| Galium | 60 | 50 | 50 | 60 | 80 | 100 | 100 | 100 | 90 | 90 | 100 | 80 | 90 | 100 |
| Goosegrass | 30 | 50 | 0 | 20 | 0 | 70 | 30 | 90 | 90 | 90 | 80 | 90 | 70 | 50 |
| Horseweed | 80 | 30 | 50 | 30 | 20 | 30 | 80 | - | 50 | 50 | 80 | 0 | 50 | 30 |
| Kochia | 50 | 20 | 30 | 20 | 20 | 60 | 80 | 90 | 70 | 70 | 70 | 30 | 50 | 40 |
| Oat, Wild | 20 | 20 | 0 | 10 | 0 | 10 | 0 | 10 | 10 | 40 | 0 | 30 | 10 | 30 |
| Pigweed, Palmer | 10 | 10 | 20 | 10 | 40 | 80 | 50 | 80 | 80 | 70 | 70 | 30 | 70 | 30 |
| Ragweed | 60 | 50 | 40 | 40 | 70 | 70 | 90 | 80 | 80 | 90 | 80 | 90 | 50 | 50 |
| Ryegrass, Italian | 60 | 0 | 0 | 0 | 0 | 50 | 40 | 50 | 90 | 70 | 0 | 0 | 0 | 0 |
| Soybean | 60 | 60 | 50 | 50 | 60 | 60 | 60 | 50 | 60 | 100 | 60 | 70 | 80 | 60 |
| Wheat | 80 | 70 | 60 | 70 | 30 | 60 | 40 | 60 | 70 | 60 | 30 | 10 | 0 | 0 |

| Table A1 | Compounds | | | | | | | | | | | |
|---|---|---|---|---|---|---|---|---|---|---|---|---|
| 125 g ai/ha | 404 | 405 | 406 | 407 | 408 | 409 | 410 | 411 | 412 | 413 | 414 | 415 |
| Postemergence | | | | | | | | | | | | |
| Blackgrass | - | - | 60 | 60 | 90 | 90 | 90 | 50 | 30 | 0 | 30 | 40 |
| Corn | 20 | 20 | 30 | 50 | 80 | 90 | 20 | 0 | 30 | 30 | 0 | 50 |
| Foxtail, Giant | 60 | 60 | 80 | 70 | 90 | 70 | 90 | 70 | 70 | 10 | 0 | 30 |
| Galium | 100 | 100 | 100 | 90 | 100 | 100 | 90 | 100 | 100 | 100 | 70 | 90 |
| Goosegrass | 40 | 80 | 90 | 90 | 90 | 90 | 90 | 90 | 40 | 0 | 0 | 90 |
| Horseweed | 80 | 100 | - | - | 70 | 90 | 50 | 70 | 90 | 90 | 70 | - |
| Kochia | 70 | 70 | 80 | 80 | 60 | 80 | 50 | 80 | 80 | 80 | 50 | 70 |
| Oat, Wild | 0 | 10 | 20 | 10 | 70 | 80 | 80 | 40 | 0 | 0 | 0 | 30 |
| Pigweed, Palmer | 60 | 40 | 60 | 30 | 50 | 30 | 20 | 60 | 60 | 30 | 10 | 20 |
| Ragweed | 90 | 90 | 90 | 90 | 90 | 90 | 70 | 90 | 90 | 90 | 70 | 70 |
| Ryegrass, Italian | 50 | 50 | 80 | 60 | 80 | 100 | 90 | 40 | 20 | 0 | 0 | 60 |
| Soybean | 40 | 70 | 60 | 60 | 50 | 80 | 70 | 90 | 90 | 80 | 30 | 30 |
| Wheat | 20 | 30 | 50 | 60 | 70 | 70 | 70 | 60 | 0 | 0 | 0 | 70 |

| Table A1 | Compounds | | | | | | | | | | | | | |
|---|---|---|---|---|---|---|---|---|---|---|---|---|---|---|
| 31 g ai/ha | 281 | 282 | 283 | 284 | 285 | 286 | 287 | 288 | 289 | 290 | 292 | 293 | 294 | 295 |
| Postemergence | | | | | | | | | | | | | | |
| Blackgrass | 0 | 0 | 0 | 0 | 0 | 0 | 0 | 0 | 0 | 0 | 0 | 0 | 0 | 0 |
| Corn | 0 | 0 | 0 | 0 | 0 | 0 | 0 | 0 | 0 | 0 | 0 | 10 | 10 | 0 |
| Foxtail, Giant | 0 | 0 | 80 | 10 | 30 | 80 | 0 | 0 | 40 | 0 | 0 | 0 | 0 | 0 |
| Galium | 0 | - | 50 | 60 | 40 | 50 | 0 | 0 | 60 | 80 | 80 | 20 | 60 | 50 |
| Goosegrass | 0 | 20 | 10 | 0 | 0 | 0 | 0 | 0 | 40 | 30 | 0 | 20 | 0 | 20 |
| Horseweed | 100 | 50 | 70 | 80 | 70 | 0 | 20 | 20 | 80 | 70 | 0 | 20 | 10 | 10 |
| Kochia | 0 | 10 | 20 | 40 | 10 | 20 | 0 | 0 | 20 | 20 | 30 | 20 | 0 | 0 |
| Oat, Wild | 0 | 0 | 0 | 0 | 0 | 0 | 0 | 0 | - | 0 | **10** | 0 | 10 | 0 |
| Pigweed, Palmer | 0 | 0 | 10 | 30 | 0 | 0 | 0 | 0 | 10 | 0 | 0 | 20 | 10 | 0 |
| Ragweed | 0 | 60 | 30 | 70 | 20 | 0 | 60 | 30 | 70 | 60 | 20 | 0 | 30 | 50 |
| Ryegrass, Italian | 0 | 30 | 0 | 0 | 0 | 0 | 0 | 0 | 30 | 0 | 0 | 0 | 0 | 0 |
| Soybean | 50 | 40 | 40 | 50 | 30 | 50 | 30 | 30 | 40 | 0 | 20 | 60 | 0 | 20 |
| Wheat | 0 | 60 | 10 | 0 | 10 | 0 | 0 | 0 | 20 | 0 | 0 | 0 | 0 | 20 |

| Table A1 | Compounds | | | | | | | | | | | | | |
|---|---|---|---|---|---|---|---|---|---|---|---|---|---|---|
| 31 g ai/ha | 296 | 297 | 298 | 299 | 300 | 301 | 302 | 303 | 304 | 305 | 306 | 307 | 308 | 309 |
| Postemergence | | | | | | | | | | | | | | |
| Blackgrass | 0 | 0 | 0 | 0 | 0 | 0 | 0 | - | - | 0 | 0 | 0 | 0 | - |
| Corn | 0 | 0 | 0 | 0 | 0 | 0 | 0 | 0 | 0 | 0 | 0 | 0 | 0 | 0 |
| Foxtail, Giant | 0 | 10 | 0 | 0 | 0 | 0 | 0 | 30 | 0 | 20 | 0 | 20 | 0 | 20 |
| Galium | 50 | 80 | 40 | 40 | 30 | 70 | 0 | 60 | 0 | 50 | 60 | 40 | 60 | 40 |
| Goosegrass | 20 | 0 | 30 | 30 | 40 | 0 | 0 | 0 | 0 | 10 | 0 | 30 | 50 | 0 |
| Horseweed | 70 | 50 | 20 | 0 | 40 | 50 | 10 | 0 | 0 | - | 0 | 30 | 0 | 10 |
| Kochia | 20 | 0 | 0 | 0 | 20 | 0 | 10 | 0 | 0 | 0 | 0 | 0 | 20 | 0 |
| Oat, Wild | 0 | 20 | 0 | 0 | 0 | 0 | 0 | 0 | 0 | 20 | 0 | 0 | 0 | 0 |
| Pigweed, Palmer | 0 | 10 | 0 | 10 | 0 | 0 | 10 | 0 | 0 | 0 | 0 20 | 0 | 0 | 0 |
| Ragweed | 20 | 30 | 40 | 40 | 20 | 60 | 30 | 30 | 0 | 0 | 0 | 0 | 50 | 0 |
| Ryegrass, Italian | 0 | 0 | 40 | 30 | 0 | 0 | 0 | 0 | 0 | 0 | 0 | 20 | 0 | 0 |
| Soybean | 30 | 30 | 20 | 40 | 30 | 10 | 20 | 30 | 20 | 10 | | 10 | 40 | 10 |
| Wheat | 0 | 10 | 0 | 30 | 0 | 0 | 0 | 0 | 0 | 50 | 0 | 30 | 10 | 0 |

| Table A1 | Compounds | | | | | | | | | | | | | |
|---|---|---|---|---|---|---|---|---|---|---|---|---|---|---|
| 31 g ai/ha | 310 | 311 | 312 | 313 | 314 | 315 | 316 | 317 | 318 | 319 | 320 | 321 | 322 | 323 |
| Postemergence | | | | | | | | | | | | | | |
| Blackgrass | 0 | 0 | 0 | 0 | 0 | 0 | 0 | 0 | 0 | 0 | 40 | 10 | 20 | 0 |
| Corn | 0 | 0 | 0 | 0 | 0 | 20 | 0 | 0 | 30 | 10 | 0 | 10 | 0 | 0 |
| Foxtail, Giant | 0 | 0 | 0 | 0 | 10 | 0 | 0 | 0 | 0 | 40 | 0 | 0 | 0 | 10 |
| Galium | 0 | 0 | 0 | 0 | 60 | 70 | 40 | 0 | 60 | 40 | 70 | 100 | 40 | 50 |
| Goosegrass | 0 | 0 | 0 | 0 | 60 | 80 | 0 | 0 | 30 | 70 | 40 | 0 | 20 | 0 |
| Horseweed | 10 | - | 0 | 0 | 0 | 20 | 30 | 0 10 | 20 | 10 | 50 | 70 | 0 | 0 |
| Kochia | 0 | 0 | 0 | 0 | 30 | 50 | 30 | 0 | 30 | 40 | 30 | 40 | 10 | 50 |
| Oat, Wild | 0 | 0 | 0 | 0 | 0 | 0 | 0 | 0 | 0 | 0 | 0 | 0 | 0 | 0 |
| Pigweed, Palmer | 0 | 0 | 0 | 0 | 10 | 30 | 30 | 0 | 30 | 0 | 40 | 50 | 0 | 20 |
| Ragweed | 0 | 0 | 0 | 0 | 0 | 20 | 0 | | 60 | 30 | 40 | 90 | 70 | 0 |
| Ryegrass, Italian | 20 | 0 | 0 | 0 | 10 | 10 | 0 | 0 | 10 | 30 | 0 | 30 | 0 | 0 |
| Soybean | 20 | 10 | 20 | 0 | 40 | 40 | 10 | 0 | 30 | 20 | 20 | 20 | 20 | 0 |
| Wheat | 0 | 0 | 0 | 0 | 50 | 30 | 10 | 0 | 50 | 70 | 0 | 0 | 0 | 0 |

| Table A1 | Compounds | | | | | | | | | | | | | |
|---|---|---|---|---|---|---|---|---|---|---|---|---|---|---|
| 31 g ai/ha | 324 | 325 | 326 | 327 | 328 | 329 | 330 | 331 | 332 | 333 | 334 | 335 | 336 | 337 |
| Postemergence | | | | | | | | | | | | | | |
| Blackgrass | 0 | 0 | 0 | 0 | 0 | 0 | 0 | 0 | 0 | 0 | 0 | 0 | 0 | 0 |
| Corn | 30 | 10 | 0 | 10 | 0 | 0 | 0 | 10 | 0 | 0 | 0 | 0 | 0 | 30 |
| Foxtail, Giant | 20 | 30 | 10 | 30 | 0 | 10 | 20 | 20 | 30 | 10 | 0 | 0 | 0 | 0 |
| Galium | 50 | 40 | 70 | 100 | 80 | 50 | 50 | 60 | 80 | 70 | 60 | 50 | 60 | 70 |
| Goosegrass | 30 | 0 | 0 | 20 | 20 | 0 | 0 | 0 | 0 | 0 | 0 | 0 | 0 | 0 |
| Horseweed | - | 90 | - | - | - | 20 | 80 | 70 | 60 | 0 | 0 | 40 | 0 | - |
| Kochia | 30 | 20 | 30 | 10 | 10 | 10 | 10 | 0 | 0 | 30 | 10 | 10 | 70 | 40 |
| Oat, Wild | 0 | 0 | 0 | 0 | 0 | 0 | 0 | 0 | 0 | 0 | 0 | 0 | 0 | 0 |
| Pigweed, Palmer | 30 | 30 | 40 | 30 | 30 | 10 | 0 | 0 | 10 | 40 | 10 | 20 | 20 | 30 |
| Ragweed | 70 | 0 | 60 | 70 | 40 | 40 | 50 | 70 | 80 | 0 | 0 | 50 | 50 | 50 |
| Ryegrass, Italian | 0 | 0 | 0 | 0 | 30 | 0 | 0 | 10 | 0 | 0 | 0 | 0 | 0 | 0 |
| Soybean | 50 | 50 | 70 | 60 | 60 | 60 | 90 | 60 | 60 | 30 | 20 | 0 | 20 | 50 |
| Wheat | 0 | 30 | 0 | 0 | 0 | 0 | 0 | 0 | 10 | 0 | 0 | 0 | 0 | 0 |

| Table A1 | Compounds | | | | | | | | | | | | | |
|---|---|---|---|---|---|---|---|---|---|---|---|---|---|---|
| 31 g ai/ha | 338 | 339 | 340 | 341 | 342 | 343 | 344 | 345 | 346 | 347 | 348 | 349 | 350 | 351 |
| Postemergence | | | | | | | | | | | | | | |
| Blackgrass | 0 | 0 | 0 | 0 | 0 | 10 | 0 | 20 | 0 | 0 | 0 | 40 | 0 | 0 |
| Corn | 10 | 0 | 20 | 0 | 10 | 10 | 20 | 0 | 20 | 0 | 0 | 40 | 0 | 0 |
| Foxtail, Giant | 10 | 0 | 10 | 0 | 0 | 0 | 10 | 30 | 30 | 20 | 20 | 30 | 10 | 30 |
| Galium | 50 | 30 | 50 | 50 | 50 | 70 | 70 | 80 | 70 | 30 | 60 | 100 | 70 | 80 |
| Goosegrass | 0 | 0 | 60 | 0 | 0 | 40 | 0 | 0 | 40 | 0 | 0 | 90 | 10 | 40 |
| Horseweed | - | 100 | - | - | - | - | - | - | - | - | - | 90 | 90 | 100 |
| Kochia | 10 | 30 | 20 | 20 | 10 | 20 | 20 | 30 | 30 | 10 | 20 | 70 | 0 | 10 |
| Oat, Wild | 0 | 0 | 0 | 0 | 0 | 0 | 20 | 20 | 20 | 10 | 0 | 0 | 0 | 0 |
| Pigweed, Palmer | 0 | 0 | 0 | 0 | 0 | 0 | 20 | 40 | 20 | 20 | 0 | 20 | 30 | 10 |
| Ragweed | 30 | 40 | 70 | 60 | 70 | 20 | 30 | 30 | 50 | 40 | 10 | 70 | 80 | 70 |
| Ryegrass, Italian | 0 | 0 | 0 | 0 | 0 | 20 | 30 | 20 | 0 | 0 | 0 | 60 | 30 | 20 |
| Soybean | 10 | 0 | 50 | 40 | 40 | 40 | 40 | 40 | 30 | 40 | 50 | 30 | 10 | 30 |
| Wheat | 20 | 0 | 50 | 0 | 30 | 30 | 40 | 30 | 30 | 0 | 0 | 30 | 0 | 0 |

| Table A1 | Compounds | | | | | | | | | | | | | |
|---|---|---|---|---|---|---|---|---|---|---|---|---|---|---|
| 31 g ai/ha | 352 | 353 | 354 | 355 | 356 | 357 | 358 | 359 | 360 | 361 | 362 | 363 | 364 | 365 |
| Postemergence | | | | | | | | | | | | | | |
| Blackgrass | 0 | 0 | 0 | 0 | 0 | 0 | 0 | 0 | 0 | 0 | 0 | 0 | 0 | 0 |
| Corn | 20 | 0 | 0 | 0 | 0 | 0 | 0 | 0 | 0 | 0 | 0 | 30 | 0 | 0 |
| Foxtail, Giant | 20 | 20 | 0 | 10 | 10 | 0 | 0 | 0 | 0 | 10 | 20 | 30 | 0 | 0 |
| Galium | 100 | 50 | 40 | 40 | 70 | 90 | 70 | 50 | 50 | 80 | 60 | 60 | 0 | 40 |
| Goosegrass | 80 | 0 | 0 | 0 | 0 | 0 | 0 | 0 | 0 | 0 | 40 | 30 | 0 | 0 |
| Horseweed | 80 | 70 | 80 | 70 | 50 | 20 | 60 | 50 | 10 | 70 | 90 | 80 | 0 | 10 |
| Kochia | 50 | 10 | 40 | 0 | 10 | 0 | 0 | 30 | 30 | 0 | 50 | 50 | 0 | 10 |
| Oat, Wild | 0 | 0 | 0 | 0 | 0 | 0 | 0 | 0 | 0 | 0 | 0 | 20 | 0 | 0 |
| Pigweed, Palmer | 20 | 0 | 0 | 0 | 0 | 0 | 0 | 0 | 20 | 10 | 10 | 0 | 0 | 0 |
| Ragweed | 70 | 20 | 40 | 60 | 70 | 80 | 80 | 30 | 70 | 60 | 70 | 60 | 0 | 0 |
| Ryegrass, Italian | 0 | 0 | 0 | 30 | 20 | 20 | 20 | 0 | 0 | 0 | 30 | 20 | 0 | 0 |
| Soybean | 20 | 50 | 50 | 10 | 50 | 40 | 50 | 50 | 20 | 50 | 50 | 60 | 0 | 0 |
| Wheat | 0 | 20 | 20 | 20 | 0 | 0 | 0 | 0 | 0 | 0 | 40 | 40 | 0 | 0 |

| Table A1 | Compounds | | | | | | | | | | | | | |
|---|---|---|---|---|---|---|---|---|---|---|---|---|---|---|
| 31 g ai/ha | 366 | 367 | 368 | 369 | 370 | 371 | 372 | 3 373 | 374 | 375 | 376 | 377 | 378 | 379 |
| Postemergence | | | | | | | | | | | | | | |
| Blackgrass | 0 | 0 | 0 | 0 | 0 | 0 | 0 | 0 | 0 | 0 | 0 | 0 | 0 | 0 |
| Corn | 0 | 0 | 40 | 0 | 0 | 60 | 20 | 0 | 60 | 0 | 0 | 0 | 0 | 0 |
| Foxtail, Giant | 30 | 10 | 80 | 20 | 10 | 90 | 0 | 0 | 0 | 0 | 0 | 0 | 0 | 0 |
| Galium | 50 | 50 | 70 | 60 | 90 | 70 | 60 | 50 | 60 | 10 | | 0 | | 0 |
| Goosegrass | 0 | 10 | 30 | 0 | 0 | 0 | 0 | 0 | 0 | 50 | 10 0 | 0 | 0 | 10 |
| Horseweed | - | - | | - | - | - | 0 | - | - | - | 0 | 30 | 0 | 0 |
| Kochia | 0 | 20 | 60 | 0 | 0 | 40 | 0 | 0 | 0 | 0 | 0 | 20 | 20 0 | 0 |
| Oat, Wild | 0 | 0 | 0 | 0 | 0 | 0 | 0 | 0 | 0 | 0 | 0 | 0 | 0 | 0 |
| Pigweed, Palmer | 10 | 40 | 20 | 0 | 0 | 0 | 30 | 0 | 10 | 0 | 0 | 0 | 0 | 0 |
| Ragweed | 40 | 50 | 50 | 20 | 50 | 40 | 40 | 50 | 60 | 0 | 20 | 20 | 0 | 20 |
| Ryegrass, Italian | 0 | 0 | 20 | 0 | 0 | 20 | 0 | 0 | 0 | 0 | 0 | 0 | 0 | 0 |
| Soybean | 50 | 50 | 70 | 20 | 50 | 60 | 30 | 50 | 50 | 30 | 20 | 0 | 0 | 0 |
| Wheat | 0 | 0 | 20 | 0 | 10 | 0 | 10 | 0 | 20 | 0 | 10 | 0 | 30 | 20 |

| Table A1 | Compounds | | | | | | | | | | | | | |
|---|---|---|---|---|---|---|---|---|---|---|---|---|---|---|
| 31 g ai/ha | 380 | 381 | 382 | 383 | 392 | 393 | 394 | 398 | 399 | 400 | 401 | 402 | 403 | 404 |
| Postemergence | | | | | | | | | | | | | | |
| Blackgrass | 20 | 0 | 0 | 0 | 0 | - | - | 0 | 40 | 0 | 0 | 0 | 0 | - |
| Corn | 0 | 0 | 0 | 0 | 0 | 0 | 0 | 30 | 0 | 0 | 10 | 0 | 0 | 0 |
| Foxtail, Giant | 10 | 0 | 0 | 0 | 0 | 0 | 0 | 20 | 10 | 0 | 0 | 0 | 0 | 0 |
| Galium | 50 | 0 | 0 | 0 | 50 | 100 | 100 | 80 | 50 | 80 | 50 | 70 | 70 | 80 |
| Goosegrass | 0 | 0 | 0 | 0 | 0 | 0 | 0 | 50 | 40 | 10 | 0 | 20 | 0 | 0 |
| Horseweed | 30 | 0 | 0 | 0 | 0 | 0 | 30 | 20 | 0 | 30 | 0 | 20 | 0 | 50 |
| Kochia | 30 | 0 | 0 | 20 | 10 | 20 | 40 | 20 | 30 | 20 | 10 | 10 | 10 | 40 |
| Oat, Wild | 0 | 0 | 0 | 0 | 0 | 0 | 0 | 0 | 10 | 0 | 0 | 10 | 0 | 0 |
| Pigweed, Palmer | 0 | 0 | 0 | 0 | 0 | 0 | 50 | 50 | 30 | 20 | 0 | 20 | 0 | 0 |
| Ragweed | 40 | 0 | 20 | 30 | 30 | 60 | 90 | 20 | 50 | 30 | 60 | 50 | 0 | 80 |
| Ryegrass, Italian | 0 | 0 | 0 | 0 | 0 | 30 | 20 | 50 | 0 | 0 | 0 | 0 | 0 | 40 |
| Soybean | 50 | 50 | 40 | 30 | 50 | 50 | 10 | 30 | 60 | 50 | 60 | 60 | 50 | 10 |
| Wheat | 0 | 20 | 0 | 0 | 0 | 30 | 10 | 60 | 60 | 30 | 0 | 0 | 0 | 0 |

| Table A1 | Compounds | | | | | | | | | | |
|---|---|---|---|---|---|---|---|---|---|---|---|
| 31 g ai/ha | 405 | 406 | 407 | 408 | 409 | 410 | 411 | 412 | 413 | 414 | 415 |
| Postemergence | | | | | | | | | | | |
| Blackgrass | - | 40 | 0 | 80 | 60 | 90 | 30 | 0 | 0 | 0 | 0 |
| Corn | 0 | 0 | 10 | 80 | 70 | 90 | 60 | 0 | 30 | 0 | 0 |
| Foxtail, Giant | 30 | 10 | 0 | 90 | 10 | 20 | 0 | 10 | 0 | 0 | 0 |
| Galium | 100 | 90 | 80 | 90 | 90 | 60 | 90 | 90 | 50 | 60 | 60 |
| Goosegrass | 20 | 30 | 0 | 90 | 80 | 80 | 50 | 0 | 0 | 0 | 70 |
| Horseweed | 100 | - | - | 20 | 20 | 20 | 0 | 20 | 40 | 0 | 60 |
| Kochia | 40 | 40 | 20 | 60 | 60 | 10 | 60 | 20 | 20 | 10 | 40 |
| Oat, Wild | 0 | 0 | 0 | 70 | 0 | 70 | 20 | 0 | 0 | 0 | 10 |
| Pigweed, Palmer | - | 10 | 0 | 30 | 30 | 0 | 0 | 20 | 20 | 0 | 0 |
| Ragweed | 70 | 80 | 70 | 40 | 70 | 50 | 80 | 60 | 60 | 40 | 30 |
| Ryegrass, Italian | 0 | 50 | 30 | 70 | 40 | 60 | 20 | 0 | 0 | 0 | 0 |
| Soybean | 30 | 60 | 30 | 40 | 30 | 60 | 30 | 80 | 80 | 20 | 10 |
| Wheat | 0 | 0 | 0 | 60 | 70 | 60 | 50 | 0 | 0 | 0 | 20 |

### TEST B

Plant species in the flooded paddy test selected from rice *(Oryza sativa),* sedge, umbrella (small-flower umbrella sedge, *Cyperus difformis*), ducksalad (*Heteranthera limosa*), and barnyardgrass (*Echinochloa crus-galli*) were grown to the 2-leaf stage for testing. At time of treatment, test pots were flooded to 3 cm above the soil surface, treated by application of test compounds directly to the paddy water, and then maintained at that water depth for the duration of the test. Treated plants and controls were maintained in a greenhouse for 13 to 15 days, after which time all species were compared to controls and visually evaluated. Plant response ratings, summarized in Table B, are based on a scale of 0 to 100 where 0 is no effect and 100 is complete control. A dash (-) response means no test result.

| Table B | Compounds | | | | | | | | | | | | | |
|---|---|---|---|---|---|---|---|---|---|---|---|---|---|---|
| 250 g ai/ha | 1 | 2 | 3 | 4 | 5 | 6 | 7 | 8 | 9 | 10 | 11 | 12 | 13 | 14 |
| Flood | | | | | | | | | | | | | | |
| Barnyardgrass | 0 | 0 | 0 | 0 | 0 | 0 | 0 | 25 | 85 | 0 | 0 | 0 | 0 | 0 |
| Ducksalad | 65 | 65 | 80 | 0 | 0 | 0 | 30 | 80 | 95 | 0 | 0 | 0 | 0 | 20 |
| Rice | 30 | 35 | 0 | 0 | 0 | 0 | 0 | 0 | 10 | 0 | 0 | 0 | 0 | 10 |
| Sedge, Umbrella | 70 | 70 | 70 | 0 | 0 | 0 | 70 | 90 | 95 | 0 | 0 | 0 | 35 | 90 |

| Table B | Compounds | | | | | | | | | | | | | |
|---|---|---|---|---|---|---|---|---|---|---|---|---|---|---|
| 250 g ai/ha | 15 | 16 | 17 | 18 | 19 | 20 | 21 | 22 | 23 | 24 | 25 | 26 | 27 | 28 |
| Flood | | | | | | | | | | | | | | |
| Barnyardgrass | 0 | 25 | 0 | 0 | 25 | 20 | 0 | 20 | 10 | 20 | 0 | 0 | 0 | 0 |
| Ducksalad | 0 | 40 | 70 | 0 | 85 | 70 | 15 | 30 | 90 | 30 | 0 | 0 | 0 | 0 |
| Rice | 0 | 0 | 0 | 0 | 10 | 0 | 0 | 15 | 15 | 45 | 25 | 0 | 0 | 0 |
| Sedge, Umbrella | 0 | 95 | 95 | 0 | 95 | 90 | 90 | 90 | 95 | 95 | 90 | 0 | 0 | 0 |

| Table B | Compounds | | | | | | | | | | | | |
|---|---|---|---|---|---|---|---|---|---|---|---|---|---|
| 250 g ai/ha | 29 | 30 | 32 | 34 | 35 | 36 | 37 | 38 | 39 | 40 | 41 | 42 | 43 |
| Flood | | | | | | | | | | | | | |
| Barnyardgrass | 0 | 0 | 0 | 0 | 0 | 0 | 65 | 0 | 0 | 0 | 0 | 0 | 20 |
| Ducksalad | 0 | 20 | 45 | 0 | 0 | 0 | 25 | 70 | 0 | 0 | 20 | 15 | 25 |
| Rice | 15 | 70 | 70 | 0 | 0 | 0 | 70 | 25 | 5 | 0 | 5 | 15 | 20 |
| Sedge, Umbrella | 85 | 35 | 65 | 0 | 85 | 0 | 45 | 75 | 80 | 0 | 75 | 75 | 25 |

| Table B | Compounds | | | | | | | | | | | | | |
|---|---|---|---|---|---|---|---|---|---|---|---|---|---|---|
| 250 g ai/ha | 44 | 45 | 46 | 47 | 50 | 51 | 52 | 53 | 54 | 55 | 56 | 57 | 58 | 59 |
| Flood | | | | | | | | | | | | | | |
| Barnyardgrass | 0 | 10 | 0 | 0 | 0 | 0 | 0 | 0 | 0 | 0 | 40 | 15 | 0 | 0 |
| Ducksalad | 0 | 25 | 50 | 0 | 0 | 0 | 0 | 0 | 0 | 45 | 20 | 30 | 0 | 0 |
| Rice | 0 | 0 | 0 | 15 | 0 | 0 | 0 | 0 | 0 | 0 | 75 | 25 | 0 | 0 |
| Sedge, Umbrella | 0 | 90 | 90 | 80 | 0 | 65 | 70 | 0 | 0 | 80 | 90 | 85 | 0 | 0 |

| Table B | Compounds | | | | | | | | | | | | | |
|---|---|---|---|---|---|---|---|---|---|---|---|---|---|---|
| 250 g ai/ha | 60 | 61 | 62 | 63 | 64 | 65 | 66 | 67 | 68 | 69 | 70 | 71 | 72 | 73 |
| Flood | | | | | | | | | | | | | | |
| Barnyardgrass | 0 | 0 | 0 | 0 | 0 | 0 | 35 | 0 | 35 | 15 | 0 | 0 | 20 | 0 |
| Ducksalad | 0 | 0 | 30 | 15 | 0 | 0 | 20 | 15 | 15 | 15 | 25 | 0 | 75 | 0 |
| Rice | 0 | 0 | 0 | 0 | 0 | 0 | 20 | 0 | 55 | 15 | 40 | 40 | 15 | 0 |
| Sedge, Umbrella | 0 | 0 | 50 | 75 | 0 | 0 | 90 | 85 | 75 | 90 | 95 | 95 | 85 | 0 |

| Table B | Compounds | | | | | | | | | | | | | |
|---|---|---|---|---|---|---|---|---|---|---|---|---|---|---|
| 250 g ai/ha | 74 | 75 | 76 | 77 | 78 | 79 | 80 | 81 | 82 | 83 | 84 | 85 | 88 | 91 |
| Flood | | | | | | | | | | | | | | |
| Barnyardgrass | 0 | 0 | 35 | 10 | 0 | 0 | 70 | 0 | 0 | 0 | 0 | 0 | 15 | 0 |
| Ducksalad | 0 | 0 | 10 | 40 | 20 | 0 | 40 | 0 | 75 | 0 | 0 | 0 | 75 | 55 |
| Rice | 0 | 0 | 45 | 15 | 0 | 20 | 55 | 0 | 0 | 0 | 0 | 0 | 0 | 0 |
| Sedge, Umbrella | 0 | 0 | 45 | 85 | 95 | 85 | 95 | 0 | 85 | 0 | 0 | 0 | 80 | 55 |

| Table B | Compounds | | | | | | | | | | | | | |
|---|---|---|---|---|---|---|---|---|---|---|---|---|---|---|
| 250 g ai/ha | 92 | 93 | 94 | 95 | 96 | 97 | 98 | 99 | 100 | 101 | 102 | 103 | 104 | 105 |
| Flood | | | | | | | | | | | | | | |
| Barnyardgrass | 75 | 25 | 35 | 0 | 45 | 0 | 10 | 65 | 90 | 35 | 90 | 85 | 0 | 10 |
| Ducksalad | 65 | 50 | 40 | 35 | 60 | 45 | 75 | 80 | 70 | 65 | 85 | 80 | 35 | 40 |
| Rice | 20 | 0 | 20 | 0 | 0 | 15 | 0 | 15 | 20 | 35 | 35 | 25 | 0 | 10 |
| Sedge, Umbrella | 85 | 70 | 45 | 40 | 65 | 70 | 90 | 95 | 80 | 90 | 80 | 85 | 75 | 85 |

| Table B | Compounds | | | | | | | | | | | | |
|---|---|---|---|---|---|---|---|---|---|---|---|---|---|
| 250 g ai/ha | 107 | 108 | 109 | 110 | 111 | 112 | 113 | 114 | 115 | 116 | 118 | 119 | 120 |
| Flood | | | | | | | | | | | | | |
| Barnyardgrass | 0 | 35 | 0 | 30 | 60 | 35 | 0 | 0 | 30 | 0 | 35 | 35 | 0 |
| Ducksalad | 30 | 65 | 80 | 75 | 65 | 50 | 55 | 0 | 65 | 90 | 50 | 75 | 55 |
| Rice | 0 | 20 | 10 | 25 | 30 | 60 | 15 | 0 | 25 | 0 | 0 | 15 | 0 |
| Sedge, Umbrella | 85 | 75 | 90 | 90 | 80 | 65 | 65 | 0 | 70 | 95 | 55 | 75 | 55 |

| Table B | Compounds | | | | | | | | | | | | | |
|---|---|---|---|---|---|---|---|---|---|---|---|---|---|---|
| 250 g ai/ha | 121 | 122 | 123 | 124 | 125 | 126 | 127 | 128 | 129 | 130 | 131 | 132 | 133 | 134 |
| Flood | | | | | | | | | | | | | | |
| Barnyardgrass | 40 | 0 | 35 | 0 | 0 | 0 | 25 | 30 | 0 | 20 | 85 | 0 | 15 | 0 |
| Ducksalad | 75 | 0 | 40 | 0 | 30 | 45 | 45 | 50 | 0 | 25 | 50 | 45 | 35 | 0 |
| Rice | 20 | 0 | 20 | 0 | 0 | 0 | 10 | 0 | 0 | 0 | 0 | 30 | 45 | 0 |
| Sedge, Umbrella | 70 | 0 | 90 | 0 | 85 | 80 | 90 | 90 | 75 | 75 | 90 | 80 | 95 | 45 |

| Table B | Compounds | | | | | | | | | | | | | |
|---|---|---|---|---|---|---|---|---|---|---|---|---|---|---|
| 250 g ai/ha | 135 | 136 | 137 | 139 | 140 | 141 | 142 | 143 | 144 | 145 | 146 | 147 | 148 | 149 |
| Flood | | | | | | | | | | | | | | |
| Barnyardgrass | 30 | 10 | 35 | 0 | 95 | 0 | 20 | 95 | 90 | 0 | 0 | 0 | 0 | 95 |
| Ducksalad | 85 | 60 | 50 | 0 | 85 | 0 | 30 | 35 | 35 | 55 | 85 | 65 | 0 | 25 |
| Rice | 10 | 20 | 40 | 0 | 0 | 0 | 0 | 0 | 15 | 0 | 0 | 0 | 0 | 0 |
| Sedge, Umbrella | 75 | 80 | 75 | 0 | 95 | 70 | 75 | 95 | 80 | 45 | 85 | 80 | 0 | 65 |

| Table B | Compounds | | | | | | | | | | | | | |
|---|---|---|---|---|---|---|---|---|---|---|---|---|---|---|
| 250 g ai/ha | 150 | 151 | 152 | 153 | 154 | 155 | 156 | 157 | 158 | 159 | 160 | 161 | 162 | 163 |
| Flood | | | | | | | | | | | | | | |
| Barnyardgrass | 95 | 85 | 95 | 15 | 15 | 90 | 50 | 20 | 35 | 65 | 0 | 90 | 25 | 0 |
| Ducksalad | 30 | 35 | 35 | 70 | 45 | 75 | 65 | 55 | 70 | 35 | 45 | 70 | 60 | 60 |
| Rice | 0 | 10 | 0 | 25 | 30 | 75 | 35 | 15 | 35 | 0 | 0 | 35 | 20 | 0 |
| Sedge, Umbrella | 80 | 65 | 70 | 65 | 65 | 85 | 85 | 70 | 90 | 70 | 75 | 90 | 70 | 90 |

| Table B | Compounds | | | | | | | | | | | | | |
|---|---|---|---|---|---|---|---|---|---|---|---|---|---|---|
| 250 g ai/ha | 164 | 165 | 166 | 167 | 168 | 169 | 170 | 171 | 172 | 173 | 174 | 175 | 176 | 177 |
| Flood | | | | | | | | | | | | | | |
| Barnyardgrass | 90 | 20 | 95 | 0 | 0 | 0 | 0 | 55 | 15 | 80 | 0 | 0 | 30 | 0 |
| Ducksalad | 85 | 75 | 70 | 0 | 0 | 0 | 0 | 30 | 65 | 75 | 60 | 70 | 60 | 70 |
| Rice | 0 | 30 | 25 | 0 | 0 | 0 | 0 | 45 | 55 | 15 | 0 | 0 | 0 | 0 |
| Sedge, Umbrella | 95 | 95 | 95 | 0 | 65 | 80 | 0 | 60 | 45 | 80 | 55 | 85 | 65 | 80 |

| Table B | Compounds | | | | | | | | | | | | | |
|---|---|---|---|---|---|---|---|---|---|---|---|---|---|---|
| 250 g ai/ha | 178 | 179 | 180 | 183 | 184 | 185 | 186 | 189 | 190 | 191 | 193 | 194 | 195 | 196 |
| Flood | | | | | | | | | | | | | | |
| Barnyardgrass | 0 | 0 | 0 | 25 | 25 | 25 | 0 | 45 | 0 | 0 | 20 | 0 | 0 | 0 |
| Ducksalad | 20 | 60 | 35 | 20 | 25 | 10 | 0 | 75 | 75 | 75 | 75 | 0 | 0 | 75 |
| Rice | 0 | 0 | 0 | 0 | 0 | 0 | 0 | 15 | 0 | 0 | 20 | 0 | 0 | 0 |
| Sedge, Umbrella | 90 | 60 | 80 | 60 | 45 | 75 | 0 | 85 | 80 | 75 | 95 | 0 | 0 | 90 |

| Table B | Compounds | | | | | | | | | | | | | |
|---|---|---|---|---|---|---|---|---|---|---|---|---|---|---|
| 250 g ai/ha | 197 | 198 | 199 | 200 | 201 | 202 | 203 | 204 | 205 | 206 | 207 | 208 | 209 | 210 |
| Flood | | | | | | | | | | | | | | |
| Barnyardgrass | 95 | 70 | 0 | 0 | 0 | 0 | 25 | 0 | 0 | 0 | 75 | 65 | 10 | 10 |
| Ducksalad | 95 | 90 | 75 | 0 | 75 | 75 | 30 | 0 | 0 | 75 | 95 | 85 | 85 | 65 |
| Rice | 0 | 0 | 0 | 0 | 0 | 0 | 20 | 0 | 0 | 0 | 0 | 15 | 0 | 15 |
| Sedge, Umbrella | 98 | 98 | 90 | 0 | 75 | 90 | 80 | 0 | 0 | 95 | 98 | 98 | 95 | 95 |

| Table B | Compounds | | | | | | | | | | | | | |
|---|---|---|---|---|---|---|---|---|---|---|---|---|---|---|
| 250 g ai/ha | 211 | 212 | 213 | 214 | 215 | 216 | 217 | 218 | 219 | 220 | 221 | 222 | 223 | 224 |
| Flood | | | | | | | | | | | | | | |
| Barnyardgrass | 15 | 0 | 0 | 0 | 0 | 0 | 0 | 20 | 55 | 15 | 0 | 20 | 0 | 15 |
| Ducksalad | 80 | 0 | 80 | 55 | 0 | 0 | 65 | 35 | 30 | 30 | 30 | 70 | 0 | 75 |
| Rice | 10 | 0 | 0 | 0 | 0 | 0 | 0 | 0 | 0 | 0 | 0 | 15 | 0 | 0 |
| Sedge, Umbrella | 75 | 0 | 65 | 25 | 0 | 0 | 80 | 90 | 90 | 95 | 95 | 95 | 0 | 90 |

| Table B | Compounds | | | | | | | | | | | | | |
|---|---|---|---|---|---|---|---|---|---|---|---|---|---|---|
| 250 g ai/ha | 225 | 226 | 227 | 228 | 229 | 230 | 231 | 232 | 233 | 234 | 235 | 236 | 237 | 238 |
| Flood | | | | | | | | | | | | | | |
| Barnyardgrass | 20 | 45 | 0 | 0 | 10 | 0 | 0 | 0 | 0 | 0 | 0 | 0 | 30 | 20 |
| Ducksalad | 75 | 70 | 0 | 75 | 75 | 85 | 70 | 70 | 0 | 0 | 80 | 50 | 35 | 85 |
| Rice | 0 | 10 | 0 | 0 | 10 | 0 | 0 | 0 | 0 | 0 | 0 | 0 | 0 | 0 |
| Sedge, Umbrella | 98 | 90 | 0 | 95 | 95 | 95 | 70 | 85 | 0 | 0 | 55 | 35 | 65 | 45 |

| Table B | Compounds | | | | | | | | | | | | | |
|---|---|---|---|---|---|---|---|---|---|---|---|---|---|---|
| 250 g ai/ha | 239 | 240 | 241 | 242 | 243 | 244 | 245 | 246 | 247 | 248 | 249 | 250 | 251 | 252 |
| Flood | | | | | | | | | | | | | | |
| Barnyardgrass | 0 | 15 | 25 | 10 | 10 | 25 | 45 | 15 | 0 | 0 | 0 | 10 | 10 | 10 |
| Ducksalad | 0 | 75 | 85 | 75 | 65 | 40 | 70 | 70 | 90 | 40 | 0 | 85 | 75 | 85 |
| Rice | 0 | 0 | 25 | 10 | 0 | 0 | 0 | 30 | 0 | 0 | 0 | 0 | 10 | 0 |
| Sedge, Umbrella | 0 | 85 | 98 | 80 | 98 | 60 | 75 | 85 | 95 | 90 | 0 | 98 | 85 | 95 |

| Table B | Compounds | | | | | | | | | | | | | |
|---|---|---|---|---|---|---|---|---|---|---|---|---|---|---|
| 250 g ai/ha | 253 | 254 | 255 | 256 | 257 | 258 | 259 | 260 | 264 | 265 | 266 | 267 | 268 | 269 |
| Flood | | | | | | | | | | | | | | |
| Barnyardgrass | 0 | 0 | 0 | 0 | 0 | 0 | 0 | 0 | 0 | 25 | 0 | 0 | 40 | 0 |
| Ducksalad | 75 | 35 | 90 | 60 | 50 | 65 | 30 | 80 | 85 | 70 | 0 | 35 | 70 | 85 |
| Rice | 0 | 35 | 0 | 0 | 0 | 0 | 0 | 0 | 0 | 0 | 0 | 0 | 0 | 0 |
| Sedge, Umbrella | 98 | 90 | 95 | 95 | 95 | 95 | 25 | 95 | 90 | 75 | 0 | 70 | 70 | 95 |

| Table B | Compounds | | | | | |
|---|---|---|---|---|---|---|
| 250 g ai/ha | 270 | 276 | 277 | 278 | 279 | 280 |
| Flood | | | | | | |
| Barnyardgrass | 25 | 15 | 25 | 0 | 0 | 95 |
| Ducksalad | 45 | 55 | 45 | 85 | 0 | 35 |
| Rice | 0 | 15 | 25 | 15 | 0 | 0 |
| Sedge, Umbrella | 90 | 85 | 90 | 85 | 0 | 95 |

### TEST B 1

Plant species in the flooded paddy test selected from bamyardgrass (*Echinochloa crus-galli*), ducksalad (*Heteranthera limosa*)*,* rice *(Oryza sativa),* and sedge, umbrella (small-flower umbrella sedge, *Cyperus difformis*) were grown to the 2-leaf stage for testing. At time of treatment, test pots were flooded to 3 cm above the soil surface, treated by application of test compounds directly to the paddy water, and then maintained at that water depth for the duration of the test. Treated plants and controls were maintained in a greenhouse for 10 to 14 days, after which time all species were compared to controls and visually evaluated. Plant response ratings, summarized in Table B, are based on a scale of 0 to 100 where 0 is no effect and 100 is complete control. A dash (-) response means no test result.

| Table B1 | Compounds | | | | | | | | | | | | | |
|---|---|---|---|---|---|---|---|---|---|---|---|---|---|---|
| 250 g ai/ha | 281 | 282 | 283 | 284 | 285 | 286 | 287 | 288 | 289 | 290 | 292 | 293 | 294 | 295 |
| Flood | | | | | | | | | | | | | | |
| Barnyardgrass | 0 | 0 | 15 | 0 | 35 | 15 | 0 | 0 | 85 | 0 | 0 | 95 | 95 | 95 |
| Ducksalad | 0 | 65 | 50 | 75 | 50 | 50 | 75 | 60 | 95 | 65 | 95 | 0 | 70 | 0 |
| Rice | 0 | 0 | 0 | 0 | 0 | 0 | 0 | 0 | 10 | 0 | 0 | 0 | 0 | 0 |
| Sedge, Umbrella | 0 | 0 | 80 | 70 | 75 | 70 | 90 | 95 | 90 | 65 | 90 | 95 | 95 | 90 |

| Table B1 | Compounds | | | | | | | | | | | | | |
|---|---|---|---|---|---|---|---|---|---|---|---|---|---|---|
| 250 g ai/ha | 296 | 297 | 298 | 299 | 300 | 301 | 302 | 303 | 304 | 305 | 306 | 307 | 308 | 309 |
| Flood | | | | | | | | | | | | | | |
| Barnyardgrass | 30 | 0 | 95 | 95 | 95 | 95 | 0 | 0 | 0 | 90 | 0 | 60 | 95 | 0 |
| Ducksalad | 20 | 50 | 90 | 55 | 90 | 50 | 70 | 0 | 0 | 90 | 85 | 70 | 90 | 60 |
| Rice | 0 | 0 | 0 | 0 | 0 | 0 | 0 | 0 | 0 | 0 | 0 | 0 | 10 | 0 |
| Sedge, Umbrella | 85 | 95 | 95 | 95 | 100 | 95 | 75 | 0 | 0 | 90 | 98 | 98 | 95 | 75 |

| Table B1 | Compounds | | | | | | | | | | | | | |
|---|---|---|---|---|---|---|---|---|---|---|---|---|---|---|
| 250 g ai/ha | 310 | 311 | 312 | 313 | 314 | 315 | 316 | 317 | 318 | 319 | 320 | 321 | 322 | 323 |
| Flood | | | | | | | | | | | | | | |
| Barnyardgrass | 0 | 0 | 0 | 0 | 98 | 98 | 95 | 0 | 95 | 90 | 0 | 15 | 55 | 0 |
| Ducksalad | 0 | 0 | 0 | 0 | 90 | 90 | 90 | 30 | 80 | 85 | 60 | 95 | 0 | 0 |
| Rice | 0 | 0 | 0 | 0 | 25 | 35 | 0 | 0 | 0 | 30 | 0 | 10 | 0 | 0 |
| Sedge, Umbrella | 0 | 0 | 0 | 0 | 90 | 90 | 90 | 0 | 80 | 80 | 90 | 98 | 0 | 0 |

| Table B1 | Compounds | | | | | | | | | | | | | |
|---|---|---|---|---|---|---|---|---|---|---|---|---|---|---|
| 250 g ai/ha | 324 | 325 | 326 | 327 | 328 | 329 | 330 | 331 | 332 | 333 | 334 | 335 | 336 | 337 |
| Flood | | | | | | | | | | | | | | |
| Barnyardgrass | 90 | 95 | 0 | 30 | 40 | 95 | 95 | 98 | 98 | 0 | 15 | 95 | 95 | 0 |
| Ducksalad | 80 | 95 | 85 | 85 | 90 | 60 | 95 | 80 | 80 | 70 | 60 | 70 | 75 | 65 |
| Rice | 0 | 15 | 0 | 0 | 0 | 20 | 0 | 0 | 0 | 0 | 10 | 45 | 45 | 25 |
| Sedge, Umbrella | 98 | 100 | 95 | 90 | 98 | 95 | 100 | 90 | 90 | 90 | 90 | 90 | 95 | 95 |

| Table B1 | Compounds | | | | | | | | | | | | | |
|---|---|---|---|---|---|---|---|---|---|---|---|---|---|---|
| 250 g ai/ha | 338 | 339 | 340 | 341 | 342 | 343 | 344 | 345 | 346 | 347 | 348 | 349 | 350 | 351 |
| Flood | | | | | | | | | | | | | | |
| Barnyardgrass | 95 | 95 | 85 | 95 | 95 | 85 | 95 | 95 | 90 | 95 | 95 | 90 | 90 | 90 |
| Ducksalad | 40 | 65 | 45 | 45 | 65 | 45 | 90 | 30 | 65 | 55 | 40 | 80 | 80 | 70 |
| Rice | 20 | 20 | 10 | 55 | 50 | 40 | 40 | 25 | 15 | 25 | 0 | 45 | 35 | 50 |
| Sedge, Umbrella | 90 | 65 | 95 | 95 | 98 | 95 | 95 | 95 | 95 | 85 | 95 | 80 | 85 | 70 |

| Table B1 | Compounds | | | | | | | | | | | | | |
|---|---|---|---|---|---|---|---|---|---|---|---|---|---|---|
| 250 g ai/ha | 352 | 353 | 354 | 355 | 356 | 357 | 358 | 359 | 360 | 361 | 362 | 363 | 364 | 365 |
| Flood | | | | | | | | | | | | | | |
| Barnyardgrass | 90 | 90 | 90 | 60 | 45 | 45 | 50 | 95 | 95 | 95 | 95 | 50 | 0 | 15 |
| Ducksalad | 65 | 65 | 60 | 45 | 45 | 60 | 0 | 25 | 65 | 65 | 50 | 70 | 0 | 25 |
| Rice | 35 | 20 | 15 | 10 | 0 | 10 | 10 | 15 | 25 | 35 | 10 | 25 | 0 | 15 |
| Sedge, Umbrella | 70 | 85 | 70 | 70 | 85 | 75 | 55 | 75 | 95 | 95 | 75 | 95 | 0 | 70 |

| Table B1 | Compounds | | | | | | | | | | | | | |
|---|---|---|---|---|---|---|---|---|---|---|---|---|---|---|
| 250 g ai/ha | 366 | 367 | 368 | 369 | 370 | 371 | 372 | 373 | 374 | 375 | 376 | 377 | 378 | 379 |
| Flood | | | | | | | | | | | | | | |
| Barnyardgrass | 15 | 60 | 70 | 90 | 95 | 95 | 20 | 95 | 40 | 50 | 95 | 0 | 95 | 95 |
| Ducksalad | 65 | 80 | 60 | 65 | 70 | 55 | 65 | 85 | 85 | 60 | 70 | 15 | 20 | 60 |
| Rice | 10 | 15 | 0 | 20 | 25 | 10 | 10 | 20 | 0 | 15 | 20 | 0 | 10 | 0 |
| Sedge, Umbrella | 95 | 98 | 98 | 98 | 98 | 98 | 75 | 95 | 95 | 70 | 95 | 0 | 80 | 80 |

| Table B1 | Compounds | | | | | | | | | | | | | |
|---|---|---|---|---|---|---|---|---|---|---|---|---|---|---|
| 250 g ai/ha | 380 | 381 | 382 | 383 | 3 392 | 393 | 394 | 397 | 398 | 399 | 400 | 401 | 402 | 403 |
| Flood | | | | | | | | | | | | | | |
| Barnyardgrass | 98 | 70 | 90 | 98 | 0 | 0 | 0 | 90 | 85 | 0 | 20 | 98 | 0 | 0 |
| Ducksalad | 70 | 50 | 25 | 25 | 65 | 95 | 85 | 75 | 95 | 75 | 55 | 45 | 45 | 15 |
| Rice | 10 | 0 | 10 | 10 | 0 | 0 | 0 | 0 | 30 | 0 | 25 | 45 | 0 | 0 |
| Sedge, Umbrella | 90 | 85 | 90 | 75 | 20 | 95 | 85 | 98 | 90 | 85 | 95 | 95 | 85 | 20 |

| Table B1 | Compounds | | | | | | | | | | | |
|---|---|---|---|---|---|---|---|---|---|---|---|---|
| 250 g ai/ha | 404 | 405 | 406 | 407 | 408 | 409 | 410 | 411 | 412 | 413 | 414 | 415 |
| Flood | | | | | | | | | | | | |
| Barnyardgrass | 80 | 55 | 35 | 45 | 98 | 40 | 95 | 98 | 0 | 40 | 0 | 85 |
| Ducksalad | 80 | 90 | 70 | 75 | 75 | 85 | 65 | 80 | 95 | 80 | 40 | 65 |
| Rice | 0 | 25 | 0 | 0 | 65 | 10 | 0 | 0 | 10 | 0 | 0 | 10 |
| Sedge, Umbrella | 98 | 98 | 90 | 65 | 90 | 95 | 85 | 85 | 98 | 95 | 90 | 85 |

## Claims

1. A compound selected from Formula **1,** all stereoisomers, *N*-oxides, and salts thereof, 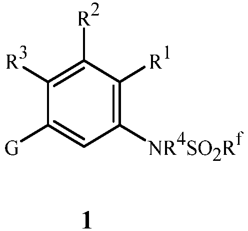 wherein
G is selected from:
| | | | | | |
|---|---|---|---|---|---|
| 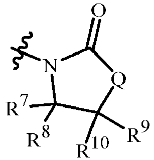 | | 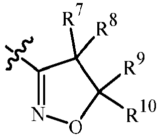 | | 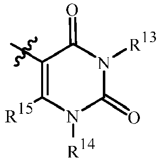 | |
| G-2 | , | G-1 | , | G-3 | , |
| 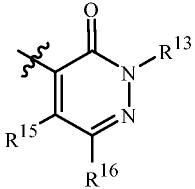 | | 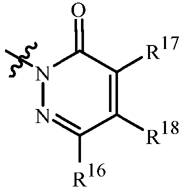 | | | |
| G-4 | and | G-5 | ; | | |
R¹ is H, C₁-C₇ alkyl, halogen, C₂-C₆ alkenyl, C₂-C₆ alkynyl, C₃-C₇ cycloalkyl or C₁-C₇ haloalkyl;
R² is H, C₁-C₇ alkyl, halogen, CN, C₁-C₇ haloalkyl, C₁-C₇ alkoxy, C₃-C₇ cycloalkyl, or C₁-C₅ alkylthio;
R³ is H, C₁-C₇ alkyl, halogen, CN, C₂-C₆ alkenyl, C₃-C₇ alkynyl, C₃-C₇ cycloalkyl, C₂-C₃ cyanoalkyl, C₁-C₇ haloalkyl, C₃-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl;
R⁴ is H, C(=O)R¹⁹, -C(=S)R¹⁹, -CO₂R¹⁹, -C(=O)SR¹⁹, -S(O)₂R¹⁹, C(=O)NR¹⁹R²⁰, -S(O)₂NR¹⁹R²⁰, S(OH)₂NR¹⁹R²⁰ or CH₂OC(=O)R¹⁹;
R⁷ is H, C₁-C₇ alkyl, halogen, CN, C₁-C₇ haloalkyl or C₁-C₇ alkoxy;
R⁸ is H, C₁-C₇ alkyl; or
R⁷ and R⁸ may be taken together to form a 3- to 7-membered ring, containing carbon atoms and optionally 1-2 oxygen, sulfur or nitrogen atoms as the ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R⁹ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R⁷ and R⁹ may be taken together to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R¹⁰ is H or C₁-C₇ alkyl; or
R⁹ and R¹⁰ may be taken together with the carbon atom to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂, said ring optionally substituted with up to 5 substituents independently selected from (R^{v})ᵣ and r is the number of the substituents;
R^{v} is independently selected from the group consisting of H, halogen, cyano, nitro, C₁-C₄ alkyl, C₃-C₆ cycloalkyl, C₁-C₄ haloalkyl, C₁-C₄ alkoxy and C₁-C₄ haloalkoxy; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂;
Q is O, S, CR¹¹R¹² or NR¹³;
R¹¹ and R¹² are taken together with the carbon atom to which they are attached to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy; or
R⁹ and R¹¹ are taken together with the carbon atom to which they are attached to form a 6 membered aromatic ring, said ring optionally substituted with up to 4 substituents independently selected from R^{w};
R^{w} is C₁-C₇ alkyl, halogen, C₁-C₇ haloalkyl or C₁-C₇ alkoxy;
r is 0, 1, 2, 3, 4 or 5;
R¹³ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R¹⁴ is H, C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₁-C₇ thioalkyl, C₃-C₇ haloalkenyl, C₂-C₇ alkoxyalkyl, C₃-C₇ alkylthioalkyl, C₁-C₇ alkoxy; C₂-C₇ alkoxyalkyl or C₄-C₇ alkylcycloalkyl;
R¹⁵ is H, C₁-C₇ alkyl, halogen, C₁-C₇ haloalkyl or C₁-C₇ alkoxy;
R¹⁶ is H, cyano, C₁-C₇ alkyl, halogen, C₁-C₄ alkylthio, C₁-C₇ haloalkyl or C₁-C₇ alkoxy;
R¹⁷ is H, C₁-C₇ alkyl, halogen, CN, C₂-C₆ alkenyl, C₂-C₆ alkynyl, C₃-C₇ cycloalkyl, C₂-C₃ cyanoalkyl, C₁-C₇ haloalkyl, C₃-C₇ haloalkenyl, C₃-C₇ haloalkynyl, C₂-C₇ alkoxyalkyl, C₁-C₇ alkoxy, C₁-C₅ alkylthio, C₂-C₃ alkoxycarbonyl or C₂-C₇ haloalkoxyalkyl;
R¹⁸ is H, C₁-C₇ alkyl, halogen, C₁-C₇ haloalkyl or C₁-C₇ alkoxy;
R¹⁹ is C₁-C₇ alkyl, C₂-C₆ alkenyl, C₂-C₆ alkynyl, C₃-C₇ cycloalkyl, C₄-C₇ cycloalkylalkyl, C₂-C₃ cyanoalkyl, C₁-C₇ haloalkyl, C₃-C₇ haloalkenyl, C₂-C₇ alkoxyalkyl, C₃-C₇ alkylthioalkyl, C₁-C₇ alkoxy; C₂-C₇ alkoxyalkyl, C₄-C₇ alkylcycloalkyl;
R²⁰ is H or C₁-C₇ haloalkyl; and
R^{f} is C₁-C₇ haloalkyl.

2. The compound of Claim 1 wherein
Q is O, S or CR¹¹R¹².

3. The compound of Claim 1 wherein
G is G-1;
R¹ is H, C₁-C₇ alkyl, halogen, C₂-C₆ alkenyl, C₂-C₆ alkynyl, C₃-C₇ cycloalkyl or C₁-C₇ haloalkyl;
R² is H, C₁-C₇ alkyl, C₃-C₆ cycloalkyl, halogen or CN;
R³ is H, C₁-C₇ alkyl, halogen, CN, C₁-C₇ alkoxy or C₁-C₇ haloalkyl;
R⁴ is H, C(=O)R¹⁹, CO₂R¹⁹, C(=O)SR¹⁹, S(O)₂R¹⁹ or CH₂OCOR¹⁹;
R^{f} is C₁-C₃ haloalkyl.

4. The compound of Claim 3 wherein
R¹ is H, C₁-C₇ alkyl, halogen or C₃-C₇ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R⁷ is H, C₁-C₇ alkyl, halogen, -CN, C₁-C₇ haloalkyl or C₁-C₇ alkoxy;
R⁸ is H or C₁-C₇ alkyl;
R⁹ is H, C₁-C₇ alkyl, C₁-C₇ haloalkyl or C₂-C₇ alkoxyalkyl;
R¹⁰ is H or C₁-C₇ alkyl; and
R^{f} is CF₃;
preferably wherein
R¹ is Me or Cl;
R³ is Me;
R⁴ is H, CH₂OCO-*t*-Bu or SO₂CF₃,
R⁷ is H;
R⁸ is H;
R⁹ is methyl, ethyl, *t*-butyl, chloromethyl or methoxymethyl; and
R¹⁰ is methyl or ethyl.

5. The compound of Claim 3 wherein
R⁷ and R⁹ are taken together to form a fused 3- to 7-membered ring, containing carbon atoms and optionally 1-2 oxygen, sulfur or nitrogen atom members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy;
R⁸ is H; and
R¹⁰ is H;
preferably wherein
R¹ is H, C₁-C₇ alkyl, halogen or C₃-C₇ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R⁷ and R⁹ are taken together to form a fused 3- to 7-membered ring, said 3- to 7-membered ring is a 5- or 6-membered ring;
more preferably wherein
R⁷ and R⁹ are taken together to form a 5- or 6-membered ring of c-pentyl, c-hexyl or tetrahydropyran, said ring unsubstituted or substituted with at least one substituent independently selected from H, halogen or C₁-C₄ alkyl.

6. The compound of Claim 3 wherein
R⁹ and R¹⁰ are taken together with the carbon atom to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂, said ring optionally substituted with up to 5 substituents independently selected from (R^{v})ᵣ and r is the number of the substituents; or
when two R^{v} are attached to the same carbon atom or attached to two adjacent carbon atoms, said two R^{v} can be taken together with the carbon atom or carbon atoms to which they are attached to form a 3- to 7-membered ring, containing carbon atoms and optionally 1-2 oxygen, sulfur or nitrogen atoms as ring members, wherein up to 2 carbon atom ring members are independently selected from C(=O) and C(=S) and the sulfur atom ring member is selected from S, S(O) or S(O)₂;
R⁷ is H; and
R⁸ is H;
preferably wherein
R¹ is H, C₁-C₃ alkyl, halogen or C₃-C₄ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R⁹ and R¹⁰ are taken together with the carbon atom to which they are attached to form a 3- to 7-membered ring, said ring is a 5- or 6-membered ring;
R^{v} is independently selected from the group consisting of H, methyl, ethyl, propyl, c-propylmethyl, propargyl or cyanomethyl; and
r is 1 or 2;
more preferably wherein
R⁹ and R¹⁰ are taken together with the carbon atom to which they are attached to form a 3- to 7-membered ring, said ring is a 5- or 6-membered ring of cyclopentane, cyclohexane, tetrahydro-2H-pyran or tetrahydro-2H-thiopyran.

7. The compound of Claim 1 wherein
G is G-2;
R¹ is H, C₁-C₇ alkyl, halogen, C₂-C₆ alkenyl, C₂-C₆ alkynyl, C₃-C₇ cycloalkyl or C₁-C₇ haloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe; and
R^{f} is C₁-C₃ haloalkyl.

8. The compound of Claim 7 wherein
Q is O or S;
R⁷ is H;
R⁸ is H;
R^{f} is CF₃;
R⁹ and R¹⁰ are taken together with the carbon atom to which they are attached to form a 3- to 7-membered ring, said ring is a 5- or 6-membered ring;
R^{v} is independently selected from the group consisting of H, methyl, ethyl, propyl, *c-*propylmethyl, propargyl or cyanomethyl; and
r is 1 or 2.

9. The compound of Claim 8 wherein
R⁹ and R¹⁰ are taken together with the carbon atom to which they are attached to form a cyclopentane, cyclohexane, tetrahydro-2*H*-pyran or tetrahydro-2*H*-thiopyran; and
R^{v} is H.

10. The compound of Claim 7 wherein
Q is CR¹¹R¹²;
R⁷ is H;
R⁸ is H;
R⁹ is H;
R¹⁰ is H;
R¹¹ and R¹² are taken together with the carbon atom to which they are attached to form a fused 3- to 7-membered ring, containing carbon atoms and optionally 1-2 oxygen, sulfur or nitrogen atoms as ring members, said ring unsubstituted or substituted with at least one substituent independently selected from the group consisting of halogen, cyano, nitro, C₁-C₄ alkyl, C₃-C₆ cycloalkyl, C₁-C₄ haloalkyl, C₁-C₄ alkoxy and C₁-C₄ haloalkoxy; and
R^{f} is CF₃;
preferably wherein
R¹¹ and R¹² are taken together with the carbon atom to which they are attached to form an unsubstituted 5- or 6-membered ring.

11. The compound of Claim 1 wherein
G is G-3;
R¹ is H, C₁-C₇ alkyl, halogen or C₃-C₇ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R¹³ is C₁-C₇ alkyl,
R¹⁴ is C₁-C₄ alkyl;
R¹⁵ is H; and
R^{f} is C₁-C₃ haloalkyl;
or wherein
G is G-4;
R¹ is H, C₁-C₇ alkyl, halogen or C₃-C₇ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R¹³ is C₁-C₇ alkyl,
R^{f} is C₁-C₃ haloalkyl;
R¹⁵ is H, C₁-C₃ alkyl or C₁-C₃ alkoxy; and
R¹⁶ is H, cyano, C₁-C₄ alkyl, halogen, C₁-C₄ alkylthio, C₁-C₄ haloalkyl or C₁-C₄ alkoxy;
or wherein
G is G-5;
R¹ is H, C₁-C₇ alkyl, halogen or C₃-C₇ cycloalkyl;
R² is H, Me or F;
R³ is H, Me, F, Cl, CN, OMe or CF₃;
R⁴ is H, SO₂CF₃, SO₂CH₃, CO₂Me, COMe, CH₂OCO-*t*-Bu, CH₂OCO-*n*-Bu, CH₂OCO-*c*-hexyl, CH₂OCO-*c*-pentyl, CH₂OCOCH₂CH₃, COMe, CH₂OCOPh, CH₂OCO-*i*-Bu, CH₂OCOMe, CH₂OCO-*sec*-Bu or COSMe;
R^{f} is C₁-C₃ haloalkyl;
R¹⁶ is H or C₁-C₄ alkyl;
R¹⁷ is H, C₁-C₄ alkyl, halogen or C₁-C₄ alkoxy; and
R¹⁸ is H or C₁-C₃ alkoxy.

12. The compound of Claim 1 selected from the group consisting of
N-[2,4-Dimethyl-5-(1-oxa-2-azaspiro[4.4]non-2-en-3-yl)phenyl]-1,1,1-trifluoromethanesulfonamide (Compound 207);
N-[2,4-Dimethyl-5-[(3aR,6aR)-3a,5,6,6a-tetrahydro-4H-cyclopent[d]isoxazol-3-yl]phenyl]-1,1,1-trifluoromethanesulfonamide (Compound 103);
N-[2,4-Dimethyl-5-(1-oxa-2-azaspiro[4.5]dec-2-en-3-yl)phenyl]-1,1,1-trifluoromethanesulfonamide (Compound 197);
N-[2,4-Dimethyl-5-(3a,4,7,7a-tetrahydro-5H-pyrano[4,3-d]isoxazol-3-yl)phenyl]-1,1,1-trifluoromethanesulfonamide (Compound 121);
N-[2,4-Dimethyl-5-(3a,6,7,7a-tetrahydro-4H-pyrano[3,4-d]isoxazol-3-yl)phenyl]-1,1,1-trifluoromethanesulfonamide (Compound 120);
N-[2,4-Dimethyl-5-(1-oxo-2-azaspiro[4.5]dec-2-yl)phenyl]-1,1,1-trifluoro-N-[(trifluoromethyl)sulfonyl]methanesulfonamide (Compound 267);
[[2,4-Dimethyl-5-(1-oxa-2-azaspiro[4.4]non-2-en-3-yl)phenyl][(trifluoromethyl)sulfonyl]amino]methyl 2,2-dimethylpropanoate (Compound 140);
[[2,4-Dimethyl-5-(1-oxa-2-azaspiro[4.5]dec-2-en-3-yl)phenyl][(trifluoromethyl)sulfonyl]amino]methyl 2,2-dimethylpropanoate (Compound 159);
[[2,4-Dimethyl-5-[(3aR,6aR)-3a,5,6,6a-tetrahydro-4H-cyclopent[d]isoxazol-3-yl]phenyl][(trifluoromethyl)sulfonyl]amino]methyl 2,2-dimethylpropanoate (Compound 100);
[[2,4-Dimethyl-5-(1-oxo-2-azaspiro[4.5]dec-2-yl)phenyl][(trifluoromethyl)sulfonyl]amino]methyl 2,2-dimethylpropanoate (Compound 268); and
[[(Trifluoromethyl)sulfonyl][2,3,4-trimethyl-5-[(3aR,6aR)-3a,5,6,6a-tetrahydro-4H-cyclopent[d]isoxazol-3-yl]phenyl]amino]methyl 2,2-dimethylpropanoate (Compound 336).

13. A herbicidal composition comprising a compound of any of Claims 1 to 12 and at least one component selected from the group consisting of surfactants, solid diluents and liquid diluents, and optionally at least one additional active ingredient selected from the group consisting of other herbicides and herbicide safeners.

14. A herbicidal mixture comprising (a) a compound of any of Claims 1 to 12, and (b) at least one additional active ingredient selected from (b1) photosystem II inhibitors, (b2) acetohydroxy acid synthase (AHAS) inhibitors, (b3) acetyl-CoA carboxylase (ACCase) inhibitors, (b4) auxin mimics, (b5) 5-enol-pyruvylshikimate-3-phosphate (EPSP) synthase inhibitors, (b6) photosystem I electron diverters, (b7) protoporphyrinogen oxidase (PPO) inhibitors, (b8) glutamine synthetase (GS) inhibitors, (b9) very long chain fatty acid (VLCFA) elongase inhibitors, (b10) auxin transport inhibitors, (b11) phytoene desaturase (PDS) inhibitors, (b12) 4-hydroxyphenyl-pyruvate dioxygenase (HPPD) inhibitors, (b13) homogentisate solanesyltransferase (HST) inhibitors, (b14) cellulose biosynthesis inhibitors, (b15) other herbicides including mitotic disruptors organic arsenicals, asulam, bromobutide, cinmethylin, cumyluron, dazomet, difenzoquat, dymron, etobenzanid, flurenol, fosamine, fosamine-ammonium, hydantocidin, metam, methyldymron, oleic acid, oxaziclomefone, pelargonic acid and pyributicarb, (b16) herbicide safeners and salts of compounds of (b1) through (b16).

15. A method for controlling the growth of undesired vegetation comprising contacting the vegetation or its environment with a herbicidally effective amount of a compound of any of Claims 1 to 12, preferably further comprising contacting the vegetation or its environment with a herbicidally effective amount of at least one additional active ingredient selected from (b1) through (b16) and salts of compounds of (b1) through (b16).
